# EUROPEAN PATENT SPECIFICATION

(11) **EP 4 372 000 B1**
(45) Date of publication and mention of the grant of the patent: **17.06.2026**
(21) Application number: 24155055.7
(22) Date of filing: 15.02.2023
(51) Int. Cl.: C07K 14/54

(54) **MASKED IL12 PROTEIN**
MASKIERTES IL12-PROTEIN
PROTEINE IL12 MASQUEE

(30) Priority: 15.02.2022 EP 22156758; 01.04.2022 EP 22166385; 02.11.2022 EP 22205062
(43) Date of publication of application: 22.05.2024
(62) Divisional of application: 23707184.0
(73) Proprietor: Tagworks Pharmaceuticals B.V., 6525 ED Nijmegen (NL)
(72) Inventor: DEN BROK, Martijn Hendrikus Martinus Gerardus Maria, 6525 ED Nijmegen (NL); ROBILLARD, Marc Stefan, 6525 ED Nijmegen (NL); ROSSIN, Raffaella, 6525 ED Nijmegen (NL); KLEIJN, Laurens Henri Johan, 6525 ED Nijmegen (NL); DE ROODE, Kim Elisa, 6525 ED Nijmegen (NL); WOUTERS, Lieke Willemijn Maria, 6525 ED Nijmegen (NL); ZIJLMANS, Lucas Hendricus Maria, 6525 ED Nijmegen (NL); VERSTEEGEN, Ronny Mathieu, 6525 ED Nijmegen (NL)
(74) Representative: V.O.

(56) References cited:
- WO-A1-2018/004338
- WO-A1-2020/256544
- WO-A1-2021/189139
- WO-A1-2021/262985

## Description

### Field of the invention

The invention disclosed herein relates to masked IL12 proteins that can be activated both *in vitro* and *in vivo.* The invention also relates to methods for using and preparing same.

### Background of the invention

Interleukin 12 (IL12) is an interleukin that is produced by dendritic cells, macrophages, neutrophils, and human B-lymphoblastoid cells, typically in response to antigenic stimulation. IL12 is composed of four alpha helices. It is a heterodimeric cytokine encoded by two separate genes. The two subunits are called the α-subunit (also p35) and the β-subunit (also p40). The active heterodimer of IL12 is also referred to as p70.

IL12 plays an important role in several processes within a living organism. For example, IL12 is involved in the differentiation of naive T cells into Th1 cells, it is known as a T cell-stimulating factor, IL12 mediates enhancement of the cytotoxic activity of NK cells and CD8+ cytotoxic T lymphocytes, and IL12 also has anti-angiogenic activity. As such, IL12 is indicated to be useful in the treatment of patients having cancer, a central nervous system (CNS) disease, an infection, an inflammation, a cardiovascular disease, psoriasis, and/or inflammatory bowel disease.

The administration of active IL12, for example recombinant IL12 protein, to subjects in need thereof may unfortunately result in side-effects. Therefore, it is desired that the IL12 protein be masked, *i.e.* partially or completely deactivated, and that the masked IL12 protein is administered instead. After administration, the masked IL12 protein can be unmasked *in vivo,* preferably at a target site where active IL12 protein is required, to minimize unwanted off-target effects.

While some efforts towards masking IL12 protein have been made, further improvements are desired. For example, WO2021/236676 A1 describes IL12 protein complexes that include a half-life extension element, a masking element, and a protease-cleavable linker. This approach, however, relies on the overexpression of a protease at the target site, which does not always occur and precludes exact timing of release. Also, non-target expression can still occur and lead to significant linker cleavage, and therefore off-target toxicity. Moreover, this approach requires genetically engineering the IL12 protein to include the other elements. Moreover, the ratio of the EC50 values of the unmasked IL12 protein and the masked protein could also be improved. Furthermore, the kinetics of the unmasking reaction may be improved as well.

Further background art uses (part of) the IL12 receptor to mask IL12. However, the (part of) the IL12 receptor needs to be co-expressed with the IL12 protein, which is laborious. Moreover, the resulting construct is rather unnatural, and typically increases the chance of anti-drug antibodies (ADA) response.

As such, there is a desire to provide masked IL12 proteins that can be activated *in vivo,* preferably specifically at the target site. It is desired that unmasking of said masked IL12 protein does not rely on inherent properties of the target site, such as overexpression of proteases or a certain pH. It is also desired that the timing of unmasking can be controlled. It is desired that such masked IL12 protein can be readily produced, *e.g.* without the need to genetically engineer said IL12 protein. Moreover, it is desired that the masking/unmasking strategy reduces unwanted off-target effects. A further desire is to provide a masking/unmasking strategy for the IL12 protein in which the ratio of the EC50 value of the unmasked IL12 protein over the EC50 value of the masked IL12 protein is improved, *i.e.* that as compared to prior art strategies the activity of the unmasked IL12 protein as compared to the activity of the masked IL12 protein is increased. The latter can be achieved by reducing the activity of the masked IL12 protein as compared to known masked IL12 proteins, and/or by increasing the activity of the unmasked IL12 protein as compared to known strategies. It is also desired that a masking/unmasking strategy be provided with faster unmasking kinetics *in vivo.* Furthermore, it is desired that masked IL12 proteins are provided that do not use (part of) the IL12 receptor.

It is desired that compounds are developed that address one or more of the abovementioned problems and/or desires.

Further background references include WO 2020/256544; WO 2018/004338; WO 2021/189139; and WO 2021/262985.

### Summary of the invention

The invention is defined by the claims, which can be combined with any other embodiment as disclosed herein.

### Brief Description of the Figures

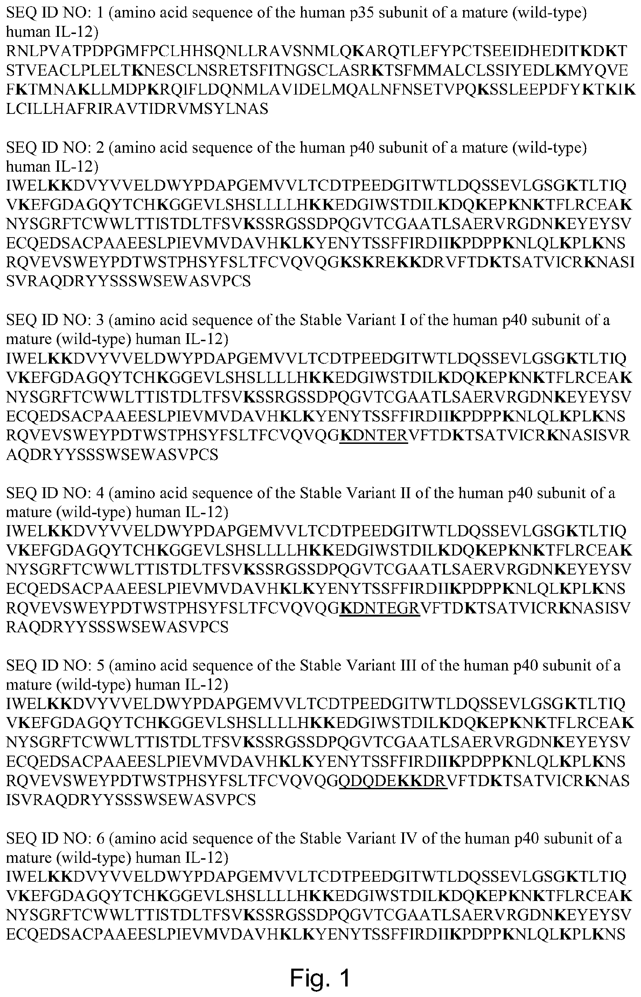

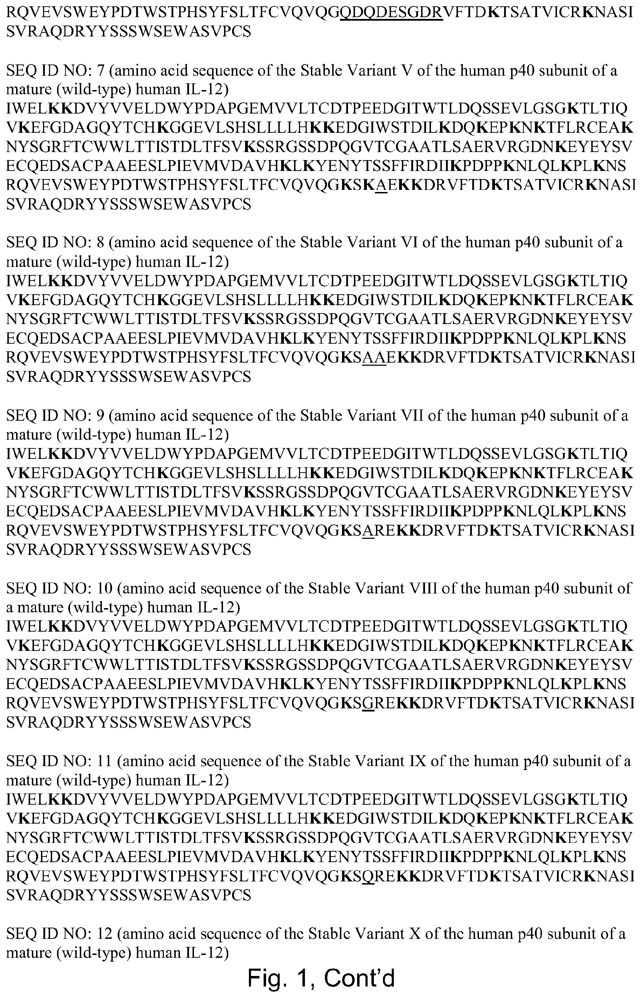

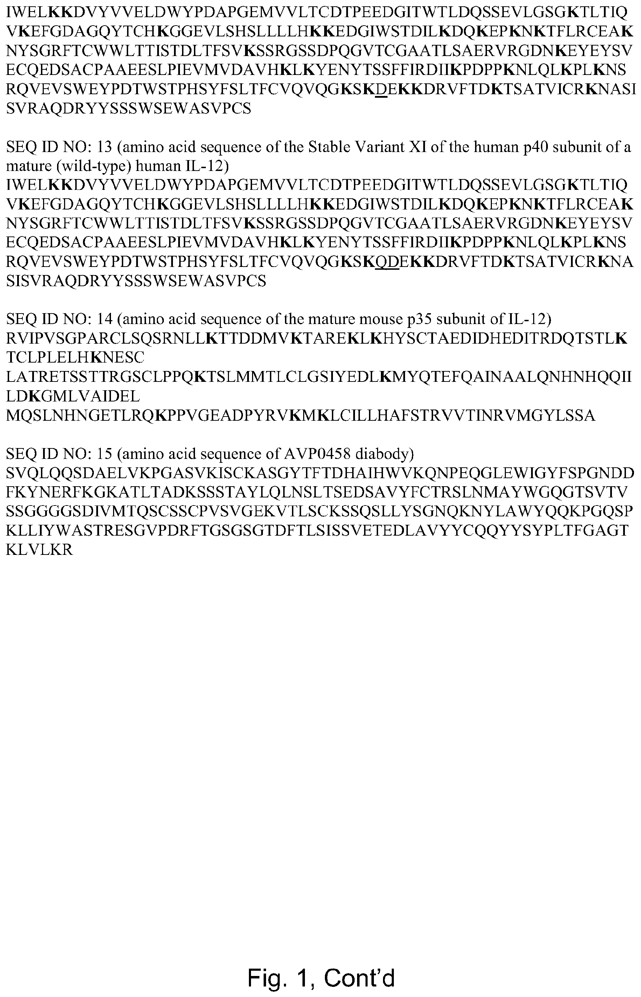

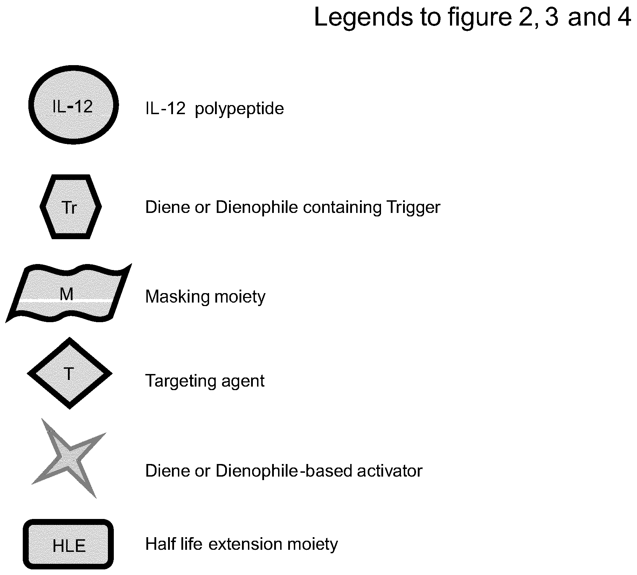

Figure 1 depicts the sequences of IL12 proteins (SEQ ID NO: 1-14) and of the AVP0458 diabody (SEQ ID NO: 15). SEQ ID NO: 1 depicts the mature amino acid sequence of the α chain, i.e., the human p35 subunit, of a mature (wild-type) human IL12. SEQ ID NO: 2 depicts the mature amino acid sequence of the β chain, i.e., the human p40 subunit, of a mature (wild-type) human IL12. SEQ ID NO: 3 up to and including SEQ ID NO: 13 are more stable variants of the p40 subunit (see US 60/755,382). Lysines are depicted in bold, differences in the amino acid sequences as compared to SEQ ID NO: 2 are underlined. SEQ ID NO: 14 is the amino acid sequence of the mature mouse p35 subunit of IL12.

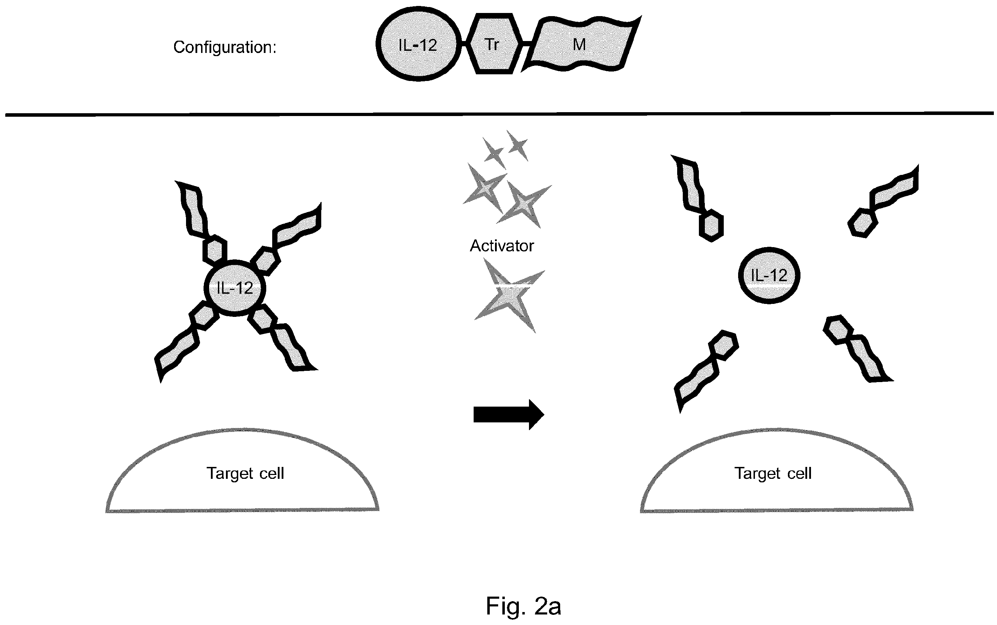

Figure 2a is a schematic illustrating a chemically activatable IL12 cytokine construct that contains an IL12 polypeptide, a chemically cleavable Trigger moiety, and a masking moiety. The masking moiety may optionally function as a half-life extension element. The IL12 cytokine is masked by conjugation to several masking moieties, e.g. a dienophile connected to a PEG chain, thereby preventing its ability to activate the IL12 receptor. Distribution into a diseased target tissue (e.g. a tumor) relies on passive diffusion and accumulation over time. When a desired distribution profile is reached an activator is administered, resulting in the traceless cleavage of the masking moieties, thereby allowing the IL12 cytokine to bind to its receptor on cells of the immune system. The cytokine now has similar PK properties as compared to the native cytokine (e.g. has a short half-life).

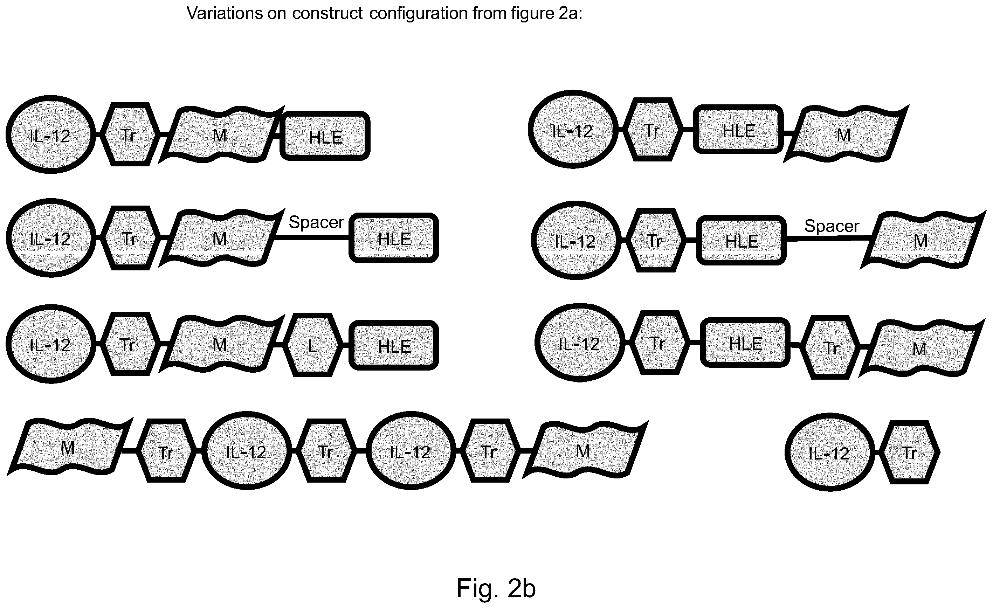

Figure 2b is a schematic illustrating variations on the IL12 protein construct as presented in figure 2a. Here, a half-life extension element (HLE, e.g. a serum albumin binder, or Fc antibody fragment) is conjugated to the construct, either to the cytokine moiety, or to the masking moiety, with a Trigger in between these elements. This Trigger can be cleavable by the activator, or optionally this connection is non-cleavable (e.g. a (biochemical linker or spacer). The HLE changes the PK properties of the cytokine so it will circulate longer as compared to the native cytokine. Shown is that also more than one IL12 moiety can be present in a construct. In some embodiments, masking of cytokine activity is achieved by the Trigger alone (i.e. without an additional masking moiety).

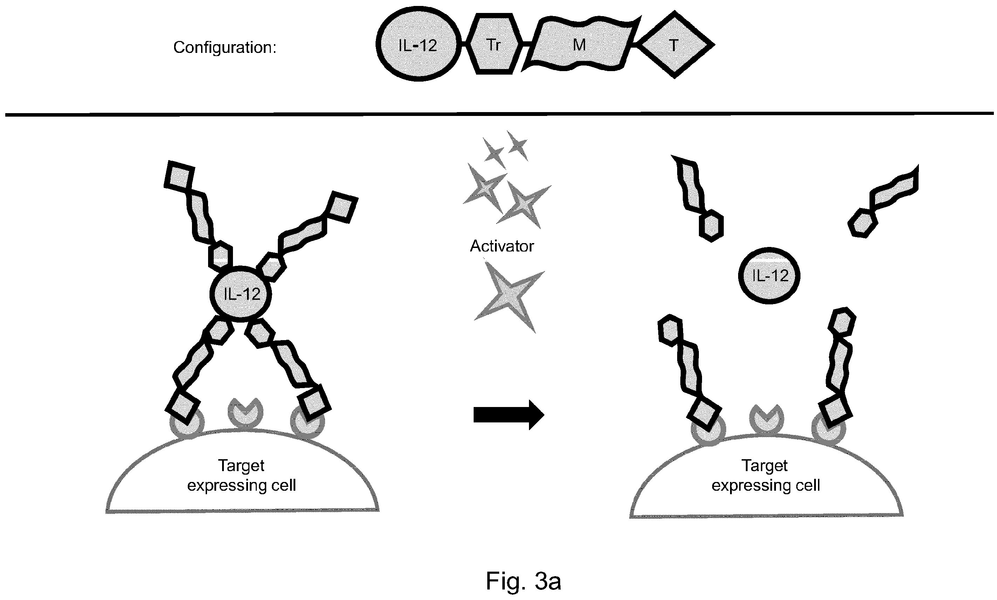

Figure 3a is a schematic illustrating a chemically activatable IL12 cytokine construct that contains an IL12 polypeptide, a chemically cleavable Trigger moiety, a masking moiety, and a targeting agent. The targeting agent serves to aid distribution to and accumulation in the diseased tissue, by binding to an expressed target on a diseased cell (e.g. a membrane-bound receptor or a specific sugar chain). The IL12 cytokine is masked by conjugation to several masking moieties, and targeted to a disease-specific cell. When a desired distribution profile is said to have been reached an activator is administered resulting in traceless cleavage of the masking moieties, thereby allowing the IL12 cytokine to bind to its receptor on cells of the immune system.

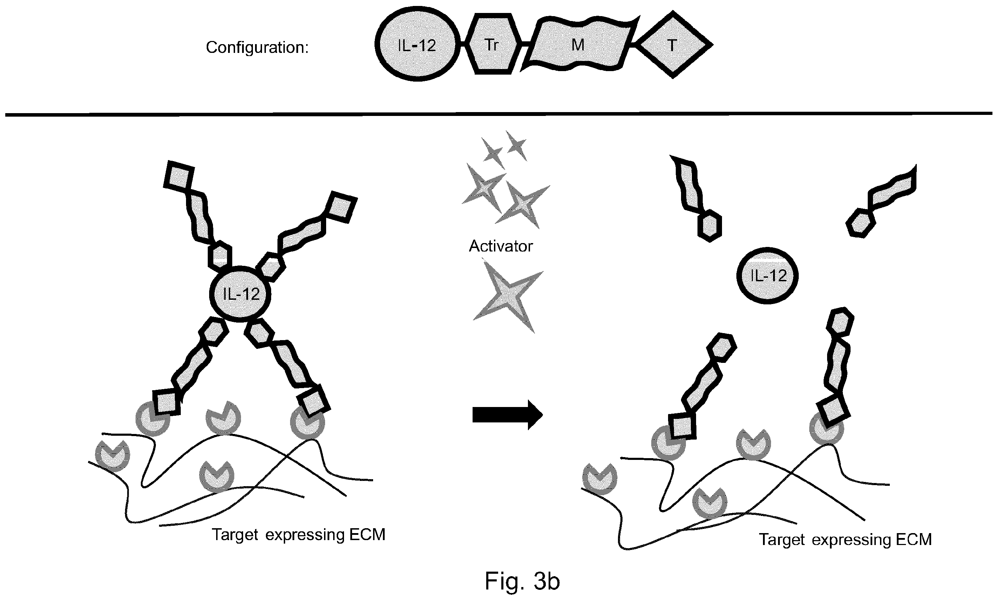

Figure 3b as Figure 3a but in this figure the targeted and masked IL12 binds to a target expressed in the extracellular matrix (ECM) present in a diseased tissue (e.g. fibronectin, or any proteoglycan).

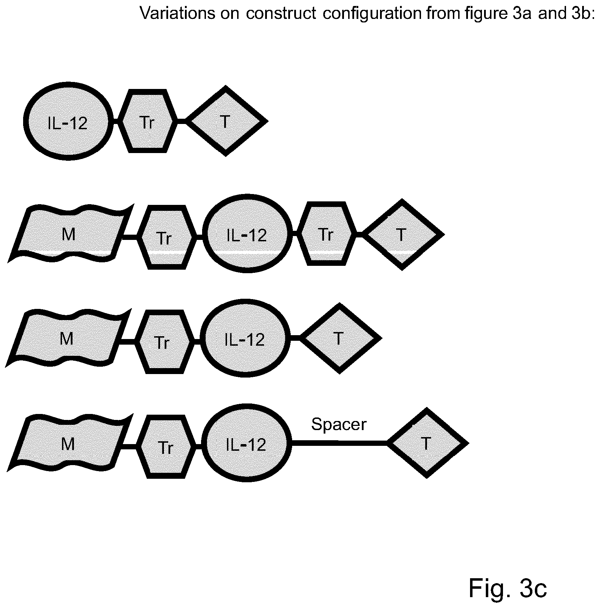

Figure 3c is a schematic illustrating variations on the IL12 protein construct as presented in figure 3a and b. Here, the targeting agent can optionally function by itself as masking moiety, or the masking moieties and the targeting agents can be conjugated to the cytokine by separate conjugations. In addition, the targeting agent can be directly conjugated to the IL12 moiety, using a non-cleavable linker with or without spacer, or both the targeting and the IL12 moiety can be expressed as a single polypeptide.

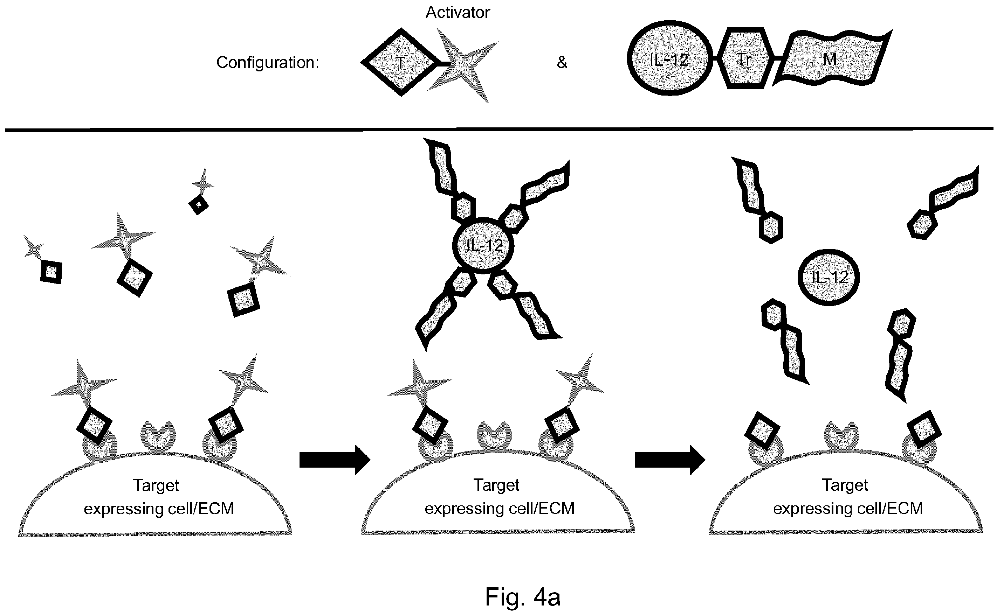

Figure 4a is a schematic illustrating a chemically activatable IL12 cytokine construct that contains an IL12 polypeptide, a chemically cleavable Trigger moiety, and a masking moiety. Here, the activator is pre-localized to the diseased target tissue by means of conjugating the activator (tetrazine or TCO, depending on the configuration of the Trigger moiety to be cleaved) to a targeting agent. The targeting agent serves to aid distribution to and accumulation of the activator in the diseased tissue, by binding to an expressed target on a diseased cell or in the extracellular matrix. After administration and target accumulation of the activator, a masked IL12 protein construct is administered, resulting in on-target cleavage of the masking moieties of the IL 12 construct in a traceless manner, thereby allowing the IL12 cytokine to bind to its receptor on cells of the immune system.

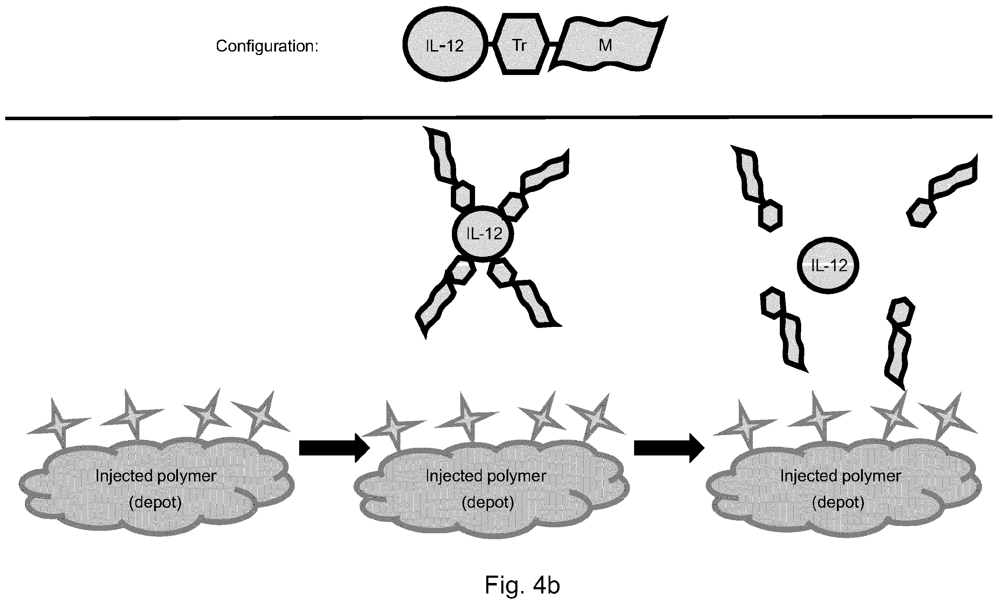

Figure 4b is a schematic illustrating a chemically activatable IL12 cytokine construct that contains an IL12 polypeptide, a chemically cleavable Trigger moiety, and a masking moiety. Here, the activator is pre-localized to the diseased target tissue by injecting it in, or in the vicinity of a diseased tissue (e.g. intra- or peri-tumorally). For this the activator (tetrazine or TCO, depending on the configuration of the Trigger moiety to be cleaved) can be conjugated to a biomolecule or polymer (e.g. hyaluronic acid) retaining it at the target site. Subsequent administration of the masked IL12 results in cleavage of the masking moieties from the IL12 construct in a traceless manner, thereby allowing the IL 12 cytokine to bind to its receptor on cells of the immune system.

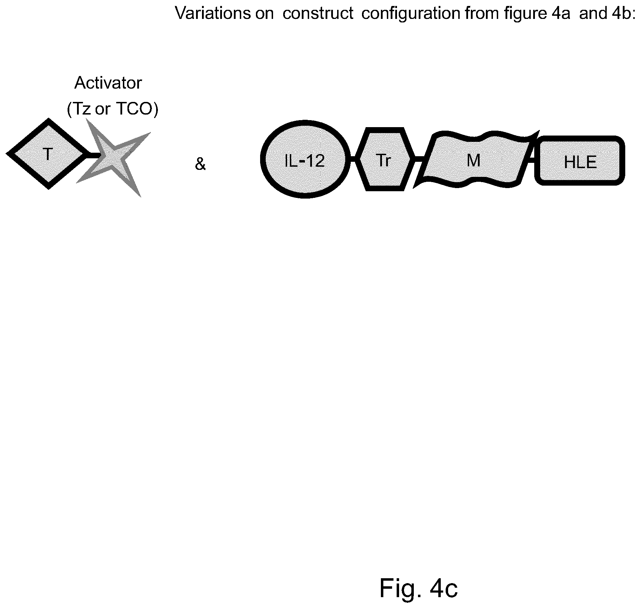

Figure 4c is a schematic illustrating variations on the IL12 protein construct as presented in figure 4a and b. Here, the construct can contain a half-life extension element to prolong the circulation time of the construct.

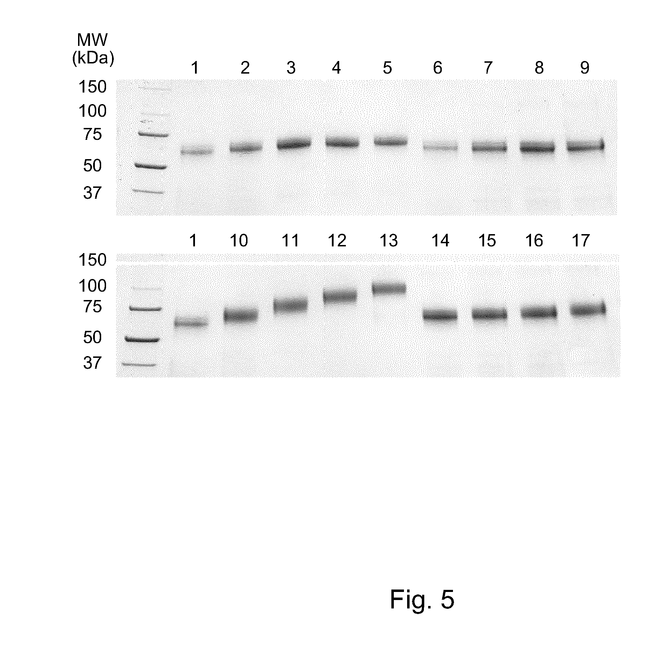

Figure 5 shows the SDS-PAGE analysis of (lanes 1) native IL12, (lanes 2 to 5) the IL12-TCO-PEG₂ conjugates **9.1, 9.3, 9.4,** and **9.5,** and (lanes 6 to 9) the same compounds after the reaction with a tetrazine **8.5** (5 eq with respect to TCO). Moreover, it shows the IL12-TCO-PEG_{1K} conjugates **9.7, 9.8, 9.9,** and **9.10** in lanes 10 to 13, or (lanes 14 to 17) the same compounds after the reaction with a tetrazine **8.5** (5 eq with respect to TCO).

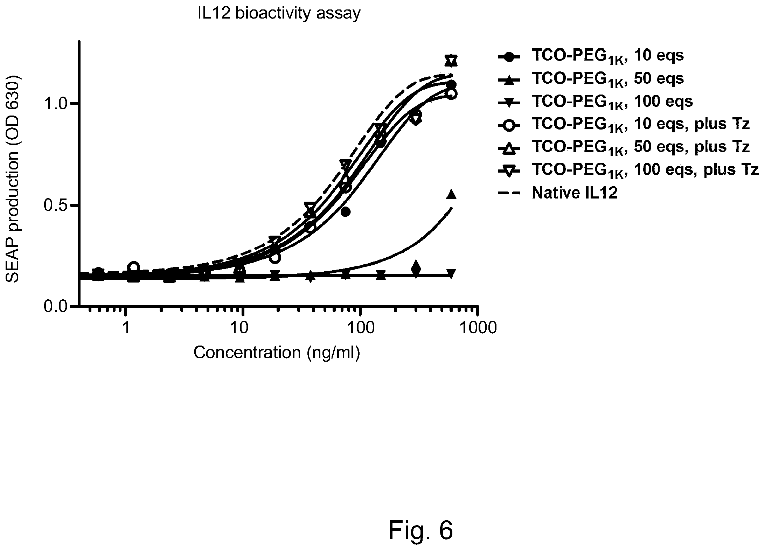

Figure 6 shows the results from a HEK-Blue IL12 reporter assay performed on IL12-TCO-PEG_{1K} constructs **9.7, 9.9,** and **9.10** and recombinant human IL12 (native IL12). Shown is biological activity of the protein constructs with (open symbols) and without (filled symbols) activation by a tetrazine **8.4,** demonstrating complete or partial IL12 deactivation depending on the modification grade. In all cases, upon reaction with a tetrazine the masking moiety is removed and IL12 bioactivity is re-established.

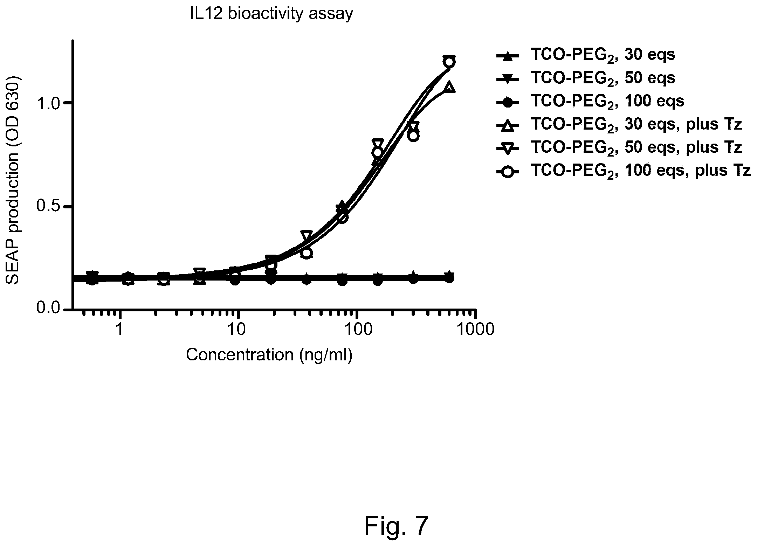

Figure 7 shows the results from a HEK-Blue IL12 reporter assay, similarly performed as in previous figure. Shown is biological activity of the IL12-TCO-PEG₂ constructs **9.3, 9.4,** and **9.5,** with (open symbols) and without (filled symbols) activation by a tetrazine **8.5** activator, demonstrating complete IL12 deactivation irrespective of the modification grade.

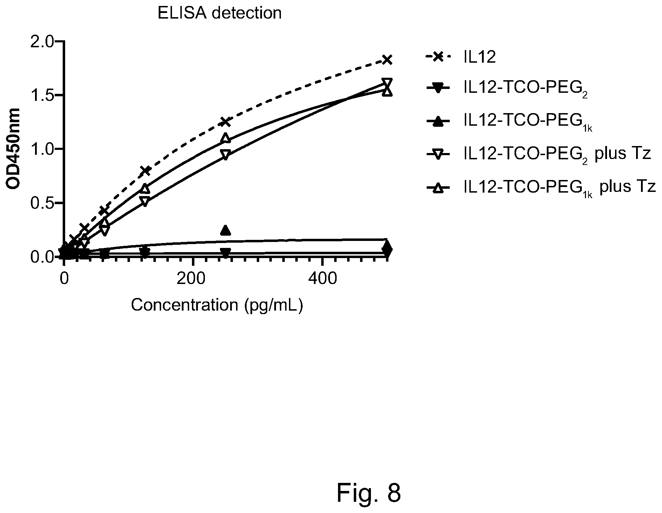

Figure 8 shows detection of IL12-TCO-PEG₂ **9.4,** or IL12-TCO-PEG1K **9.10** constructs using a hIL12-p70 ELISA assay ('sandwich setup'). Shown is the inability to detect masked cytokine constructs (filled symbols), while release of the PEG masks enables a concentration dependent detection similar to native IL12 (open symbols).

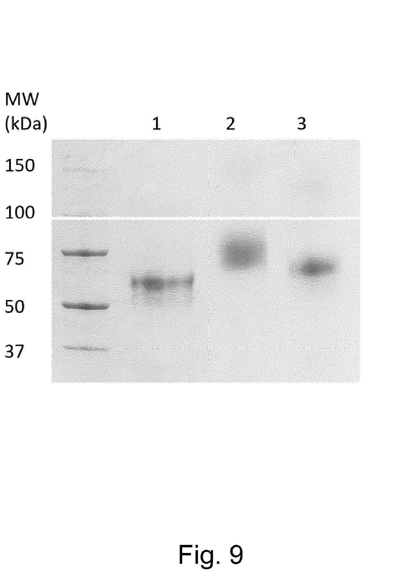

Figure 9 shows an SDS-PAGE analysis of (lanes 1) native IL12, (lane 2) the IL12-Tz-PEG conjugate **12.3,** and (lane 3) the same compound after the reaction with a small molecule TCO activator, confirming release of PEG masks.

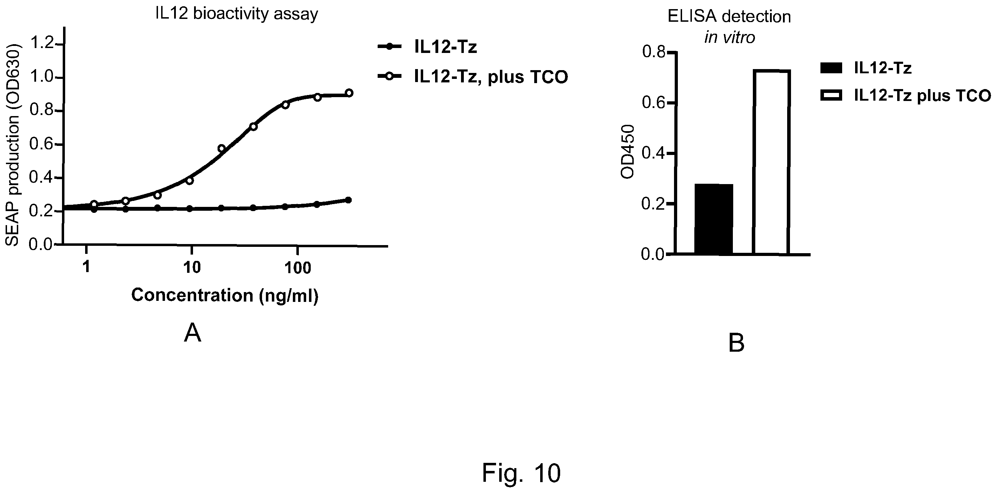

Figure 10 shows the results from a HEK-Blue IL12 bioactivity assay (A), and an ELISA detection assay (B) similarly performed as in previous figures. IL12 protein was masked using conjugation with Tz-PEG (compound **12.4).** Figures show attenuated biological activity and ELISA detection of Tz-masked protein construct without (filled symbols/bar), and regained activity with TCO activator (open symbols/bar).

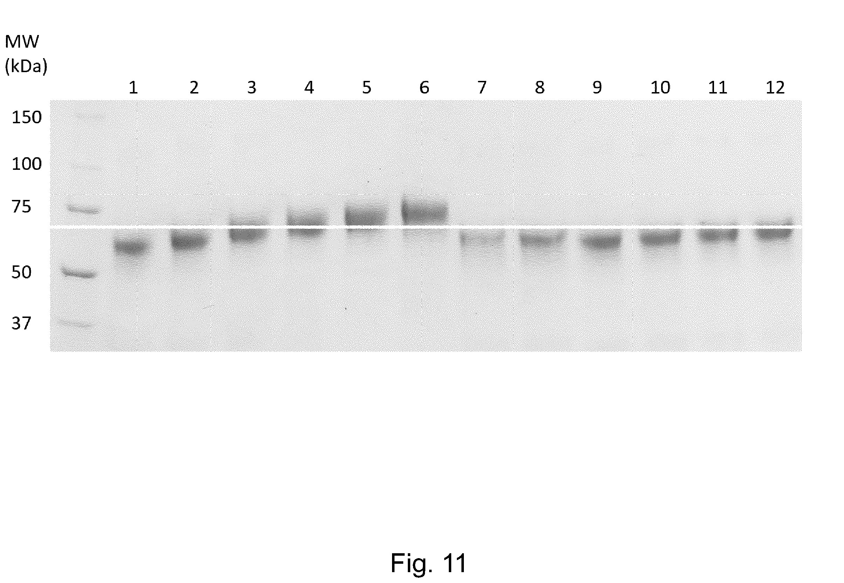

Figure 11 shows a SDS-PAGE analysis of (lane 1 and 7) native IL12, (lane 2-6) the IL12-TCO-PEG8-keto conjugates **(15.1, 15.2, 15.3, 15.4,** and **15.5** resp.). Lane 8-12 show the same conjugates after reaction with a tetrazine **8.5,** confirming release of the masks.

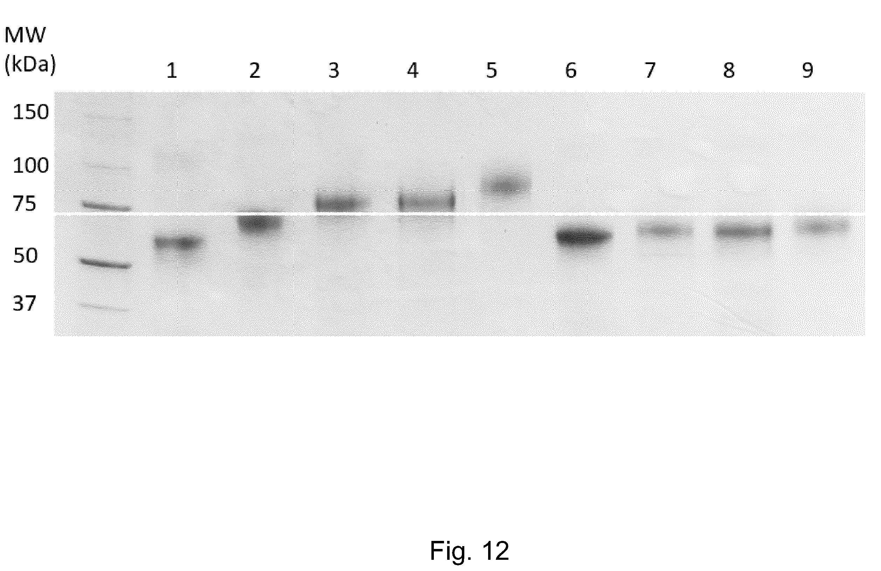

Figure 12 shows a SDS-PAGE analysis of (lane 1) native IL12, (lane 2) the IL12-TCO-PEG8-keto conjugate **(15.4),** and (lane 3-5) the conjugates obtained after reaction of **(15.4)** with peptide 1, peptide 2 or peptide 3 resp. **(16.1-16.3).** Lanes 6-9 show these same compounds after incubation with tetrazine activator **(8.5),** confirming release of the PEG8-keto or PEG8-keto-peptide masks.

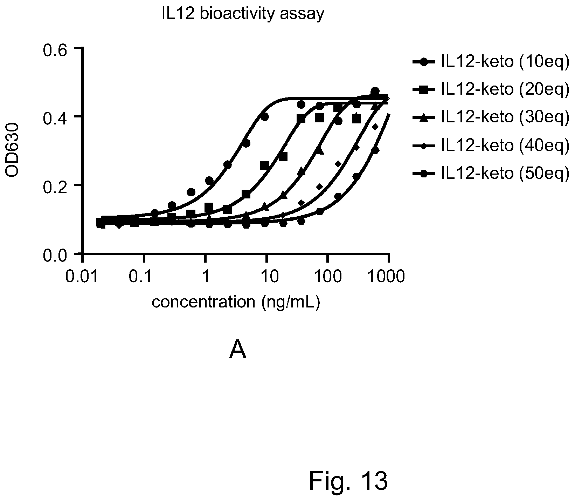

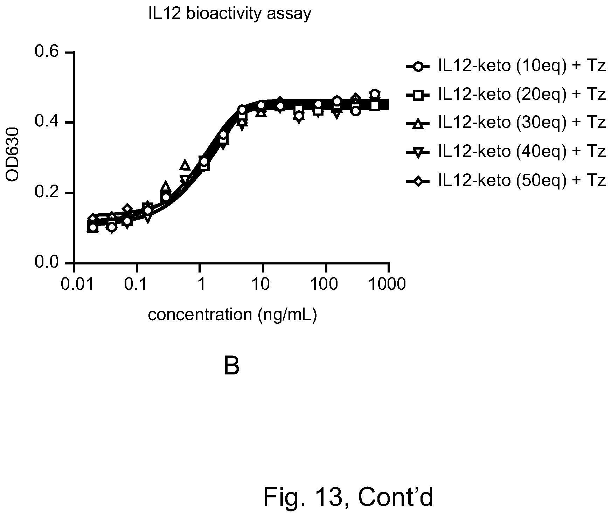

Figure 13 shows the results from a HEK-Blue IL12 bioactivity assay similarly performed as in previous figures. Here, IL12 protein was masked with TCO-PEG8-keto conjugates **(15.1-15.5).** Figures show increasing attenuation of biological activity with increased modification grade (A) and complete recovery upon reaction with a tetrazine activator **(8.5)** (B).

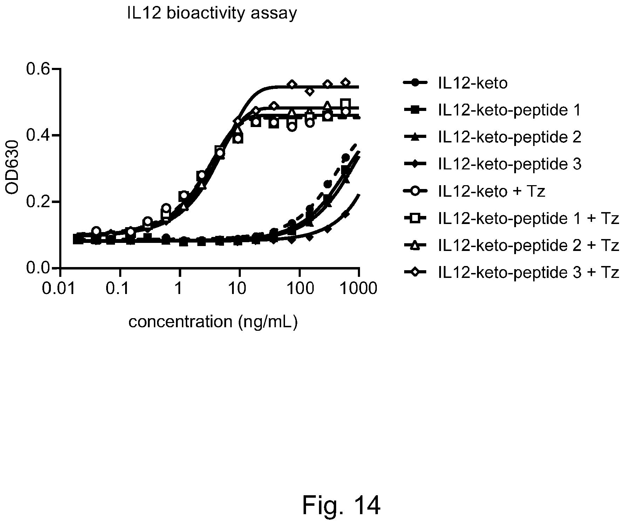

Figure 14 shows the results from a HEK-Blue IL12 reporter assay, similarly performed as in previous figures. Here, the IL12-keto-peptide conjugates **16.1-16.3** were incubated with or without **8.5.** Figure shows recovery of attenuated biological activity upon reaction of the masked constructs with tetrazine.

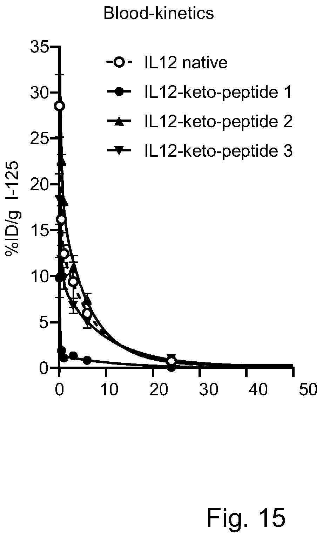

Figure 15. Blood kinetics of ¹²⁵I -labelled peptide-masked IL12 constructs. Peptide masked constructs **16.1-16.3** were administered i.v. in tumour-free nude mice (10ug/mouse), followed by detection of radioactivity in consecutive blood samples. All constructs show a comparable clearance pattern as wildtype IL12. Data represent the mean percentage injected dose per gram (% ID g-1) with s.d. (n=3).

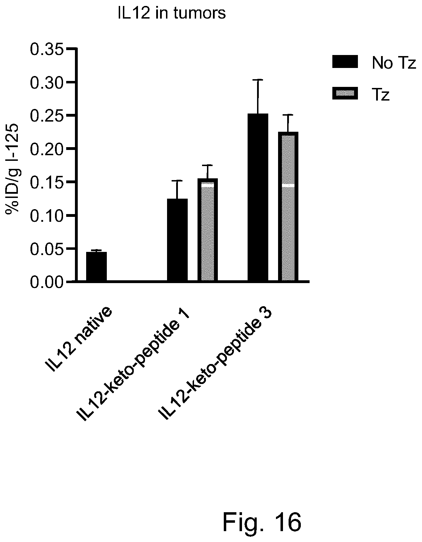

Figure 16. Detection of ¹²⁵I-labelled peptide-masked IL12 constructs in tumor-bearing nude mice. Peptide masked constructs **16.1 and 16.3** were administered i.v. in PC3 tumor-bearing nude mice (10ug/mouse), 24hr after injection of the constructs mice received an i.v. injection of a tetrazine **(8.5),** followed by euthanasia 1hr later. IL12 content of the tumor was determined using ¹²⁵I activity in isolated tumors. Data show significantly less tumor retention of wildtype IL12, as compared to the peptide-targeted constructs, and relatively low washout after Tz unmasking. Data represent the mean percentage injected dose per gram (% ID g-1) with SEM, (n=4).

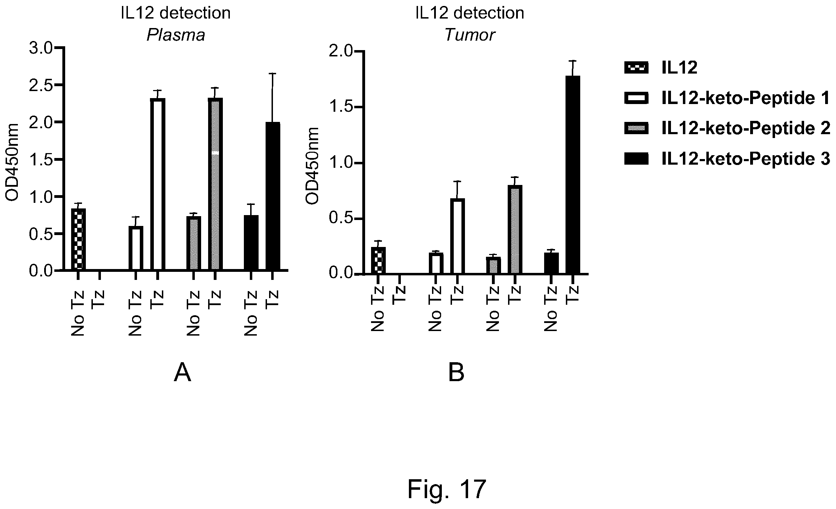

Figure 17. Detection of IL12 constructs in tumor-bearing nude mice by ELISA. Mice were treated similarly as in fig 16. Tumors were isolated and homogenized, after which IL12 in the homogenate and simultaneously isolated blood plasma was detected using ELISA. Data show that release of the masks by Tz injection leads to significantly more detection of IL12 in blood (A) as well as in the tumor (B).

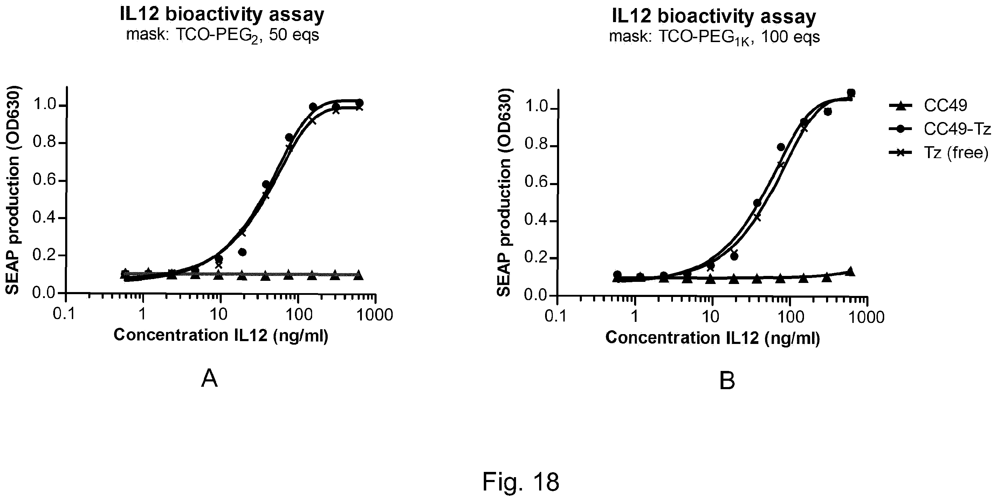

Figures 18 shows the results from a HEK-Blue IL12 reporter assay, similarly performed as in previous figures, with the exception that the tetrazine activator was provided as a conjugate to a CC49 IgG, which targets TAG72 expressed in tumors (CC49-Tz, compound **21.4).** IL12 proteins were masked using conjugation with TCO-PEG₂ (compound **9.4,** figure 16A) or TCO-PEG_{1K} (compound **9.10,** figure 16B). Shown is biological activity of the IL12 constructs after addition of CC49 (triangles), CC49-Tz (circles), or non-conjugated tetrazine activator **(8.5)** (crosses).

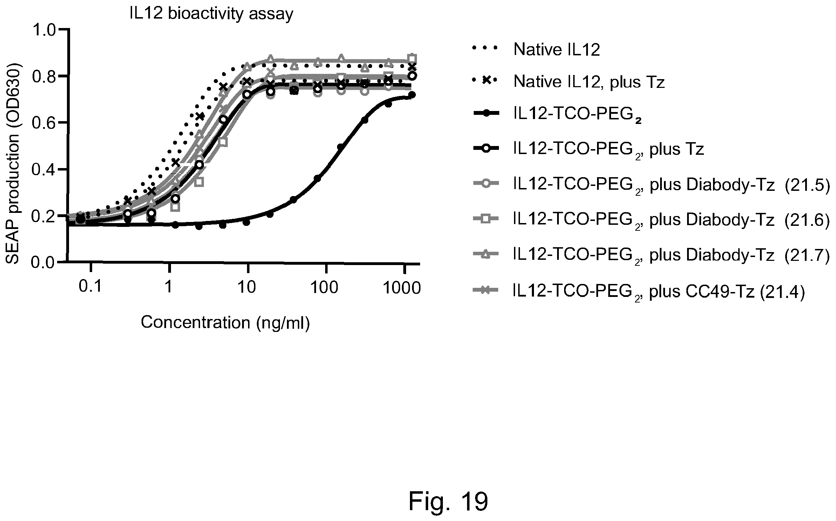

Figure 19 shows the results from a HEK-Blue IL12 reporter assay, similarly performed as in previous figures, with the exception that the tetrazine activator was provided as a conjugate to AVP0458 diabodies, which target TAG72 expressed in tumors. Figure shows bioactivity of IL12-TCO-PEG₂ construct **(9.2),** reacted with unconjugated tetrazine **(8.5),** the diabody-Tz constructs **21.5, 21.6,** and **21.7,** or CC49-Tz **(21.4).**

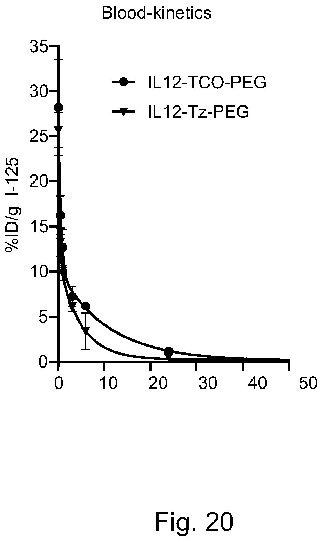

Figure 20. Blood kinetics of ¹²⁵I -labelled masked IL12 constructs after activator pre-targeting. TCO/Tz-PEG-masked IL12 constructs **9.2** and **12.3** were radiolabelled with ¹²⁵I and administered i.v. in tumour-free mice (10ug/mouse), followed by detection of radioactivity in blood samples collected at various timepoints (2, 30, 60min, 3, 6, 24, and 72hrs post injection). All constructs show a comparable clearance pattern as wildtype IL12. Data represent the mean percentage injected dose per gram (% ID g-1) with s.d. (n=3).

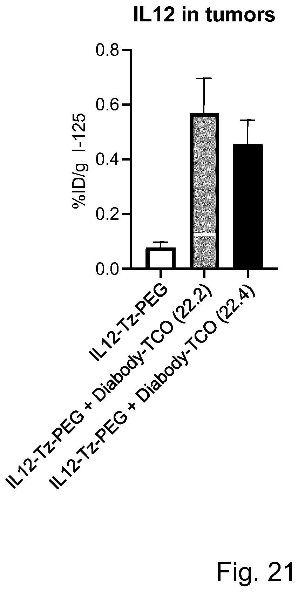

Figure 21. Detection of ¹²⁵I -labelled Tz-masked IL 12 constructs in tumors after activator pre-targeting. TCO-functionalized AVP0458 compounds **22.2** and **22.4** were administered i.v. in LS174T tumor-bearing nude mice (40ug/mouse). 24h **(22.2),** or 48h **(22.4)** later, when diabodies were virtually cleared from blood, ¹²⁵I -labeled Tz-PEG-masked IL12 construct **(12.3)** was injected i.v. (10ug/mouse). 24hrs later, plasma and tumors were isolated and IL12 content was determined using ¹²⁵I counts. Data show more tumor retention of masked IL12-Tz-PEG when TCO activator was pre-targeted via the diabodies. Data represent the mean percentage injected dose per gram (% ID g-1) with SEM, (n=4).

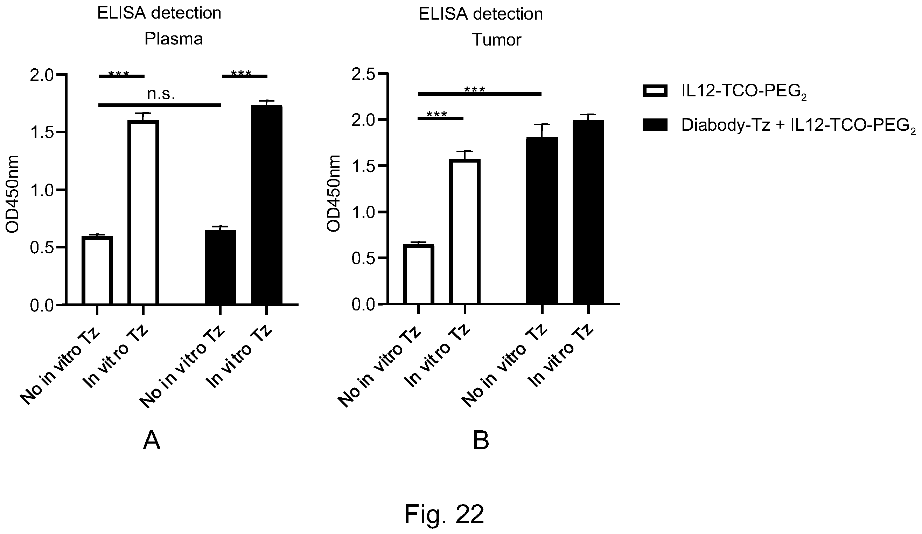

Figure 22. ELISA detection of masked IL12 constructs in tumors and plasma after activator pre-targeting. Tz-functionalized AVP0458 compound **21.6** was administered i.v. in mice bearing LS174T tumors (40ug/mouse). 24hrs later, when **21.6** was virtually cleared from blood, TCO-PEG-masked IL12 construct **(9.2)** was injected i.v. (10ug/mouse). 24hrs later, plasma and tumors were isolated. Tumors were homogenized, with or without addition of 100 eqs of small-molecule Tz activator **8.5** to unmask remaining IL12 constructs ("in vitro Tz"). IL12 in the homogenate (B) and simultaneously isolated blood plasma (A) was detected using ELISA. Data represent mean OD450nm values with SEM, (n=4).

### Detailed Description of the Invention

The invention, in a broad sense, is based on the judicious insight that IL12 protein can be deactivated if one or more of its lysine residues is modified with a Trigger as described herein, and then be reactivated after contacting the masked IL12 protein with a diene (if the Trigger is a dienophile) or a dienophile (if the Trigger is a diene). Surprisingly, even a small Trigger moiety as described herein is able to significantly lower IL12 activity. Moreover, the activity of the masked IL12 protein can be restored after contacting it with a diene, which can be administered to a subject separately from the masked IL12 protein. This makes unmasking independent from the microenvironment of the target site, and the timing of unmasking can be controlled. Furthermore, the masked IL12 proteins and masked subunits of the invention can be readily produced, and allow the use of wild-type IL12 proteins and subunits. This may also lower the anti-drug antibody (ADA) response in subjects, as masked IL12 proteins and/or subunits are used that are highly similar to the wildtype protein and/or subunits.

In addition, the activity of the masked IL12 protein of the invention is generally lower than those of the very few known masked IL12 proteins. Simultaneously, the unmasking strategy of the invention involves contacting the masked IL12 protein with an activator, viz. a diene (if the Trigger is a dienophile) or a dienophile (if the Trigger is a diene). Thereafter, the Trigger moiety is cleaved off the lysine residue of the IL12 protein, leaving an unmodified lysine residue. As such, a wild-type IL12 protein can be obtained after unmasking, and its activity is fully or almost fully restored. Moreover, the reaction between the masked IL12 protein and the activator proceeds very rapidly, thus improving the unmasking kinetics as compared to known strategies.

For at least the above reasons, the invention addresses one or more of the problems and desires as identified herein.

Chemical cleavage of one or more of the Trigger moieties present in the masked IL12 protein is achieved by provision of an activator molecule. As described herein, the activator molecule comprises a compound with a diene, preferably a tetrazine, structure, if the Trigger is a dienophile. Such compounds are able to react with the dienophile moiety. Conversely, if the Trigger is a diene, the activator comprises a dienophile.

The masked IL12 protein can include a masking moiety that also provides additional advantageous properties. For example, the masked IL12 protein can contain a masking moiety that simultaneously extends the serum half-life and/or targets the masked IL12 protein to a desired site of cytokine activity. Preferably, this element is removed chemically, by administration of an activator compound, at the desired body location (e.g., in the tumor microenvironment), restoring pharmacokinetic properties to the payload molecule (e.g., IL12) substantially similar to the naturally occurring payload molecule.

The masked IL12 proteins may be targeted to a desired cell or tissue in an active or passive way. As described herein active targeting is typically accomplished through the action of a targeting agent, e.g. a small molecule, peptide, aptamer, protein, DARPins, antibody, or antibody fragment, binding a target expressed on the target location (for example a tumor-specific antigen, receptor, or antigen present in the extracellular matrix of a tumor). Passive targeting on the other hand relies on the passive accumulation of the masked IL12 protein at the desired cell or tissue by modulating pharmacokinetics and distribution via molecular properties of the masked IL12 protein (e.g. size). Typically, in these situations the masked IL12 protein contains one or more moieties that extends and modulates the half-life of the construct (e.g. PEG, albumin binders). Preferably the masked IL 12 is designed to passively target the tumor by means of the Enhanced Permeability and Retention (EPR) effect.

The targeting agent may be attached to the cytokine via a chemically cleavable or non-cleavable linker. If attached by a non-cleavable linker, the targeting domain may further aid in retaining the cytokine at the target site, for example a tumor, and may be considered a retention domain. The targeting domain does not necessarily need to be directly linked to the cytokine moiety, and may be linked via another element of the masked IL12 protein.

In one aspect the invention provides a masked IL12 protein comprising an IL12 protein or subunit thereof, and a masking moiety, *e.g*. a polyethylene glycol (PEG) masking domain. The masking moiety is conjugated to the IL12 protein, through a linker, and can be separated from the IL12 protein by cleavage of the construct due to reaction of the Trigger with a separately provided activator. For example, when the IL12 protein is conjugated to a masking moiety through a linker that contains a TCO cleavage site, the IL12 protein is released from the masking moiety and can bind its receptor, upon tetrazine-driven cleavage of the linker.

Preferably, the masked IL12 protein is designed to be targeted to the site of desired cytokine activity, for example the tumor microenvironment, while the presence of the masked IL12 protein at non-desired locations (e.g. non-tumor tissue, or blood circulation), is decreasing over time. Administering the activator separated in time from the masked IL12 protein, offers selective cytokine activity concentrated at the desired locations, while avoiding off-target activity and reducing overall toxicity of cytokine therapy.

In one embodiment, the masked IL12 protein is provided before the activator. Herein, the activator is provided only once the masked IL12 protein is present at the desired location and at the desired local concentration, while at the same time the masked IL12 protein has been cleared from circulation and non-target tissues to a desired reduced level as compared to the initial level occurring directly after the administration.

In another embodiment, the activator is administered to the subject before the masked IL12 protein is administered to said subject. Herein, the activator may typically be prelocalized at the target site, for example the tumor microenvironment. Such prelocalization can be achieved by injecting a construct comprising one or more activator moieties at or in the vicinity of the target site. Such construct can comprise a biomolecule or a polymer, such as hyaluronic acid, to which the one or more activator moieties are covalently linked. The construct typically has a size that enables it to remain at or in the vicinity of the target site, and it is not cleared efficiently. Prelocalization of the activator can also be achieved by conjugation of the activator to a targeting agent such as a small molecule, peptide, aptamer, protein, DARPin, antibody, or antibody fragment, binding a target expressed on the target location (for example a tumor-specific antigen). This targeting agent will provide the activator with active tissue targeting upon systemic administration. In conjunction, a masked IL12 protein of the invention may next be injected only once the targeted activator is present at the desired location and at the desired local concentration. If a targeted activator is used, the masked IL12 protein is preferably administered when the activator has been cleared from circulation and non-target tissues to a desired reduced level as compared to the initial level directly after administration of the activator. As described herein the prelocalized activator will activate the attenuated masked IL12 protein by cleavage of the Trigger being part of the masked IL12 protein, thereby restoring complete or nearly complete IL 12-receptor activating activity of the IL12 moiety present in the masked IL12 protein. In other embodiments the activator is targeted to the target tissue in a passive way, as described herein for the masked IL12, for example by comprising one or more activator moieties in a construct that targets the tumor by means of the EPR effect (e.g. diene-containing nanoparticles, diene polymer conjugates) (Maeda et al. J. Pers Med. 2021, volume 11, page 229). In other embodiments both the masked IL12 and the activator are targeted to the target tissue by either passive or active targeting as described herein.

In one embodiment, a masked IL12 protein is provided that includes, [B] an IL12 masking moiety, wherein said masked IL12 protein has attenuated IL12-receptor activating activity. Optionally, the masked IL12 protein additionally includes [H] a half-life extension domain, or [S] a spacer to provide a certain distance between the various moieties in the masked IL12 protein. Typically, the IL 12-receptor activating activity of the masked IL12 protein is at least about 10 fold less than the IL12-receptor activating activity of the masked IL12 protein that is produced by cleavage of the Trigger moiety. The serum half-life of the unmasked IL12 protein that is produced by activator-driven cleavage of the Trigger moiety is typically comparable to the half-life of naturally occurring IL12.

The masked IL12 protein can have the formula: [A]-[L]; [A]-[L]-[B]; [A]-[L]-[B]-[H]; [A]-[L]-[B]-[S]-[H]; [A]-[L]-[B]-[L]-[H]; [A]-[L]-[H]; [A]-[L]-[H]-[B]; [A]-[L]-[H]-[S]-[B]; [A]-[L]-[H]-[L]-[B]; [H]-[A]-[L]-[B]; [H]-[S]-[A]-[L]-[B]; [H]-[L]-[A]-[L]-[B]; or [B]-[L]-[A]-[L]-[A]-[L]-[B]; wherein [A] is an interleukin 12 (IL12) protein, [B] is one or more masking moieties, [L] is one or more chemically cleavable linkers (*i.e.* one or more dienophile moieties as described herein), and [S] is one or more chemical or polypeptide linkers/spacers that is typically non cleavable, and [H] is one or more half-life extension elements. The interleukin 12 (IL12) polypeptide [A] can be further defined by its molecular subunits: [A1] IL12 p35 subunit polypeptide, and [A2] IL12 p40 subunit polypeptide. As is known in the art, both IL12 subunits can be joined by a linker or spacer, still giving the total construct the full IL12 receptor activating activity as the naturally occurring IL12 protein. Therefore, in a further embodiment, in the above masked IL12 protein formulas the IL12 protein [A] may be substituted by: [A1]-[S]-[A2]; or [A2]-[S]-[A1]; wherein [A1] is an IL12 p35 subunit polypeptide, and [A2] is an IL12 p40 subunit polypeptide.

The masked IL12 protein can further include a targeting agent such as a small molecule, peptide, aptamer, DARPin, antibody, or antibody fragment, binding a target expressed on the target location (for example a tumor-specific antigen). This targeting agent will provide the masked IL12 protein with active tissue targeting. For example, the targeting agent can be linked to any one of [A], [B], or [H] by a chemically cleavable or non-cleavable linker. As described herein, when the masked IL12 protein includes a targeting agent, preferably the activator is provided separately only once the distribution of the masked IL12 protein throughout the body is favourable.

In a different embodiment, the activator is conjugated to a targeting agent, comprising a small molecule, (oligo)nucleotide, aptamer, carbohydrate, protein, peptide, peptoid, DARPin, antibody, or antibody fragment, binding a target expressed on the target location (for example a tumor-specific antigen). The diene, preferably a tetrazine, or a dienophile, preferably a *trans*-cyclooctene, and the targeting agent may be joined directly, or through a non-cleavable linker.

The IL12-receptor activating activity of the masked and unmasked IL12 proteins of the invention can be assessed by reporter assays, for example using reporter cells like the HEK Blue reporter cells (Invivogen). These assays use equal amounts on a mole basis of the IL12 protein and/or the IL12 masked IL12 protein.

The half-life extension element of the masked IL12 protein can be, for example, human serum albumin, an antigen-binding polypeptide or small molecule that binds human serum albumin, an immunoglobulin Fc, or a water-soluble polypeptide such as polysarcosine, or an optionally branched or multi-armed polyethylene glycol (PEG), all previously employed in the art to extend serum half-life.

In some embodiments, the masking moiety can also function as a serum half-life extension element (e.g. polyethylene glycol). In some other embodiments, the masked IL12 protein comprises a separate serum half-life extension element.

Preferably, the masking agent also functions as the targeting agent. Preferably masking agents that also function as targeting agents are selected from the list comprisising amino acids, peptides, peptoids, proteins, antibody fragments, carbohydrates, nucleotides, oligonucleotides, aptamers, steroids, glycan small organic molecules, small inorganic molecules, and combinations thereof. Preferably, the masking agents that also function as targeting agents have a molecular weight of at most 20 kDa, more preferably at most 10 kDa, more preferably at most 5 kDa, more preferably at most 2 kDa, more preferably at most 1 kDa, and most preferably at most 500 Da. In other embodiments, said masking agents have a molecular weight of between 300 and 2000 Da more preferably between 500 and 1000 Da.

In other embodiments, the dienophile or diene moiety itself is the masking agent, and no additional masking agents are comprised in the masked IL12.

In addition to serum half-life extension and/or masking moieties, the pharmaceutical compositions described herein preferably comprise at least one, or more targeting agents that bind to one or more target antigens or one or more regions on a single target antigen. It is contemplated herein that a masked IL12 protein of the invention is cleaved, for example, after the masked IL12 protein has bound to a target antigen present in a disease-specific microenvironment. At least one target antigen is involved in and/or associated with a disease, disorder or condition. Exemplary target antigens include those associated with a proliferative disease, a tumorous disease, an inflammatory disease, an immunological disorder, an autoimmune disease, an infectious disease, a viral disease, an allergic reaction, a parasitic reaction, a graft-versus-host disease or a host-versus-graft disease.

In some embodiments, the target antigen is a cell surface molecule such as a protein, lipid or polysaccharide. In some embodiments, the target antigen is present on a tumor cell, virally infected cell, bacterially infected cell, damaged red blood cell, arterial plaque cell, or fibrotic tissue cell. Cell surface target antigens typically are expressed on the surface of a diseased cell or tissue, for example a tumor or a cancer cell, or an immune cell. Such target antigens for tumors include but are not limited to Trophoblast glycoprotein (5T4), Tumor-associated calcium signal transducer 2 (Trop2), gpA33, CGS-2, EpCAM, EGFR, HER-2, HER-3, c-Met, FOLR1, TAG72, and CEA.

In other embodiments, the target antigen is a molecule such as a protein, lipid or polysaccharide present in the direct vicinity of the diseased cell, e.g. the tumor microenvironment. Typically these molecules are found in the extracellular matrix, and not anymore directly connected to a cell. Such target antigens for tumors include but are not limited to Fibroblast activation protein alpha (FAPa), Tenacin C, Tenacin W, Fibronectin EDB (EDB-FN), Fibronectin EDA (EDA-FN), and fibronectin EIIIB domain.

In some embodiments, the targeting polypeptides independently comprise a scFv, a VH domain, a VL domain, a non-Ig domain, or a ligand that specifically binds to the target antigen. In some embodiments, the targeting polypeptide serves as a retention domain and is attached to the cytokine via a non-cleavable linker.

This disclosure also relates to pharmaceutical compositions that comprise a masked IL12 protein according to the invention. Typically, the pharmaceutical composition contains one or more physiologically acceptable carriers and/or excipients.

### Compounds of the invention

The invention relates to a masked IL12 protein or salts, hydrates or solvates thereof. At least one lysine residue of said masked IL12 protein has a structure according to Formula (1), which means that at least one lysine residue of the IL12 protein has been modified with a dienophile as defined herein to yield a masked IL12 protein. The masked IL12 protein has a much lower activity than the unmasked IL12 protein, but the activity can be restored by contacting the masked IL12 protein with a diene, which reacts with the dienophile so as to release an active IL12 protein.

As the skilled person is aware, typically the IL12 protein has two subunits, an α-chain that is also referred to as the p35 subunit, and a β-chain that is also referred to as the p40 subunit. In nature, the p35 and p40 subunits together form an active IL12 protein via noncovalent interactions. It is known, however, that the IL12 subunits can also be covalently linked, generally via short peptide, to yield an active IL12 protein. Typically, such a covalent linker is used to make expression more efficient, since only one vector needs to be expressed instead of two vectors for each separate subunit. Such linkers for connecting the two IL12 subunits typically take the form of -[X]ₙ-, wherein X is an amino acid residue, preferably selected from the group consisting of G, S, A, and D; and n is an integer in the range of from 3 to 20, preferably of from 5 to 15. Examples of such linkers include GGGGS, GGGGSGGGGS, GGGGSGGGGSGGGGS, SGGGG, SGGGGSGGGG, SGGGGSGGGGSGGGG, GSADGG, GGSGG, and GGGAGGGAGGGA.

In preferred embodiments, the IL12 protein or a subunit thereof is a recombinant protein.

For the masked IL12 protein, it will be understood that at least one of the p35 subunit and the p40 subunit needs to be masked, *i.e.* contain at least one lysine residue according to Formula (1). In preferred embodiments, the masked IL12 protein comprises a masked p35 subunit and a non-masked p40 subunit. In other preferred embodiments, the masked IL12 protein comprises a non-masked p40 subunit and a masked p40 subunit. Most preferably, the masked IL12 protein comprises a masked p35 subunit and a masked p40 subunit.

It will be understood that in the phrase "a masked IL12 protein or a masked subunit thereof" as used herein, said masked subunit relates to a masked p35 subunit or a masked p40 subunit. Such masked subunits are also part of the invention, as they are intermediate products. For example, a masked p35 subunit can be bound to a non-masked p40 subunit to yield a masked IL12 protein, and vice versa.

The masked p35 subunit has a sequence similarity of at least 80%, preferably at least 85%, more preferably at least 90%, even more preferably at least 93%, yet more preferably at least 95%, and most preferably at least 99%, with an amino acid sequence selected from the group consisting of SEQ ID NO: 1 or SEQ ID NO: 14. In preferred embodiments, the masked p35 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 1. In preferred embodiments, the masked p35 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 14.

In preferred embodiments, the masked p35 subunit has a sequence identity of at least 80%, preferably at least 85%, more preferably at least 90%, even more preferably at least 93%, yet more preferably at least 95%, and most preferably at least 99%, with an amino acid sequence selected from the group consisting of SEQ ID NO: 1 or SEQ ID NO: 14. In preferred embodiments, the masked p35 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 1. In preferred embodiments, the masked p35 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 14.

In preferred embodiments, the p35 subunit has a sequence similarity of at least 80%, preferably at least 85%, more preferably at least 90%, even more preferably at least 93%, yet more preferably at least 95%, and most preferably at least 99%, with an amino acid sequence selected from the group consisting of SEQ ID NO: 1 or SEQ ID NO: 14. In preferred embodiments, the p35 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 1. In preferred embodiments, the p35 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 14.

In preferred embodiments, the p35 subunit has a sequence identity of at least 80%, preferably at least 85%, more preferably at least 90%, even more preferably at least 93%, yet more preferably at least 95%, and most preferably at least 99%, with an amino acid sequence selected from the group consisting of SEQ ID NO: 1 or SEQ ID NO: 14. In preferred embodiments, the p35 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 1. In preferred embodiments, the p35 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 14.

The masked p40 subunit has a sequence similarity of at least 80%, preferably at least 85%, more preferably at least 90%, even more preferably at least 93%, yet more preferably at least 95%, and most preferably at least 99%, with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 2. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 3. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 4. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 5. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 6. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 7. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 8. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 9. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 10. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 11. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 12. In preferred embodiments, the masked p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 13.

In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 2. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 3. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 4. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 5. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 6. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 7. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 8. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 9. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 10. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 11. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 12. In preferred embodiments, the masked p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 13.

In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 2. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 3. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 4. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 5. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 6. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 7. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 8. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 9. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 10. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 11. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 12. In preferred embodiments, the p40 subunit has a sequence similarity of at least 80%, preferably at least 85%, more preferably at least 90%, even more preferably at least 93%, yet more preferably at least 95%, and most preferably at least 99%, with an amino acid sequence of SEQ ID NO: 13.

In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 2. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 3. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 4. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 5. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 6. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 7. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 8. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 9. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 10. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 11. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 12. In preferred embodiments, the p40 subunit has a sequence identity of at least 80%, preferably at least 85%, more preferably at least 90%, even more preferably at least 93%, yet more preferably at least 95%, and most preferably at least 99%, with an amino acid sequence of SEQ ID NO: 13.

In preferred embodiments, the p35 subunit is an amino acid sequence according to SEQ ID NO: 1. In other preferred embodiments, the p35 subunit is an amino acid sequence according to SEQ ID NO: 14.

In preferred embodiments, the masked p35 subunit is an amino acid sequence according to SEQ ID NO: 1. In other preferred embodiments, the masked p35 subunit is an amino acid sequence according to SEQ ID NO: 14.

In preferred embodiments, the p40 subunit is an amino acid sequence according to SEQ ID NO: 2. In preferred embodiments, the p40 subunit is an amino acid sequence according to SEQ ID NO: 3. In preferred embodiments, the p40 subunit is an amino acid sequence according to SEQ ID NO: 4. In preferred embodiments, the p40 subunit is an amino acid sequence according to SEQ ID NO: 5. In preferred embodiments, the p40 subunit is an amino acid sequence according to SEQ ID NO: 6. In preferred embodiments, the p40 subunit is an amino acid sequence according to SEQ ID NO: 7. In preferred embodiments, the p40 subunit is an amino acid sequence according to SEQ ID NO: 8. In preferred embodiments, the p40 subunit is an amino acid sequence according to SEQ ID NO: 9. In preferred embodiments, the p40 subunit is an amino acid sequence according to SEQ ID NO: 10. In preferred embodiments, the p40 subunit is an amino acid sequence according to SEQ ID NO: 11. In preferred embodiments, the p40 subunit is an amino acid sequence according to SEQ ID NO: 12. In preferred embodiments, the p40 subunit is an amino acid sequence according to SEQ ID NO: 13.

In preferred embodiments, the masked p40 subunit is an amino acid sequence according to SEQ ID NO: 2. In preferred embodiments, the masked p40 subunit is an amino acid sequence according to SEQ ID NO: 3. In preferred embodiments, the masked p40 subunit is an amino acid sequence according to SEQ ID NO: 4. In preferred embodiments, the masked p40 subunit is an amino acid sequence according to SEQ ID NO: 5. In preferred embodiments, the masked p40 subunit is an amino acid sequence according to SEQ ID NO: 6. In preferred embodiments, the masked p40 subunit is an amino acid sequence according to SEQ ID NO: 7. In preferred embodiments, the masked p40 subunit is an amino acid sequence according to SEQ ID NO: 8. In preferred embodiments, the masked p40 subunit is an amino acid sequence according to SEQ ID NO: 9. In preferred embodiments, the masked p40 subunit is an amino acid sequence according to SEQ ID NO: 10. In preferred embodiments, the masked p40 subunit is an amino acid sequence according to SEQ ID NO: 11. In preferred embodiments, the masked p40 subunit is an amino acid sequence according to SEQ ID NO: 12. In preferred embodiments, the masked p40 subunit is an amino acid sequence according to SEQ ID NO: 13.

In particular, the IL12 protein or a subunit thereof according to the invention may comprise further modified amino acid residues, preferably lysine residues, that are coupled to e.g. targeting agents as defined herein.

In other preferred embodiments, said modified amino acid residues, that are coupled to e.g. targeting agents as defined herein, are cysteine, tyrosine, non-natural amino acid residues, or chemically or enzymatically modified amino acid residues.

In preferred embodiments, the masked IL12 protein or masked subunit according to the invention has at least two, preferably at least three, more preferably at least four, even more preferably at least five, more preferably still at least ten lysine residues that have a structure according to Formula (1).

Preferably, the masked IL12 protein has a number of lysine residues having a structure according to Formula (1) of from 1 to 40, more preferably of from 3 to 30, even more preferably of from 4 to 25, more preferably still of from 5 to 20, more preferably of from 6 to 15, more preferably of from 7 to 14, and most preferably of from 8 to 12.

Preferably, the masked p35 subunit has a number of lysine residues having a structure according to Formula (1) of from 1 to 15, more preferably of from 2 to 14, even more preferably of from 3 to 13, more preferably still of from 4 to 12, more preferably of from 5 to 11, more preferably of from 6 to 10, and most preferably of from 7 to 9.

Preferably, the masked p40 subunit has a number of lysine residues having a structure according to Formula (1) of from 1 to 25, more preferably of from 2 to 22, even more preferably of from 3 to 20, more preferably still of from 4 to 18, more preferably of from 5 to 16, more preferably of from 6 to 14, and most preferably of from 7 to 12.

If the Trigger is a dienophile, preferably the masked IL12 protein has a number of lysine residues having a structure according to Formula (1) of from 1 to 40, more preferably of from 3 to 30, even more preferably of from 4 to 25, more preferably still of from 5 to 20, more preferably of from 6 to 15, more preferably of from 7 to 14, and most preferably of from 8 to 12.

If the Trigger is a dienophile, preferably the masked p35 subunit has a number of lysine residues having a structure according to Formula (1) of from 1 to 15, more preferably of from 2 to 14, even more preferably of from 3 to 13, more preferably still of from 4 to 12, more preferably of from 5 to 11, more preferably of from 6 to 10, and most preferably of from 7 to 9.

If the Trigger is a dienophile, preferably the masked p40 subunit has a number of lysine residues having a structure according to Formula (1) of from 1 to 25, more preferably of from 2 to 22, even more preferably of from 3 to 20, more preferably still of from 4 to 18, more preferably of from 5 to 16, more preferably of from 6 to 14, and most preferably of from 7 to 12.

If the Trigger is a tetrazine, preferably the masked IL12 protein has a number of lysine residues having a structure according to Formula (1) of from 10 to 40, more preferably of from 11 to 35, even more preferably of from 12 to 30, more preferably still of from 13 to 25, more preferably of from 14 to 23, more preferably of from 15 to 21, and most preferably of from 16 to 19.

If the Trigger is a tetrazine, preferably the masked p35 subunit has a number of lysine residues having a structure according to Formula (1) of from 10 to 40, more preferably of from 11 to 35, even more preferably of from 12 to 30, more preferably still of from 13 to 25, more preferably of from 14 to 23, more preferably of from 15 to 21, and most preferably of from 16 to 19.

In preferred embodiments, the masked IL12 protein or masked subunit according to the invention has a functionalization grade of at least two, more preferably at least three, yet more preferably at least four, even more preferably at least five, more preferably still at least ten.

Preferably, the masked IL12 protein has a functionalization grade of from 1 to 40, more preferably of from 3 to 30, even more preferably of from 4 to 25, more preferably still of from 5 to 20, more preferably of from 6 to 15, more preferably of from 7 to 14, and most preferably of from 8 to 12.

Preferably, the masked p35 subunit has a functionalization grade of from 1 to 15, more preferably of from 2 to 14, even more preferably of from 3 to 13, more preferably still of from 4 to 12, more preferably of from 5 to 11, more preferably of from 6 to 10, and most preferably of from 7 to 9.

Preferably, the masked p40 subunit has a functionalization grade of from 1 to 25, more preferably of from 2 to 22, even more preferably of from 3 to 20, more preferably still of from 4 to 18, more preferably of from 5 to 16, more preferably of from 6 to 14, and most preferably of from 7 to 12.

If the Trigger is a dienophile, preferably the masked IL12 protein has a functionalization grade of from 1 to 40, more preferably of from 3 to 30, even more preferably of from 4 to 25, more preferably still of from 5 to 20, more preferably of from 6 to 15, more preferably of from 7 to 14, and most preferably of from 8 to 12.

If the Trigger is a dienophile, preferably the masked p35 subunit has a functionalization grade of from 1 to 15, more preferably of from 2 to 14, even more preferably of from 3 to 13, more preferably still of from 4 to 12, more preferably of from 5 to 11, more preferably of from 6 to 10, and most preferably of from 7 to 9.

If the Trigger is a dienophile, preferably the masked p40 subunit has a functionalization grade of from 1 to 25, more preferably of from 2 to 22, even more preferably of from 3 to 20, more preferably still of from 4 to 18, more preferably of from 5 to 16, more preferably of from 6 to 14, and most preferably of from 7 to 12.

If the Trigger is a tetrazine, preferably the masked IL12 protein has a functionalization grade of from 10 to 40, more preferably of from 11 to 35, even more preferably of from 12 to 30, more preferably still of from 13 to 25, more preferably of from 14 to 23, more preferably of from 15 to 21, and most preferably of from 16 to 19.

If the Trigger is a tetrazine, preferably the masked p35 subunit has a functionalization grade of from 10 to 40, more preferably of from 11 to 35, even more preferably of from 12 to 30, more preferably still of from 13 to 25, more preferably of from 14 to 23, more preferably of from 15 to 21, and most preferably of from 16 to 19.

If the Trigger is a tetrazine, preferably the masked p40 subunit has a functionalization grade of from 10 to 40, more preferably of from 11 to 35, even more preferably of from 12 to 30, more preferably still of from 13 to 25, more preferably of from 14 to 23, more preferably of from 15 to 21, and most preferably of from 16 to 19.

Preferably, for the masked p35 subunit the at least one lysine residue having the structure according to Formula (1) is selected from the group consisting of K122, K128, K168, K170, K110, K117, K158, K172, K32, K54, K56, K70, and K93, wherein said numbers refer to the amino acid residues of SEQ ID NO:1 or equivalent positions thereof, *e.g.* in SEQ ID NO:14. More preferably, said at least one lysine residue is selected from the group consisting of K122, K128, K168, K170, K110, K117, K158, and K172. Most preferably, said at least one lysine residue is selected from the group consisting of K122, K128, K168, and K170. Without wishing to be bound by theory, the inventors believe that modification of K122, K128, K168, and/or K170 has the largest impact on the activity of the IL12 protein.

Preferably, for the masked p40 subunit the at least one lysine residue having the structure according to Formula (1) is selected from the group consisting of K58, K195, K197, K51, K70, K258, K260, K263, K264, K280, K5, K6, K84, K85, K96, K99, K102, K104, K112, K135, K163, K212, K217, K222, K225, and K271; wherein said numbers refer to the amino acid residues of SEQ ID NO: 2 or equivalent positions thereof in other sequences such as those of SEQ ID NO: 3 up to and including SEQ ID NO: 13. More preferably, said at least one lysine residue having the structure according to Formula (1) is selected from the group consisting of K58, K195, K197, K51, K70, K258, K260, K263, K264, and K280. Even more preferably, said at least one lysine residue having the structure according to Formula (1) is selected from the group consisting of K58, K195, K197, K51, and K70. Most preferably, said at least one lysine residue having the structure according to Formula (1) is selected from the group consisting of K58, K195, and K197. Without wishing to be bound by theory, the inventors believe that modification of K58, K195, and/or K197 has the largest impact on the activity of the IL12 protein.

In the masked IL12 protein or the masked subunit thereof according to the invention, at least one lysine residue has a structure according to Formula (1): 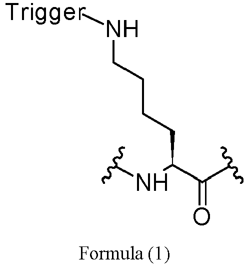 wherein the wiggly lines indicate bonds to other amino acid residues of the masked IL12 protein or the masked subunit. The Trigger, which is a dienophile or a tetrazine, is linked to the ε-amine of the lysine residue of Formula (1) via a bond C^{A}. It will be understood that C^{A} is a covalent bond. The Trigger can herein also be denoted as T^{R}.

If the Trigger is a tetrazine, it is a tetrazine according to Formula (2): 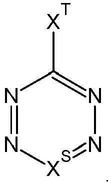 X^{S} is an optionally substituted carbon atom, preferably substituted with a group RG1 not being hydrogen or RG5. Preferably, X^{S} is a carbon atom substituted with a group according to Q₁ or Q₂ as disclosed for Formula (4), or Yₐ or Y_{b} as disclosed herein for Formula (14), including any one of the more specific embodiments of said Q₁, Q₂, Yₐ, and Y_{b}. In another preferred embodiment, X^{S} is a carbon atom substituted with RT¹. More preferably, X^{s} is a carbon atom substituted with phenyl or alkyl, wherein said phenyl and alkyl are optionally substituted.

X^{T} is -A^{T}-(B^{T})_{b}-X^{C}-C(C^{T})-(L^{C})_{f}-C^{A}, and A^{T} and B^{T} are each independently selected from the group consisting of an optionally substituted carbon atom, an optionally substituted nitrogen atom, O, S, S(=O), and S(=O)₂, optionally the bond between A^{T} and B^{T} is a double bond. Preferably, A^{T} and B^{T} are each independently a carbon attached to two groups RG1. In other preferred embodiments, A^{T} and B^{T} are each independently from the group consisting of CRT²₂, C=O, C=CRT³₂, C=NRT⁶, NRT⁴, O, S, S(=O), or S(=O)₂, provided that no sets consisting of adjacent atoms are present selected from the group consisting of -O-O-, -S-N(RT⁴)-, -O-S-, -O-S(=O)-, -O-S(=O)₂-, and -S-S-, and wherein one RT² or RT⁴ group from B^{T} and one RT² or RT⁴ group from A^{T} can together be a bond, so as to form a double bond, and wherein two or more groups selected from RT², RT³, RT⁴, RT⁵, RT⁶ can together form a C₆-C₁₂ aryl, or a 3-to 8-membered heterocycle. wherein each RT¹, RT², RT³ and RT⁵ is independently selected from the group consisting of H, (hetero)alkyl, aryl, heterocycle, carbocycle, F, CF₃, CF₂-RT', ORT', STR', CN, C(=O)RT', S(=O)₂RT", S(=O)₂ORT', OS(=O)₂RT", PO₃RT'₂, Si-RT"₃, Si-O-RT"₃, B(ORT')₂, S(=O)₂NRT'₂, NRT'S(=O)₂RT", C(=O)NRT'₂, C(=S)NRT'₂, NRT'₂, NRT"₃⁺, NRT'C(=O)RT', NRT'C(=S)RT', NRT'C(=O)NRT'₂, NRT'C(=S)NRT'₂ wherein each RT' is independently selected from the group consisting of H, (hetero)alkyl, aryl, carbocycle, heterocycle, and each RT" is independently selected from the group consisting of (hetero)alkyl, aryl, carbocycle, heterocycle, wherein all (hetero)alkyl, aryl, carbocycle, heterocycle moieties comprised in RT¹, RT², RT³ and RT⁵ can be unsubstituted or substituted, and wherein two RT' or RT" groups can together form a ring;
each RT⁴ and RT⁶ is independently selected from the group consisting of H, (hetero)alkyl, aryl, heterocycle, carbocycle, ORT', C(=O)RT', C(=O)NRT'₂, C(=S)NRT'₂, C(=NRT')NRT'₂, NRT'₂, preferably such that no sets consisting of adjacent atoms are present selected from the group consisting of -N-N-, -N-O-, -N-S-, -N-Si- and -N-P-, and wherein each RT' is independently selected from the group consisting of hydrogen, (hetero)alkyl, aryl, carbocycle, heterocycle, wherein all (hetero)alkyl, aryl, carbocycle, heterocycle moieties comprised in RT⁴ and RT⁶ can be unsubstituted or substituted, and wherein two RT' groups can together form a ring;
wherein, optionally, in all of the above embodiments, one or more of RT¹, RT², RT³, RT⁴, RT⁵, RT⁶ and the self-immolative linker L^{C}, is bound to a Construct B C^{B}, and/or S^{P}-C^{B}; wherein S^{P} is a spacer;
wherein Construct B is selected from the group consisting of small molecules, organic molecules, metal coordination compounds, inorganic molecules, organometallic molecules, carbohydrates, peptides, peptoids, lipids, proteins, enzymes, oligonucleotides, DNA, RNA, PNA, LNA, aptamers, hormones, toxins, steroids, cytokines, antibodies, antibody fragments, antibody fusions, polymers, resins, particles, glass particles, liposomes, polymersomes, gels, surfaces, cells, biological tissues, pathogens, and combinations thereof.
More preferably, A^{T} is CH₂. More preferably, B^{T} is CH₂.

X^{C} is O, S, or an optionally substituted nitrogen atom. Preferably, if f is 1, and X^{C} is an optionally substituted nitrogen atom, then L^{C} is not coupled to C(C^{T}) via a nitrogen atom. Preferably, if f is 1, and X^{C} is an O or S, then L^{C} is not coupled to C(C^{T}) via an O or S. Preferably, if f is 0, then X^{C} is O or S.

C^{T} is O or S; preferably O. In Formula (2), f is 0 or 1. L^{C} is a self-immolative linker. Preferably, when f is 1, L^{C} is directly linked to C(O) via a moiety selected from the group consisting of a secondary amine, and a tertiary amine, wherein said moiety is part of L^{C}.

Preferably the group -A^{T}-(B^{T})_{b}-X^{C}-C(C^{T})- is selected from the group consisting of: 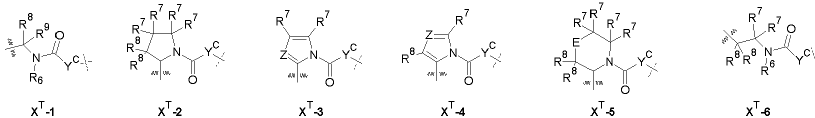 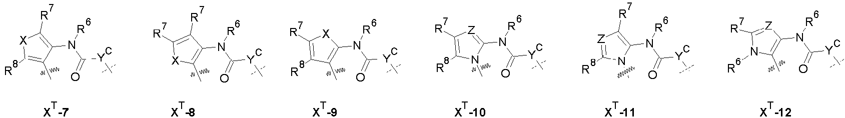 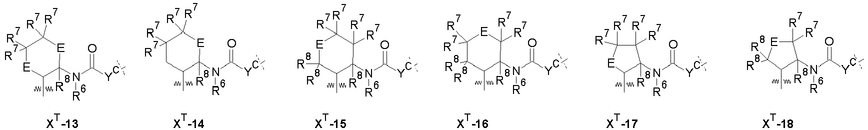 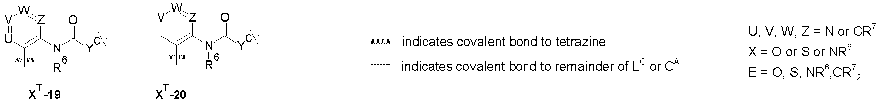 wherein U, V, W, Z are each independently chosen from the group consisting of N and CRT⁷; X is independently chosen from the group consisting of O, S and NRT⁶; E is independently chosen from the group consisting of O, S, NRT⁶ and CRT⁷₂; with RT⁷, RT⁸ and RT⁹ each independently being selected from the group defined above for RT¹, RT², RT³, RT⁵, wherein two RT⁶, RT⁷, RT⁸ and/or RT⁹ can together form a C₆-C₁₂ aryl, or a 3-to 8-membered heterocycle; wherein, optionally one or more of RT⁶, RT⁷, RT⁸, RT⁹, and L^{C} is bound to a C^{B}, and/or S^{P}-C^{B}, wherein preferably the group -A^{T}-(B^{T})_{b}-X^{C}-C(C^{T})- satisfies one of the following structures 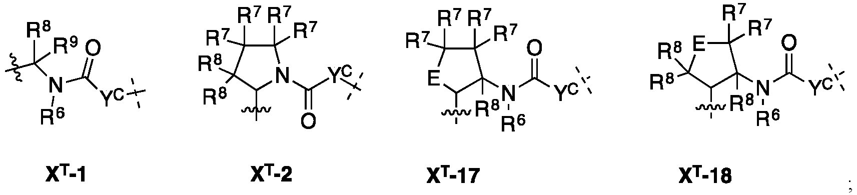 or wherein the group -A^{T}-(B^{T})_{b}-X^{C}-C(C^{T})- satisfies the following structure: 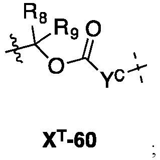 wherein the wiggly line indicates a bond to tetrazine, and the dashed line indicates a bond to the remainder of L^{C} if f is 1, or if is 0 the remainder of the masked IL12 protein or masked IL12 subunit; wherein for X^{T}-1 to X^{T}-20 when f is 1, Y^{C} is O or S, wherein Y^{C} is part of L^{C}, and when f is 0, Y^{C} is bond C^{A}; wherein for X^{T}-60 when f is 1, Y^{C} is O, N, or S, preferably secondary or tertiary N, wherein Y^{C} is part of L^{C}, and when f is 0, Y^{C} is bond C^{A}.

It will be understood that in the schemes showing X^{T}-1 up to and including X^{T}-20, and X^{T}-60, R⁶ refers to RT⁶, R⁷ refers to RT⁷, R⁸ refers to RT⁸, and R⁹ refers to RT⁹.

Preferably, each of RT¹, R T², RT³, RT⁴, R T⁵ RT⁶, RT⁷, RT⁸, and RT⁹ are hydrogen or methyl, more preferably hydrogen.

The dienophile Trigger comprises an eight-membered non-aromatic cyclic mono-alkenylene moiety comprising at least one allylic carbon, preferably two allylic carbons, and optionally comprising one or more heteroatoms. It will be understood that the eight-membered non-aromatic cyclic mono-alkenylene moiety is at least substituted at the at least one allylic carbon, but may comprise further substituents. Similarly, the tetrazine Trigger may also comprise substituents. For any Trigger, these substituents are typically organic molecules or inorganic molecules, more specifically a masking moiety, targeting agent, a Construct B as defined herein, or a radical according to RG1, RG3, RG4, and/or RG5 as described herein. Preferably, the substituent comprises a polysarcosine or polyethylene glycol (PEG) moiety, and more preferably the substituent is polyethylene glycol. Most preferably, the substituent is PEG. The PEG moiety can be linear or branched.

Preferably, the PEG moiety is coupled to a targeting agent as defined herein, optionaly via a spacer S^{P} as defined herein. Preferably, the PEG moiety is at most 5,000 Da, more preferably at most 2,500 Da, more preferably still at most 1,000 Da, most preferably at most 500 Da. Preferably, the PEG moiety has of from 1 to 114 repeating units, more preferably of from 2 to 57 repeating units, even more preferably of from 3 to 30 repeating units, and most preferably of from 4 to 20 repeating units. Preferably, the PEG moiety has at most 24 repeating units, more preferably at most 2 repeating units.

The skilled person is familiar with the fact that the dienophile activity is not necessarily dependent on the presence of all carbon atoms in the ring, since also heterocyclic monoalkenylene eight-membered rings are known to possess dienophile activity. Thus, in general, the invention is not limited to strictly *trans*-cyclooctene. The person skilled in organic chemistry will be aware that other eight-membered ring-based dienophiles exist, which comprise the same endocyclic double bond as the *trans*-cyclooctene, but which may have one or more heteroatoms elsewhere in the ring.

Preferably, for the Trigger the dienophile is a cyclooctene, and more preferably a *trans*-cyclooctene. Preferably, the *trans*-cyclooctene is an all-carbon ring, which may be substituted as defined herein.

In the dienophile Trigger, the at least one allylic carbon is
(a) directly linked to the ε-amine of the lysine residue via a moiety M^{R} selected from the group consisting of -OC(O)-, - OC(S)-, -SC(O)-, and -SC(S), forming a cleavable carbamate, thiocarbamate or dithiocarbamate moiety together with said ε-amine and C^{A}; preferably M^{R} is -OC(O)-; or
(b) directly linked to a first end of a self-immolative linker via a cleavable moiety M^{B} selected from the group consisting of carbamate, thiocarbamate, carbonate, thiocarbonate, ether, ester, amine, amide, thioether, thioester, sulfoxide, and sulfonamide; and a second end of the self-immolative linker is directly linked to the ε-amine of the lysine residue via a moiety M^{R} that is part of the self-immolative linker, forming a carbamate, thiocarbamate or dithiocarbamate moiety together with said ε-amine and C^{A}.

The Trigger moiety as described herein is capable of reacting in an inverse electron-demand Diels-Alder reaction (IEDDA) when it is contacted with a diene, preferably a tetrazine, if the Trigger is a dienophile, or when it is contacted with a dienophile if the Trigger is a tetrazine. After said IEDDA reaction, an electron-cascade-based elimination takes place, termed the "pyridazine elimination", which is a 1,4-elimination reaction. Multiple reaction mechanisms for the combined IEDDA pyridazine elimination reaction are well-known to the skilled person. Upon the IEDDA pyridazine elimination reaction, the ε-amine of the at least one lysine residue of the masked IL12 protein is released, yielding an unmasked and active IL12 protein. Importantly, only groups are released that are attached to the allylic carbon of the eight-membered non-aromatic cyclic mono-alkenylene moiety. Other groups attached to other parts of said mono-alkenylene moiety are not released and do not negatively impact on the IEDDA pyridazine elimination reaction. Various groups, such as amines, thiols, hydroxyl groups, acids, and the like can be released when they are directly attached to the allylic carbon as being part of a moiety selected from the group consisting of carbamate, thiocarbamate, carbonate, thiocarbonate, ether, ester, amine, amide, thioether, thioester, sulfoxide, and sulfonamide. The best results are obtained when said moiety is selected from the group consisting of carbamate, thiocarbamate, carbonate, thiocarbonate, ether, ester, and thioester; most preferably said moiety is a carbamate.

As such, in case (a), the moiety M^{R} is directly linked to the allylic carbon of the dienophile, and the moiety M^{R} and the ε-amine of the lysine residue together form a carbamate, thiocarbamate or dithiocarbamate; preferably a carbamate. Thus, upon contacting the masked IL12 protein of the invention with a diene, the ε-amine of the at least one lysine residue is released in conjunction with the formation of respectively CO₂, COS, or CS₂, and the masked IL12 protein is unmasked.

In case (b), the allylic carbon is coupled to a self-immolative linker, preferably one as described herein. The self-immolative linker is coupled to said allylic carbon via a releasable moiety. In this case, upon reacting the masked IL12 protein of the invention with a diene, the first end of the self-immolative linker is released, which then affects the carbamate, thiocarbamate or dithiocarbamate moiety formed at the other end of the self-immolative linker together with said ε-amine of the at least one lysine residue and C^{A} via an intramolecular reaction or an electron cascade elimination This intramolecular reaction or electron cascade elimination then releases the ε-amine of the at least one lysine residue so as to again yield an unmasked IL12 protein. Various self-immolative linkers are known in the art, and the skilled person is well aware of selecting suitable self-immolative linkers. Non-limiting examples thereof are described herein below.

It will be understood that moiety M^{B} generally takes the form of for example 1-OC(Y^{C2})-y^{C1}-2, 1-OC(Y^{C2})-2, 1-0-2, 1-SC(Y^{C2})-2, or 1-SC(Y^{C2})-Y^{C1}-2, wherein 1 indicates the bond to the allylic carbon, and 2 the bond to the remainder of the self-immolative linker L^{C}; Y^{C1} is selected from the group consisting of -O-, -S-, and -NR₃₆-; Y^{C2} is O or S. R₃₆ is a radical according to Radical Group 1, preferably R₃₆ is hydrogen or methyl. Preferably, M^{B} is selected from the group consisting of I-OC(O)-NR₃₆-2, 1-OC(O)-O-2, 1-OC(O)-S-2, 1-OC(O)-2, and 1-O-2. Importantly, in both cases (a) and (b) no part of the dienophile moiety remains on the IL12 protein after a reaction with the diene, and unmodified lysine residues are obtained.

Similarly, if the Trigger is a tetrazine according to Formula (2), upon reaction with a dienophile, unmodified lysine residues are obtained. The structure of moiety X^{T} in Formula (2) ensures that the ε-amine of the lysine residue is released, with no part of the tetrazine Trigger remaining on the IL12 protein.

Thus, typically a wild-type protein is obtained after reacting the masked IL12 protein with an activator, and activity is fully or almost fully restored. This is surprising, since it was entirely unexpected that small groups on lysine residues can already deactivate IL12 protein, and bare lysine groups need to be obtained to fully reactivate IL12 protein. Moreover, it was surprising that the activity of the IL12 protein can be fully or almost fully restored after unmasking, and that apparently the complex heterodimer protein is not for example damaged by some irreversible change in *e.g.* the tertiary and/or quaternary structure leading to loss of function. At any rate, this is advantageous, as for toxicitiy studies and the like of the product that is released data on wild-type IL12 protein can be used, and no further studies with remnants of *e.g.* a dienophile moiety on the IL12 protein are to be required. This makes the clinical studies using the masked IL12 protein of the invention also more efficient, which is a clear advantage over strategies that do not yield a bare, wild-type IL12 protein.

### Construct B (C^{B})

Preferably, the dienes and dienophiles as disclosed herein, especially Trigger moieties, also contain one or more, preferably at most two, and most preferably one, construct B (C^{B}) as defined herein. C^{B} may be attached to the remainder of the dienophile or diene via a spacer S^{P} as defined herein. In general, each C^{B} is independently an organic molecule or an inorganic molecule. C^{B} may have one or more functions, and may have multiple functions at the same time. C^{B} may act as a masking moiety, a targeting agent, and/or a pharmacokinetics-modulating moiety (*e.g*. a half-life extension moiety). In its latter purpose, C^{B} can for example be used to optimize the aqueous solubility of the dienophile, diene, and/or the entire masked IL12 protein, for example if C^{B} is an amino acid, in particular glycine.

Preferably, each C^{B} is independently selected from the group consisting of organic molecules, and inorganic molecules. More preferably, Construct B is according to RG3 or RG4. Thus, C^{B} is preferably selected from the group consisting of a nucleic acid, a peptide, a protein, a carbohydrate, an aptamer, a hormone, a toxin, a steroid, a cytokine, a lipid, a small organic molecule as defined herein, a polymer, LNA, PNA, an amino acid, a peptoid, a chelating moiety, a molecule comprising a radionuclide, a fluorescent dye, a phosphorescent dye, a drug, a resin, a bead, an organic particle, a gel, an organic surface, an organometallic compound, a cell, an inorganic surface, an inorganic particle, an allotrope of carbon, an inorganic drug, a radionuclide, a lipsomome, a polymersome, a micelle, and combinations thereof.

If C^{B} is part of the Trigger, C^{B} preferably comprises a polymer, and more preferably C^{B} comprises polyethylene glycol (PEG). This is advantageous, as the polymer typically enables further masking of the IL12 protein or subunit thereof. In particular, C^{B} is a PEG moiety coupled to a targeting agent as defined herein, optionally via a spacer S^{P} as defined herein. Preferably, C^{B} comprises or is a PEG moiety of at most 5,000 Da, more preferably at most 2,500 Da, more preferably still at most 1,000 Da, most preferably at most 500 Da. Preferably, C^{B} comprises or is a PEG moiety having of from 1 to 114 repeating units, more preferably of from 2 to 57 repeating units, even more preferably of from 3 to 30 repeating units, and most preferably of from 4 to 20 repeating units. Preferably, C^{B} comprises or is a PEG moiety having at most 24 repeating units, more preferably at most 2 repeating units. The PEG moiety of C^{B} can be linear or branched. Preferably, C^{B} is a polymer, and more preferably C^{B} is polyethylene glycol (PEG).

If C^{B} is part of the Trigger, C^{B} preferably is a Targeting Agent, preferably an antibody fragment or a peptide, linked, optionally via a spacer S^{P}as defined herein, preferably a polymer, more preferably PEG, to the remainder of the Trigger. In this embodiment, it is preferred that the polymer is closest to the eight-membered non-aromatic cyclic mono-alkenylene moiety or the tetrazine moiety, *i.e.* that the polymer acts as a linker between said moiety and the Targeting Agent.

Preferably, especially if C^{B} acts as a targeting agent, C^{B} is a small molecule, a carbohydrate, biotin, peptide, peptoid, lipid, protein, oligonucleotide, DNA, RNA, PNA, LNA, aptamer, hormone, toxin, steroid, cytokine, DARPIN, antibody, antibody fragment (e.g. Fab2, Fab, scFV, diabody, triabodies, VHH), and antibody (fragment) fusions (e.g. bi-specific and trispecific mAb fragments). In other embodiments C^{B} is a drug or an imaging probe such as a fluorscent dye.

Preferably, C^{B} comprises a targeting peptide selected from the group consisting of PPRRGLIKLKTS, FHKHKSPALSPV, [KTVRTSADE-NH₂], and CSSPIQGSWTWENGK[X]WTWGIIRLEQ. Preferably, C^{B} is a targeting peptide selected from the group consisting of PPRRGLIKLKTS, FHKHKSPALSPV, [KTVRTSADE-NH₂], and CSSPIQGSWTWENGK[X]WTWGIIRLEQ. Therein, [KTVRTSADE-NH₂] is a cyclic peptide cyclized via the side chains of the K and E residues. CSSPIQGSWTWENGK[X]WTWGIIRLEQ is a bipodal aptide wherein a group X is attached to the side chain of the K residue. Group X may be a radical according to RG1, RG3, RG4, or RG5 and may contain a further Construct B. Preferably, X comprises an oligoethylene glycol moiety, more preferably PEG₂.

The invention also relates to targeting peptides comprising PPRRGLIKLKTS, FHKHKSPALSPV, [KTVRTSADE-NH₂], and CSSPIQGSWTWENGK[X]WTWGIIRLEQ, wherein the targeting peptide further comprises an aminoxy moiety. Preferably, the targeting peptides are selected from the group consisting of HA-PEG₂-PPRRGLIKLKTS, HA-PEG₂-FHKHKSPALSPV, HA-PEG₂-[KTVRTSADE-NH₂], and CSSPIQGSWTWENGK[PEG₂-HA]WTWGIIRLEQ. Therein, HA denotes hydroxylamine, and PEG denotes polyethylene glycol.

The targeting peptides of the invention can advantageously be used to modify ketones and aldehydes that can be readily incorporated in masked IL12 protein or subunits thereof of the invention. The reaction of the aminoxy moiety with the ketone or aldehyde moiety yiels a stable oxime ether group. This approach makes synthesis easier as compared to other methods. Moreover, without wishing to be bound by theory, the targeting peptides of the invention may result in increased targeting efficiency and higher target vs non-target ratios.

Construct B can also be a radical according to RG1f or a moiety comprising RG1f, as defined herein, wherein RG1f can be used to bind to a further Construct B. For example, Construct B can be RGlf being a maleimide or photocrosslinker that is bound to the remainder of the dienophile (of which the eight-membered non-aromatic cyclic mono-alkenylene moiety may be referred to as T^{R}) via a Spacer S^{P}. The maleimide or photocrosslinker can be used to further conjugate the T^{R} to another Construct-B such as a protein. In this particular embodiment C^{B} is a biomolecule-binding moiety.

In another preferred embodiment, each C^{B} is independently a radical according to RG5 as defined herein. Preferably, each C^{B} is independently a masking moiety as defined herein. Preferably, each C^{B} is independently a targeting agent as defined herein. As noted herein, the masking moiety may also act as a targeting agent and vice versa, so that C^{B} can also have multiple functions.

Preferably, at most three C^{B} are comprised in the dienophile, more preferably at most two, most preferably at most one C^{B} is comprised in the dienophile.

When C^{B} is present in the Trigger, Preferably C^{B} is bound to the remainder of the molecule via a residue of RG1f as defined herein, wherein preferably said residue of RG1f equals or is comprised in a Spacer. A person skilled in the art will understand that "residue of RG1f" means the conjugation reaction product of RG1f with another chemical group so as to form a conjugate between C^{B} with the Trigger, L^{C} or S^{P}.

Preferably, when C^{B} is present in the dienophile, C^{B} is bound to the remainder of the molecule via a radical according to any one of RG2a, RG2b, and RG2c as defined herein, wherein preferably RG2a, RG2b, and RG2c equals or is comprised in a Spacer.

### Dienophiles

First, dienophiles in general will be discussed, which can be used in the invention as an Activator if the Trigger is a tetrazine. Thereafter, specific dienophiles are described that can be used in the invention if the Trigger is a dienophile. It will be understood that herein, dienophiles can be provided as a salt, hydrate, or solvate.

A dienophile as used herein preferably comprises an eight-membered non-aromatic cyclic mono-alkenylene moiety comprising at least one allylic carbon, and optionally comprising one or more heteroatoms, preferably the heteroatom is N, O, or Si. The eight-membered non-aromatic cyclic mono-alkenylene moiety is optionally substituted, preferably with a radical according to RG1. Preferably, the eight-membered non-aromatic cyclic mono-alkenylene moiety is a cyclooctene moiety, more preferably a *trans*-cyclooctene (TCO) moiety. Most preferably, the *trans*-cyclooctene moiety is an all-carbon ring.

Preferably, at least five, more preferably at least six, and most preferably at least seven, members of the eight-membered non-aromatic cyclic mono-alkenylene moiety are not substituted. Preferably, the vinylic carbons are not substituted, *i.e.* are CH. As such, if the dienophile comprises a *trans*-cyclooctene moiety that is an all-carbon ring, then the *trans*-cyclooctene moiety preferably is a ring with one double bond between two CH moieties, *i.e.* -CH=CH-, and the ring further comprises at least at least three, more preferably at least four, and most preferably at least five CH₂ moieties. The substituted members are preferably N or C, more preferably C. If the substituted member is C, then it is preferably CH substituted with a radical according to RG1 not being hydrogen, more preferably -(S^{P})ᵢ-C^{B}.

The dienophile can comprise a payload that is released upon reacting with a diene. The payload is preferably according to RG3 or RG4, more preferably the payload is a drug. It will be understood that if the dienophile is the Trigger, then the payload is the IL12 protein or a subunit thereof.

If the dienophile comprises a payload, then at least one allylic carbon of the eight-membered non-aromatic cyclic mono-alkenylene moiety is directly linked to a cleavable bond. The cleavable bond comprises at least one S, N, NH, or O, and is selected from the group consisting of carbamate, thiocarbamate, carbonate, thiocarbonate, ether, ester, amine, amide, thioether, thioester, sulfoxide, and sulfonamide bonds, such that the atom connecting the cleavable bond to the allylic carbon is not a carbon atom. Preferably, the cleavable bond is selected from the group consisting of carbamate, thiocarbamate, carbonate, thiocarbonate, ether, ester, thioether, and thioester bonds. More preferably, the cleavable bond is selected from the group consisting of carbamate, ether, and ester bonds. Most preferably, the cleavable bond is a carbamate. The at least one S, N, NH, or O of the cleavable bond is part of the payload, or part of an optional spacer between the cleavable bond and the payload. Preferably, the optional spacer is a self-immolative linker, preferably as described herein.

It will be understood that if the Trigger is a dienophile, the eight-membered non-aromatic cyclic mono-alkenylene moiety is at least substituted at the at least one allylic carbon, but may comprise further substituents. These substituents are typically organic molecules or inorganic molecules, more specifically a masking moiety, targeting agent, a Construct B as defined herein, or a radical according to RG5 as described herein. Preferably, the substituent comprises a polysarcosine or polyethylene glycol (PEG) moiety, and more preferably the substituent is polyethylene glycol. Most preferably, the substituent is PEG.

Without wishing to be bound by theory, it is believed that optionally present other substituents on the eight-membered non-aromatic cyclic mono-alkenylene moiety do not qualitatively influence the release of the payload upon reaction with a diene. In other words, the payload will be released upon reaction with a diene, regardless of whether other substituents are present on the eight-membered non-aromatic cyclic mono-alkenylene moiety. Several mechanisms for the release of the payload are known in the art. These are for example described in WO 2020/256546, in particular in Scheme 2 on page 39.

The dienophiles for use in the invention can be synthesized by the skilled person, on the basis of known synthesis routes to cyclooctenes and corresponding hetero atom(s)-containing rings. The skilled person further is aware of the wealth of cyclooctene derivatives that can be synthesized via the ring closing metathesis reaction using Grubbs catalysts. As mentioned above, the TCO possibly includes one or more heteroatoms in the ring. This is as such sufficiently accessible to the skilled person [e.g. WO2016025480]. Reference is made, e.g., to the presence of a thioether in TCO: [Cere et al. J. Org. Chem. 1980, 45, 261]. Also, e.g., an - O-SiR₂-O moiety in TCO: [Prevost et al. J. Am. Chem. Soc. 2009, 131, 14182]. References to TCO syntheses wherein the allylic positioned leaving group (R₄₈) is an ether, ester, carbonate, carbamate or a thiocarbamate are: [Versteegen et al Angew. Chem. Int. Ed. 2018, 57, 10494], and [Steiger et al Chem Comm 2017, 53, 1378].

It should be noted that, depending on the choice of nomenclature, the TCO dienophile may also be denoted E-cyclooctene. With reference to the conventional nomenclature, it will be understood that, as a result of substitution on the cyclooctene ring, depending on the location and molecular weight of the substituent, the same cyclooctene isomer may formally become denoted as a Z-isomer. In the present invention, any substituted variants of the invention, whether or not formally "E" or "Z," or "cis" or "trans" isomers, will be considered derivatives of unsubstituted trans-cyclooctene, or unsubstituted E-cyclooctene. The terms "trans-cyclooctene" (TCO) as well as E-cyclooctene are used interchangeably and are maintained for all dienophiles according to the present invention, also in the event that substituents would formally require the opposite nomenclature. I.e., the invention preferably relates to cyclooctene in which carbon atoms 1 and 6 as numbered below in Formula (3a) are in the E *(entgegen)* or *trans* position.

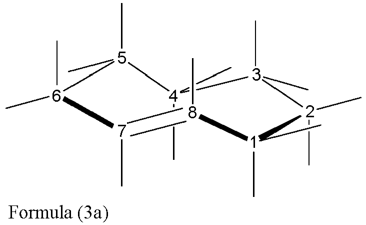

Preferably, the dienophile is according to Formula (3b):

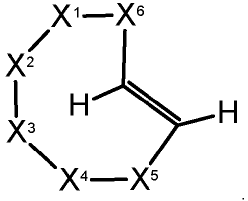

In Formula (3b), X¹, X², X³, and X⁴ together form an optionally substituted four-membered aliphatic or heteroaliphatic moiety. Preferably, each of X¹, X², X³, and X⁴ is independently selected from the group consisting of C(R₄₇)₂, C=C(R₄₇)₂, C=O, C=S, C=NR₃₇, S, SO, SO₂, O, NR₃₇, and Si(R₄₇)₂, with at most three of Y, Z, X, and Q being selected from the group consisting of C=C(R₄₇)₂, C=O, C=S, and C=NR₃₇, wherein two R moieties together may form a ring, and with the proviso that no adjacent pairs of atoms are present selected from the group consisting of O-O, O-NR₃₇, S-NR₃₇, O-S, O-S(O), O-S(O)₂, and S-S, and such that Si is only adjacent to C(R₄₇)₂ or O. More preferably, each of X¹, X², X³, and X⁴ is independently selected from the group consisting of -C(R₄₇)₂-, -NR₃₇-, -C(O)-, and -O-, such that at most two, more preferably at most one, of X¹, X², X³, X⁴ are not -C(R₄₇)₂-, and with the proviso that no sets consisting of adjacent atoms are present selected from the group consisting of -O-O-, -O-N-, -C(O)-O-, N-N-, and -C(O)-C(O)-.

Preferably, X¹, X², X³, and X⁴ are all -C(R₄₇)₂- and at most 3, more preferably at most 2, at most preferably at most one, of R₄₇ are not H. Preferably, at most three R₄₇ in Formula (3b) are not H. Preferably, all X in Formula (3b) are - C(R₄₇)₂- if the dienophile is an Activator.

In Formula (3b), X⁵ and X⁶ are optionally substituted carbons.

Preferably, X⁵ is -C(R₄₇)₂-, and more preferably, X⁵ is -CHR₄₇, and most preferably X⁵ is -CH₂. In some embodiments, X⁵ is -CHR₄₉. In relation to Formula (3b), R₄₉ is selected from the group consisting of -M^{R}-C^{B}, and -M^{B}-L^{C}-C^{B}, wherein C^{B} is preferably a drug. As X⁵ is an allylic carbon in the dienophile moiety of Formula (3b), the moiety C^{B} attached thereto via -M^{R}- or -M^{B}-L^{C}- will be released upon the reaction between the dienophile moiety and the activator. This can be especially advantageous if the dienophile is the Trigger, and X⁶ is connected to the IL12 protein or subunit thereof. Then, upon unmasking the masked IL 12 protein or subunit thereof, preferably at the target site, C^{B}, which is preferably a drug, is released as well. This may greatly enhance the efficacy of the treatment.

Preferably, X⁶ is -C(R₄₇)₂-, and more preferably, X⁶ is -CHR₄₇. If the Trigger in Formula (1) is a tetrazine, then X⁶ is most preferably -CH₂. If the Trigger of Formula (1) is a dienophile, however, X⁶ is -CHR₄₈. In particularly favorable embodiments, R₄₈ is in the axial position. This further improves the kinetics of the payload release.

R₄₈ is -M^{R}-C^{A} or -M^{B}-L^{C}-C^{A}. L^{C} is a self-immolative linker, preferably as described herein, directly linked via a group M^{R}, that is part of L^{C}, and the bond C^{A} to the ε-amine of the at least one lysine residue of Formula (1). Optionally, L^{C} is linked to one or more groups -(S^{P})ᵢ-C^{B} and/or one or more groups -(S^{P})ᵢ-D^{D} with i being an integer in a range of from 0 to 4, preferably i is 1. D^{D} is a drug. If the optional at least one C^{B} is present, it is linked, directly or via a spacer S^{P}, to a moiety selected from the group consisting of X¹, X², X³, X⁴, X⁵, and L^{C}. It will be understood that if C^{B} is linked, directly or via a spacer S^{P}, to a moiety selected from the group consisting of X¹, X², X³, X⁴, and X⁵, C^{B} or -S^{P}-C^{B} is considered to be R₄₇.

In relation to Formula (3b), each of R₄₇ and R₃₇ are independently selected from the group consisting of hydrogen, organic molecules, and inorganic molecules. Preferably, each R₄₇ is independently selected from a group according to RG1, RG3, RG4, or RG5, more preferably RG1. Preferably, as used herein throughout this application, each R₃₇ is independently selected from a group according to RG1, RG3, RG4, or RG5, more preferably RG1.

Preferably, in relation to Formula (3b) each C^{B} is a radical according to RG3 or RG4, more preferably RG3.

Preferably, X¹, X², X⁴, and X⁵ are all -CH₂-, and X³ is -C(R₄₇)₂-, wherein preferably for X³ one R₄₇ is hydrogen, OH, or methyl, and the other R₄₇ is -(S^{P})ᵢ-C^{B}. Preferably, therein i is 1; preferably S^{P} is a radical according to RG2a, more preferably -C(O)NH-; and C^{B} is a polymer, preferably polyethylene glycol, optionally coupled to an antibody, diabody, or antibody fragment.

In some embodiments, two R₄₇ and/or R₃₇ are comprised in a ring so as to form a ring fused to the eight-membered non-aromatic cyclic mono-alkenylene moiety. It is preferred that these rings fused to the eight-membered trans-ring are C₃-C₇ cycloalkylene groups and C₄-C₇ cycloalkenylene groups, optionally substituted and containing heteroatoms as described for R₄₇. Preferably, when in Formula (3b) two R₃₇, and/or R₄₇ groups are comprised in a ring so as to form a ring fused to the eight-membered trans-ring, this ring fused to the eight-membered trans-ring is as defined in WO 2012/156919 A1. More preferably the ring fused to the eight-membered trans-ring is as defined in WO 2012/156919 A1 on page 15, line 25 to page 18, line 9.

Optionally, the dienophile comprises at least two exocyclic bonds fixed in substantially the same plane, and/or it optionally comprises at least one substituent in the axial position, and not the equatorial position. The person skilled in organic chemistry will understand that the term "fixed in substantially the same plane" refers to bonding theory according to which bonds are normally considered to be fixed in the same plane. Typical examples of such fixations in the same plane include double bonds and strained fused rings. E.g., the at least two exocyclic bonds can be the two bonds of a double bond to an oxygen (i.e. C=O). The at least two exocyclic bonds can also be single bonds on two adjacent carbon atoms, provided that these bonds together are part of a fused ring (i.e. fused to the TCO ring) that assumes a substantially flat structure, therewith fixing said two single bonds in substantially one and the same plane. Examples of the latter include strained rings such as cyclopropyl and cyclobutyl. Without wishing to be bound by theory, the inventors believe that the presence of at least two exocyclic bonds in the same plane will result in an at least partial flattening of the TCO ring, which can lead to higher reactivity.

It is preferred that the at least two exocyclic bonds fixed in the same plane are selected from the group consisting of (a) the single bonds of a fused cyclobutyl ring, (b) the hybridized bonds of a fused aromatic ring, (c) an exocyclic double bond to an oxygen, (d) an exocyclic double bond to a carbon, (e) the single bonds of a fused dioxalane ring, (f) the single bonds of a fused cyclopropyl ring.

Preferred dienophiles according to this invention are the racemic and enantiomerically pure compounds listed below: 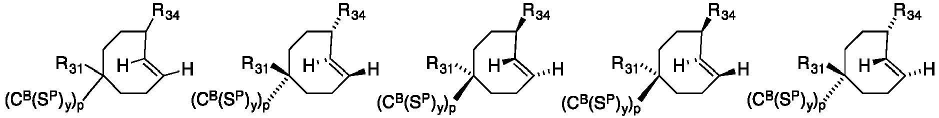 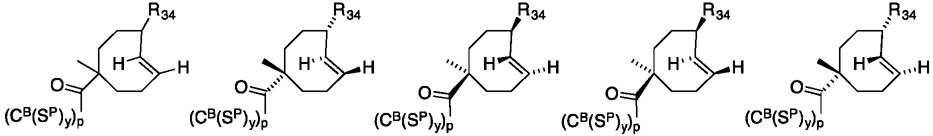 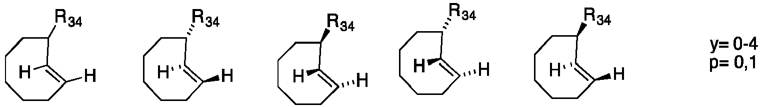 Herein, R₃₁ is a radical according to RG1. Preferably, R₃₁ is selected from the group consisting of hydrogen, C₁-C₆ (hetero)alkyl, C₆ aryl, C₄-C₅ heteroaryl, C₃-C₆ (hetero)cycloalkyl, and combinations thereof, which insofar not being H are optionally substituted with a moiety selected from the group consisting of -Cl, -F, -Br, -I, NO₂, SO₃H, PO₃H, -PO₄H₂, -OR', -N(R')₂, -CF₃, =O, =NR', and -SR'. R' is a radical according to RG1, preferably R' is hydrogen or methyl, most preferably R' is hydrogen. Preferably, R₃₁ is hydrogen, methyl, or hydroxyl. In some embodiments, R₃₁ is -CH₃. In some embodiments, R₃₁ is OH. In some embodiments, R₃₁ is H.

Especially preferred TCO compounds according to this invention are the enantiomerically pure compounds listed below: 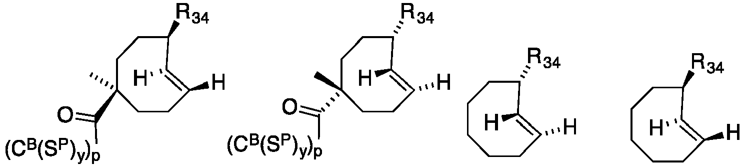 R₃₄ is identical to R₄₇ or R₄₈ as described herein.
Other preferred TCO compounds are: 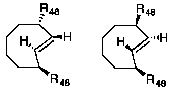 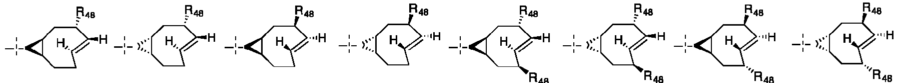 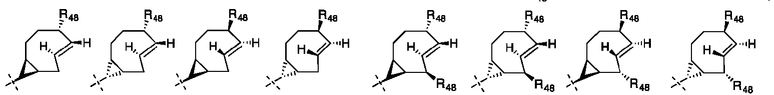 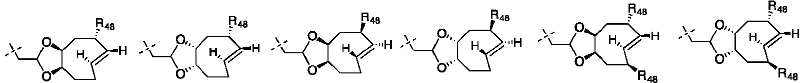 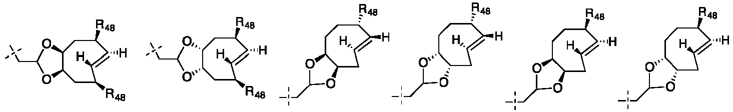 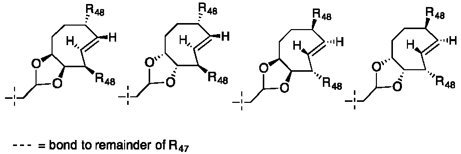 wherein if the dienophile comprises one R₄₈ moiety, this is as defined herein for R₄₈ in relation to Formula (3b); and if the dienophile comprises two R₄₈ moieties, one is as defined herein for R₄₈ in relation to Formula (3b) and the other R₄₈ is as defined herein for R₄₇ or R₄₉ in relation to Formula (3b).

In some embodiments, the dienophile is as defined in WO 2012/156918 A1, in particular as in any one of claims 5-7 thereof. In some embodiments, the dienophile is as defined in WO 2012/156919 A1, in particular as in any one of claims 1-4 thereof. In some embodiments, the dienophile is as defined in WO 2012/156920 A1, in particular as in any one of claims 1-6 thereof. In some embodiments, the dienophile is as defined in WO 2014/081303 A1, in particular as in any one of claims 1-9 thereof. In some embodiments, the dienophile is as defined in WO 2019/212357 A1, in particular as in any one of claims 1-3 thereof. In some embodiments, the dienophile is as defined in WO 2020/256544 A1, in particular as in Section 9 thereof. In some embodiments, the dienophile is as defined in WO 2020/256546 A1, in particular as in any one of claims 1-4 thereof. In some embodiments, the dienophile is as defined in WO 2020/256545 A1, in particular as in any one of claims 1-5 thereof. It will be understood if the dienophiles disclosed therein are used in the present invention, the releasable construct, such as a Construct A, as mentioned in said references should be interpreted as the at least one lysine residue of the IL12 protein as described herein, or the bond C^{A} as described herein, whichever is more appropriate.

The following structures are non limiting examples of suitable dienophiles, wherein it will be understood that the wiggly lines represent the bond C^{A} or the remainder of L^{C}-C^{A}, wherein optionally -C^{B} or -S^{P}-C^{B} is coupled to L^{C} 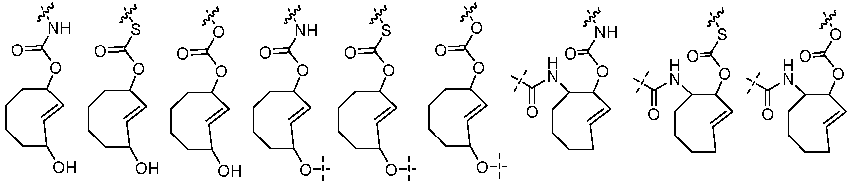 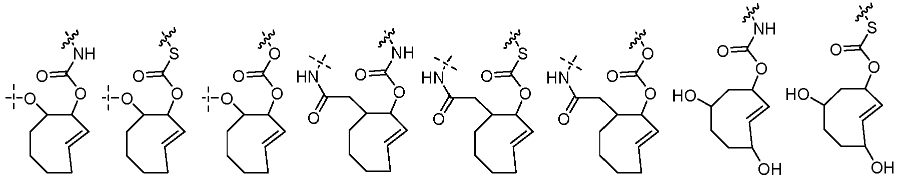 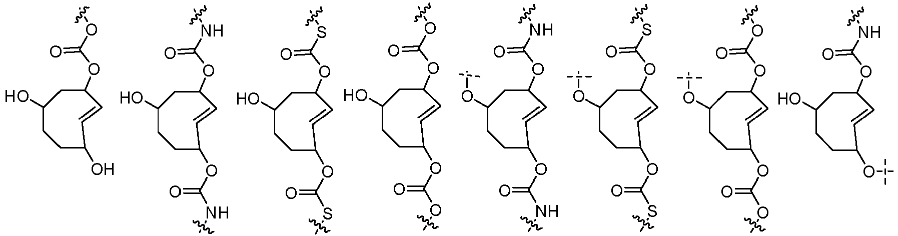 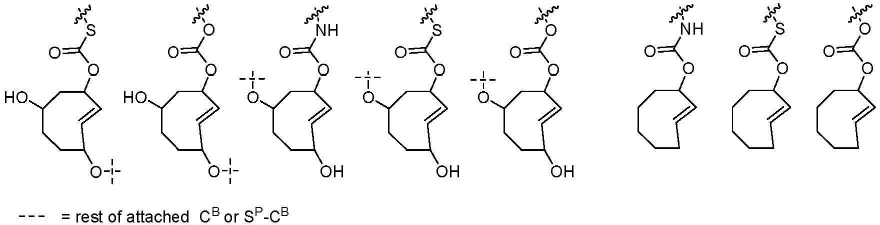 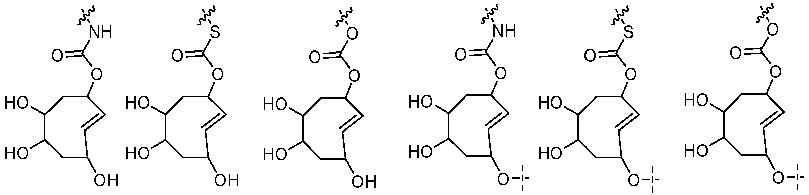 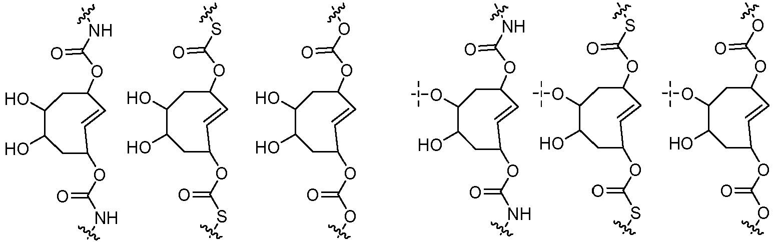 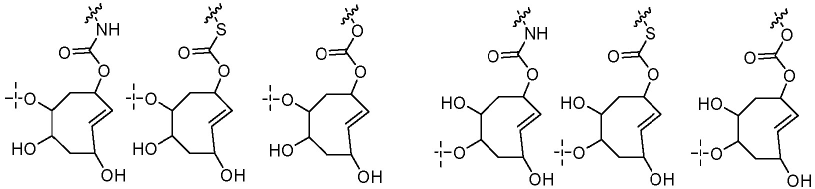 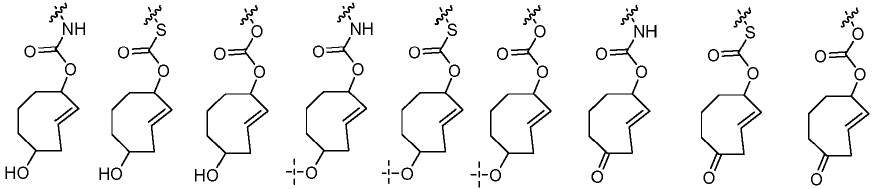 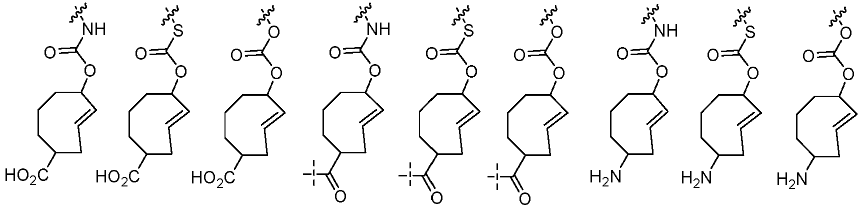 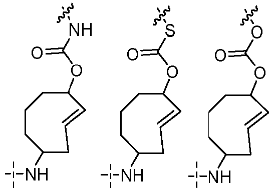 --- = rest of attached C^{B} or S^{P}-C^{B} 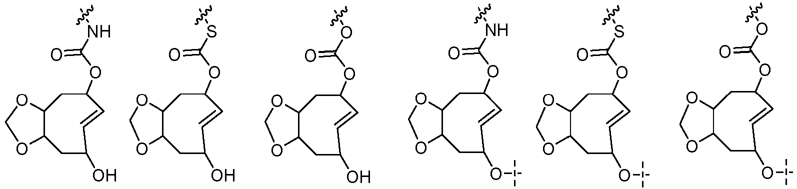 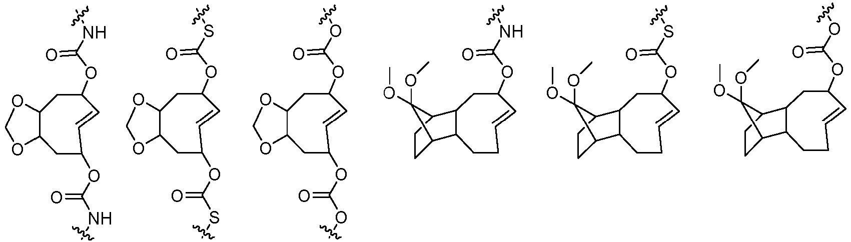 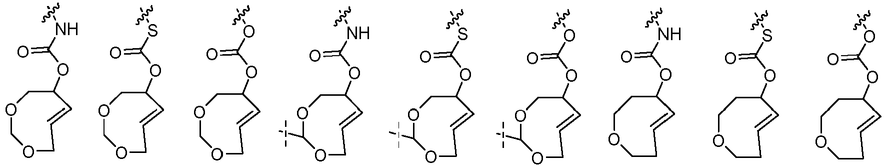 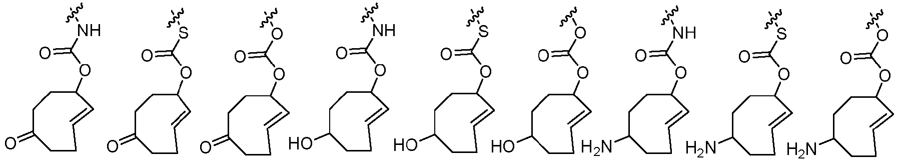 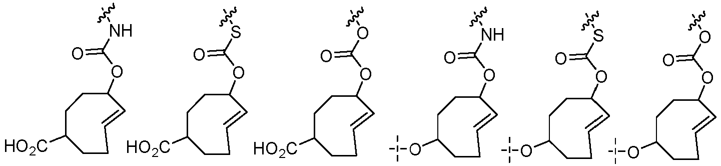 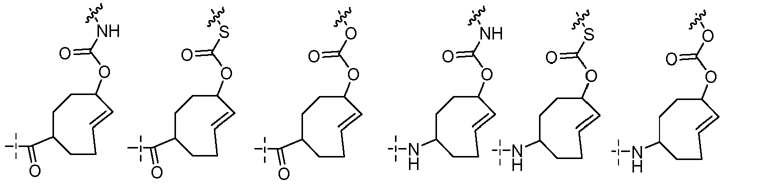 --- = rest of attached C^{B} or S^{P}-C^{B}

Preferably, the dienophile satisfies Formula (3c), which is a preferred embodiment of the dienophile according to Formula (3b): 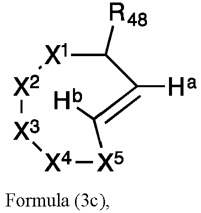
wherein R₄₈ is as defined herein in relation to Formula (3c); wherein at least one of conditions (a)-(c) is met:
   (a) at least one of X¹ , X², X³, X⁴, X⁵, is CR₄₇Y^{T1}; and Y^{T1} is positioned cis relative to H^{a};
   (b) X³ is Y^{T3}; and
   (c) two adjacent X moieties selected from X¹, X², X³, X⁴, X³, are part of a fused ring satisfying one of the Formulae (3c1)-(3c7): 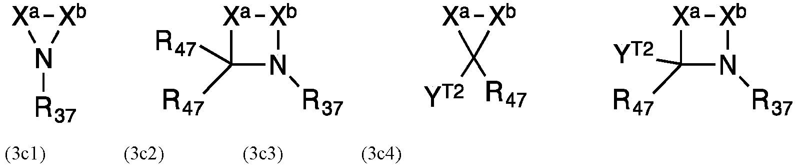 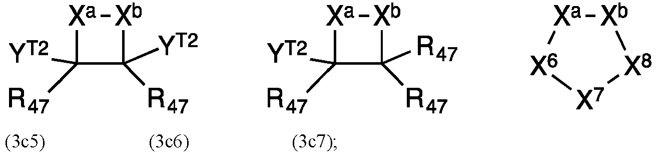
Y^{T2} is positioned *syn* relative to the 8-membered dienophile ring;
wherein X^{a} and X^{b} are part of the 8-membered ring of Formula (3c), such that X^{a}-X^{b} or X^{b}-X^{a} is X¹-X², X²-X³, X³-X⁴, or X⁴-X⁵;
for Formulae (3c1)-(3c3), X^{a} and X^{b} are CR₄₇, preferably CH;
for Formulae (3c4)-(3c7), X^{a} and X^{b} are independently CR₄₇ or N, preferably CR₄₇, more preferably CH;
X⁶ and X⁸ are each independently selected from the group consisting of Y^{T3}, C(R₄₇)Y^{T2}, C(R₄₇)₂, O, S, C(O), C(S), and S(O)₂;
X⁷ is selected from the group consisting of Y^{T3} and Z^{T};
when X⁶ is Y^{T3}, then X⁷ is Z^{T};
when X⁷ is Y^{T3}, then X⁶ and X⁸ are each independently selected from the group consisting of C(R₄₇)Y^{T2}, C(R₄₇)₂, O, and S; preferably C(R₄₇)Y^{T2} or C(R₄₇)₂;
wherein the fused ring satisfying Formula (3c7) comprises at least one Y^{T2} or Y^{T3} moiety;
for Formulae (3c2) and (3c6), two R₄₇ directly connected to the same carbon may together be =C-(R₄₇)₂, =S, or =O; with the proviso that X¹-X², X²-X³, X³-X⁴, and X⁴-X⁵ are not -O-O-, -N-N-, -O-N- or -N-O-;
the direct bonds from X^{a} and X^{b} to the remainder of the ring fused to the 8-membered dienophile ring of Formulae (3c1)-(3c7) are cis relative to one another, and cis relative to H^{a};
preferably no adjacent pairs of atoms being -O-O- are part of the fused rings of Formulae (3c1)-(3c7);
Y^{T1} is selected from the group consisting of OH, SH, N(R₃₈)₂, C(O)OH, C(S)OH, C(O)SH, C(S)SH, O-N(R₃₈)₂, SO₄H, SO₃H, SO₂H, PO₄H₂, PO₃H, PO₂H, and C(N)N(R₃₈)₂,
Y^{T2} is selected from the group consisting of OH, SH, and N(R₃₈)₂,
Y^{T3} is NR₃₈ and is not flanked by C(O), C(S), S(O), or S(O)₂;
the remaining X¹, X², X³, X⁴, X³, are independently selected from the group consisting of C(R₄₇)₂ and O, such that at most two of X¹, X², X³, X⁴, X⁵ are O, NR₃₈ or N;
wherein each R₃₇ and R₃₆ are independently as defined herein;
wherein R₃₈ is as defined for R₃₇ and R₃₆, with the proviso that R₃₈ is not attached to the remainder of the molecule via C(O), C(S), S(O), or S(O)₂,
wherein each R₄₇ is independently as defined herein,
wherein two R₃₇, R₃₈, R₄₇ groups are optionally comprised in a ring,
wherein two R₃₇, R₃₈, R₄₇ groups are optionally comprised in a ring so as to form a ring fused to the eight-membered trans-ring.

For Formula (3c), Z^{T} is according to RG1, preferably according to RG1b, RG1c, RG1d, or RG1e. Preferably, the Z^{T} groups are not substituted. Preferably, the Z^{T} groups do not contain heteroatoms.

Preferably, in Formula (3c) only condition (a) is met. Preferably, in Formula (3c) only condition (b) is met. Preferably, in Formula (3c) only condition (c) is met. Preferably, when in Formula (3c) two R₃₇, R₃₈, R₄₇ groups are optionally comprised in a ring so as to form a ring fused to the eight-membered trans-ring, this ring fused to the eight-membered trans-ring is as defined in WO 2012/156919 A1. More preferably the ring fused to the eight-membered trans-ring is as defined in WO 2012/156919 A1 on page 15, line 25 to page 18, line 9.

Preferred dienophiles according to Formula (3c) are selected from the group consisting of 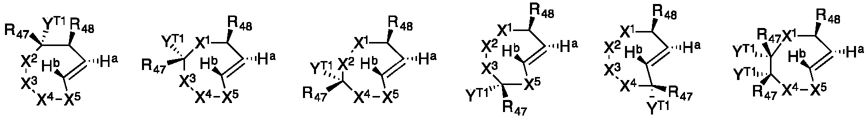 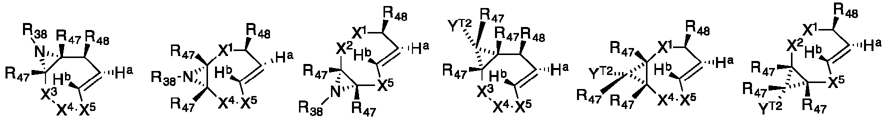 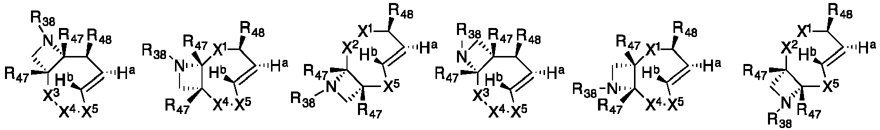 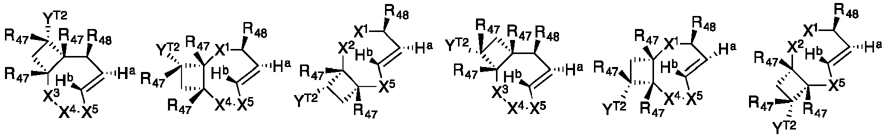 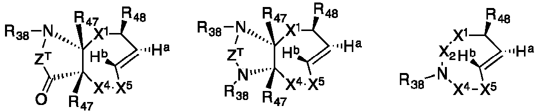 and enantiomers thereof in line with Formula (3c).

Other preferred dienophiles according to Formula (3c) are selected from the group consisting of:

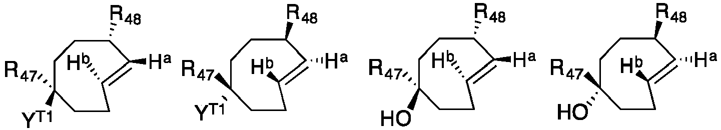

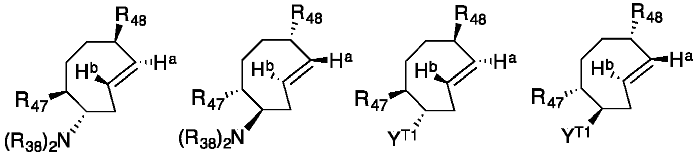

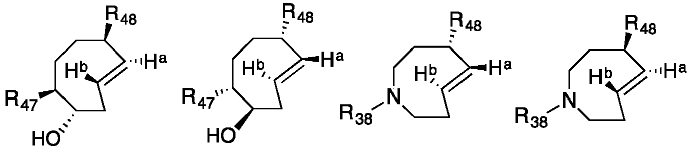

Further preferred dienophiles according to Formula (3c) are selected from the group consisting of 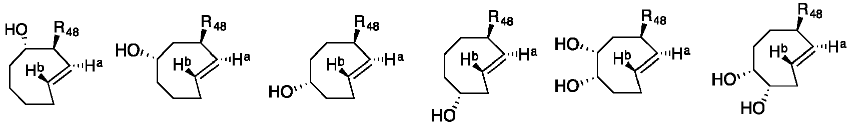 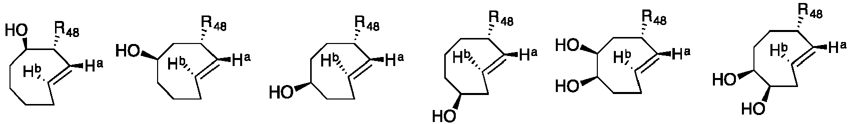 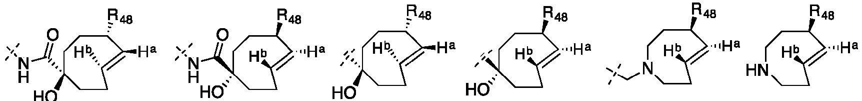 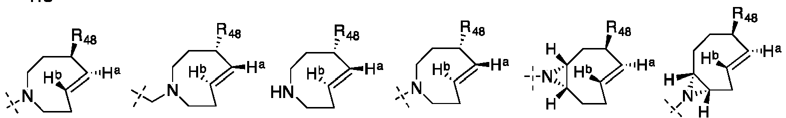 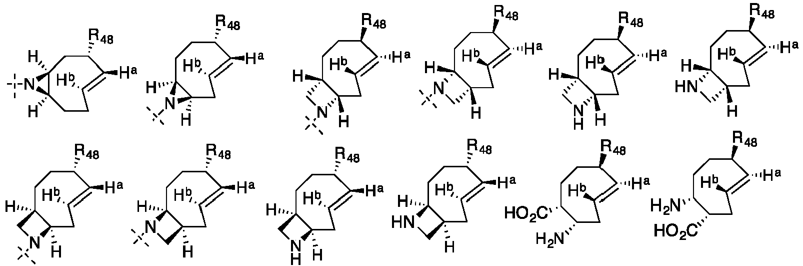 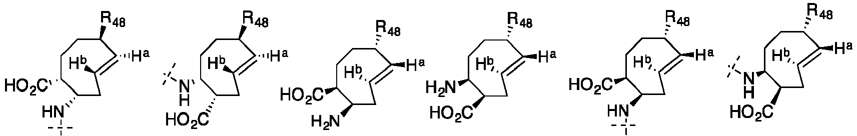 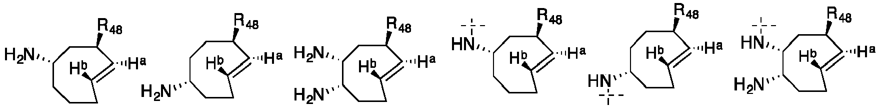 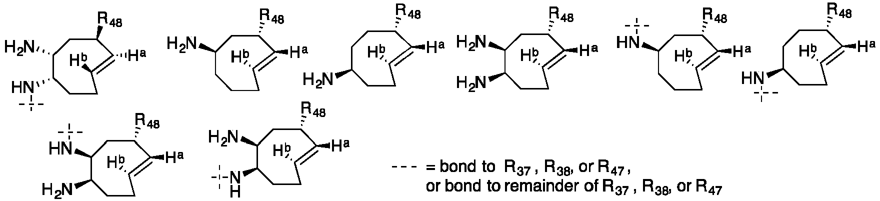 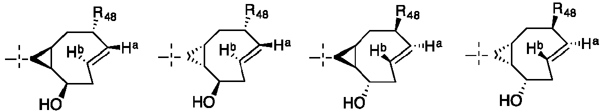 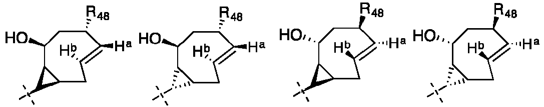 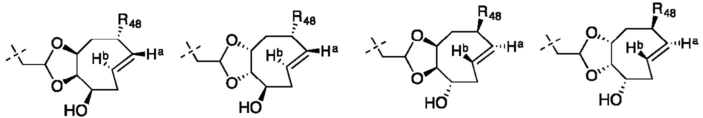 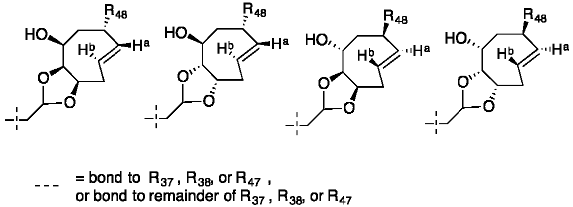 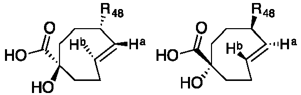 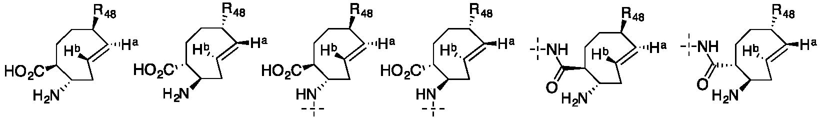 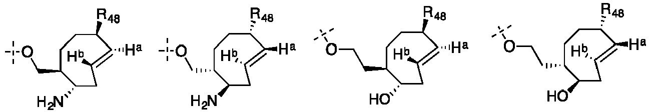 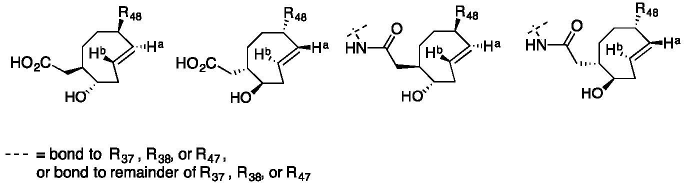 Especially preferred dienophiles of the invention are: 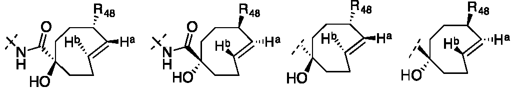 wherein the dashed line indicates a bond to R₃₇, R₃₈, or R₄₇; or a bond to the remainder of R₃₇, R₃₈, or R₄₇.

Preferably, Y^{T1} is OH, N(R₃₈)₂, C(O)OH, O-N(R₃₈)₂, SH, more preferably OH, N(R₃₈)₂, O-N(R₃₈)₂, more preferably OH, N(R₃₈)₂, most preferably Y^{T1} is OH. In other preferred embodiments, Y^{T1} is N(R₃₈)₂, more preferably NR₃₈H, most preferably NH₂. Preferably, no Y^{T2} and Y^{T3} are present and Y^{T1} is OH, N(R₃₈)₂, C(O)OH, O-N(R₃₈)₂, more preferably OH, N(R₃₈)₂, most preferably Y^{T1} is OH. Preferably, Y^{T2} is OH. Preferably, no Y^{T1} and Y^{T3} are present and Y^{T2} is OH. It is preferred that X¹ and X⁵ are not O.

Preferably, when a fused ring is present satisfying any of the Formulae (3c1)-(3c7) that there are no other rings fused to the 8-membered dienophile ring.

Preferably, R₃₇, R₃₈, R₄₇ groups are not OH, SH, N(R₃₈)₂, O-N(R₃₈)₂, C(N)N(R₃₈)₂. Preferably, R₃₇, R₃₈, R₄₇ groups are not OH, SH, N(R₃₈)₂, C(O)OH, C(S)OH, C(O)SH, C(S)SH, O-N(R₃₈)₂, SO₄H, SO₃H, SO₂H, PO₄H₂, PO₃H, PO₂H, C(N)N(R₃₈)₂.

Preferably, the compound of Formula (3c) comprises at most three moieties each independently selected from the group consisting of Y^{T1}, Y^{T2}, and Y^{T3}, more preferably at most two moieties, even more preferably one moiety.

Preferably, one of X², X³, X⁴ is CR₄₇Y^{T1}, most preferably X³ or X⁴ is CR₄₇Y^{T1}. Preferably, one of X², X³, X⁴ is CR₄₇Y^{T1}, most preferably X³ or X⁴ is CR₄₇Y^{T1}, and the remaining X¹, X², X³, X⁴, X⁵ are C(R₄₇)₂, preferably CH₂. Preferably, two of X², X³, X⁴ are CR₄₇Y^{T1}, and the remaining X¹, X², X³, X⁴, X⁵ are C(R₄₇)₂, preferably CH₂. Preferably, X³ is Y^{T3}, and the remaining X¹, X², X³, X⁴, X⁵ are C(R₄₇)₂, preferably CH₂. Preferably, X² and X³ are part of a fused ring satisfying one of the Formulae (3c1)-(3c7), and the remaining X¹, X², X³, X⁴, X⁵ are C(R₄₇)₂, preferably CH₂.

Preferably, X³ and X⁴ are part of a fused ring satisfying one of the Formulae (3c1)-(3c7), and the remaining X¹, X², X³, X⁴, X⁵ are C(R₄₇)₂, preferably CH₂.

Preferably, the fused ring satisfies Formula (3c1). Preferably, the fused ring satisfies Formula (3c2). Preferably, the fused ring satisfies Formula (3c3).

For Formula (3c7) it is preferred that when X⁶ and X⁸ are both Y^{T3} that X⁷ is not C₁ alkylene. For Formula (3c7) it is preferred that X⁶ and X⁸ are both Y^{T3}, preferably NR₃₈, and that X⁷ is C₂ alkylene. For Formula (3c7) it is preferred that X⁶ and X⁸ are both C(R₄₇)₂, preferably CH₂, and X⁷ is Y^{T3}, preferably NR₃₈.
Preferably, X^{a} and X^{b} are CH. Preferably, the fused ring satisfies Formula (3c1), and that X^{a} and X^{b} are CH.

Preferably, at most 4 of R₃₇, R₃₈, R₄₇ comprised in X¹, X², X³, X⁴, and X⁵ (*i.e.* in total for X¹-X⁵, not per moiety X) are not H, preferably at most 3 are not H, more preferably at most 2 are not H, most preferably at most 1 is not H.

It is preferred that when two R₃₇, R₃₈, R₄₇ groups comprised in X¹, X², X³, X⁴, X⁵ are comprised in a ring so as to form a ring fused to the eight-membered trans-ring, that these rings fused to the eight-membered trans-ring are C₃-C₇ cycloalkylene groups and C₄-C₇ cycloalkenylene groups, optionally substituted and containing heteroatoms as described for R₄₇.

### Dienes

It will be understood that all dienes herein, either acting as Trigger, Activator, or otherwise, can be provided as a salt, hydrate, or solvate.

If the Trigger is a tetrazine, it is one according to Formula (2) as described herein. If the Trigger in relation to the invention is a dienophile, then the Activator can be any diene. In particular, then the Activator comprises a diene, preferably a tetrazine. The diene typically reacts with a dienophile. Preferably, the Activator is a tetrazine. Synthesis routes to tetrazines in general are readily available to the skilled person, based on standard knowledge in the art. References to tetrazine synthesis routes include for example Lions et al, J. Org. Chem., 1965, 30, 318-319; Horwitz et al, J. Am. Chem. Soc., 1958, 80, 3155-3159; Hapiot et al, New J. Chem., 2004, 28, 387-392, Kaim et al, Z. Naturforsch., 1995, 50b, 123-127; Yang et al., Angew. Chem. 2012, 124, 5312 -5315; Mao et al., Angew. Chem. Int. Ed. 2019, 58, 1106-1109; Qu et al. Angew. Chem. Int. Ed. 2018, 57, 12057 -12061; Selvaraj et al., Tetrahedron Lett. 2014, 55, 4795-4797; Fan et al., Angew. Chem. Int. Ed. 2016, 55, 14046-14050.

Preferably, the diene is a tetrazine satisfying Formula (4) or a salt, solvate, or hydrate thereof: 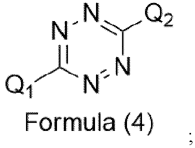 wherein each moiety Q₁ and Q₂ is independently selected from the group consisting of hydrogen, organic molecules, and inorganic molecules; and preferably at least one of moieties Q₁ and Q₂ is not hydrogen. Preferably, each moiety Q₁ and Q₂ is independently selected from RG1, RG3, RG4, and RG5.

Preferably, the tetrazine is symmetrical, *i.e.* Q₂ equals Q₁. This is advantageous, as it typically simplifies the synthesis of the tetrazine.

Preferably, in Formula (4) Q₁ and Q₂ are selected from the group of hydrogen, (cyclo)alkyl, (cyclo)alkenyl, (cyclo)alkynyl, hetero(cyclo)alkyl, hetero(cyclo)alkenyl, hetero(cyclo)alkynyl, (hetero)aryl, phenyl, 2-pyridyl, 3-pyridyl, 4-pyridyl, 2,6-pyrimidyl, 2,5-pyrimidyl, 3,5-pyrmidyl, 2,4-pyrimidyl, and linear or cyclic vinyl ethers; and Q₁ and Q₂ not being hydrogen are optionally substituted, preferably with one or more moieties according to RG1 not being hydrogen. Preferably, each individual Q₁ and Q₂ group comprises at most 4 substituents, more preferably at most 3 substituents, even more preferably at most 2 substituents, and most preferably at most 1 substituent.

In some embodiments, the diene is a multimeric compound, comprising a plurality of tetrazines. These multimeric compounds can be peptide, peptoid, protein, oligonucleotide, oligosaccharide, polymersome, biomolecules, polymers, dendrimers, liposomes, micelles, particles, nanoparticles, microparticles, polymer particles, or other polymeric constructs. If the diene is a multimeric compound, it is preferred that it comprises a tetrazine coupled, optionally via a spacer, to a polymer, more preferably hyaluronic acid.

In Formula (4), the Q₁ and Q₂ are optionally bound to a moiety according to RG3 or RG4, preferably RG3, more preferably a polymer or a protein.

In some embodiments, the Q₁ and Q₂ not being hydrogen are not substituted.

Preferably, in Formula (4):
(a) Q₁ and Q₂ are independently selected from the group consisting of 2-pyridyl, 3-pyridyl, and 4-pyridyl;
(b) Q₁ is selected from the group consisting of 2,6-pyrimidyl, 2,5-pyrimidyl, 3,5-pyrmidyl, and 2,4-pyrimidyl; and Q₂ is (hetero)alkyl,
(c) Q₁ is phenyl and Q₂ is hydrogen;
(d) Q₁ is phenyl and Q₂ is phenyl;
(e) Q₁ is phenyl and Q₂ is C₁-C₈ (hetero)alkyl,
(f) Q₁ and Q₂ are C₁-C₈ (hetero)alkyl;
(g) Q₁ and Q₂ are C₃-C₈ (cyclo)alkenyl;
(h) Q₁ and Q₂ are vinyl ether, preferably cyclic vinyl ether;(i) Q₁ is selected from the group consisting of 2,6-pyrimidyl, 2,5-pyrimidyl, 3,5-pyrmidyl, and 2,4-pyrimidyl; and Q₂ is RG1, RG3, RG4, or RG5, optionally via RG2;(j) Q₁ is selected from the group consisting of 2-pyridyl, 3-pyridyl, and 4-pyridyl and Q2 is RG5;(k) Q₁ is (hetero)alkyl and Q2 is RG1, RG3, RG4, or RG5 optionally via RG2;(l) Q₁ is (hetero)aryl and Q2 is RG1, RG3, RG4, or RG5 optionally via RG2;(m) Q₁ is vinyl ether and Q2 is RG5 optionally via RG2;
and in (a)-(m) all Q₁ and Q₂ not being hydrogen are optionally substituted by a group according to RG1 not being hydrogen, RG3 not being hydrogen, RG4 not being hydrogen, and/or RG5 not being hydrogen, wherein the substituents RG1, RG3, RG4, or RG5 are optionally coupled to the remainder of Q₁ and Q₂ via RG2.

Preferably, Q₁ in Formula (4) is selected from the group consisting of phenyl, vinyl ether, and C₃-C₅ heteroaryl, and is optionally further substituted with at least one moiety RG5, preferably not more than two, more preferably not more than one moiety RG5. Herein, preferred heteroaryls are 2-pyridyl, 3-pyridyl, 4-pyridyl, 2,6-pyrimidyl, 3,5-pyrimidyl, 2,5-pyrimidyl, 2,4-pyrimidyl, 2,4 imidazyl, 2,5 imidazyl, 2,3-pyrazyl, 3,4-pyrazyl, oxazol, isoxazol, thiazol, oxazoline, 2-pyrryl, 3-pyrryl, 2-thiophene, and 3-thiophene.

Preferably, Q₁ in Formula (4) is C₃-C₅ heteroaryl or vinyl ether, and is optionally further substituted with at least one moiety RG5, preferably not more than two, more preferably not more than one moiety RG5, and Q₂ is C₃-C₅ heteroaryl or vinyl ether, and is optionally further substituted with one or more moieties RG5, preferably not more than two, more preferably not more than one moiety RG5. Herein, preferred heteroaryls are 2-pyridyl, 3-pyridyl, 4-pyridyl, 2,6-pyrimidyl, 3,5-pyrimidyl, 2,5-pyrimidyl, 2,4-pyrimidyl, 2,4 imidazyl, 2,5 imidazyl, 2,3-pyrazyl, 3,4-pyrazyl, oxazol, isoxazol, thiazol, oxazoline, 2-pyrryl, 3-pyrryl, 2-thiophene, and 3-thiophene.

In some embodiments, Q₁ in Formula (4) is C₃-C₅ heteroaryl, and is optionally further substituted with at least one moiety RG5, preferably not more than two, more preferably not more than one moiety RG5, and Q₂ is H. In some embodiments, Q₁ in Formula (4) is a phenyl ring, and is optionally further substituted with at least one moiety RG5, preferably not more than two, more preferably not more than one moiety RG5, and Q₂ is -H. In some embodiments, Q₁ in Formula (4) is a phenyl ring, and is optionally further substituted with at least one moiety RG5, preferably not more than two, more preferably not more than one moiety RG5, and Q₂ is a phenyl ring, and is optionally further substituted with at least one moiety RG5, preferably not more than two, more preferably not more than one moiety RG5. In some embodiments, Q₁ in Formula (4) is a phenyl ring, and is optionally further substituted with at least one moiety RG5, preferably not more than two, more preferably not more than one moiety RG5, and Q₂ is selected from the group consisting of C₆ aryl, and C₃₋₅ heteroaryl, and is optionally further substituted with at least one moiety RG5, preferably not more than two, more preferably not more than one moiety RG5. In some embodiments, Q₁ in Formula (4) is C₁-C₁₂ (hetero)alkyl, and is optionally further substituted with at least one moiety RG5, preferably not more than two, more preferably not more than one moiety RG5, and Q₂ selected from the group consisting of C₆ aryl, and C₃₋₅ heteroaryl, and is optionally further substituted with at least one moiety RG5, preferably not more than two, more preferably not more than one moiety RG5. In some embodiments, Q₁ in Formula (4) is C₁-C₁₂ (hetero)alkyl, and is optionally further substituted with at least one moiety RG5, preferably not more than two, more preferably not more than one moiety RG5, and Q₂ in Formula (4) is C₁-C₁₂ (hetero)alkyl, and is optionally further substituted with at least one moiety RG5, preferably not more than two, more preferably not more than one moiety RG5. In some embodiments, Q₁ in Formula (4) is C₃-C₁₂ (hetero)alkenyl, and is optionally further substituted with at least one moiety RG5, preferably not more than two, more preferably not more than one moiety RG5, and Q₂ in Formula (4) is C₁-C₁₂ (hetero)alkenyl, and is optionally further substituted with at least one moiety RG5, preferably not more than two, more preferably not more than one moiety RG5.

Preferably, the tetrazine satisfies Formula (4x): 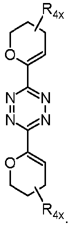 Herein, each R₄ₓ is independently according to RG1, preferably -(S^{P})ᵢ-C^{B}. Preferably, the C^{B} in R₄ₓ is a Targeting Agent as defined herein. Preferably, the C^{B} in R₄ₓ is an antibody or a diabody. Most preferably, the C^{B} in R₄ₓ is the antibody CC49 or the diabody AVP0458.

Preferably, the S^{P} in R₄ₓ is coupled to the vinyl ether ring via a moiety -C₁₋₃ (hetero)alkylene-O-, more preferably - CH₂-O-.

Preferably, the S^{P} in R₄ₓ comprises a polymer, more preferably polyethylene glycol (PEG). Preferably, the S^{P} in R₄ₓ comprises or is a moiety -C₁₋₃ (hetero)alkylene-(R₄ₓ₁-CH₂-(CH₂-O-CH₂)ₚ₁-CH₂)ₚ₂-R₄ₓ₂. Therein, p1 is an integer of from 1 to 24, preferably of from 5 to 15, more preferably of from 8 to 10, and p2 is 0 or 1. More preferably, the S^{P} in R₄ₓ comprises or is a moiety -CH₂-(R₄ₓ₁-CH₂-(CH₂-O-CH₂)ₚ₁-CH₂)ₚ₂-R₄ₓ₂.

R₄ₓ₁ is according to RG2a, RG2b, or RG2c, and is preferably selected from the group consisting of -OC(O)-, - C(O)O-, -OC(O)-NH-, -NH-C(O)O-, -OC(S)-, -C(S)O-, -OC(S)-NH-, -NH-C(S)O-, -NHC(O)-, -C(O)NH-, -NHC(S)-, - C(S)NH-, -NHC(O)O-, -O-C(O)NH-, -NHC(S)O-, -O-C(S)NH-, -NHC(O)NH-, and -NHC(S)NH-. Preferably, R₄ₓ₁ is - OC(O)-NH-. R₄ₓ₂ is -C(O)-, -C(S)-, -OC(O)-, -OC(S)-, -OC(O)-NH-, OC(S)-NH-, -NH-, -NHC(O)-, -NHC(S)-, NHC(O)NH-, -NHC(S)NH-, -OC(O)-NH-C₁₋₃ (hetero)alkylene-, or -NH-C(O)-C₁₋₃ (hetero)alkylene-, -OC(O)-NH-C₁₋₃ (hetero)alkylene-R₄ₓ₃, or -NH-C(O)-C₁₋₃ (hetero)alkylene-R₄ₓ₃. It will be understood that R₄ₓ₂ is coupled to the C^{B}, optionally via another spacer. Preferably, the C₁₋₃ (hetero)alkylene is a C₁ (hetero)alkylene. Preferably, if p1 is 0, then R₄ₓ₂ is -OC(O)-, -NHC(O)-, -OC(O)-NH-C₁₋₃ (hetero)alkylene-R₄ₓ₃, or -NH-C(O)-C₁₋₃ (hetero)alkylene-R₄ₓ₃.

R₄ₓ₃ is according to RG2a, RG2b, or RG2c, preferably RG2b. More preferably, R₄ₓ₃ is 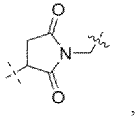 wherein the wiggly line indicates a bond to the C₁₋₃ (hetero)alkylene and the dashed line a bond to the C^{B}.

Tetrazines of Formula (4x) are particularly advantageous, as they have excellent stability and reactivity, also in physiological conditions. As such, tetrazines according to Formula (4x) are particularly useful in embodiments wherein the tetrazine of Formula (4x) is administered to the subject prior to administering the masked IL12 protein as described herein. Then, the tetrazine of Formula (4x), which comprises a Targeting Agent, can first accumulate at the target site, after which the masked IL12 protein can be administered to said subject. The masked IL 12 protein then does not necessarily comprise a Targeting Agent, but is simply only unmasked at the target site after reaction with the tetrazine of Formula (4x) that is accumulated at said target site. Preferably, the tetrazine according to Formula (4x) is a tetrazine according to Formula (4x1):

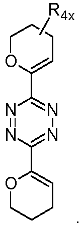

Most preferably, the tetrazine according to Formula (4x) is a tetrazine according to Formula (4x2):

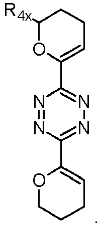

Preferably, in particular for the embodiment wherein the Activator is administered after the masked IL12 protein or masked subunit of the invention, all compounds disclosed herein comprising a group Q, at least one of these has a molecular weight in a range of from 100 Da to 3000 Da. Preferably, at least one of these has a molecular weight in a range of from 100 Da to 2000 Da. More preferably, at least one of these has a molecular weight in a range of from 100 Da to 1500 Da, even more preferably in a range of from 150 Da to 1500 Da. Even more preferably still, at least one of these has a molecular weight in a range of from 150 Da to 1000 Da, most preferably in a range of from 200 Da to 1000 Da.

Preferably, for all compounds disclosed herein comprising a group Q, none of these has a molecular weight of more than 3000 Da, in particular in the case the Activator needs to efficiently extravasate into tissues.

If, however, the Activator is administrated to a subject prior to administering the masked IL12 protein and/or masked subunit of the invention, then there is preferably no restriction to the molecular weight and/or size of the compounds comprising a group Q. In that case, the Activator can be coupled to large structures such as proteins, nanoparticles, and the like.

### Formula (14)

In some embodiments, the diene is a tetrazine satisfying Formula (14): 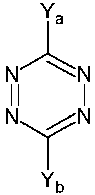 Formula (14), and salts, solvates, and hydrates thereof, wherein Yₐ is selected from the group consisting of Y₁, Y₂, Y₃, Y₄, Y₅, Y₆, Y₇, and Y₈:

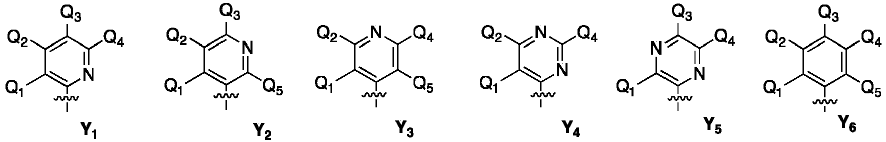

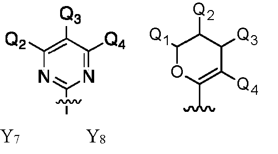

In Formula (14), Y_{b} is according to RG1, and is preferably selected from the group consisting of Y₁, Y₂, Y₃, Y₄, Y₅, Y₆, Y₇, Y₈, hydrogen, X₄₇, and -(S^{P})ᵢ-C^{B}; wherein i is 0 or 1. For Formula (14), each Q₁ and Q₅, are independently selected from the group consisting of X₄₅, hydrogen, X₄₇ and -(S^{P})ᵢ-C^{B}; each Q₂ and Q₄, are independently selected from the group consisting of X₄₆, hydrogen, X₄₇, and -(S^{P})ᵢ-C^{B}; each Q₃ is independently selected from the group consisting of hydrogen, X₄₇, and -(S^{P})ᵢ-C^{B}.

Preferably, the compound of Formula (14) comprises at least one X₄₅ or X₄₆ group. Each X₄₅ is independently selected from the group consisting of N(X₅₀)₂, C(X₅₁)₂N(X₅₀)_{2,} NX₅₀C(O)X₅₁, NX₅₀C(S)X_{51,} OH, SH, C(O)OH, C(S)OH, C(O)SH, C(S)SH, NX₅₀C(O)OX_{51,} NX₅₀C(S)OX_{51,} NX₅₀C(O)SX_{51,} NX₅₀C(S)SX_{51,} NX₅₀C(O)N(X₅₁)₂, NX₅₀C(S)N(X₅₁)₂, NX₅₀SO₂X_{51,} NX₅₀SO₃X_{51,} NX₅₀OX₅₁, SO₃H, and PO₃H₂.

Each X₄₆ is independently selected from the group consisting of N(X₅₀)₂, C(X₅₁)₂N(X₅₀)_{2,} NX₅₀C(O)X₅₁, NX₅₀C(S)X_{51,}, OH, SH, C(O)OH, C(S)OH, C(O)SH, C(S)SH, NX₅₀C(O)OX_{51,} NX₅₀C(S)OX_{51,} NX₅₀C(O)SX_{51,} NX₅₀C(S)SX_{51,} NX₅₀C(O)N(X₅₁)₂, NX₅₀C(S)N(X₅₁)₂, NX₅₀SO₂X_{51,} NX₅₀SO₃X₅₁, NX₅₀OX₅₁, SO₃H, and PO₃H₂.

Each X₅₀ and X₅₁ is independently selected from the group consisting of hydrogen, X₄₈, and -(S^{P})ᵢ-C^{B}. Each X₄₈ is independently selected from the group consisting of hydrogen, C₁-C₄ (hetero)alkyl, C₂-C₄ (hetero)alkenyl, and C₄₋₆ (hetero)aryl; wherein for X₄₈ the (hetero)alkyl, (hetero )alkenyl, and (hetero)aryl are optionally substituted with a moiety selected from the group consisting of -Cl, -F, -Br, -I, -OH, -NH₂, =O, -SH, -SO₃H, -PO₃H_{,} -PO₄H₂, and -NO₂; and optionally contain at most two heteroatoms selected from the group consisting of -O-, -S-, -NH-, -P-, and -Si-, wherein the N, S, and P atoms are optionally oxidized. Preferably, X₅₀ is hydrogen.

Each X₄₇ is independently selected from the group consisting of -F, -Cl, -Br, -I, -OX₄₉, -N(X₄₉)₂, -SO₃, -PO₃⁻_{,} -NO₂, - CF₃, -SX₄₉, S(=O)₂N(X₄₉)_{2,} OC(=O)X₄₉, SC(=O) X₄₉, OC(=S)X₄₉, SC(=S)X_{49,} NX₄₉C(=O)-X₄₉, NX₄₉C(=S)-X₄₉, NX₄₉C(=O)O-X₄₉, NX₄₉C(=S)O-X₄₉, NX₄₉C(=O)S-X₄₉, NX₄₉C(=S)S-X₄₉, OC(=O)N(X₄₉)₂, SC(=O)N(X₄₉)₂, OC(=S)N(X₄₉)₂, SC(=S)N(X₄₉)₂, NX₄₉C(=O)N(X₄₉)₂, NX₄₉C(=S)N(X₄₉)_{2,} C(=O)X_{49,} C(=S)X_{49,} C(=O)N(X₄₉)₂, C(=S)N(X₄₉)₂, C(=O)O-X₄₉, C(=O)S-X₄₉, C(=S)O-X₄₉, C(=S)S-X₄₉, -S(O)X₄₉, -S(O)₂X₄₉, NX₄₉S(O)₂X₄₉, -ON(X₄₉)_{2,} - NX₄₉OX_{49,} C₁-C₈ (hetero)alkyl, C₂-C₈ (hetero)alkenyl, C₂-C₈ alkynyl, C₆-C₁₂ aryl, C₂-C₁₂ heteroaryl, C₃-C₈ cycloalkyl, C₅-C₈ cycloalkenyl, C₃-C₁₂ (hetero)alkyl(hetero)aryl, C₃-C₁₂ (hetero)arylalkyl, C₄-C₁₂ (hetero)alkylcycloalkyl, C₄-C₁₂ cycloalkylalkyl, C₅-C₁₂ cycloalkyl(hetero)aryl, C₅-C₁₂ (hetero)arylcycloalkyl, and combinations thereof, wherein the (hetero)alkyl, (hetero)alkenyl, alkynyl, aryl, heteroaryl, cycloalkyl, cycloalkenyl, (hetero)alkyl(hetero)aryl, (hetero)arylalkyl, (hetero)alkylcycloalkyl, cycloalkylalkyl, cycloalkyl(hetero)aryl and (hetero)arylcycloalkyl are optionally substituted with a moiety selected from the group consisting of -Cl, -F, -Br, -I, -OX₄₉, -N(X₄₉)₂, -SO₃X₄₉, - PO₃(X₄₉)_{2,} -PO₄(X₄₉)_{2,} -NO₂, -CF₃, =O, =NX₄₉, and -SX₄₉.

Each X₄₉ is independently selected from the group consisting of hydrogen, C₁-C₈ (hetero)alkyl, C₂-C₈ (hetero)alkenyl, C₂-C₈ alkynyl, C₆-C₁₂ aryl, C₂-C₁₂ heteroaryl, C₃-C₈ cycloalkyl, C₅-C₈ cycloalkenyl, C₃-C₁₂ (hetero)alkyl(hetero)aryl, C₃-C₁₂ (hetero)arylalkyl, C₄-C₁₂ (hetero)alkylcycloalkyl, C₄-C₁₂ cycloalkylalkyl, C₅-C₁₂ cycloalkyl(hetero)aryl and C₅-C₁₂ (hetero)arylcycloalkyl, wherein X₄₉ not being hydrogen is optionally substituted with a moiety selected from the group consisting of -Cl, -F, -Br, -I, -OH, -NH₂, -SO₃H, -PO₃H_{,} -PO₄H_{2,} -NO₂, -CF₃, =O, =NH, and -SH.

Preferably, at most two, more preferably at most one of Q₁, Q₂, Q₃, Q_{4,} and Q₅ are C^{B}; wherein the compound according to Formula (14) preferably comprises for each individual Yₐ and Y_{b} at most four C^{B}, more preferably at most two C^{B}, most preferably at most one C^{B}; wherein the compound according to Formula (14) preferably comprises at least one C^{B}; wherein preferably for each individual Yₐ and Y_{b} at most three, more preferably at most two of Q₁, Q₂, Q₃, Q₄, and Q₅ are not hydrogen; wherein preferably for each individual Yₐ and Y_{b} at most two of Q₁, Q₂, Q₃, Q₄, and Q₅ are X₄₅ or X₄₆, wherein preferably for each individual Yₐ and Y_{b} one of Q₁, Q₂, Q₄, and Q₅ is X₄₅ or X₄₆, wherein preferably both Yₐ and Y_{b} comprise at least one X₄₅ or X₄₆,wherein preferably both Yₐ and Y_{b} comprise one X₄₅ or X₄₆, wherein preferably both Yₐ and Y_{b} comprise one X₄₅ or X₄₆, whereby the X₄₅ comprised in Yₐ is the same as the X₄₅ comprised in Y_{b}, and/or the X₄₆ comprised in Yₐ is the same as the X₄₆ comprised in Y_{b}, wherein preferably Yₐ and Y_{b} are both independently selected Y₁, or both independently selected Y₂, or both independently selected Y₃, or both independently selected Y₄, or both independently selected Y₅; or both independently selected Y₇; or both independently selected Y₈.

Preferably, in Formula (14), when Q₁ is a X₄₇ or -(S^{P})ᵢ-C^{B}, then for Q₁ the X₄₇ and -(S^{P})ᵢ-C^{B} are not a group in accordance with the definition of X₄₅. Preferably, in Formula (14), when Q₅ is a X₄₇ or -(S^{P})ᵢ-C^{B}, then for Q₅ the X₄₇ and -(S^{P})ᵢ-C^{B} are not a group in accordance with the definition of X₄₅. Preferably, in Formula (14), when Q₂ is a X₄₇ or - (S^{P})ᵢ-C^{B}, then for Q₂ the X₄₇ and -(S^{P})ᵢ-C^{B} are not a group in accordance with the definition of X₄₆. Preferably, in Formula (14), when Q₄ is a X₄₇ or -(S^{P})ᵢ-C^{B}, then for Q₄ the X₄₇ and -(S^{P})ᵢ-C^{B} are not a group in accordance with the definition of X₄₆. Preferably Yₐ equals Y_{b}.

Preferably Yₐ is selected from Y₁, Y₂, Y₃, Y₄ or Y₅ and Y_{b} is hydrogen, X₄₇ or -(S^{P})ᵢ-C^{B}. Preferably Yₐ is selected from Y₁, Y₂, Y₃, Y₄ or Y₅ and Y_{b} is hydrogen. Preferably the compound according to Formula (14) does not comprise - (S^{P})ᵢ-C^{B}.

Preferably when X₄₅ or X₄₆ is N(X₅₀)₂, then one X₅₀ is hydrogen and one X₅₀ is X₄₈ or -(S^{P})ᵢ-C^{B}. Preferably Formula (14) does not comprise X₄₆. Preferably, both Q₁ in Formula (14) are X₄₅. Preferably, both Q₂ in Formula (14) are X₄₆. Preferably, both Q₅ in Formula (14) are X₄₅. Preferably, both Q₄ in Formula (14) are X₄₆.

Preferably, each X₄₅ is independently selected from the group consisting of N(X₅₀)₂, NX₅₀C(O)X₅₁, NX₅₀C(S)X₅₁, OH, SH, NX₅₀C(O)OX₅₁, NX₅₀C(S)OX₅₁, NX₅₀C(O)SX₅₁, NX₅₀C(S)SX₅₁, NX₅₀C(O)N(X₅₁)₂, NX₅₀C(S)N(X₅₁)₂, NX₅₀SO₂X₅₁, NX₅₀SO₃X₅₁, and NX₅₀OX₅₁. More preferably, each X₄₅ is independently selected from the group consisting of N(X₅₀)₂, NX₅₀C(O)X₅₁, NX₅₀C(S)X₅₁, OH and SH.

In some embodiments, X₄₅ is selected from the group consisting of NHX₅₀, C(X₅₁)₂NH₂, CHX₅₁NH₂, CH₂N(X₅₀)₂, CH₂NHX₅₀, NHC(O)X₅₁, NHC(S)X₅₁, OH, and SH.
In some embodiments, X₄₅ is NHX₅₀. In some embodiments, X₄₅ is C(X₅₁)₂NH₂. In some embodiments, X₄₅ is CHX₅₁NH₂. In some embodiments, X₄₅ is CH₂N(X₅₀)₂. In some embodiments, X₄₅ is CH₂NHX₅₀. In some embodiments, X₄₅ is NH₂. In some embodiments, X₄₅ is CH₂NH₂. In some embodiments, X₄₅ is NHC(O)X₅₁. In some embodiments, X₄₅ is NHC(S)X₅₁. In some embodiments, X₄₅ is OH. In some embodiments, X₄₅ is SH.

Preferably, X₄₆ is independently selected from the group consisting of N(X₅₀)₂, NX₅₀C(O)X_{51,} NX₅₀C(O)OX₅₁, and NX₅₀C(O)N(X₅₁)₂. More preferably, X₄₆ is selected from the group consisting of N(X₅₀)₂, and NX₅₀C(O)X_{51,}. Most preferably, X₄₆ is selected from the group consisting of NHX₅₀ and NHC(O)X₅₁. In some embodiments, X₄₆ is NHX₅₀. In some embodiments, X₄₆ is NH₂. In some embodiments, X₄₆ is NHC(O)X₅₁.

Preferably, each X₄₇ is independently selected from the group consisting of F, -OH, -NH₂, -SO₃⁻_{,} -NO₂, -CF₃, -SH, C₁-C₆ (hetero)alkyl, C₆ aryl, C₄-C₅ heteroaryl, C₅-C₈ (hetero)alkyl(hetero)aryl, C₅-C₈ (hetero)arylalkyl, C₄-C₈ (hetero)alkylcycloalkyl, and C₄-C₈ cycloalkylalkyl . In a more preferred embodiment, each X₄₇ is independently selected from the group consisting of F, -SO₃⁻_{,} -NO₂, -CF₃, C₁-C₆ (hetero)alkyl, C₆ aryl, C₄-C₅ heteroaryl, C₅-C₈ (hetero)alkyl(hetero)aryl, C₅-C₈ (hetero)arylalkyl, C₄-C₈ (hetero)alkylcycloalkyl, and C₄-C₈ cycloalkylalkyl . Preferably, the X₄₇ substituents do not contain heteroatoms. Preferably, X₄₇ is not substituted. In another preferred embodiment, X₄₇ does not contain heteroatoms.

Preferably, each X₄₈ is independently selected from the group consisting of hydrogen, C₁-C₄ (hetero)alkyl, C₂-C₄ (hetero)alkenyl, and C₄₋₆ (hetero)aryl. For X₄₈ the (hetero)alkyl, (hetero)alkenyl, and (hetero)aryl are optionally substituted with a moiety selected from the group consisting of -Cl, -F, -Br, -I, -OH, -NH₂, =O, -SH, -SO₃H, -PO₃H_{,} - PO₄H₂ and -NO₂, and optionally contain at most two heteroatoms selected from the group consisting of -O-, -S-, -NH-, - P-, and -Si-, wherein the N, S, and P atoms are optionally oxidized.

Preferably, X₄₈ is C₁-C₄ (hetero)alkyl. Preferably, the X₄₈ substituents do not contain heteroatoms. Preferably, X₄₈ is not substituted. In another preferred embodiment, X₄₈ does not contain heteroatoms.

Preferably, X₄₉ is selected from the group consisting of hydrogen, C₁-C₈ (hetero)alkyl, C₂-C₈ (hetero)alkenyl, C₂-C₈ (hetero)alkynyl, C₆-C₁₂ aryl, C₂-C₁₂ heteroaryl, C₃-C₈ (hetero)cycloalkyl, C₅-C₈ (hetero)cycloalkenyl, C₃-C₁₂ (hetero)alkyl(hetero)aryl, C₃-C₁₂ (hetero)arylalkyl, C₄-C₁₂ (hetero)alkylcycloalkyl, C₄-C₁₂ cycloalkylalkyl, C₅-C₁₂ cycloalkyl(hetero)aryl and C₅-C₁₂ (hetero)arylcycloalkyl, wherein X₄₉ not being hydrogen is optionally substituted with a moiety selected from the group consisting of -Cl, -F, -Br, -I, -OH, -NH₂, -SO₃H, -PO₃H_{,} -PO₄H_{2,} -NO₂, -CF₃, =O, =NH, and -SH.

Preferably, X₄₉ is selected from the group consisting of hydrogen, C₁-C₄ (hetero)alkyl, C₂-C₄ (hetero)alkenyl, C₂-C₄ (hetero)alkynyl, C₆-C₈ aryl, C₂-C₈ heteroaryl, C₃-C₆ cycloalkyl, C₅-C₆ cycloalkenyl, C₃-C₁₀ (hetero)alkyl(hetero)aryl, C₃-C₁₀ (hetero)arylalkyl, C₄-C₈ (hetero)alkylcycloalkyl, C₄-C₈ cycloalkylalkyl, C₅-C₁₀ cycloalkyl(hetero)aryl and C₅-C₁₀ (hetero)arylcycloalkyl, wherein the X₄₉ not being hydrogen are optionally substituted with a moiety selected from the group consisting of -Cl, -F, -Br, -I, -OH, -NH₂, -SO₃H, -PO₃H_{,} -PO₄H_{2,} -NO₂, -CF₃, =O, =NH, and -SH, and optionally contain one or more heteroatoms selected from the group consisting of O, S, NH, P, and Si, wherein the N, S, and P atoms are optionally oxidized, wherein the N atoms are optionally quaternized.

Preferably, X₄₉ substituents do not contain heteroatoms. Preferably, X₄₉ is not substituted. In another preferred embodiment, X₄₉ does not contain heteroatoms.

Preferably, each X₅₀ is independently selected from the group consisting of hydrogen, X₄₈, and -(S^{P})ᵢ-C^{B}. In some embodiments, X₅₀ is X₄₈. In some embodiments, X₅₀ is -(S^{P})ᵢ-C^{B}. Preferably, X₅₀ is H.

Preferably, each X₅₁ is independently selected from the group consisting of hydrogen, X₄₈, and -(S^{P})ᵢ-C^{B}. In some embodiments, X₅₁ is X₄₈. In some embodiments, X₅₁ is -(S^{P})ᵢ-C^{B}. Preferably, X₅₁ is H.

Preferably, in Formula (14) Q₁ is selected from the group consisting of hydrogen, X₄₇, and -(S^{P})ᵢ-C^{B}. In some embodiments, Q₁ in Formula (14) is hydrogen. In some embodiments, Q₁ in Formula (14) is X₄₇. Preferably, Q₁ in Formula (14) is -(S^{P})ᵢ-C^{B}, and preferably Q₂, Q₃, Q₄, Q₅, Q₆, Q₇, Q₈, Q₉, and Q₁₀ are X₄₅, X₄₆, or hydrogen.

Preferably, Q₂ in Formula (14) is selected from the group consisting of hydrogen, X₄₇, and -(S^{P})ᵢ-C^{B}. In some embodiments, Q₂ in Formula (14) is hydrogen. In some embodiments, Q₂ is in Formula (14) X₄₇. Preferably, Q₂ in Formula (14) is -(S^{P})ᵢ-C^{B}, and preferably Q₁, Q₃, Q₄, Q₅, Q₆, Q₇, Q₈, Q₉, and Q₁₀ are X₄₅, X₄₆, or hydrogen.

Preferably, Q₃ in Formula (14) is selected from the group consisting of hydrogen, X₄₇, and -(S^{P})ᵢ-C^{B}. In some embodiments, Q₃ in Formula (14) is hydrogen. In some embodiments, Q₃ in Formula (14) is X₄₇. Preferably, Q₃ in Formula (14) is -(S^{P})ᵢ-C^{B}, and preferably Q₁, Q₂, Q₄, Q₅, Q₆, Q₇, Q₈, Q₉, and Q₁₀ are X₄₅, X₄₆, or hydrogen.

Preferably, Q₄ in Formula (14) is selected from the group consisting of hydrogen, X₄₇, and -(S^{P})ᵢ-C^{B}. In some embodiments, Q₄ in Formula (14) is hydrogen. In some embodiments, Q₄ in Formula (14) is X₄₇. Preferably, Q₄ in Formula (14) is -(S^{P})ᵢ-C^{B}, and preferably Q₁, Q₂, Q₃, Q₅, Q₆, Q₇, Q₈, Q₉ and Q₁₀ are X₄₅, X₄₆, or hydrogen.

Preferably, Q₅ in Formula (14) is selected from the group consisting of hydrogen, X₄₇, and -(S^{P})ᵢ-C^{B}. In some embodiments, Q₅ in Formula (14) is hydrogen. In some embodiments, Q₅ in Formula (14) is X₄₇. Preferably, Q₅ is -(S^{P})ᵢ-C^{B}, and preferably Q₁, Q₂, Q₃, Q₄, Q₆, Q₇, Q₈, Q₉ and Q₁₀ are X₄₅, X₄₆, or hydrogen.

Preferably, Q₆ in Formula (14) is selected from the group consisting of hydrogen, X₄₇, and -(S^{P})ᵢ-C^{B}. In some embodiments, Q₆ in Formula (14) is hydrogen. In some embodiments, Q₆ in Formula (14) is X₄₇. Preferably, Q₆ in Formula (14) is -(S^{P})ᵢ-C^{B}, and preferably Q₁, Q₂, Q₃, Q₄, Q₅, Q₇, Q₈, Q₉ and Q₁₀ are X₄₅, X₄₆, or hydrogen.

Preferably, Q₇ in Formula (14) is selected from the group consisting of hydrogen, X₄₇, and -(S^{P})ᵢ-C^{B}. In some embodiments, Q₇ in Formula (14) is hydrogen. In some embodiments, Q₇ in Formula (14) is X₄₇. Preferably, Q₇ in Formula (14) is -(S^{P})ᵢ-C^{B}, and preferably Q₁, Q₂, Q₃, Q₄, Q₅, Q₆, Q₈, Q₉ and Q₁₀ are X₄₅, X₄₆, or hydrogen.

Preferably, Q₈ in Formula (14) is selected from the group consisting of hydrogen, X₄₇, and -(S^{P})ᵢ-C^{B}. In some embodiments, Q₈ in Formula (14) is hydrogen. In some embodiments, Q₈ in Formula (14) is X₄₇. Preferably, Q₈ in Formula (14) is -(S^{P})ᵢ-C^{B}, and preferably Q₁, Q₂, Q₃, Q₄, Q₅, Q₆, Q₇, Q₉ and Q₁₀ are X₄₅, X₄₆, or hydrogen.

Preferably, Q₉ in Formula (14) is selected from the group consisting of hydrogen, X₄₇, and -(S^{P})ᵢ-C^{B}. In some embodiments, Q₉ in Formula (14) is hydrogen. In some embodiments, Q₉ in Formula (14) is X₄₇. Preferably, Q₉ in Formula (14) is -(S^{P})ᵢ-C^{B}, and preferably Q₁, Q₂, Q₃, Q₄, Q₅, Q₆, Q₇, Q₈ and Q₁₀ are X₄₅, X₄₆, or hydrogen.

Preferably, Q₁₀ in Formula (14) is selected from the group consisting of hydrogen, X₄₇, and -(S^{P})ᵢ-C^{B}. In some embodiments, Q₁₀ in Formula (14) is hydrogen. In some embodiments, Q₁₀ in Formula (14) is X₄₇. Preferably, Q₁₀ in Formula (14) is -(S^{P})ᵢ-C^{B}, and preferably Q₁, Q₂, Q₃, Q₄, Q₅, Q₆, Q₇, Q₈ and Q₉ are X₄₅, X₄₆, or hydrogen.

For Y₈, it is preferred that Q₂, Q₃, Q₄ are hydrogen and Q₁ is as defined for R₄ₓ

If the Trigger is a tetrazine, it is a tetrazine according to Formula (2) as described herein.

### Methods for preparing a masked IL12 protein of the invention

The invention relates to a method for preparing a masked IL12 protein or masked subunit according to the invention, wherein a IL12 protein or subunit thereof is contacted with an activated tetrazine or activated dienophile. For both the activated dienophile and activated tetrazine, it is preferred that R^{R} is -O-N-succinimidyl or -O-pentafluorophenyl.

The activated tetrazine is according to Formula (2a), wherein X^{S}, A^{T}, B^{T}, b, X^{C}, C^{T}, L^{C}, and f are as defined for Formula (2). The activated tetrazine and the tetrazine of Formula (2) only differ in respect of whether they are bound to a moiety R^{R} or the ε-amine of the at least one lysine residue of the masked IL12 protein, respectively. As such, all embodiments of the tetrazine as described for Formula (2) also hold for the activated tetrazine, with the difference being that C^{A} should be replaced with R^{R}.

Preferably, activated tetrazines to prepare the masked IL12 proteins of the invention are according to Formula (2a-2): 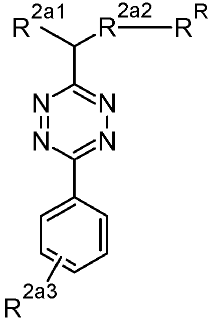 Formula (2a-2),
wherein R^{R} is as defined for Formula (2a); R^{2a1} is hydrogen or methyl, preferably methyl, R^{2a2} is -X^{C}-C(C^{T})-(L^{C})_{f}- as defined for Formula (2a), and R^{2a3} is hydrogen or -S^{P}-C^{B}, wherein S^{P} is a spacer, preferably as defined herein, and C^{B} is a Construct B, preferably as defined herein.

Preferably, in relation to Formula (2a-2), S^{P} is according to RG2, more preferably S^{P} is a C₁-C₆ heteroalkyl, optionally substituted with =O, more preferably S^{P} is 1-CH₂-NH-C(O)-CH₂-CH₂-2, wherein 1 denotes a bond to the phenyl ring, and 2 denotes a bond to C^{B}.

Preferably, in relation to Formula (2a-2), C^{B} comprises a linear or branched polyethylene glycol moiety, preferably a linear polyethylene glycol moiety, more preferably C^{B} is -(O-CH₂-CH₂)_{q}-O-CH₃, wherein q is an integer of from 1 to 500, preferably of from 2 to 250, more preferably of from 3 to 125, even more preferably of from 4 to 75, more preferably still of from 5 to 50, more preferably still of from 6 to 30, even more preferably of from 7 to 20, more preferably still of from 8 to 15, and most preferably of from 9 to 12.

More preferably, activated tetrazines to prepare the masked IL12 proteins of the invention are according to Formulae (2a-3): 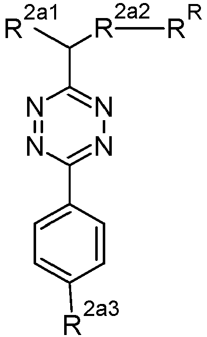 Formula (2a-3); wherein R^{2a1}, R^{2a2}, R^{2a3}, and R^{R} are as defined for Formula (2a-2).

More preferably still, activated tetrazines to prepare the masked IL12 proteins of the invention are according to any one of Formulae (2a-4) and (2a-5): 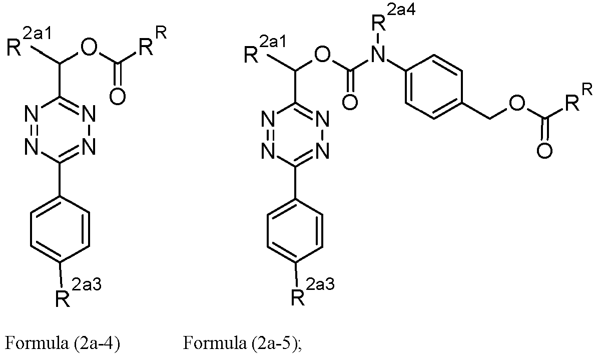 wherein R^{2a1}, R^{2a3}, and R^{R} are as defined in relation to Formula (2a) and/or Formula (2a-2). In Formula (2a-5), R^{2a4} is hydrogen or methyl.

Most preferably, activated tetrazines to prepare the masked IL12 proteins of the invention are selected from the group consisting of:

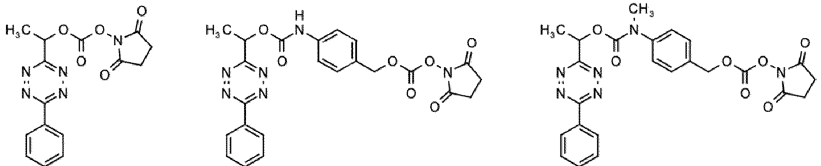

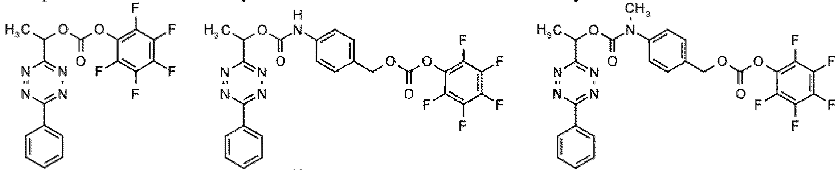

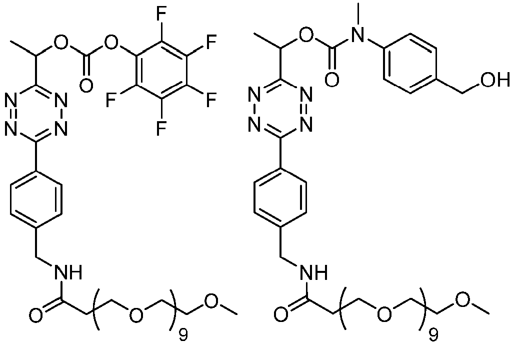

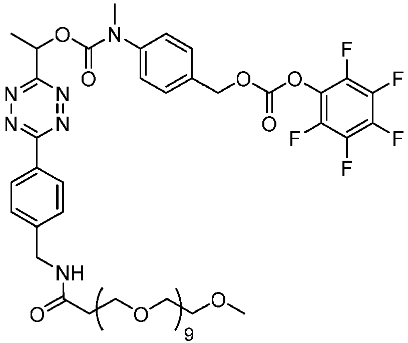

Likewise, it will be understood that the activated dienophile and the dienophile in a structure according to Formula (1) only differ in respect of whether they are bound to a moiety R^{R} or the ε-amine of the at least one lysine residue of the masked IL12 protein, respectively. As such, all embodiments of the dienophile as described for Formula (1) also hold for the activated dienophile, with the difference being that C^{A} should be replaced with R^{R}.

Preferably, activated dienophiles to prepare the masked IL12 protein or masked subunit of the invention are according to Formula (2b-2): 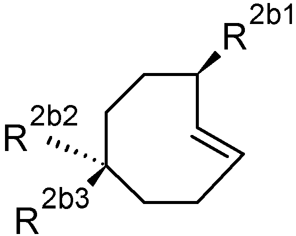 Formula (2b-2); wherein R^{2b1} is M^{R}-R^{R} or -M^{B}-L^{C}-R^{R}; M^{R}, M^{B}, and L^{C} are as defined in relation to Formula (1); wherein R^{2b2} is a radical according to RG1, RG3, RG4, or RG5, preferably -OH or methyl, most preferably -OH; wherein R^{2b3} is hydrogen or -S^{P}-C^{B}, preferably -S^{P}-C^{B}; wherein S^{P} is a spacer, preferably as defined herein, and C^{B} is a Construct B, preferably as defined herein.

Preferably, in relation to Formula (2b-2), S^{P} is according to RG2, most preferably 1-CCO)NH-(CH₂)_{z}-2, wherein 1 denotes a bond to the *trans*-cyclooctene ring, and 2 denotes a bond to C^{B}; wherein z is an integer of from 1 to 4, preferably 2.

Preferably, in relation to Formula (2b-2), C^{B} is -CH₂-(4-acetylphenyl) or C^{B} comprises a linear or branched polyethylene glycol moiety, preferably a linear polyethylene glycol moiety. More preferably, in relation to Formula (2b-2), C^{B} is -(O-CH₂-CH₂)_{q}-O-CH₃, wherein q is an integer of from 1 to 500, preferably of from 3 to 250, more preferably of from 5 to 125, even more preferably of from 7 to 100, more preferably still of from 5 to 75, more preferably still of from 10 to 60, even more preferably of from 12 to 50, more preferably still of from 15 to 40, and most preferably of from 20 to 30.

Most preferably, activated dienophiles to prepare the masked IL12 protein or masked subunit of the invention are selected from the group consisting of: 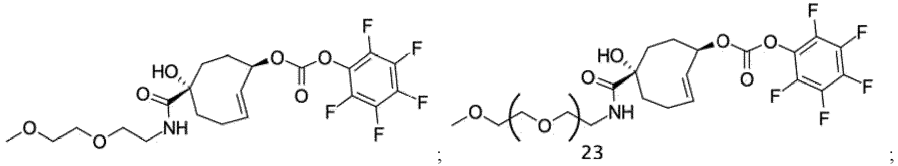 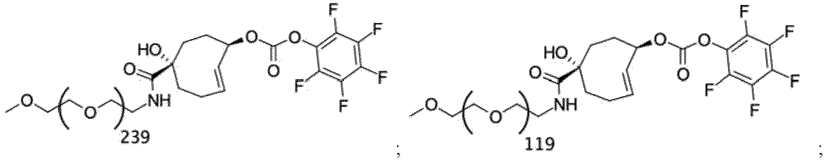 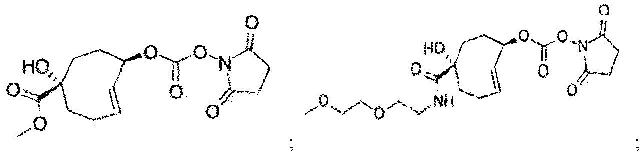 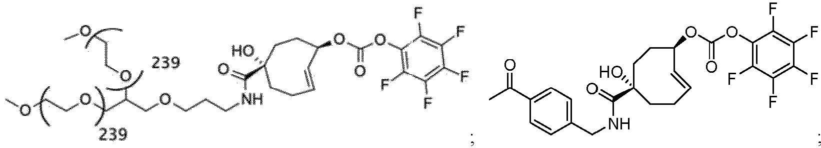 and

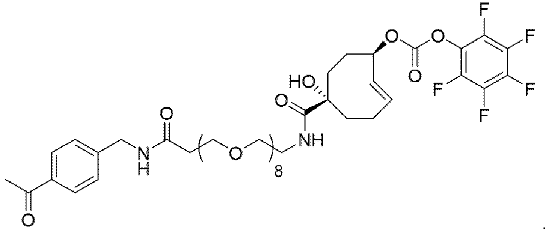

In said method for preparing a masked IL12 protein or masked subunit according to the invention, it is preferred that the molar ratio of the IL12 protein or a subunit thereof as compared to the activated dienophile or activated tetrazine is at least 1:10, preferably at least 1:25, more preferably at least 1:50, and most preferably at least 1:100. Surprisingly, even at relatively low ratios, sufficient masking of the IL12 protein is achieved. It is preferred that said molar ratio is at most 1:300, more preferably at most 1:250, even more preferably at most 1:200, and most preferably at most 1:150. Surprisingly, the inventors found that good masking of IL12 is achieved even at low molar ratios of the IL12 protein as compared to the activated dienophile or activated tetrazine, especially if there are no substituents on the Trigger, or when such substituents are present but are relatively small, such as substituents having a molecular weight of at most 500 Da, more preferably at most 200 Da.

The method for preparing a masked IL12 protein of the invention can be performed in any suitable solvent, typically an aqueous solution, such as an aqueous buffer.

General synthesis methods for activated dienophiles and activated tetrazines are described in the Examples. A general synthesis route providing access to dienophiles is also described in WO2022/197182A1. An alternative route is shown directly below. The synthesis method is as reported in Rossin et al Nature Communications 2018, 9, 1484 and Rossin et al Bioconj.Chem., 2016 27(7):1697-1706 with the exception of the conversion of D to F, which now is conducted by mixing D with hydroxide solution in methanol, followed by evaporation and reaction with iodomethane. For the sake of clarity only one of the two enantiomers of E to K is shown. A person skilled in the art understands that the enantiomers can be separated at various stages in the synthesis using established chiral resolution methods to obtain enantiomerically pure compounds for example, such as chiral salts.

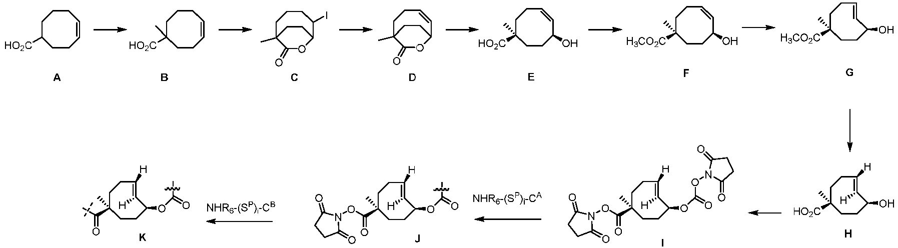

### Method for unmasking the masked IL12 protein

The invention also relates to a method for unmasking the masked IL12 protein or masked subunit according to the invention, said method comprising the step of contacting said masked IL12 protein with a diene (if the Trigger is a dienophile) or a dienophile (if the Trigger is a diene) as defined herein. Typically, this method is a non-therapeutic method and is for example aimed at *in vitro* reactions, such as those for testing reactivation of the masked IL12 protein upon contacting it with a diene or dienophile, whichever is appropriate, or for testing the response of cells that are kept in a cell culture. In principle, unmasking can also take place *in vivo*, after which the unmasked IL12 protein may have a therapeutic effect.

### Method for imaging

The invention also pertains to a non-therapeutic method for imaging the masked IL12 protein or masked subunit according to the invention, in a subject, preferably a human, said non-therapeutic method comprising the steps of
(a) administering the masked IL12 protein according to the invention comprising a label, to the subject;
(b) imaging the masked IL12 protein according to the invention, present in the subject; wherein the label is selected from the group consisting of radionuclides, fluorescent dyes, and phosphorescent dyes.

It will be understood that in the non-therapeutic imaging method of the invention, the masked IL12 protein is labelled. This can be performed by labeling the IL12 protein, and then masking the labelled IL12 protein using a method as described herein, by labeling the masked IL12 protein according to the invention, and/or by including a label in the dienophile or diene that is part of Formula (1), for example as C^{B}. Any combination of these strategies is conceivable, and multiple labels can be used. Preferably, the method of administering is as defined herein.

### Use of the masked IL12 protein

The invention further pertains to the use of a masked IL12 protein according to the invention, or a combination according to the invention, in a bioorthogonal reaction. Preferably, the use is *in vitro.* In particular, the *in vitro* use is for applications in monitoring IL12 activation and activity, cellular responses to IL12, *in vitro* diagnostics kits, and the like. Preferably, the use is in a chemical environment. Preferably, the use is in a biological environment. Preferably, the use is in a physiological environment. Preferably, the use is *in vitro.* Preferably, the use is *in vivo.*

### Combinations in relation to the invention

The invention also pertains to a combination comprising the masked IL12 protein or masked subunit according to the invention, and (A) a diene, preferably a tetrazine, if the Trigger is a dienophile, or (B) a dienophile, preferably a dienophile comprising an eight-membered non-aromatic cyclic mono-alkenylene moiety, if the Trigger is a diene . The combination of the invention may be presented as a kit, wherein the masked IL12 protein of the invention and the diene are provided in separate containers.

### Spacers S^{P}

As the skilled person is aware, the specific structure of a spacer used in either a dienophile or diene as described herein does not typically influence whether the payload is released. However, in some cases specific spacers are preferred. For example, if a payload is to be released, the spacer between e.g. the allylic carbon of the eight-membered non-aromatic cyclic mono-alkenylene moiety and the payload is preferably a self-immolative linker. Such a linker, which is typically referred to as L^{C} herein, ensures that upon release of the end of the linker connected to said allylic carbon, a further rearrangement or reaction takes place, after which the payload is decoupled from the linker L^{C}. Below, first spacers in general are discussed, and thereafter the more specific self-immolative linkers.

In general, a spacer S^{P} as used herein is a moiety according to RG2, more preferably any one of the preferred and/or specific embodiments thereof.

Preferably, a spacer S^{P} consists of one or multiple Spacer Units S^{U} arranged linearly and/or branched and may be connected to one or more C^{B} moieties and/or one or more L^{C} or T^{R} moieties. The Spacer may be used to connect C^{B} to one T^{R} (Example A below; with reference to Formula 5a and 5b: f, e, a = 1) or more T^{R} (Example B and C below; with reference to Formula 5a and 5b: f, e = 1, a ≥ 1), but it can also be used to modulate the properties, e.g. pharmacokinetic properties, of the C^{B}-T^{R}-C^{A} conjugate (Example D below; with reference to Formula 5a and 5b: one or more of c,e,g,h ≥ 1). Thus a Spacer unit does not necessarily connect two entities together, it may also be bound to only one component, e.g. the T^{R} or L^{C}. Alternatively, the Spacer may comprise a Spacer Unit linking C^{B} to T^{R} and in addition may comprise another Spacer Unit that is only bound to the Spacer and serves to modulate the properties of the conjugate (Example F below; with reference to Formula 5a and 5b: e ≥ 1). The Spacer may also consist of two different types of S^{U} constructs, e.g. a PEG linked to a peptide, or a PEG linked to an alkylene moiety (Example E below; with reference to Formula 5a and 5b: e ≥ 1). For the sake of clarity, Example B depicts a S^{U} that is branched by using a multivalent branched S^{U} Example C depicts a S^{U} that is branched by using a linear S^{U} polymer, such as a peptide, whose side chain residues serve as conjugation groups. 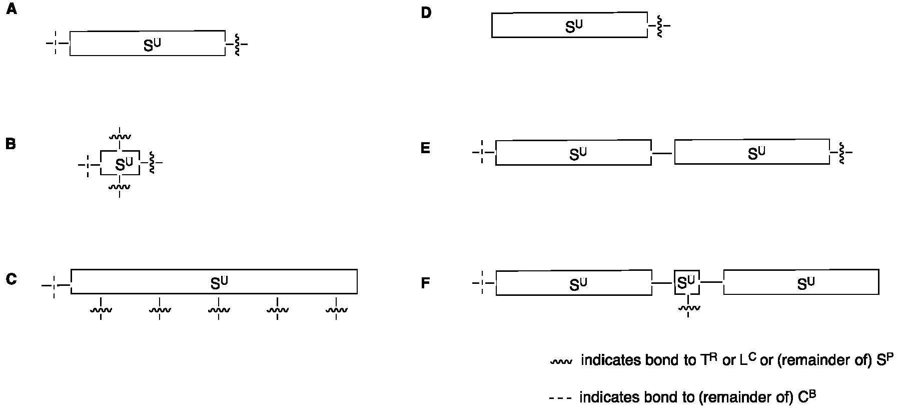 The Spacer may be bound to the Activator in similar designs such as depicted in above examples A- F.

Each individual spacer unit S^{U} may be independently selected from the group of radicals according to RG2. The Spacer Units include but are not limited to amino acids, nucleosides, nucleotides, and biopolymer fragments, such as oligo- or polypeptides, oligo- or polypeptoids, or oligo- or polylactides, or oligo- or poly-carbohydrates, varying from 2 to 200, particularly 2 to 113, preferably 2 to 50, more preferably 2 to 24 and more preferably 2 to 12 repeating units. Preferred biopolymer S^{U} are peptides. Preferably each S^{U} comprises at most 50 carbon atoms, more preferably at most 25 carbon atoms, more preferably at most 10 carbon atoms. In some embodiments the S^{U} is independently selected from the group consisting of (CH₂)ᵣ, (C₃-C₈ carbocyclo), O-(CH₂)ᵣ, arylene, (CH₂)ᵣ-arylene, arylene-(CH₂)ᵣ, (CH₂)ᵣ -(C₃-C₈ carbocyclo), (C₃-C₈ carbocyclo)-(CH₂)ᵣ, (C₃-C₈ heterocyclo), (CH₂)ᵣ -(C₃-C₈ heterocyclo), (C₃-C₈ heterocyclo)-(CH₂)ᵣ, - (CH₂)ᵣC(O)NR'(CH₂)ᵣ, (CH₂CH₂O)ᵣ, (CH₂CH₂O)ᵣCH₂,(CH₂)ᵣC(O)NR'(CH₂ CH₂O)ᵣ, (CH₂)ᵣCCO)NR'(CH₂CH₂O)ᵣCH_{2,} (CH₂CH₂O)ᵣ C(O)NR'(CH₂CH₂O)ᵣ, (CH₂CH₂O)ᵣ C(O)NR'(CH₂CH₂O)ᵣCH₂, (CH₂CH₂O)ᵣC(O)NR'CH₂; wherein r is independently an integer from 1 -10. As used herein, each R'is independently selected from the group consisting of radicals according to RG1. Preferably, R'is hydrogen. Other examples of Spacer Units S^{U} are linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, -OCH₂CH₃, OCH₂CH₂CO₂H. Other polymeric Spacer Units are polymers and copolymers such as poly-(2-oxazoline), poly(N-(2-hydroxypropyl)methacrylamide) (HPMA), polylactic acid (PLA), polylactic-glycolic acid (PLGA), polyglutamic acid (PG), polysarcosine, dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used as S^{U} are cellulose, amylose, dextran, dextrin, levan, fucoidan, carraginan, inulin, pectin, amylopectin, glycogen, lixenan, agarose, hyaluronan, chondroitinsulfate, dermatansulfate, keratansulfate, alginic acid and heparin. In yet other exemplary embodiments, the polymeric S^{U} comprises a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Preferred polymeric S^{U} are PEG, HPMA, PLA, PLGA, PVP, PHF, dextran, oligopeptides, and polypeptides. In some embodiments, polymers used in a S^{U} have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa. Other exemplary S^{U} are dendrimers, such as poly(propylene imine) (PPI) dendrimers, PAMAM dendrimers, and glycol-based dendrimers. The S^{U} of the invention expressly include but are not limited to conjugates prepared with commercially available cross-linker reagents such as BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, sulfo-SMPB, and SVSB, DTME, BMB, BMDB, BMH, BMOE, BM(PEO)₃ and BM(PEO)₄. To construct a branching Spacer one may use a S^{U} based on one or several natural or non-natural amino acids, amino alcohol, aminoaldehyde, or polyamine residues or combinations thereof that collectively provide the required functionality for branching. For example serine has three functional groups, i.e. acid, amino and hydroxyl groups and may be viewed as a combined amino acid an aminoalcohol residue for purpose of acting as a branching S^{U}. Other exemplary amino acids are lysine and tyrosine. In some embodiments, the Spacer consists of one Spacer Unit, therefore in those cases S^{P} equals S^{U}. Preferably the Spacer consists of two, three or four Spacer Units. In some embodiments, S^{P} has a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, or from 500 dalton to 5 kDa. In some embodiments, the S^{P} has a mass of no more than 5000 daltons, no more than 4000 daltons, no more than 3000 daltons, no more than 2000 daltons, no more than 1000 daltons, no more than 800 daltons, no more than 500 daltons, no more than 300 daltons, no more than 200 daltons. In some aspects the S^{P} has a mass from 100 daltons, from 200 daltons, from 300 daltons to 5000 daltons. In some aspects of the S^{P} has a mass from 30, 50, or 100 daltons to 1000 daltons, from about 30, 50, or 100 daltons to 500 daltons.

Preferably, S^{P} comprises a moiety RG2a, RG2b, RG2c, or a residue of RG1f, as described herein. Preferably, said RG2a, RG2b, RG2c, or a residue of RG1f connects the S^{P} to C^{B}, L^{C}, or T^{R}.

### Self-immolative linkers L^{C}

L^{C} is an optional self-immolative linker, which may consist of multiple units arranged linearly and/or branched, and which may be connected to multiple ε-amines of different lysine residues of the masked IL12 protein via multiple bonds C^{A}. By way of further clarification, if r is 0 in relation to Formula (1) or f is 0 in relation to Formula (2), the ε-amine of the at least one lysine residue directly constitutes the leaving group of the release reaction, and if r > 0, the self-immolative linker L^{C} constitutes the leaving group of the release reaction. The possible L^{C} structures, their use, position and ways of attachment of linkers L^{C}, C^{A} and C^{B}, and the T^{R} are known to the skilled person, see for example [Papot et al., Anticancer Agents Med. Chem., 2008, 8, 618-637]. Nevertheless, preferred but non-limiting examples of self-immolative linkers L^{C} are benzyl-derivatives, such as those drawn below. There are two main self-immolation mechanisms: electron cascade elimination and cyclization-mediated elimination. The preferred example below on the left functions by means of the cascade mechanism, wherein the bond between the allylic carbon of the Trigger and the -O- or -S- attached to said carbon is cleaved, and an electron pair of Y^{C1}, for example an electron pair of NR⁶, shifts into the benzyl moiety resulting in an electron cascade and the formation of 4-hydroxybenzyl alcohol, CO₂ and the liberated ε-amine of the at least one lysine residue. The preferred example in the middle functions by means of the cyclization mechanism, wherein cleavage of the bond to the NR⁶ on the side of the Trigger leads to nucleophilic attack of the amine on the carbonyl, forming a 5-ring 1,3-dimethylimidazolidin-2-one and liberating the ε-amine of the at least one lysine residue. The preferred example on the right combines both mechanisms. This linker will degrade not only into CO₂ and one unit of 4-hydroxybenzyl alcohol (when Y^{C1} is O), but also into one 1,3-dimethylimidazolidin-2-one unit. 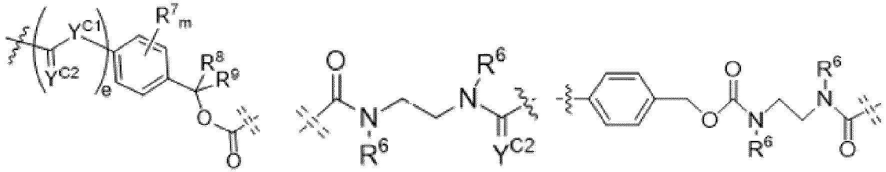 wherein the wiggly line indicates a bond to -O- or -S- on the allylic position of the trans-cyclooctene, and the double dashed line indicates C^{A}.

In relation to the self-immolative linkers, R⁶, R⁷, R⁸, and R⁹ are each independently according to RG1, RG3, RG4, or RG5, preferably RG1.

By substituting the benzyl groups of aforementioned self-immolative linkers L^{C}, it is possible to tune the rate of release of the ε-amine of the at least one lysine residue, caused by either steric and/or electronic effects on the cyclization and/or cascade release. Synthetic procedures to prepare such substituted benzyl-derivatives are known to the skilled person (see for example [Greenwald et al, J. Med. Chem., 1999, 42, 3657-3667] and [Thornthwaite et al, Polym. Chem., 2011, 2, 773-790]. Some preferred substituted benzyl-derivatives with different release rates are drawn below.

Self-immolative linkers that undergo cyclization include but are not limited to substituted and unsubstituted aminobutyric acid amide, appropriately substituted bicyclo[2.2.1] and bicyclo[2.2.2] ring system, 2-aminophenylpropionic acid amides, and trimethyl lock-based linkers, see e.g. [Chem. Biol. 1995, 2, 223], [J. Am. Chem. Soc. 1972, 94, 5815], [J. Org. Chem. 1990, 55, 5867]. Further preferred examples of L^{C} can be found in WO2009017394(A1), US7375078, WO2015038426A1, WO2004043493, Angew. Chem. Int. Ed. 2015, 54, 7492 - 7509.

Preferably the L^{C} has a mass of no more than 1000 daltons, no more than 500 daltons, no more than 400 daltons, no more than 300 daltons, or from 10, 50 or 100 to 1000 daltons, from 10, 50, 100 to 400 daltons, from 10, 50, 100 to 300 daltons, from 10, 50, 100 to 200 daltons, e.g., 10-1000 daltons, such as 50-500 daltons, such as 100 to 400 daltons.

A person skilled in the art will know that one L^{C} may be connected to another L^{C} that is bound to C^{A}, wherein upon reaction of the Activator with the Trigger T^{R}, L^{C}-L^{C}-C^{A} is released from the T^{R}, leading to self-immolative release of both L^{C} moietes and the ε-amine of the at least one lysine residue. With respect to the L^{C} formulas disclosed herein, the L^{C} linking the T^{R} to the other L^{C} then does not release the ε-amine of the at least one lysine residue but an L^{C} that is bound via Y^{C1} and further links to C^{A}. The skilled person will acknowledge that this principle also holds for further linkers L^{C} linked to L^{C}, *e*.*g*. L^{C}-L^{C}-L^{C}-L^{C}-C^{A}.

In all schemes presented below depicting preferred L^{C} structures, it will be understood that in the legend "indicates a bond to C^{A}" should be interpreted as "indicates the bond C^{A}" or "indicates a bond to the ε-amine of the at least one lysine residue", and that moiety (C(Y^{C2})Y^{C1}), if present, should be interpreted as a part of M^{B}, which is not a part of L^{C}.

Preferably, L^{C} is selected from the group consisting of linkers according to Group I, and Group II, as defined in this Section below.

### Linkers according to Group I are

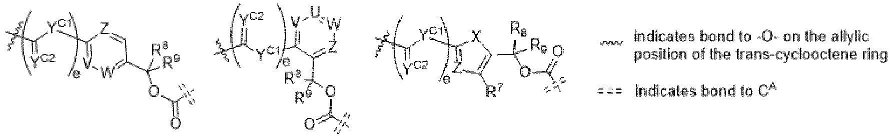

, wherein the wiggly line may also indicate a bond to -S- on the allylic position of the trans-cyclooctene, wherein U, V, W, Z are each independently selected from the group consisting of -CR⁷-, and -N-, wherein e is 0 or 1, wherein X is selected from the group consisting of -O-, -S- and -NR⁶-, wherein preferably each R⁸ and R⁹ are independently selected from the group consisting of hydrogen, C₁-C₄ (hetero)alkyl, C₂-C₄ (hetero)alkenyl, and C₄₋₆ (hetero)aryl; wherein for R⁸ and R⁹ the (hetero)alkyl, (hetero)alkenyl, and (hetero)aryl are optionally substituted with a moiety selected from the group consisting of -Cl, -F, -Br, -I, -OH, -NH₂, =O, -SH, -SO₃H, -PO₃H_{,} -PO₄H₂ and -NO₂ and preferably contain at most two heteroatoms selected from the group consisting of -O-, -S-, -NH-, -P-, and -Si-, wherein the N, S, and P atoms are optionally oxidized. Preferably, for linkers of Group I both R⁸ and R⁹ are hydrogen.

The linker according to Group II is 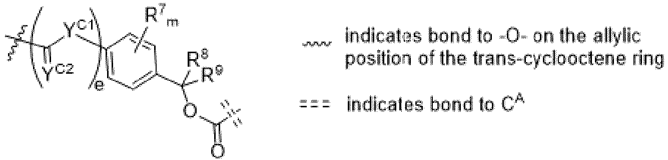
, wherein the wiggly line may also indicate a bond to -S- on the allylic position of the trans-cyclooctene, wherein m is an integer between 0 and 2, preferably m is 0,
wherein e is 0 or 1. Preferably, for linkers of Group II both R⁸ and R⁹ are hydrogen. Preferably, for linkers of Group II R⁷ is methyl or isopropyl. Optionally, R⁶, R⁷, R⁸, R⁹ comprised in said Group I, and II, are -(S^{P})ᵢ-C^{B}.

For all linkers according to Group I and Group II Y^{C1} is selected from the group consisting of -O-, -S-, and -NR⁶-, preferably -NR⁶-. For all linkers according to Group I, and Group II, Y^{C2} is selected from the group consisting of O and S, preferably O.

In another preferred embodiment, L^{C} is selected from the group consisting of linkers according to Group III and Group IV, as defined below in this Section.

Linkers according to Group III are 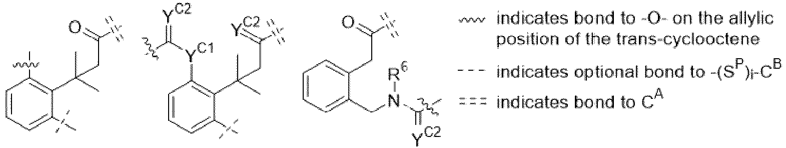 , wherein the wiggly line may also indicate a bond to -S- on the allylic position of the trans-cyclooctene.

Linkers according to Group IV are 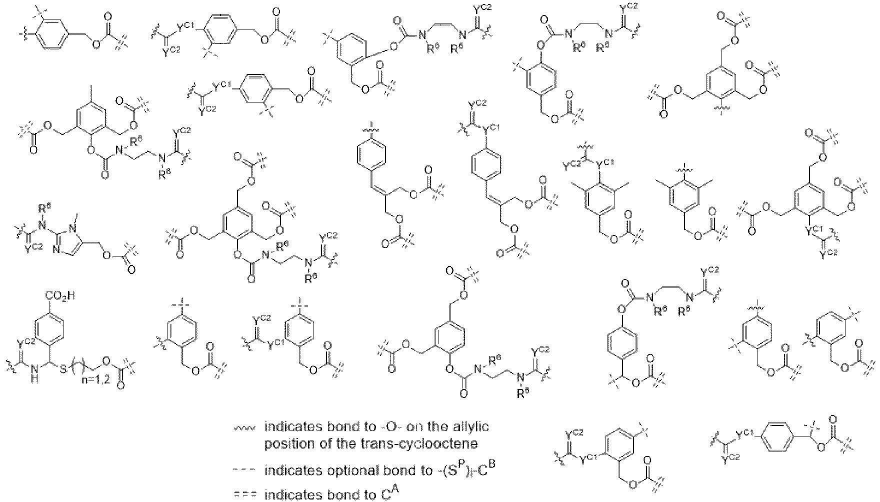 , wherein the wiggly line may also indicate a bond to -S- on the allylic position of the trans-cyclooctene.

For all linkers according to Group III and Group IV, the moiety -C(Y^{C2})-Y^{C1}- should be considered as a bond to M^{B}, or alternatively, that the moiety -C(Y^{C2})-Y^{C1}- is part of M^{B} and not part of L^{C}. For Groups IV-VII preferably R⁶ and R⁷ are as defined in for linkers of Groups I-III, and i and j are independently an integer of from 0 to 4, preferably 1. Preferably, R⁶, R⁷, R⁸, R⁹ are according to RG1 or any preferred embodiment thereof. Preferably, R⁶, R⁷, R⁸, R⁹ used in this Section are not substituted. Most preferably, R⁶, R⁷, R⁸, R⁹ used in this Section are hydrogen.

### Definitions

### General definitions

The present invention will further be described with respect to particular embodiments and with reference to certain drawings but the invention is not limited thereto but only by the claims. Any reference signs in the claims shall not be construed as limiting the scope. The drawings described are only schematic and are non-limiting. In the drawings, the size of some of the elements may be exaggerated and not drawn on scale for illustrative purposes. Where an indefinite or definite article is used when referring to a singular noun e.g. "a" or "an", "the", this includes a plural of that noun unless something else is specifically stated.

As used herein, "TZ" or "Tz" refers to tetrazine.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present invention, the only relevant components of the device are A and B.

The compounds disclosed in this description and in the claims may comprise one or more asymmetric centres, and different diastereomers and/or enantiomers may exist of the compounds. The description of any compound in this description and in the claims is meant to include all diastereomers, and mixtures thereof, unless stated otherwise. In addition, the description of any compound in this description and in the claims is meant to include both the individual enantiomers, as well as any mixture, racemic or otherwise, of the enantiomers, unless stated otherwise. When the structure of a compound is depicted as a specific enantiomer, it is to be understood that the invention of the present application is not limited to that specific enantiomer, unless stated otherwise. When the structure of a compound is depicted as a specific diastereomer, it is to be understood that the invention of the present application is not limited to that specific diastereomer, unless stated otherwise.

The compounds may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer, unless stated otherwise.

The compounds disclosed in this description and in the claims may further exist as exo and endo diastereoisomers. Unless stated otherwise, the description of any compound in the description and in the claims is meant to include both the individual exo and the individual endo diastereoisomers of a compound, as well as mixtures thereof. When the structure of a compound is depicted as a specific endo or exo diastereomer, it is to be understood that the invention of the present application is not limited to that specific endo or exo diastereomer, unless stated otherwise.

Unless stated otherwise, the compounds of the invention and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

In several formulae, groups or substituents are indicated with reference to letters such as "A", "B", "X", "Y", and various (numbered) "R" groups. In addition, the number of repeating units may be referred to with a letter, e.g. n in - (CH₂)ₙ-. The definitions of these letters are to be read with reference to each formula, i.e. in different formulae these letters, each independently, can have different meanings unless indicated otherwise.

In several chemical formulae and texts below reference is made to "alkyl", "heteroalkyl", "aryl", "heteroaryl", "alkenyl", "alkynyl", "alkylene", "alkenylene", "alkynylene", "arylene", "cycloalkyl", "cycloalkenyl", "cycloakynyl", and the like. The number of carbon atoms that these groups have, excluding the carbon atoms comprised in any optional substituents as defined below, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

Unsubstituted alkyl groups have the general formula CₙH₂ₙ₊₁ and may be linear or branched. Optionally, the alkyl groups are substituted by one or more substituents further specified in this document. Examples of alkyl groups include methyl, ethyl, propyl, 2-propyl, t-butyl, 1-hexyl, 1-dodecyl, etc.

A cycloalkyl group is a cyclic alkyl group. Unsubstituted cycloalkyl groups comprise at least three carbon atoms and have the general formula CₙH₂ₙ₋₁. Optionally, the cycloalkyl groups are substituted by one or more substituents further specified in this document. Examples of cycloalkyl groups include cyclopropyl, cyclobutyl, cyclopentyl and cyclohexyl.

An alkenyl group comprises one or more carbon-carbon double bonds, and may be linear or branched. Unsubstituted alkenyl groups comprising one C-C double bond have the general formula CₙH₂ₙ₋₁. Unsubstituted alkenyl groups comprising two C-C double bonds have the general formula CₙH₂ₙ₋₃. An alkenyl group may comprise a terminal carbon-carbon double bond and/or an internal carbon-carbon double bond. A terminal alkenyl group is an alkenyl group wherein a carbon-carbon double bond is located at a terminal position of a carbon chain. An alkenyl group may also comprise two or more carbon-carbon double bonds. Examples of an alkenyl group include ethenyl, propenyl, isopropenyl, t-butenyl, 1,3-butadienyl, 1,3-pentadienyl, etc. Unless stated otherwise, an alkenyl group may optionally be substituted with one or more, independently selected, substituents as defined below.

A cycloalkenyl group is a cyclic alkenyl group. An unsubstituted cycloalkenyl group comprising one double bond has the general formula CₙH₂ₙ₋₃. Optionally, a cycloalkenyl group is substituted by one or more substituents further specified in this document. An example of a cycloalkenyl group is cyclopentenyl.

An alkynyl group comprises one or more carbon-carbon triple bonds, and may be linear or branched. Unsubstituted alkynyl groups comprising one C-C triple bond have the general formula CₙH₂ₙ₋₃. An alkynyl group may comprise a terminal carbon-carbon triple bond and/or an internal carbon-carbon triple bond. A terminal alkynyl group is an alkynyl group wherein a carbon-carbon triple bond is located at a terminal position of a carbon chain. An alkynyl group may also comprise two or more carbon-carbon triple bonds. Unless stated otherwise, an alkynyl group may optionally be substituted with one or more, independently selected, substituents as defined below. Examples of an alkynyl group include ethynyl, propynyl, isopropynyl, t-butynyl, etc.

A cycloalkynyl group is a cyclic alkynyl group. An unsubstituted cycloalkynyl group comprising one triple bond has the general formula CₙH₂ₙ₋₅. Optionally, a cycloalkynyl group is substituted by one or more substituents further specified in this document. An example of a cycloalkynyl group is cyclooctynyl.

An aryl group refers to an aromatic hydrocarbon ring system that comprises six to twenty-four carbon atoms, more preferably six to twelve carbon atoms, and may include monocyclic and polycyclic structures. When the aryl group is a polycyclic structure, it is preferably a bicyclic structure. Optionally, the aryl group may be substituted by one or more substituents further specified in this document. Examples of aryl groups are phenyl and naphthyl. Preferably, an aryl groups is phenyl.

Arylalkyl groups and alkylaryl groups comprise at least seven carbon atoms and may include monocyclic and bicyclic structures. Optionally, the arylalkyl groups and alkylaryl may be substituted by one or more substituents further specified in this document. An arylalkyl group is for example benzyl. An alkylaryl group is for example 4-*tert-*butylphenyl.

Preferably, heteroaryl groups comprise five to sixteen carbon atoms and contain between one to five heteroatoms. Heteroaryl groups comprise at least two carbon atoms (i.e. at least C₂) and one or more heteroatoms N, O, P or S. A heteroaryl group may have a monocyclic or a bicyclic structure. Optionally, the heteroaryl group may be substituted by one or more substituents further specified in this document. Examples of suitable heteroaryl groups include pyridinyl, quinolinyl, pyrimidinyl, pyrazinyl, pyrazolyl, imidazolyl, thiazolyl, pyrrolyl, furanyl, triazolyl, benzofuranyl, indolyl, purinyl, benzoxazolyl, thienyl, phospholyl and oxazolyl.

Heteroarylalkyl groups and alkylheteroaryl groups comprise at least three carbon atoms (i.e. at least C₃) and may include monocyclic and bicyclic structures. Optionally, the heteroaryl groups may be substituted by one or more substituents further specified in this document.

Where an aryl group is denoted as a (hetero)aryl group, the notation is meant to include an aryl group and a heteroaryl group. Similarly, an alkyl(hetero)aryl group is meant to include an alkylaryl group and an alkylheteroaryl group, and (hetero)arylalkyl is meant to include an arylalkyl group and a heteroarylalkyl group. A C₂-C₂₄ (hetero)aryl group is thus to be interpreted as including a C₂-C₂₄ heteroaryl group and a C₆-C₂₄ aryl group. Similarly, a C₃-C₂₄ alkyl(hetero)aryl group is meant to include a C₇-C₂₄ alkylaryl group and a C₃-C₂₄ alkylheteroaryl group, and a C₃-C₂₄ (hetero)arylalkyl is meant to include a C₇-C₂₄ arylalkyl group and a C₃-C₂₄ heteroarylalkyl group.

In general, when (hetero) is placed before a group, it refers to both the variant of the group without the prefix hetero-as well as the group with the prefix hetero-. Herein, the prefix hetero- denotes that the group contains one or more heteroatoms selected from the group consisting of O, N, S, P, and Si. Preferably, the one or more heteroatoms is selected from the group consisting of O, N, S, and P. It will be understood that for any compound containing a heteroatom, the N, S, and P atoms are optionally oxidized and the N atoms are optionally quaternized. Preferably, up to two heteroatoms are consecutive, such as in for example -CH₂-NH-OCH₃ and -CH₂-O-Si(CH₃)₃. More preferably, however, the heteroatoms are not directly bound to one another.

Examples of heteroalkyls include -CH₂CH₂-O-CH₃, -CH₂CH₂-NH-CH₃, -CH₂CH₂-S(O)-CH₃, -CH=CH-O-CH₃, CH₂CH₂-NH₂, CH₂CH₂-SH, -CH₂CH₂-OH, -CH₂CH₂-COOH, -CH₂C(O)H, -C(O)HCH₃, and -Si(CH₃)₃. Preferably, a C₁-C₄ heteroalkyl contains at most 2 heteroatoms.

Herein, it will be understood that when the prefix hetero- is used for combinations of groups, the prefix hetero- only refers to the one group before it is directly placed. For example, heteroarylalkyl denotes the combination of a heteroaryl group and an alkyl group, not the combination of a heteroaryl and a heteroalkyl group.

Herein, the prefix cyclo- denotes that groups are cyclic. It will be understood that when the prefix cyclo- is used for combinations of groups, the prefix cyclo- only refers to the one group before it is directly placed. For example, cycloalkylalkenylene denotes the combination of a cycloalkylene group (see the definition of the suffix -ene below) and an alkenylene group, not the combination of a cycloalkylene and a cycloalkenylene group. In general, when (cyclo) is placed before a group, it refers to both the variant of the group without the prefix cyclo- as well as the group with the prefix cyclo-.

Herein, the suffix -ene denotes divalent groups, i.e. that the group is linked to at least two other moieties. An example of an alkylene is propylene (-CH₂-CH₂-CH₂-), which is linked to another moiety at both termini. It is understood that if a group with the suffix -ene is substituted at one position with -H, then this group is identical to a group without the suffix. For example, an alkylene attached to an -H is identical to an alkyl group. I.e. propylene, -CH₂-CH₂-CH₂-, attached to an-H at one terminus, -CH₂-CH₂-CH₂-H, is logically identical to propyl, -CH₂-CH₂-CH₃.

Herein, when combinations of groups are listed with the suffix -ene, it refers to a divalent group, i.e. that the group is linked to at least two other moieties, wherein each group of the combination contains one linkage to one of these two moieties. As such, for example alkylarylene is understood as a combination of an arylene group and an alkylene group. An example of an alkylarylene group is -phenyl-CH₂-, and an example of an arylalkylene group is -CH₂-phenyl-.

Unless indicated otherwise, a hetero group may contain a heteroatom at non-terminal positions or at one or more terminal positions. In this case, "terminal" refers to the terminal position within the group, and not necessarily to the terminal position of the entire compound. For example, C₂ heteroalkylene may refer to -NH-CH₂-CH₂-, -CH₂-NH-CH₂-, and -CH₂-CH₂-NH-. For example, C₂ heteroalkyl may refer to -NH-CH₂-CH₃, -CH₂-NH-CH₃, and -CH₂-CH₂-NH₂.

Herein, it is understood that cyclic compounds (i.e. aryl, cycloalkyl, cycloalkenyl, etc.) are understood to be monocyclic, polycyclic or branched. It is understood that the number of carbon atoms for cyclic compounds not only refers to the number of carbon atoms in one ring, but that the carbon atoms may be comprised in multiple rings. These rings may be fused to the main ring or substituted onto the main ring. For example, C₁₀ aryl optionally containing heteroatoms may refer to *inter alia* a naphthyl group (fused rings) or to *e.g.* a bipyridyl group (substituted rings, both containing an N atom).

Unless stated otherwise, any group disclosed herein that is not cyclic is understood to be linear or branched. In particular, (hetero)alkyl groups, (hetero)alkenyl groups, (hetero)alkynyl groups, (hetero)alkylene groups, (hetero)alkenylene groups, (hetero)alkynylene groups, and the like are linear or branched, unless stated otherwise.

The general term "sugar" is herein used to indicate a monosaccharide, for example glucose (Glc), galactose (Gal), mannose (Man) and fucose (Fuc). The term "sugar derivative" is herein used to indicate a derivative of a monosaccharide sugar, i.e. a monosaccharide sugar comprising substituents and/or functional groups. Examples of a sugar derivative include amino sugars and sugar acids, e.g. glucosamine (GlcNH₂), galactosamine (GalNH₂) N-acetylglucosamine (GlcNAc), N-acetylgalactosamine (GalNAc), sialic acid (Sia) which is also referred to as N-acetylneuraminic acid (NeuNAc), and N-acetylmuramic acid (MurNAc), glucuronic acid (GlcA) and iduronic acid (ldoA). A sugar may be without further substitution, and then it is understood to be a monosaccharide. A sugar may be further substituted with at one or more of its hydroxyl groups, and then it is understood to be a disaccharide or an oligosaccharide. A disaccharide contains two monosaccharide moieties linked together. An oligosaccharide chain may be linear or branched, and may contain from 3 to 10 monosaccharide moieties.

The term "amino acid" is used herein in its normal scientific meaning. In particular, amino acids in relation to the invention comprise both natural and unnatural amino acids. Preferably, amino acids as used herein are selected from the group consisting of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, valine, azidolysine, beta-alanine (bAla), 4-aminomethyl phenylalanine (Amf), 4-guanidine phenylalanine (Gnf), 4-aminomethyl-N-isopropyl phenylalanine (Iaf), 3-pyridyl alanine (Pya), 4-piperidyl alanine (Ppa), 4-aminomethyl cyclohexyl alanine (Ama), 4-aminocyclohexyl alanine (Aca), ornithine (Orn), citrulline, hydroxylysine (Hyl), allo-hydroxylysine (aHyl), 6-N-methyllysine (MeLys), desmosine (Des), isodesmosine (Ide), 2-aminoadipic acid (Aad), 3-aminoadipic acid (bAad), 2-aminobutyric acid (Abu), 4-aminobutyric acid (4Abu), 6-aminohexonic acid (Acp), 2-aminoheptanoic acid (Ahe), 2-aminoisobutyric acid (Aib), 3-aminoisobutyric acid (bAib), 2-aminopimelic acid (Apm), 2,4-diaminobutyric acid (Dbu), 2,2'-diaminopimelic acid (Dpm), 2-3-diaminopropionic acid (Dpr), N-ethylglycine (EtGly), N-ethylasparagine (EtAsn), 3-hydroxyproline (3Hyp), 4-hydroxyproline (4Hyp), allo-isoleucine (Alle), sarcosine (MeGly), N-methylisoleucine (Melle), N-methylvaline (MeVal), norvaline (Nva), and norleucine (Nle).

The term "protein" is herein used in its normal scientific meaning. Herein, polypeptides comprising about 10 or more amino acids are considered proteins. A protein may comprise natural, but also unnatural amino acids. The term "protein" herein is understood to comprise antibodies and antibody fragments.

The term "peptide" is herein used in its normal scientific meaning. Herein, peptides are considered to comprise a number of amino acids in a range of from 2 to 9.

The term "peptoid" is herein used in its normal scientific meaning.

An antibody is a protein generated by the immune system that is capable of recognizing and binding to a specific antigen. While antibodies or immunoglobulins derived from IgG antibodies are particularly well-suited for use in this invention, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)₂, Fab', Fab'-SH, F(ab')₂, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), DARPINs, chimeric antibodies, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Canc. Gen. Prot. 2013 10, 1-18], and [BioDrugs 2014, 28, 331-343]. "Antibody fragment" refers to at least a portion of the variable region of the immunoglobulin that binds to its target, i.e. the antigen-binding region. Other embodiments use antibody mimetics as Drug D^{D} or Targeting Agent T^{T}, such as but not limited to Affimers, Anticalins, Avimers, Alphabodies, Affibodies, DARPins, and multimers and derivatives thereof; reference is made to [Trends in Biotechnology 2015, 33, 2, 65]. For the avoidance of doubt, in the context of this invention the term "antibody" is meant to encompass all of the antibody variations, fragments, derivatives, fusions, analogs and mimetics outlined in this paragraph, unless specified otherwise. Preferred antibodies in relation to the invention are CC49 and AVP0458. The amino acid sequence of AVP0458 is depicted in Figure 1 (SEQ ID NO: 15).

A spacer is herein defined as a moiety that connects two or more elements of a compound. The terms "spacer" and "linker" are used herein interchangeably. Typically, a spacer is herein denoted as S^{P}, and the more specific self-immolative linkers as L^{C}. It will be understood that when herein, it is stated that "each individual S^{P} is linked at all ends to the remainder of the structure" this refers to the fact that the spacer S^{P} connects multiple moieties within a structure, and therefore the spacer has multiple ends by defintion. The spacer S^{P} may be linked to each individual moiety via different or identical moieties that may be each individually selected. Typically, these linking moieties are to be seen to be part of spacer S^{P} itself. In case the spacer S^{P} links two moieties within a structure, "all ends" should be interpreted as "both ends". As an example, if the spacer connects a trans-cylooctene moiety to a Construct B, then "the remainder of the molecule" refers to the trans-cylooctene moiety and Construct B, while the connecting moieties between the spacer and the trans-cyclooctene moiety and Construct B (i.e. at both ends) may be individually selected.

As used herein, an organic molecule is defined as a molecule comprising a C-H bond. Organic compound and organic molecule are used synonymously.

As used herein, an inorganic molecule is defined as any molecule not being an organic molecule, *i.e.* not comprising a C-H bond. It will be understood that "inorganic molecule" typically also comprises hydrogen, -COOH, etc.

As used herein, a "small molecule" is preferably a small organic molecule. In general, a small molecule has a molecular weight of at most 2 kDa, more preferably at most 1 kDa, more preferably at most 750 Da, more preferably at most 500 Da, and most preferably at most 300 Da. Preferably, a small molecule has a molecular weight of at least 15 Da, more preferably at least 50 Da, more preferably at least 75 Da, and most preferably at least 100 Da.

As used herein, "particle" is preferably defined as a microparticle or a nanoparticle.

A "primary target" as used in the present invention can be any molecule, which is present in an organism, tissue or cell. Preferably, a "primary target" relates to a target for a targeting agent for therapy, imaging, theranostics, diagnostics, or in vitro studies.

The term "salt thereof" means a compound formed when an acidic proton, typically a proton of an acid, is replaced by a cation, such as a metal cation or an organic cation and the like. The term "salt thereof" also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted salt" means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

The unified atomic mass unit or Dalton is herein abbreviated to Da. The skilled person is aware that Dalton is a regular unit for molecular weight and that 1 Da is equivalent to 1 g/mol (grams per mole).

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example,
-((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂- before the individual R₅₁ and R₅₂ moieties are selected, rather than first selecting moieties R₅₁ and R₅₂ and then writing out the formula.

It will be understood that the disclosure is divided into several Sections. It will be understood that the embodiments in each Section can be combined with embodiments from other sections, or in general with any embodiment disclosed herein. In the event that the symbols describing variables (e.g. R-groups, Formula numbers, single letters describing an integer, and the like) in a Section are identical to those of a different Section or another part of the disclosure, it will be understood that said symbols are as defined within the same Section. For example, if Section 2 and Section 3 both describe an R1-group with different definitions, R1 in Section 2 should be interpreted as defined in Section 2. Regardless of these possible identical symbols, it will be understood that the different embodiments between sections may be combined, and the symbols may be redefined (e.g. renumbered) if necessary.

### 7.2 Further definitions

The percentage of similarity of an amino acid sequence, or the term "% sequence similarity", is defined herein as the percentage of residues in a candidate amino acid or nucleic acid sequence that is similar, *i.e.* allowing conservative substitutions, with the residues in a reference sequence after aligning the two sequences and introducing gaps, if necessary, to achieve the maximum percent similarity. The skilled person is well aware of conservative substitions in relation to the IL12 protein, and can readily provide a similarity-scoring matrix without undue burden. Conservative substitutions of amino acids include, but are not limited to, substitutions made amongst amino acids within the following groups: (a) M, I, L, V; (b) F, Y, W; (c) K, R, H; (d) A, G; (e) S, T; (f) Q, N; and (g) E, D.

The percentage of identity of an amino acid sequence, or the term "% sequence identity", is defined herein as the percentage of residues in a candidate amino acid or nucleic acid sequence that is identical with the residues in a reference sequence after aligning the two sequences and introducing gaps, if necessary, to achieve the maximum percent identity. Methods and computer programs for the alignment are well known in the art, for example "Align 2".

It will be understood that herein, for the determination of the sequence identity and the sequence similarity, amino acid residues which are modified, for example because they are coupled to a dienophile or targeting agent as described herein, are generally considered the same as non-modified amino acid residues. As such, the at least one lysine residue having a structure according to Formula (1) will be considered a lysine residue. For example, a sequence MARK with K4 being a lysine residue according to Formula (1) has 100% sequence identity with a sequence MARK wherein K is a non-modified lysine residue.

It will be understood that herein, "IL12" and "IL-12" are used interchangeably.

As used herein, "IL12 protein" refers to an IL12 protein comprising a p35 subunit and a p40 subunit as described herein, *i.e.* the p70 heterodimer. In some embodiments, said subunits are covalently linked as described herein. Preferably, the IL12 protein consists of a p35 subunit and a p40 subunit as described herein. Preferably, the IL12 protein is a human IL12 protein or a mouse IL12 protein, more preferably a human IL12 protein. In particular, the p35 subunit can be a human or a mouse p35 subunit, but a human p35 subunit is preferred. For the p40 subunit, it is also preferred that it is a human p40 subunit.

It will be understood that in the phrase "a masked IL12 protein or a masked subunit thereof" as used herein, said masked subunit relates to a masked p35 subunit or a masked p40 subunit. Such masked subunits are also part of the invention, as they are intermediate products. For example, a masked p35 subunit can be bound to a non-masked p40 subunit to yield a masked IL12 protein, and vice versa.

As used herein, "a (masked) IL12 protein" should be interpreted as "a masked IL12 protein or an IL12 protein". Similarly, "a (masked) subunit" should be interpreted as "a masked subunit or a subunit".

As used herein, "Prodrug" refers to the masked IL12 protein unless indicated otherwise, as the activity of the IL12 protein is significantly reduced by masking, and the masked IL12 protein can be reactivated if desired as described herein.

As used herein, "functionalization grade" is defined as the average number, preferably the exact number, of Trigger moieties attached to a masked IL12 protein or subunit thereof. The Trigger moiety may be connected to a lysine residue of said masked IL12 protein or subunit thereof as shown in Formula (1), but said Trigger moiety may also be connected to another residue, preferably a serine residue and/or a cysteine residue, of said masked IL12 protein or subunit thereof."

### Radical groups (RG)

### Radical Group 1: terminal groups

For Radical Group 1 (RG1), the radical is selected from the group consisting of -H, -Cl, -F, -Br, -I, -OH, -NH₂,-COOH, -CONH₂, -CN, -N₃, -NCS, -SCN, -SO₃H, -PO₃H, -PO₄H₂,-NO₂, -CF₃, -CF₂H, -CFH₂, =O, =NH, -SH, -SO₂H,-(S^{P})ᵢ-C^{B}, (hetero)alkyl, (hetero)alkenyl, (hetero)alkynyl, (hetero)cycloalkyl, (hetero)cycloalkenyl, (hetero)cycloalkynyl, (hetero)aryl, and combinations thereof. Herein, S^{P} is a spacer as defined herein, C^{B} is Construct B as defined herein, and i is an integer in a range of from 0 to 4, preferably i is 0 or 1.

For RG1, "combinations thereof" in particular refers to (hetero)alkylcycloalkyl, (hetero)alkylcycloalkenyl, (hetero)alkylcycloalkynyl, (hetero)cycloalkylalkyl, (hetero)cycloalkenylalkyl, (hetero)cycloalkynylalkyl, (hetero)alkenylcycloalkyl, (hetero)alkenylcycloalkenyl, (hetero)alkenylcycloalkynyl, (hetero)cycloalkylalkenyl, (hetero)cycloalkenylalkenyl, (hetero)cycloalkynylalkenyl, (hetero)alkynylcycloalkyl, (hetero)alkynylcycloalkenyl, (hetero)alkynylcycloalkynyl, (hetero)cycloalkylalkynyl, (hetero)cycloalkenylalkynyl, (hetero)cycloalkynylalkynyl, (hetero)arylalkyl, (hetero)arylalkenyl, (hetero)arylalkynyl, alkyl(hetero)aryl, alkenyl(hetero)aryl, alkynyl(hetero)aryl, cycloalkyl(hetero)aryl, cycloalkenyl(hetero)aryl, cycloalkynyl(hetero)aryl, (hetero)arylcycloalkyl, (hetero)arylcycloalkenyl, and (hetero)arylcycloalkynyl. In addition, "combinations thereof" in relation to RG1 also refers to *e.g.* an alkyl group substituted with one or more -Cl and/or -OH groups. As such, RG1 also comprises radicals such as -NH-CH₂-COOH (a glycine residue), which is a combination of a heteroalkyl and -COOH.

Preferably, for RG1 the radical is selected from the group RGla consisting of -H, -Cl, -F, -Br, -I, -OH, -NH₂,-COOH, -CONH₂, -SO₃H, -PO₃H, -PO₄H₂, -NO₂, -CF₃, =O, =NH, -SH, -(S^{P})ᵢ-C^{B}, C₁-C₂₄ (hetero)alkyl, C₂-C₂₄ (hetero)alkenyl, C₂-C₂₄ (hetero)alkynyl, C₃-C₂₄ cycloalkyl, C₂-C₂₄ heterocycloalkyl, C₅-C₂₄ cycloalkenyl, C₃-C₂₄ heterocycloalkenyl, C₇-C₂₄ cycloalkynyl, C₅-C₂₄ (hetero)cycloalkynyl, C₆-C₂₄ aryl, C₂-C₂₄ heteroaryl, and combinations thereof.

More preferably, for RG1 the radical is selected from the group RG1b consisting of -H, -Cl, -F, -Br, -I, -OH, -NH₂,-COOH, -CONH₂, -SO₃H, -PO₃H, -PO₄H₂, -NO₂, -CF₃, =O, =NH, -SH, -(S^{P})ᵢ-C^{B}, C₁-C₁₂ (hetero)alkyl, C₂-C₁₂ (hetero)alkenyl, C₂-C₁₂ (hetero)alkynyl, C₃-C₁₂ cycloalkyl, C₂-C₁₂ heterocycloalkyl, C₅-C₁₂ cycloalkenyl, C₃-C₁₂ heterocycloalkenyl, C₇-C₁₂ cycloalkynyl, C₅-C₁₂ (hetero)cycloalkynyl, C₆-C₁₂ aryl, C₂-C₁₂ heteroaryl, and combinations thereof.

Even more preferably, for RG1 the radical is selected from the group RG1c consisting of -H, -Cl, -F, -Br, -I, -OH,-NH₂, -COOH, -CONH₂, -SO₃H, -PO₃H, -PO₄H₂, -NO₂, -CF₃, =O, =NH, -SH, -(S^{P})ᵢ-C^{B}, C₁-C₈ (hetero)alkyl, C₂-C₈ (hetero)alkenyl, C₂-C₈ (hetero)alkynyl, C₃-C₈ cycloalkyl, C₂-C₈ heterocycloalkyl, C₅-C₈ cycloalkenyl, C₃-C₈ heterocycloalkenyl, C₇-C₈ cycloalkynyl, C₅-C₈ (hetero)cycloalkynyl, C₆-C₈ aryl, C₂-C₈ heteroaryl, and combinations thereof.

More preferably still, for RG1 the radical is selected from the group RGld consisting of -H, -Cl, -F, -Br, -I, -OH,-NH₂, -COOH, -CONH₂, -SO₃H, -PO₃H, -PO₄H₂, -NO₂, -CF₃, =O, =NH, -SH, -(S^{P})ᵢ-C^{B}, C₁-C₆ (hetero)alkyl, C₂-C₆ (hetero)alkenyl, C₂-C₆ (hetero)alkynyl, C₃-C₆ cycloalkyl, C₂-C₆ heterocycloalkyl, C₅-C₇ cycloalkenyl, C₃-C₅ heterocycloalkenyl, C₈ cycloalkynyl, C₆-C₇ (hetero)cycloalkynyl, phenyl, C₃-C₅ heteroaryl, and combinations thereof.

Most preferably, for RG1 the radical is selected from the group RGle consisting of -H, -Cl, -F, -Br, -I, -OH, -NH₂,-COOH, -CONH₂, -SO₃H, -PO₃H, -PO₄H₂, -NO₂, -CF₃, =O, =NH, -SH, -(S^{P})ᵢ-C^{B}, C₁-C₃ (hetero)alkyl, C₃-C₆ cycloalkyl, C₂-C₅ heterocycloalkyl, phenyl, C₄-C₅ heteroaryl, and combinations thereof.

In some embodiments, for RG1 the radical is a conjugation moiety, which is a chemical group that can be used for binding, conjugation or coupling of a Construct, such as Construct-B, or a Spacer, or another molecule or construct of interest. The person skilled in the art is aware of the myriad of strategies that are available for the chemoselective or -unselective or enzymatic coupling or conjugation of one molecule or construct to another.

In some embodiments, RG1 is a moiety that allows conjugation to a protein comprising natural and/or non-natural amino acids. Moieties suitable for conjugation are known to the skilled person. Conjugation strategies are for example found in [O. Boutureira, G.J.L. Bernardes, Chem. Rev., 2015, 115, 2174-2195].

If RG1 is a conjugation moiety, it is preferably selected from the group RG1f consisting of N-maleimidyl, halogenated N-alkylamido, sulfonyloxy N-alkylamido, vinyl sulfone, (activated) carboxylic acids, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (imino)sydnones, methylsulfonyl phenyloxadiazole, aminooxy, 2-amino benzamidoxime, ethynylphosphonamidates, reactive in the Pictet-Spengler ligation and hydrazine- Pictet-Spengler (HIPS) ligation, DNA intercalators, tetrazine, trans-cyclooctene, and photocrosslinkers. More preferably, RG1f is N-maleimidyl.

In other embodiments RG1f is selected from the group consisting of hydroxyl, amine, halogens, vinyl pyridine, disulfide, pyridyl disulfide, sulfonyloxy, mercaptoacetamide, anhydride, sulfonylated hydroxyacetamido, sulfonyl chlorides, thiosemicarbazone, hydrazine carboxylate, and arylhydrazide. In yet other embodiments RG1f is a group that can be connected to another group by means of an enzyme, for example sortase or Tubulin tyrosine ligase.

### Radical Group 2: connecting groups

For Radical Group 2 (RG2), the radical is selected from the group consisting of (hetero)alkylene, (hetero)alkenylene, (hetero)alkynylene, (hetero)cycloalkylene, (hetero)cycloalkenylene, (hetero)cycloalkynylene, (hetero)arylene, amino acid, peptide, protein, polymer, oligonucleotide, nucleotide, carbohydrate, RG2a, RG2b, RG2c, and combinations thereof.

The radicals from RG2 are optionally attached to one or more radicals according to RG1. Thus, RG2 also covers e.g. -NH-CH(CH₂OH)-C(O)- (*i.e.* a serine residue), which is a heteroalkylene attached to -OH and =O.

For RG2, "combinations thereof" in particular, but not exclusively, refers to alkyl(hetero)arylene, (hetero)arylalkylene, (hetero)arylalkenylene, (hetero)arylalkynylene, alkenyl(hetero)arylene, and alkynyl(hetero)arylene.

Preferably, for RG2 the radical is selected from the group consisting of C₁-C₂₄ (hetero)alkylene, C₂-C₂₄ (hetero)alkenylene, C₂-C₂₄ (hetero)alkynylene, C₃-C₂₄ cycloalkylene, C₂-C₂₄ heterocycloalkylene, C₅-C₂₄ cycloalkenylene, C₃-C₂₄ heterocycloalkenylene, C₇-C₂₄ cycloalkynylene, C₅-C₂₄ (hetero)cycloalkynylene, C₆-C₂₄ arylene, C₂-C₂₄ heteroarylene, amino acid, peptide, protein, polymer, oligonucleotide, nucleotide, carbohydrate, RG2a, RG2b, RG2c, and combinations thereof.

More preferably, for RG2 the radical is selected from the group consisting of C₁-C₁₂ (hetero)alkylene, C₂-C₁₂ (hetero)alkenylene, C₂-C₁₂ (hetero)alkynylene, C₃-C₁₂ cycloalkylene, C₂-C₁₂ heterocycloalkylene, C₅-C₁₂ cycloalkenylene, C₃-C₁₂ heterocycloalkenylene, C₇-C₁₂ cycloalkynylene, C₅-C₁₂ (hetero)cycloalkynylene, C₆-C₁₂ arylene, C₂-C₁₂ heteroarylene, amino acid, peptide, protein, polymer, oligonucleotide, nucleotide, carbohydrate, RG2a, RG2b, RG2c, and combinations thereof.

Even more preferably, for RG2 the radical is selected from the group consisting of C₁-C₈ (hetero)alkylene, C₂-C₈ (hetero)alkenylene, C₂-C₈ (hetero)alkynylene, C₃-C₈ cycloalkylene, C₂-C₈ heterocycloalkylene, C₅-C₈ cycloalkenylene, C₃-C₈ heterocycloalkenylene, C₇-C₈ cycloalkynylene, C₅-C₈ (hetero)cycloalkynylene, C₆-C₈ arylene, C₂-C₈ heteroarylene, amino acid, peptide, protein, polymer, oligonucleotide, nucleotide, carbohydrate, RG2a, RG2b, RG2c, and combinations thereof.

More preferably still, for RG2 the radical is selected from the group consisting of C₁-C₆ (hetero)alkylene, C₂-C₆ (hetero)alkenylene, C₂-C₆ (hetero)alkynylene, C₃-C₆ cycloalkylene, C₂-C₆ heterocycloalkylene, C₅-C₇ cycloalkenylene, C₃-C₅ heterocycloalkenylene, C₈ cycloalkynylene, C₆-C₇ (hetero)cycloalkynylene, phenylene, C₃-C₅ heteroarylene, amino acid, peptide, protein, polymer, oligonucleotide, nucleotide, carbohydrate, RG2a, RG2b, RG2c, and combinations thereof.

Even more preferably still, for RG2 the radical is selected from the group consisting of C₁-C₃ (hetero)alkylene, C₃-C₆ cycloalkylene, C₂-C₅ heterocycloalkylene, phenylene, C₄-C₅ heteroarylene, amino acid, peptide, protein, polymer, oligonucleotide, nucleotide, carbohydrate, RG2a, RG2b, RG2c, and combinations thereof.

RG2a is selected from the group consisting of -O-, -S-, -SS-, -NR₄-, -N=N-, -C(O)-, -C(O)NR₄-, -OC(O)-, -C(O)O-,-OC(O)O-, -OC(O)NR₄-, -NR₄C(O)-, -NR₄C(O)O-, -NR₄C(O)NR₄-, -SC(O)-, -C(O)S-, -SC(O)O-, -OC(O)S-,-SC(O)NR₄-, -NR₄C(O)S-, -S(O)-, -S(O)₂-, -OS(O)₂-, -S(O₂)O-, -OS(O)₂O-, -OS(O)₂NR₄-, -NR₄S(O)₂O-,-C(O)NR₄S(O)NR₄-, -OC(O)NR₄S(O)₂NR₄-, -OS(O)-, -OS(O)O-, -OS(O)NR₄-, -ONR₄C(O)-, -ONR₄C(O)O-,-ONR₄C(O)NR₄-, -NR₄OC(O)-, -NR₄OC(O)O-, -NR₄OC(O)NR₄-, -ONR₄C(S)-, -ONR₄C(S)O-, -ONR₄C(S)NR₄-,-NR₄OC(S)-, -NR₄OC(S)O-, -NR₄OC(S)NR₄-, -OC(S)-, -C(S)O-, -OC(S)O-, -OC(S)NR₄-, -NR₄C(S)-, -NR₄C(S)O-,-SS(O)₂-, -S(O)₂S-, -OS(O₂)S-, -SS(O)₂O-, -NR₄OS(O)-, -NR₄OS(O)O-, -NR₄OS(O)NR₄-, -NR₄OS(O)₂-, -NR₄OS(O)₂O-, -NR₄OS(O)₂NR₄-, -ONR₄S(O)-, -ONR₄S(O)O-, -ONR₄S(O)NR₄-, -ONR₄S(O)₂O-, -ONR₄S(O)₂NR₄-, -ONR₄S(O)₂-,-OP(O)(R₄)₂-, -SP(O)(R₄)₂-, and -NR₄P(O)(R₄)₂-.
Herein, R₄ is according to RG1, preferably R₄ is hydrogen or methyl, more preferably R₄ is hydrogen.

Preferably, RG2a is selected from the group consisting of -O-, -S-, -SS-, -NR₄-, -N=N-, -C(O)-, -C(O)NR₄-, -OC(O)-, -C(O)O-, -OC(O)NR₄-, -NR₄C(O)-, -NR₄C(O)O-, -NR₄C(O)NR₄-, -SC(O)-, -C(O)S-, -SC(O)O-, -OC(O)S-, -SC(O)NR₄-, -NR₄C(O)S-, -S(O)-, -S(O)₂-, -C(O)NR₄S(O)₂NR₄-, -OC(O)NR₄S(O)₂NR₄-, -OC(S)-, -C(S)O-, -OC(S)O-, -OC(S)NR₄-,-NR₄C(S)-, -NR₄C(S)O-, and -SS(O)₂-.

More preferably, for RG2 the radical is RG2b or RG2c, most preferably RG2b.

RG2b is selected from the group consisting of 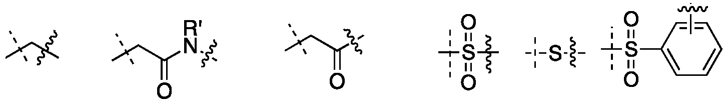 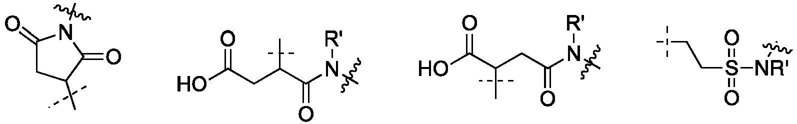 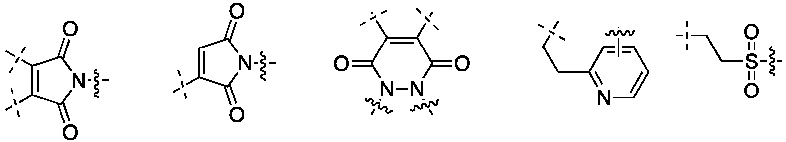 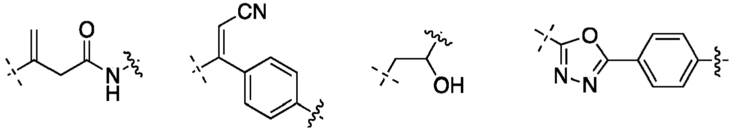 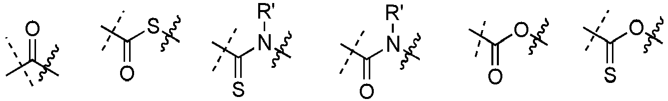 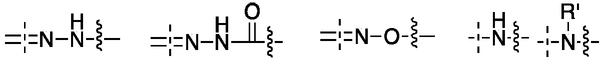 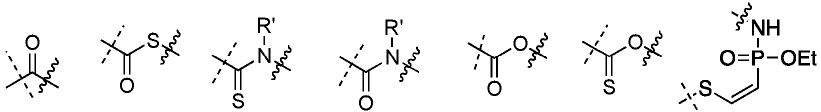 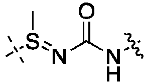 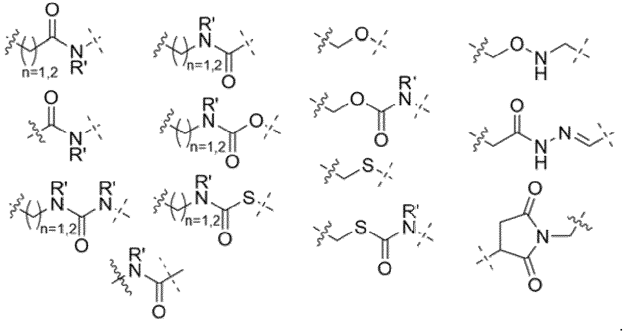 Therein, R' is a radical according to RG1, preferably R' is hydrogen or C₁₋₃ alkyl. The dashed and wiggly lines denote bonds to the other parts of the molecule.

RG2c is selected from the group consisting of 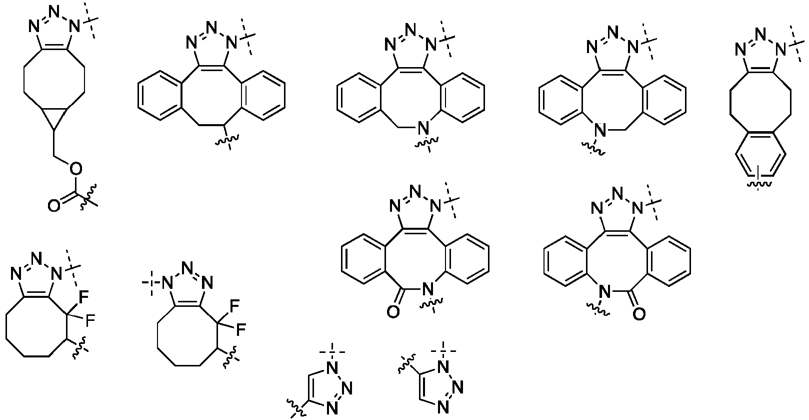 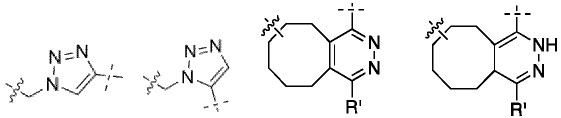 Therein, R' is a radical according to RG1, preferably R' is hydrogen or C₁₋₃ alkyl. The dashed and wiggly lines denote bonds to the other parts of the molecule.

### Radical Group 3: organic molecule

For Radical Group 3 (RG3) the radical is an organic molecule selected from the group consisting of a nucleic acid, a peptide, a protein, a carbohydrate, an aptamer, a hormone, a toxin, a steroid, a cytokine, a lipid, a small organic molecule as defined herein, a polymer, LNA, PNA, an amino acid, a peptoid, a chelating moiety, a molecule comprising a radionuclide, a fluorescent dye, a phosphorescent dye, a drug, a resin, a bead, an organic particle, a gel, an organic surface, an organometallic compound, a cell, and combinations thereof.

Preferably, for RG3 the radical is a a nucleic acid, a peptide, a protein, a carbohydrate, a lipid, a polymer, an amino acid, a chelating moiety, a drug, or a gel.

As used herein, a nucleic acid is preferably selected from the group consisting of a nucleotide, an oligonucleotide, a polynucleotide, DNA, and RNA.

As used herein, a protein is preferably an antibody or a diabody. A preferred antibody is CC49, and a preferred diabody is AVP0458.

As used herein, a carbohydrate is preferably selected from the group consisting of a monosaccharide, an oligosaccharide, and a polysaccharide.

As used herein, a polymer is typically selected from the group consisting of polyethyleneglycol (PEG), poly(N-(2-hydroxypropyl)methacrylamide) (HPMA), polylactic acid (PLA), polylactic-glycolic acid (PLGA), polyglutamic acid (PG), polyvinylpyrrolidone (PVP), poly(1-hydroxymethylethylene hydroxymethyl-formal (PHF), polysarcosine, copolymers of a polyacetal/polyketal and a hydrophilic polymer selected from the group consisting of polyacrylates, polyvinyl polymers, polyesters, polyorthoesters, polyamides, oligopeptides, polypeptides and derivatives thereof, oligopeptides, polypeptides, glycopolysaccharides, and polysaccharides such as dextran and hyaluronan, copolymers thereof, and combinations thereof. Preferably, a polymer as used herein is polyethylene glycol (PEG).

As used herein, a resin is preferably a polystyrene resin or an agarose resin.

As used herein, an organic particle is preferably a liposome or a polymersome.

As used herein, a chelating moiety is preferably selected from the group consisting of DTPA (diethylenetriaminepentaacetic acid), DOTA (1,4,7,10- tetraazacyclododecane-N,N',N",N"-tetraacetic acid), NOTA (1,4,7-triazacyclononane-N,N',N"-triacetic acid), TETA (1,4,8,11-tetraazacyclotetradecane-N,N',N",N'-tetraacetic acid), OTTA (N1-(*p*-isothiocyanatobenzyl)-diethylenetriamine-N₁,N₂,N₃,N₃-tetraacetic acid), deferoxamine or DFA (N'-[5-[[4-[[5-(acetylhydroxyamino)pentyl]amino]-1,4-dioxobutyl]hydroxyamino]pentyl]-N-(5-aminopentyl)-N-hydroxybutanediamide) or HYNIC (hydrazinonicotinamide), EDTA (ethylenediaminetetraacetic acid), OTAM, TACN, sarcophagine, and 3,4-HOPO-based chelators.

More preferably, herein a chelating moiety is selected from the group consisting of 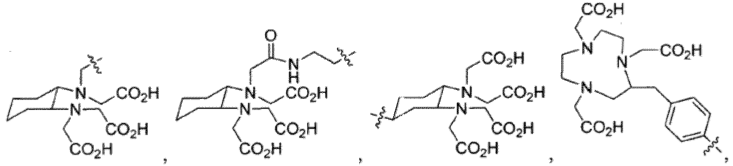 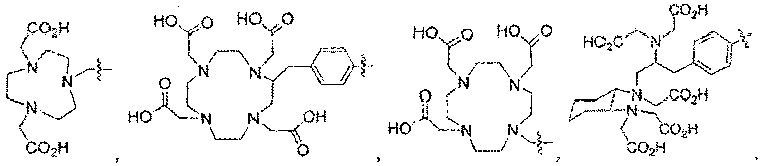 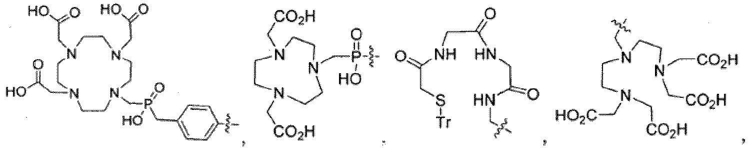 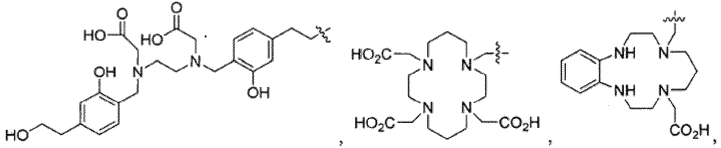 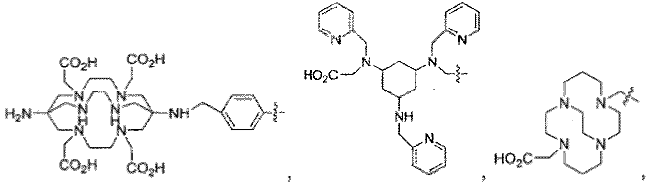 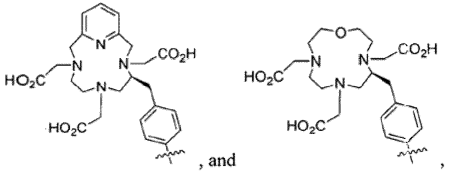 wherein the wiggly line denotes a bond to the remaining part of the molecule, optionally bound via -C(O)NH-, wherein the chelator moieties according to said group optionally chelate a metal, wherein the metal is preferably selected from the group consisting of ⁴⁴Sc,⁶²Cu, ⁶⁴Cu, ⁶⁶Ga, ⁶⁷Ga, ⁶⁷Cu, ⁶⁸Ga, ⁸⁶Y, ⁸⁹Zr, ⁹⁰Y, ^{99m}Tc, ¹¹¹In, ¹⁶⁶Ho, ¹⁷⁷Lu, ¹⁸⁶Re, ¹⁸⁸Re, ²¹¹Bi, ²¹²Bi, ²¹²Pb, ²¹³Bi, ²¹⁴Bi, and ²²⁵Ac.

### Radical Group 4: inorganic molecule

For Radical Group 4 (RG4), the radical is an inorganic molecule selected from the group consisting of an inorganic surface, an inorganic particle, an allotrope of carbon, an inorganic drug, a radionuclide, and combinations thereof.

As used herein, an inorganic surface is preferably selected from the group consisting of chips, wafers, metal such as gold, and silica-based surfaces such as glass.

As used herein, an inorganic particle is preferably selected from the group consisting of beads, silica-based particles, polymer-based materials, and iron oxide particles. Preferably, a bead is a magnetic bead or a gold bead.

As used herein, an allotrope of carbon is preferably selected from the group consisting of fullerenes such as Buckminsterfullerene; graphite, graphene, diamond, Lonsdaleite, Q-carbon, linearn acetylenic carbon, amorphous carbon, and carbon nanotubes.

As used herein, an inorganic drug is preferably cisplatin.

### Radical group 5: further terminal groups

For RG5 the radical is: 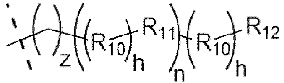 wherein the dashed line indicates a bond to the remaining part of the dienophile or diene.

For RG5, each R₁₀ is independently selected from RG2, preferably from RG2a.

For RG5, each R₁₁ is independently selected from RG2, preferably not being RG2a, RG2b, or RG2c.

For RG5, R₁₂ is selected from RG1, RG3, or RG4, preferably RG3, more preferably a peptide, protein, polymer, or chelating moiety.

Preferably, z is an integer in a range of from 0 to 12, preferably from 0 to 10, more preferably from 0 to 8, even more preferably from 1 to 6, most preferably from 2 to 4. Preferably, z is 0. In case the compound according to the invention comprises more than one moiety RG5, each z is independently selected.

Preferably, h is 0 or 1. In case the compound according to the invention comprises more than one moiety RG5, each h, z, and n is independently selected. Preferably, each n belonging to RG5 is an integer independently selected from a range of from 0 to 24, preferably from 1 to 12, more preferably from 1 to 6, even more preferably from 1 to 3. Preferably, n is 1. In other preferred embodiments n is an integer in the range from 12 to 24.

Preferably, z is 0, and n is 1. In other embodiments, z is 1, and n is 1. Preferably, the moiety RG5 has a molecular weight in a range of from 100 Da to 3000 Da, preferably, in a range of from 100 Da to 2000 Da, more preferably, in a range of from 100 Da to 1500 Da, even more preferably in a range of from 150 Da to 1500 Da. Even more preferably still, the moiety RG5 has a molecular weight in a range of from 150 Da to 1000 Da, most preferably in a range of from 200 Da to 1000 Da.

Preferably, RG5 is selected from the group RG5a consisting of: 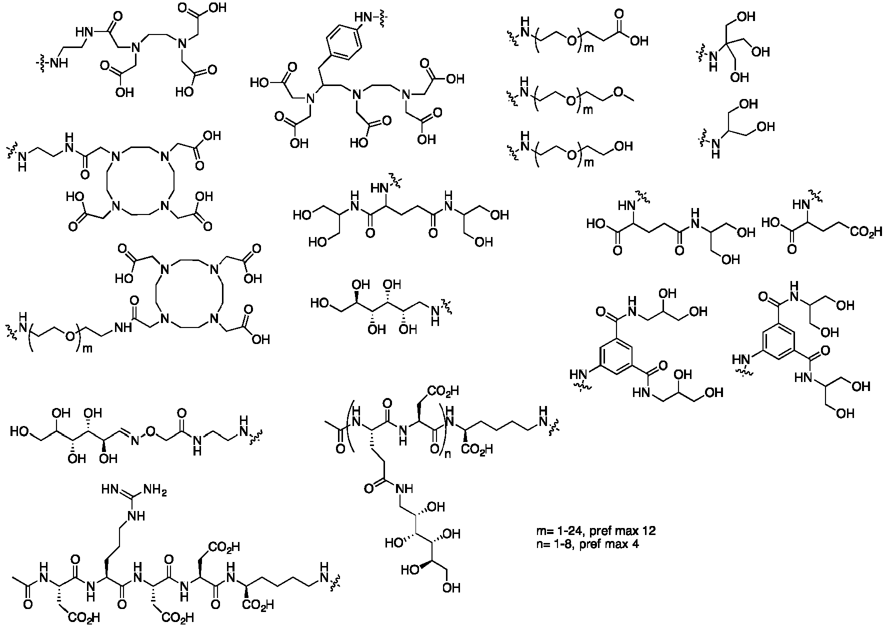 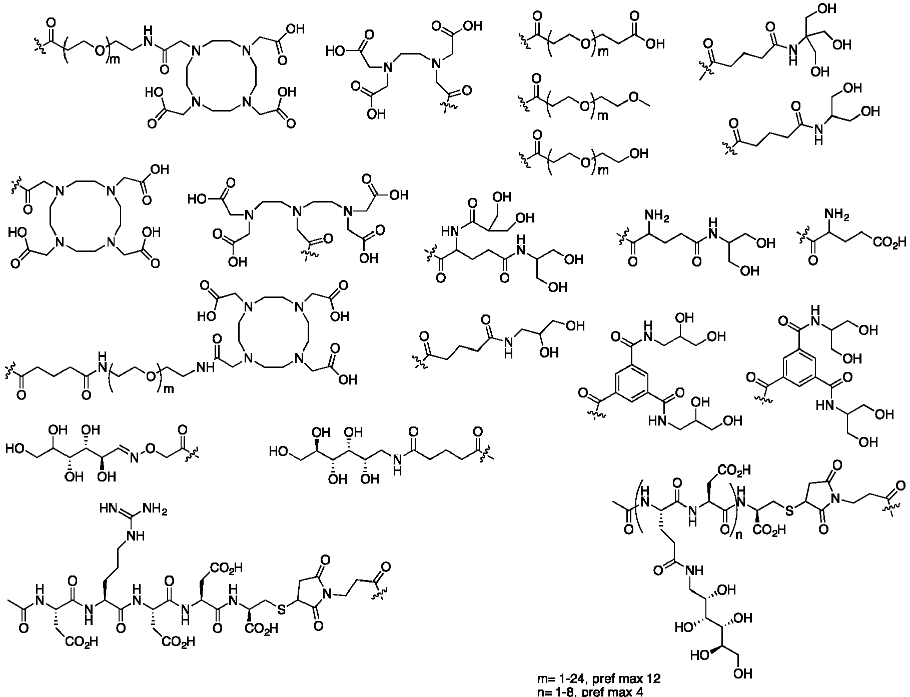 , wherein the wiggly line denotes a bond to the remainder of the molecule.

It is understood that when n is more than 1, -(R₁₀)ₕ-R₁₁)ₙ-(R₁₀)ₕ-R₁₂ may be preceded by a group -(R₁₀)ₕ-R₁₁- so as to form a group -(R₁₀)ₕ-R₁₁-((R₁₀)ₕ-R₁₁)ₙ-(R₁₀)ₕ-R₁₂ . It is understood that this follows from the definition of how to write out the repeating units, i.e. -((R₁₀)ₕ-R₁₁)₂- would first be written as -(R₁₀)ₕ-R₁₁-(R₁₀)ₕ-R₁₁- before R₁₀, h, and R₁₁ are independently selected.

### Targeting Agents T^{T}

A Targeting Agent, T^{T}, binds to a Primary Target. In order to allow specific targeting of the above-listed Primary Targets, the Targeting Agent T^{T} can comprise compounds including but not limited to antibodies, antibody derivatives, antibody fragments, antibody (fragment) fusions (e.g. bi-specific and tri-specific mAb fragments or derivatives), proteins, peptides, e.g. octreotide and derivatives, VIP, MSH, LHRH, chemotactic peptides, cell penetrating peptide, membrane translocation moiety, bombesin, elastin, peptide mimetics, organic compounds, inorganic compounds, carbohydrates, monosaccharides, oligosacharides, polysaccharides, oligonucleotides, aptamers, viruses, whole cells, phage, drugs, polymers, liposomes, chemotherapeutic agents, receptor agonists and antagonists, cytokines, hormones, steroids, toxins. Examples of organic compounds envisaged within the context of the present invention are, or are derived from, dyes, compounds targeting CAIX and PSMA, estrogens, e.g. estradiol, androgens, progestins, corticosteroids, methotrexate, folic acid, and cholesterol. Examples of Targeting Agents of protein nature include insulin, transferrin, fibrinogen-gamma fragment, thrombospondin, claudin, apolipoprotein E, Affibody molecules such as for example ABY-025, Ankyrin repeat proteins, ankyrin-like repeat proteins, interferons, e.g. alpha, beta, and gamma interferon, interleukins, lymphokines, colony stimulating factors and protein growth factor, such as tumor growth factor, e.g. alpha, beta tumor growth factor, platelet-derived growth factor (PDGF), uPAR targeting protein, apolipoprotein, LDL, annexin V, endostatin, and angiostatin. Examples of peptides as targeting agents include LHRH receptor targeting peptides, EC-1 peptide, RGD peptides, HER2-targeting peptides, PSMA targeting peptides, somatostatin-targeting peptides, bombesin. Other examples of targeting agents include lipocalins, such as anticalins. One particular embodiment uses Affibodies^{™} and multimers and derivatives.

In one embodiment antibodies are used as the T^{T}. While antibodies or immunoglobulins derived from IgG antibodies are particularly well-suited for use in this invention, immunoglobulins from any of the classes or subclasses may be selected, e.g. IgG, IgA, IgM, IgD and IgE. Suitably, the immunoglobulin is of the class IgG including but not limited to IgG subclasses (IgG1, 2, 3 and 4) or class IgM which is able to specifically bind to a specific epitope on an antigen. Antibodies can be intact immunoglobulins derived from natural sources or from recombinant sources and can be immunoreactive portions of intact immunoglobulins. Antibodies may exist in a variety of forms including, for example, polyclonal antibodies, monoclonal antibodies, camelized single domain antibodies, recombinant antibodies, anti-idiotype antibodies, multispecific antibodies, antibody fragments, such as, Fv, VHH, Fab, F(ab)₂, Fab', Fab'-SH, F(ab')₂, single chain variable fragment antibodies (scFv), tandem/bis-scFv, Fc, pFc', scFv-Fc, disulfide Fv (dsFv), bispecific antibodies (bc-scFv) such as BiTE antibodies, trispecific antibody derivatives such as tribodies, camelid antibodies, minibodies, nanobodies, resurfaced antibodies, humanized antibodies, fully human antibodies, single domain antibodies (sdAb, also known as Nanobody^{™}), chimeric antibodies, chimeric antibodies comprising at least one human constant region, dual-affinity antibodies such as dual-affinity retargeting proteins (DART^{™}), and multimers and derivatives thereof, such as divalent or multivalent single-chain variable fragments (e.g. di-scFvs, tri-scFvs) including but not limited to minibodies, diabodies, triabodies, tribodies, tetrabodies, and the like, and multivalent antibodies. Reference is made to [Trends in Biotechnology 2015, 33, 2, 65], [Trends Biotechnol. 2012, 30, 575-582], and [Canc. Gen. Prot. 2013 10, 1-18], and [BioDrugs 2014, 28, 331-343]. "Antibody fragment" refers to at least a portion of the variable region of the immunoglobulin that binds to its target, i.e. the antigen-binding region. Other embodiments use antibody mimetics as T^{T}, such as but not limited to Affimers, Anticalins, Avimers, Alphabodies, Affibodies, DARPins, and multimers and derivatives thereof, reference is made to [Trends in Biotechnology 2015, 33, 2, 65]. For the avoidance of doubt, in the context of this invention the term "antibody" is meant to encompass all of the antibody variations, fragments, derivatives, fusions, analogs and mimetics outlined in this paragraph, unless specified otherwise.

Preferably the T^{T} is selected from antibodies and antibody derivatives such as antibody fragments, fragment fusions, proteins, peptides, peptide mimetics, organic molecules, dyes, fluorescent molecules, enzyme substrates.

Preferably the T^{T} being an organic molecule has a molecular weight of less than 2000 Da, more preferably less than 1500 Da, more preferably less than 1000 Da, even more preferably less than 500 Da.

In another preferred embodiment the T^{T} is selected from antibody fragments, fragment fusions, and other antibody derivatives that do not contain a Fc domain.

In another embodiment the T^{T} is a polymer and accumulates at the Primary Target by virtue of the EPR effect. Typical polymers used in this embodiment include but are not limited to polyethyleneglycol (PEG), poly(N-(2-hydroxypropyl)methacrylamide) (HPMA), polylactic acid (PLA), polylactic-glycolic acid (PLGA), polyglutamic acid (PG), polyvinylpyrrolidone (PVP), poly(1-hydroxymethylethylene hydroxymethyl-formal (PHF), polysarcosine. Other examples are copolymers of a polyacetal/polyketal and a hydrophilic polymer selected from the group consisting of polyacrylates, polyvinyl polymers, polyesters, polyorthoesters, polyamides, oligopeptides, polypeptides and derivatives thereof. Other examples are oligopeptides, polypeptides, glycopolysaccharides, and polysaccharides such as dextran and hyaluronan. In addition reference is made to [G. Pasut, F.M. Veronese, Prog. Polym. Sci. 2007, 32, 933-961].

In some embodiments the T^{T} can be a cell penetrating moiety, such as cell penetrating peptide. In other embodiments, the T^{T} is a polymer, particle, gel, biomolecule or another above listed T^{T} moiety and is locally injected to create a local depot of Prodrug, which can subsequently be activated by the Activator. In another embodiment the targeting agent T^{T} is a solid material such as but not limited to polymer, metal, ceramic, wherein this solid material is or is comprised in a cartridge, reservoir, depot, wherein preferably said cartridge, reservoir, depot is used for drug release in vivo. In some embodiments, the targeting agent T^{T} also acts as a Drug, denoted as D^{D}.

### Masking moieties

A Masking Moiety as used herein may also be denoted as M^{M}. For the avoidance of doubt, in the context of this invention wherein a M^{M} is removed from a masked IL12 protein, the unmasked IL 12 protein itself can optionally bind to one or more Primary Targets without the use of an additional Targeting Agent T^{T}. In this context, the Primary target is preferably the therapeutic target.

Masking moieties M^{M} can for example be an antibody, protein, peptide, polymer, polyethylene glycol, polypropylene glycol carbohydrate, aptamers, oligopeptide, oligonucleotide, oligosaccharide, carbohydrate, as well as peptides, peptoids, steroids, organic molecule, or a combination thereof that further shield the bound drug D^{D} or Prodrug. This shielding can be based on e.g. steric hindrance, but it can also be based on a non covalent interaction with the drug D^{D}. Such Masking Moiety may also be used to affect the in vivo properties (e.g. blood clearance; biodistribution, recognition by the immune system) of the drug D^{D} or Prodrug. Preferably the Masking Moiety is an albumin binding moiety. Preferably, the Masking Moiety equals a Targeting Agent. Preferably, the Masking Moiety is bound to a Targeting Agent. Preferably the Drug D^{D}, is modified with multiple M^{M}, being C^{B}, wherein at least one of the bound M^{M} is T^{T}.

Preferably the T^{R} can itself act as a Masking Moiety. For the sake of clarity, in these embodiments the size of the T^{R} without the attachment of a M^{M} is sufficient to deactivate the IL 12 protein.

### Drugs

Drugs D^{D} that can be used in a Prodrug relevant to this invention are pharmaceutically active compounds. Preferably the pharmaceutically active compound is selected from the group consisting of cytotoxins, antiproliferative/antitumor agents, antiviral agents, antibiotics, anti-inflammatory agents, chemosensitizing agents, radio sensitizing agents, immunomodulators, immunosuppressants, immunostimulants, anti-angiogenic factors, and enzyme inhibitors.

Preferably these pharmaceutically active compounds are selected from the group consisting of antibodies, antibody derivatives, antibody fragments, proteins, aptamers, oligopeptides, oligonucleotides, oligosaccharides, carbohydrates, as well as peptides, peptoids, steroids, toxins, hormones, cytokines, and chemokines.

Most preferably, the drug is monomethyl auristatin E.

Preferably these drugs are low to medium molecular weight compounds, preferably organic compounds (e.g. about 200 to about 2500 Da, preferably about 300 to about 1750 Da, more preferably about 300 to about 1000 Da).

Exemplary cytotoxic drug types for use as conjugates to the Trigger and to be released upon IEDDA reaction with the Activator, for example for use in cancer therapy, include but are not limited to DNA damaging agents, DNA crosslinkers, DNA binders, DNA alkylators, DNA intercalators, DNA cleavers, microtubule stabilizing and destabilizing agents, topoisomerases inhibitors, radiation sensitizers, anti-metabolites, natural products and their analogs, peptides, oligonucleotides, enzyme inhibitors such as dihydrofolate reductase inhibitors and thymidylate synthase inhibitors.

Examples include but are not limited to colchinine, vinca alkaloids, anthracyclines (e.g. doxorubicin, epirubicin, idarubicin, daunorubicin), camptothecins, taxanes, taxols, vinblastine, vincristine, vindesine, calicheamycins, tubulysins, tubulysin M, cryptophycins, methotrexate, methopterin, aminopterin, dichloromethotrexate, irinotecans, enediynes, amanitins, deBouganin, dactinomycines, CC1065 and its analogs, duocarmycins, maytansines, maytansinoids, dolastatins, auristatins, pyrrolobenzodiazepines and dimers (PBDs), indolinobenzodiazepines and dimers, pyridinobenzodiazepines and dimers, mitomycins (e.g. mitomycin C, mitomycin A, caminomycin), melphalan, leurosine, leurosideine, actinomycin, tallysomycin, lexitropsins, bleomycins, podophyllotoxins, etoposide, etoposide phosphate, staurosporin, esperamicin, the pteridine family of drugs, SN-38 and its analogs, platinum-based drugs, cytotoxic nucleosides.

Other exemplary drug classes are angiogenesis inhibitors, cell cycle progression inhibitors, P13K/m-TOR/AKT pathway inhibitors, MAPK signaling pathway inhibitors, kinase inhibitors, protein chaperones inhibitors, HDAC inhibitors, PARP inhibitors, Wnt/Hedgehog signaling pathway inhibitors, and RNA polymerase inhibitors.

In some embodiments, the drug is an auristatin. Examples of auristatins include dolastatin 10, monomethyl auristatin E (MMAE), auristatin F, monomethyl auristatin F (MMAF), auristatin F hydroxypropylamide (AF HPA), auristatin F phenylene diamine (AFP), monomethyl auristatin D (MMAD), auristatin PE, auristatin EB, auristatin EFP, auristatin TP and auristatin AQ. MMAE is a preferred auristatin. Suitable auristatins are also described in U.S. Publication Nos. 2003/0083263, 2011/0020343, and 2011/0070248; PCT Application Publication Nos. WO09/117531, WO2005/081711, WO04/010957; WO02/088172 and WO01/24763, and U.S. Patent Nos. 7,498,298; 6,884,869; 6,323,315; 6,239,104; 6,124,431; 6,034,065; 5,780,588; 5,767,237; 5,665,860; 5,663,149; 5,635,483; 5,599,902; 5,554,725; 5,530,097, 5,521,284; 5,504,191; 5,410,024; 5,138,036; 5,076,973; 4,986,988; 4,978,744; 4,879,278; 4,879,278; 4,816,444; and 4,486,414.

Exemplary drugs include the dolastatins and analogues thereof including: dolastatin A (U.S. Pat No. 4,486,414), dolastatin B (U.S. Pat No. 4,486,414), dolastatin 10 (U.S. Pat No. 4,486,444, 5,410,024, 5,504,191, 5,521,284, 5,530,097, 5,599,902, 5,635,483, 5,663,149, 5,665,860, 5,780,588, 6,034,065, 6,323,315), dolastatin 13 (U.S. Pat No. 4,986,988), dolastatin 14 (U.S. Pat No. 5,138,036), dolastatin 15 (U.S. Pat No. 4,879,278), dolastatin 16 (U.S. Pat No. 6,239,104), dolastatin 17 (U.S. Pat No. 6,239,104), and dolastatin 18 (U.S. Pat No. 6,239,104). Exemplary maytansines, maytansinoids, such as DM-1 and DM-4, or maytansinoid analogs, including maytansinol and maytansinol analogs, are described in U.S. Patent Nos. 4,424,219; 4,256,746; 4,294,757; 4,307,016; 4,313,946; 4,315,929; 4,331,598; 4,361,650; 4,362,663; 4,364,866; 4,450,254; 4,322,348; 4,371,533; 5,208,020; 5,416,064; 5,475,092; 5,585,499; 5,846,545; 6,333,410; 6,441,163; 6,716,821 and 7,276,497. Other examples include mertansine and ansamitocin. Pyrrolobenzodiazepines (PBDs), which expressly include dimers and analogs, include but are not limited to those described in [Denny, Exp. Opin. Ther. Patents, 10(4):459-474 (2000)], [Hartley et al., Expert Opin Investig Drugs. 2011, 20(6):733-44], Antonow et al., Chem Rev. 2011, 111(4), 2815-64]. Calicheamicins include, e.g. enediynes, esperamicin, and those described in U.S. Patent Nos. 5,714,586 and 5,739,116. Examples of duocarmycins and analogs include CC1065, duocarmycin SA, duocarmycin A, duocarmycin B1, duocarmycin B2, duocarmycin C1, duocarmycin C2, duocarmycin D, DU-86, KW-2189, adozelesin, bizelesin, carzelesin, seco- adozelesin, CPI, CBI. Other examples include those described in, for example, US Patent No. 5,070,092; 5,101,092; 5,187,186; 5,475,092; 5,595,499; 5,846,545; 6,534,660; 6,548,530; 6,586,618; 6,660,742; 6,756,397; 7,049,316; 7,553,816; 8,815,226; US20150104407; 61/988,011 filed may 2, 2014 and 62/010,972 filed June 11, 2014. Exemplary vinca alkaloids include vincristine, vinblastine, vindesine, and navelbine, and those disclosed in U.S. Publication Nos. 2002/0103136 and 2010/0305149, and in U.S. Patent No. 7,303,749. Exemplary epothilone compounds include epothilone A, B, C, D, E, and F, and derivatives thereof. Suitable epothilone compounds and derivatives thereof are described, for example, in U.S. Patent Nos. 6,956,036; 6,989,450; 6,121,029; 6,117,659; 6,096,757; 6,043,372; 5,969,145; and 5,886,026; and WO97/19086; WO98/08849; WO98/22461; WO98/25929; WO98/38192; WO99/01124; WO99/02514; WO99/03848; WO99/07692; WO99/27890; and WO99/28324. Exemplary cryptophycin compounds are described in U.S. Patent Nos. 6,680,311 and 6,747,021. Exemplary platinum compounds include cisplatin, carboplatin, oxaliplatin, iproplatin, ormaplatin, tetraplatin. Exemplary DNA binding or alkylating drugs include CC-1065 and its analogs, anthracyclines, calicheamicins, dactinomycines, mitromycines, pyrrolobenzodiazepines, indolinobenzodiazepines, pyridinobenzodiazepines and the like. Exemplary microtubule stabilizing and destabilizing agents include taxane compounds, such as paclitaxel, docetaxel, tesetaxel, and carbazitaxel; maytansinoids, auristatins and analogs thereof, vinca alkaloid derivatives, epothilones and cryptophycins. Exemplary topoisomerase inhibitors include camptothecin and camptothecin derivatives, camptothecin analogs and non-natural camptothecins, such as, for example, CPT-11, SN-38, topotecan, 9-aminocamptothecin, rubitecan, gimatecan, karenitecin, silatecan, lurtotecan, exatecan, diflometotecan, belotecan, lurtotecan and S39625. Other camptothecin compounds that can be used in the present invention include those described in, for example, J. Med. Chem., 29:2358-2363 (1986); J. Med. Chem., 23:554 (1980); J. Med Chem., 30:1774 (1987). Angiogenesis inhibitors include, but are not limited to, MetAP2 inhibitors, VEGF inhibitors, PIGF inhibitors, VGFR inhibitors, PDGFR inhibitors, MetAP2 inhibitors. Exemplary VGFR and PDGFR inhibitors include sorafenib, sunitinib and vatalanib. Exemplary MetAP2 inhibitors include fumagillol analogs, meaning compounds that include the fumagillin core structure. Exemplary cell cycle progression inhibitors include CDK inhibitors such as, for example, BMS-387032 and PD0332991; Rho-kinase inhibitors such as, for example, AZD7762; aurora kinase inhibitors such as, for example, AZD1152, MLN8054 and MLN8237; PLK inhibitors such as, for example, BI 2536, BI6727, GSK461364, ON-01910; and KSP inhibitors such as, for example, SB 743921, SB 715992, MK-0731, AZD8477, AZ3146 and ARRY-520. Exemplary P13K/m-TOR/AKT signalling pathway inhibitors include phosphoinositide 3-kinase (P13K) inhibitors, GSK-3 inhibitors, ATM inhibitors, DNA-PK inhibitors and PDK-1 inhibitors. Exemplary P13 kinases are disclosed in U.S. Patent No. 6,608,053, and include BEZ235, BGT226, BKM120, CAL263, demethoxyviridin, GDC-0941, GSK615, IC87114, LY294002, Palomid 529, perifosine, PF-04691502, PX-866, SAR245408, SAR245409, SF1126, Wortmannin, XL147 and XL765. Exemplary AKT inhibitors include, but are not limited to AT7867. Exemplary MAPK signaling pathway inhibitors include MEK, Ras, JNK, B-Raf and p38 MAPK inhibitors. Exemplary MEK inhibitors are disclosed in U.S. Patent No. 7,517,944 and include GDC-0973, GSK1120212, MSC1936369B, AS703026, RO5126766 and RO4987655, PD0325901, AZD6244, AZD8330 and GDC-0973. Exemplary B-raf inhibitors include CDC-0879, PLX-4032, and SB590885. Exemplary B p38 MAPK inhibitors include BIRB 796, LY2228820 and SB 202190. Exemplary receptor tyrosine kinases inhibitors include but are not limited to AEE788 (NVP-AEE 788), BIBW2992 (Afatinib), Lapatinib, Erlotinib (Tarceva), Gefitinib (Iressa), AP24534 (Ponatinib), ABT-869 (linifanib), AZD2171, CHR-258 (Dovitinib), Sunitinib (Sutent), Sorafenib (Nexavar), and Vatalinib. Exemplary protein chaperon inhibitors include HSP90 inhibitors. Exemplary inhibitors include 17AAG derivatives, BIIB021, BIIB028, SNX-5422, NVP-AUY-922 and KW-2478. Exemplary HDAC inhibitors include Belinostat (PR₄₈101), CUDC-101, Droxinostat, ITF2357 (Givinostat, Gavinostat), JNJ-26481585, LAQ824 (NVP-LAQ824, Dacinostat), LBH-589 (Panobinostat), MC1568, MGCD0103 (Mocetinostat), MS-275 (Entinostat), PCI-24781, Pyroxamide (NSC 696085), SB939, Trichostatin A and Vorinostat (SAHA). Exemplary PARP inhibitors include iniparib (BSI 201), olaparib (AZD-2281), ABT-888 (Veliparib), AG014699, CEP9722, MK 4827, KU-0059436 (AZD2281), LT-673, 3-aminobenzamide, A-966492, and AZD2461. Exemplary Wnt/Hedgehog signalling pathway inhibitors include vismodegib, cyclopamine and XAV-939. Exemplary RNA polymerase inhibitors include amatoxins. Exemplary amatoxins include alpha-amanitins, beta amanitins, gamma amanitins, eta amanitins, amanullin, amanullic acid, amanisamide, amanon, and proamanullin. Exemplary immunomodulators are APRIL, cytokines, including IL-2, IL-7, IL-10, IL12, IL-15, IL-21, TNF, interferon gamma, GMCSF, NDV-GMCSF, and agonists and antagonists of STING, agonists and antagonists of TLRs including TLR1/2, TLR3, TLR4, TLR7/8, TLR9, TLR12, agonists and antagonists of GITR, CD3, CD28, CD40, CD74, CTLA4, OX40, PD1, PDL1, RIG, MDA-5, NLRP1, NLRP3, AIM2, IDO, MEK, cGAS, and CD25, NKG2A. Other exemplary drugs include puromycins, topetecan, rhizoxin, echinomycin, combretastatin, netropsin, estramustine, cemadotin, discodermolide, eleutherobin, mitoxantrone, pyrrolobenzimidazoles (PBI), gamma-interferon, Thialanostatin (A) and analogs, CDK11, immunotoxins, comprising e.g. ricin A, diphtheria toxin, cholera toxin. In exemplary embodiments of the invention, the drug moiety is a mytomycin compound, a vinca alkaloid compound, taxol or an analogue, an anthracycline compound, a calicheamicin compound, a maytansinoid compound, an auristatin compound, a duocarmycin compound, SN38 or an analogue, a pyrrolobenzodiazepine compound, a indolinobenzodiazepine compound, a pyridinobenzodiazepine compound, a tubulysin compound, a non-natural camptothecin compound, a DNA binding drug, a kinase inhibitor, a MEK inhibitor, a KSP inhibitor, a P13 kinase inhibitor, a topoisomerase inhibitor, or analogues thereof. In one preferred embodiment the drug is a non-natural camptothecin compound, vinca alkaloid, kinase inhibitor, (e.g. P13 kinase inhibitor: GDC-0941 and PI-103), MEK inhibitor, KSP inhibitor, RNA polymerase inhibitor, PARP inhibitor, docetaxel, paclitaxel, doxorubicin, dolastatin, calicheamicins, SN38, pyrrolobenzodiazepines, pyridinobenzodiazepines, indolinobenzodiazepines, DNA binding drugs, maytansinoids DM1 and DM4, auristatin MMAE, CC1065 and its analogs, camptothecin and its analogs, SN-38 and its analogs.

In another preferred embodiment the drug is selected from DNA binding drugs and microtubule agents, including pyrrolobenzodiazepines, indolinobenzodiazepines, pyridinobenzodiazepines, maytansinoids, maytansines, auristatins, tubulysins, duocarmycins, anthracyclines, taxanes.

In another preferred embodiment the drug is selected from colchinine, vinca alkaloids, tubulysins, irinotecans, an inhibitory peptide, amanitin and deBouganin.

In another preferred embodiment the drug is a radioactive moiety, said moiety comprising a radioactive isotope for radiation therapy. A radionuclide used for therapy is preferably an isotope selected from the group consisting of ²⁴Na, ³²P, ³³P, ⁴⁷Sc, ⁵⁹Fe, ⁶⁷Cu, ⁷⁶As, ⁷⁷As, ⁸⁰Br, ⁸²Br, ⁸⁹Sr, ⁹⁰Nb, ⁹⁰Y, ¹⁰³Ru, ¹⁰⁵Rh, ¹⁰⁹Pd, ¹¹¹Ag, ¹¹¹In, ¹²¹Sn, ¹²⁷Te, ¹³¹I, ¹⁴⁰La, ¹⁴¹Ce, ¹⁴²Pr, ¹⁴³Pr, ¹⁴⁴Pr, ¹⁴⁹Pm, ¹⁴⁹Tb, ¹⁵¹Pm, ¹⁵³Sm, ¹⁵⁹Gd, ¹⁶¹Tb, ¹⁶⁵Dy, ¹⁶⁶Dy, ¹⁶⁶Ho, ¹⁶⁹Er, ¹⁷²Tm, ¹⁷⁵Yb, ¹⁷⁷Lu, ¹⁸⁶Re, ¹⁸⁸Re, ¹⁹⁸Au, ¹⁹⁹Au, ²¹¹At, ²¹¹Bi, ²¹²Bi, ²¹²Pb, ²¹³Bi, ²¹⁴Bi, ²²³Ra, ²²⁴Ra, ²²⁵Ac, and ²²⁷Th. When the radioactive moiety is intended to comprise a metal, such as ¹⁷⁷Lu, such radiometal is preferably provided in the form of a chelate. In such a case the radioactive moiety preferably comprises a structural moiety capable of forming a coordination complex with such a metal. A good example hereof are macrocylic lanthanide(III) chelates derived from 1,4,7,10-tetraazacyclododecane-1,4,7,10-tetraacetic acid (H₄dota). Preferably, the structural moiety capable of forming a coordination complex with such a metal is a chelating moiety as defined herein. In other embodiments the radioactive moiety comprises a prosthetic group (i.e. a phenol) that is bound by a non-metal radionuclide, such as ¹³¹I.

Drugs optionally include a (portion of a) membrane translocation moiety (e.g. adamantine, poly-lysine/arginine, TAT, human lactoferrin) and/or a targeting agent (against e.g. a tumor cell receptor) optionally linked through a stable or labile linker. Exemplary references include: Trends in Biochemical Sciences, 2015,. 40, 12, 749; J. Am. Chem. Soc. 2015, 137, 12153-12160; Pharmaceutical Research, 2007, 24, 11, 1977.

It will further be understood that, in addition to one or more targeting agents (or C^{B}) that may be attached to the Trigger or Linker L^{C} a targeting agent T^{T} may optionally be attached to a drug, optionally via a spacer S^{P}.

Alternatively, it will be further understood that the targeting agent (or C^{B}) may comprise one or more additional drugs which are bound to the targeting agent by other types of linkers, e.g. cleavable by proteases, pH, thiols, or by catabolism.

It will be understood that chemical modifications may also be made to the desired compound in order to make reactions of that compound more convenient for purposes of preparing conjugates of the invention.

Drugs containing an amine functional group for coupling to the Trigger include mitomycin-C, mitomycin-A, daunorubicin, doxorubicin, aminopterin, actinomycin, bleomycin, 9-amino camptothecin, N8-acetyl spermidine, 1-(2 chloroethyl)1,2-dimethanesulfonyl hydrazide, tallysomycin, cytarabine, dolastatins (including auristatins) and derivatives thereof. Drugs containing a hydroxyl function group for coupling to the Trigger include etoposide, camptothecin, taxol, esperamicin, 1,8-dihydroxy-bicyclo[7.3.1]trideca-4-9-diene-2,6-diyne-13-one (U.S. Pat No. 5,198,560), podophyllotoxin, anguidine, vincristine, vinblastine, morpholine-doxorubicin, n-(5,5-diacetoxy-pentyl)doxorubicin, and derivatives thereof. Drugs containing a sulfhydryl functional group for coupling to the Trigger include esperamicin and 6-mecaptopurine, and derivatives thereof.

### Examples

### Example 1 - General methods

All reagents, chemicals, materials and solvents were obtained from commercial sources and were used as received, including nitrile starting compounds that have not been described. IL12 (native human sequence produced in HEK cells) was obtained from PeproTech or WuXi Biologics. Compounds **2.1** and **2.10** were prepared as described in patent WO2020256546. All solvents were of AR quality. [¹¹¹In]Indium chloride and sodium [¹²⁵I]iodide solutions were purchased from Curium and Perkin Elmer, respectively. Sulpho-Cy5-TCO, mPEG24-maleimide, and t-Boc-aminooxy-PEG₂-CH₂CO₂H were purchased from Broadpharm. PEG reagents were purchased at Broadpharm, Creative Pegworks and NOF EUROPE. Compound **1.1** (dioxopyrrolidin-1-yl 2-[(4E)-cyclooct-4-en-1-yloxy]acetate) was prepared as described in Rossin et al., Mol Pharm 2014. Compound **1.2** (2,5-dioxopyrrolidin-1-yl 41,45-dioxo-45-((6-(6-(pyridin-2-yl)-1,2,4,5-tetrazin-3-yl)pyridin-3-yl)amino)-4,7,10,13,16,19,22,25,28,31,34,37-dodecaoxa-40-azapentatetracontan-1-oate) was prepared as described in Rossin et al., J Nucl Med 2013, 54, 1989-1995; compound 10 in article. TCO4-PEG₂-maleimide **1.3,** was purchased from SiChem. Compounds *t*-butyl (4-(6-(1-(((4-nitrophenoxy)carbonyl)oxy)ethyl)-1,2,4,5-tetrazin-3-yl)benzyl)carbamate **(3.1)** and *t*-butyl 4-(6-(1-hydroxyethyl)-1,2,4,5-tetrazin-3-yl)benzylcarbamate **(3.8)** were prepared as described in Van Onzen et al., JACS 2020. Compound **1.4** (TCO-oxymethylacetamide) was prepared as in Rossin et al. Mol. Pharm. 2014. Amicon Ultra centrifugal devices (MW cut-off 10 and 30 kDa) were purchased from Millipore. Analytical thin layer chromatography was performed on Kieselgel F-254 precoated silica plates. Column chromatography was carried out on Screening Devices B.V. silica gel (flash: 40-63 µm mesh; normal: 60-200 µm mesh). ¹H NMR and ¹³C NMR spectra were recorded on a Bruker Avance III HD (400 MHz for ¹H NMR and 100 MHz for ¹³C NMR) spectrometer or a JEOL (500 MHz for ¹H NMR) at 298 K. Chemical shifts are reported in ppm downfield from TMS at rt. Abbreviations used for splitting patterns are s = singlet, d = doublet, dd = double doublet, t = triplet, q = quartet, m = multiplet and br = broad. HPLC-PDA/MS was performed using a Shimadzu LC-10 AD VP series HPLC coupled to a diode array detector (Finnigan Surveyor PDA Plus detector, Thermo Electron Corporation) and an Ion-Trap (LCQ Fleet, Thermo Scientific). HPLC-analyses were performed using a Alltech Alltima HP C₁₈ 3µ column using an injection volume of 1-4 µL, a flow rate of 0.2 mL/min and typically a gradient (5 % to 100 % in 10 min, held at 100 % for a further 3 min) of MeCN in H₂O (both containing 0.1 % formic acid) at 298 K. Size exclusion chromatography (SEC) was performed on an Akta system equipped with a Superdex75 10/300 or a 16/600 (Hi-Load) column (Cytiva) eluted at 0.5-1.0 mL/min with PBS. Radio-HPLC was performed on an Agilent 1200 Infinity system, equipped with a Gabi Star radioactive detector (Raytest). The samples were loaded on an Alltima C₁₈ column (4.6 × 250mm, 5µ; HiChrom), which was eluted at 1 mL/min with a linear gradient of water (A) and MeCN (B) containing 0.1 % TFA v/v% (Gradient 1: 3 to 20 % B in 3 min followed by 20 to 40% B in 26 min; Gradient 2: 3 to 20% B in 4 min followed by 20 to 70% B in 26 min; Gradient 3: 3 to 20% B in 4 min followed by 20 to 40% B in 86 min). Radio-ITLC was performed on ITLC-SG strips (Varian Inc.) eluted with 200 mM EDTA in saline solution (¹¹¹In-labeling) or MeOH/EtOAc 1:1 (¹²⁵I-labeling). The radioactivity distribution on ITLC strips was monitored with a Typhoon FLA 7000 phosphor imager (GE Healthcare Life Science) using the AIDA software. In 200 mM EDTA the ¹¹¹In-labeled tetrazine remains at the base while unbound ¹¹¹In migrates with a R_{f} of 0.9. In MeOH/EtOAc the ¹²⁵I-labeled proteins remain at the based while ¹²⁵I-SHPP (Bolton-Hunter reagent) migrates with a R_{f} 0.5-0.9. SDS-PAGE was performed on a Mini-PROTEAN Tetra Cell system using 4-20% precast Mini-PROTEAN TGX gels and Precision Plus Protein All Blue protein standards (BioRad Laboratories). The gels were stained with Coomassie Brilliant Blue for protein detection. MALDI spectra were recorded on a Bruker Microflex LRF Maldi-tof system, operating in reflective mode. Sinapinic acid was used as matrix. HEK-Blue-IL 12 reporter cells were purchased from Invivogen and were grown according to manufacturer's instructions in DMEM, high glucose, GlutaMAX Supplement, pyruvate (Gibco), under selection pressure. Human IL12-p70 ELISA kits were purchased from ThermoFisher Scientific and used with Nunc Maxisorp 96-well plates. The CC49 full Ig was purified from hybridoma supernatant as described elsewhere (Rossin et al., Nature Commun 2018). The diabody AVP0458 was produced as described elsewhere (Rossin et al. Angew. Chem. Int. Ed. Engl, 2010). Experiments involving animals were performed according to national and European guidelines and approved by the local animal ethics committee.

### Example 2: Synthesis of conjugatable TCO-PEG masking moieties

### (1R,2E,6S)-6-Hydroxy-6-{[2-(2-methoxyethoxy)ethyl]carbamoyl}cyclooct-2-en-1-yl-2,3,4,5,6-pentafluorophenyl carbonate (2.3)

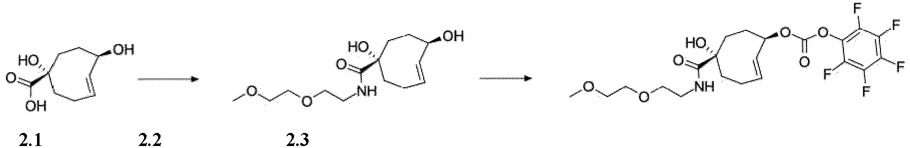

Compound **2.1** (106 mg, 0.57 mmol), mPEG2-amine (136 mg, 1.14 mmol), PyBOP (296 mg, 0.57 mmol) and DiPEA (298 µL, 1.71 mmol) were dissolved in dry MeCN and stirred for 16h. Following concentration, the reaction mixture was applied to a silica column in CH₂Cl₂ and eluted over a CH₂Cl₂:MeOH gradient (100:0 to 96:4). Following concentration, the residue was redissolved in CH₂Cl₂ and dried over MgSO₄. Intermediate **2.2** was isolated after filtration and concentration in 135.2 mg yield (0.47 mmol). Intermediate **2.2** (68 mg, 0.24 mmol), pentafluorophenyl carbonate (274 mg, 0.70 mmol), DMAP (1.5 mg, 12 µmol) and DiPEA (152 µL, 0.87 mmol) were dissolved in dry MeCN (2 mL) and stirred for 2 days at rt. The reaction mixture was concentrated and applied to a C₁₈ reverse phase preparative HPLC column followed by elution using a 5% to 95% gradient of MeCN in H₂O over 40 min yielding **2.3** in 58 mg yield (0.12 mmol) after lyophilization. Analysis **2.2:** ESI-MS calculated for C₁₄H₂₅NO₅: 287.17; found [M+H]⁺ 288.00. ¹H NMR (400 MHz, CD₃OD): δ 6.04-5.97 (m, 1H), 5.74 (dd, *J* = 16.6, 2.5 Hz, 1H), 4.41 (m, 1H), 3.63-3.61 (m, 2H), 3.56-3.53 (m, 4H), 3.39-3.35 (m, 4H), 2.50-2.40 (m, 1H), 2.23-2.03 (m, 4H), 1.93-1.89 (m, 1H), 1.74-1.69 (m, 1H), 1.63-1.59 (m, 1H) ppm. Analysis **2.3** ESI-MS calculated for C₂₁H₂₄F₅NO-: 497.15; found [M+H]⁺ 497.92, [M+Na]⁺ 520.00. ¹H NMR (400 MHz, CDCl₃): δ 6.31 (s, 1H), 6.08-5.93 (m, 1H), 5.79 (dd, *J* = 16.5, 2.3 Hz, 1H), 5.34 (s, 1H), 3.63-3.61 (m, 2H), 3.58-3.53 (m, 5H), 3.49-3.45 (2H), 3.41 (s, 3H), 2.58-2.48 (m, 1H), 2.33-2.22 (m, 2H), 2.06-1.99 (m, 4H), 1.80-1.74 (m, 1H) ppm.

### mPEG₁ₖ-TCO-PFP (2.5)

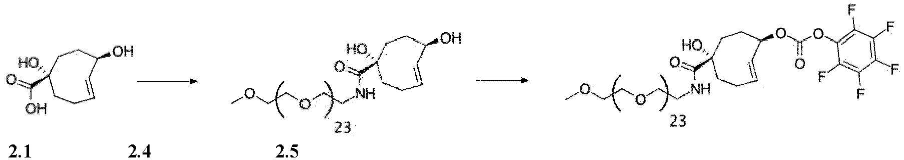

Compound **2.1** (27.5 mg, 148 µmol), mPEG₁ₖ-amine (98.1 mg, ca. 98 µmol), PyBOP (83.4 mg, 160 µmol) and DiPEA (100 µL, 574 µmol) were dissolved in dry MeCN/DMF and stirred overnight at rt. The reaction mixture was concentrated and co-evaporated with MeCN. The residue was applied to a C₁₈ reverse phase preparative HPLC column followed by elution using a 5% to 95% gradient of MeCN in H₂O over 40 min. After lyophilization, the residue was dissolved in CH₂Cl₂ and dried over MgSO₄. Intermediate **2.4** was isolated after filtration and concentration in 71 mg yield (ca. 57 µmol). **2.4** (71 mg, ca. 57 µmol), pentafluorophenyl carbonate (92.1 mg, 234 µmol), DMAP (0.7 mg, 6 µmol) and DiPEA (41 µL, 0.24 mmol) were dissolved in dry MeCN and stirred for 5 days at rt. The reaction mixture was concentrated and applied to a C₁₈ reverse phase preparative HPLC column followed by elution using a 20% to 95% gradient of MeCN in H₂O over 40 min, yielding **2.5** in 7.6 mg yield (ca. 5.2 µmol) after lyophilization. **Analysis 2.4:** ESI-MS calculated for C₅₈H₁₁₃NO₂₇: 1255.75; found [M+H]⁺ 1256.24. ¹H NMR (400 MHz, CDCl₃): δ 6.63 (s, 1H), 6.02 (ddd, *J* = 15.4, 11.3, 3.4 Hz, 1H), 5.75 (dd, *J* = 16.5, 2.3 Hz, 1H), 4.49 (s, 1H), 3.69 - 3.58 (m, ca. 97H), 3.55 - 3.51 (m, 4H), 3.36 (s, 3H), 2.46 (qd, *J* = 11.9, 4.7 Hz, 1H), 2.25 (d, *J* = 12.2 Hz, 1H), 2.12 - 1.89 (m, 5H), 1.74 (dd, *J* = 14.4, 6.0 Hz, 1H), 1.62 (dd, *J =* 14.9, 6.2 Hz, 1H) ppm. **Analysis 2.5:** ESI-MS calculated for C₆₅H₁₁₂F₅NO₂₉: 1465.72; found [M+2H]²⁺ 733.64. ¹H NMR (400 MHz, CDCl₃): δ 6.57 (s, 1H), 6.01 (ddd, *J=* 15.4, 11.3, 3.5 Hz, 1H), 5.78 (dd, *J* = 16.6, 2.3 Hz, 1H), 5.33 (s, 1H), 3.67 - 3.62 (m, ca. 96H), 3.58 - 3.53 (m, 4H), 3.38 (s, 3H), 2.52 (qd, *J =* 12.0, 4.8 Hz, 2H), 2.35 - 2.21 (m, 3H), 2.14 - 1.94 (m, 4H), 1.78 (dd, *J =* 15.7, 5.6 Hz, 1H) ppm.

### mPEG₅ₖ-TCO-PFP (2.7)

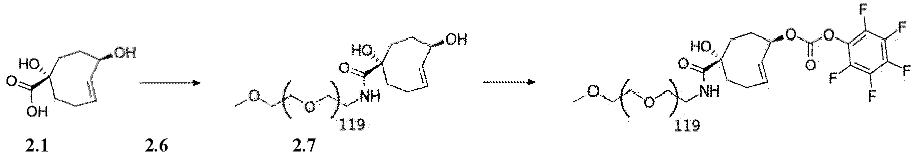

Compound **2.1** (15 mg, 80 µmol), mPEG₅ₖ-amine (200 mg, ca. 40 µmol), PyBOP (31.2 mg, 60 µmol) and DiPEA (34.8 µL, 200 µmol) were dissolved in dry CH₂Cl₂/DMF and agitated for 16h. The reaction mixture was filtered over a plug of cotton and added dropwise to Et₂O (45 mL) in a falcon tube. After spinning and decanting of the supernatant, the residue was washed twice more (2x45 mL Et₂O). The residue was dried under vacuum to yield intermediate **2.6** as a white powder in ca. 200 mg yield (ca. 38 µmol) **2.6,** (112 mg, 21.5 µmol), pentafluorophenyl carbonate (51 mg, 129 µmol), DMAP (0.1 mg, 1 µmol) and DiPEA (15 µL, 86 µmol) were dissolved in dry CH₂Cl₂ (2 mL) and agitated for 16h at rt. The reaction mixture was filtered over a plug of cotton and added dropwise to Et₂O (45 mL) in a falcon tube. After spinning and decanting of the supernatant, the residue was washed twice more (2x45 mL Et₂O). The residue was dried under vacuum to yield **2.7** as a white powder in ca. 81 mg yield (ca. 15 µmol)

### mPEG₁₀ₖ-TCO-PFP (2.9)

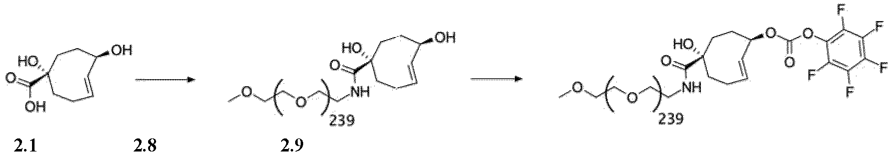

Compound **2.1** (7.5 mg, 40 µmol), mPEG₁₀ₖ-amine (200 mg, ca. 20 µmol), PyBOP (15.6 mg, 30 µmol) and DiPEA (17.4 µL, 100 µmol) were dissolved in dry CH₂Cl₂/DMF and agitated for 16h. The reaction mixture was filtered over a plug of cotton and added dropwise to Et₂O (45 mL) in a falcon tube. After spinning and decanting of the supernatant, the residue was washed twice more (2x45 mL Et₂O). The residue was dissolved in CH₂Cl₂ and dried over MgSO₄. Intermediate **2.8** was isolated after filtration and concentration. **2.8,** (up to 20 µmol), pentafluorophenyl carbonate (95 mg, 240 µmol), DMAP (0.2 mg, 2 µmol) and DiPEA (30 µL, 160 µmol) were dissolved in dry CH₂Cl₂ (2 mL) and agitated for 16h at rt. The reaction mixture was filtered over a plug of cotton and added dropwise to Et₂O (45 mL) in a falcon tube. After spinning and decanting of the supernatant, the residue was washed twice more (2x45 mL Et₂O). The residue was dried under vacuum to yield intermediate **2.9** as a white powder in 145 mg yield (ca. 14 µmol).

### Methyl (1S,4E,6R)-6-({[(2,5-dioxopyrrolidin-1-yl)oxy]carbonyl}oxy)-1-hydroxycyclo oct-4-ene-1-carboxylate (2.11)

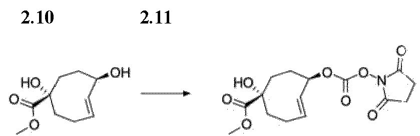

Compound **2.10** (115 mg, 0.57 mmol), N,N'-disuccinimidyl carbonate (560 mg, 2.19 mmol), DMAP (267 mg, 2.19 mmol) were dissolved in dry MeCN by slight heating and the resulting clear reaction mixture was stirred for 6 days at rt. After concentration of the reaction mixture, the residue was mixed with CHCl₃ (25 mL) and gently washed with 20 mM HCl (4x25 mL). The organic phase was dried over MgSO₄ and concentrated to give 169 mg crude **2.11** (**2.11:2.10,** 8:2 mol:mol based on ¹H NMR). Crude **2.11** (50 mg) was applied to a C₁₈ reverse phase preparative HPLC column followed by elution using a 5% to 95% gradient of MeCN in H₂O over 40 min yielding **2.11** in 4.5 mg yield (13.2 µmol) after lyophilization.

### 2,5-Dioxopyrrolidin-1-yl (1R,2E,6S)-6-hydroxy-6-{[2-(2-methoxyethoxy)ethyl] carbamoyl} cyclooct-2-en-1-yl carbonate (2.12)

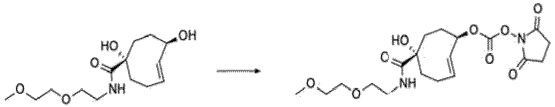

Compound **2.2** (68 mg, 0.24 mmol), N,N'-disuccinimidyl carbonate (178 mg, 0.70 mmol), DMAP (1.5 mg, 12 µmol) and DiPEA (152 µL, 0.87 mmol) were dissolved in dry MeCN and stirred for 2 days at rt. The reaction mixture was concentrated and applied to a C₁₈ reverse phase preparative HPLC column followed by elution using a 5% to 95% gradient of MeCN in H₂O over 40 min yielding **2.12** in 7.8 mg yield (18.2 µmol) after lyophilization. Analysis **2.12** ESI-MS calculated for C₁₉H₂₈N₂O₉: 428.18; found [M+H]⁺ 428.92.

### mPEG₂₀ₖ-TCO-PFP (2.14)

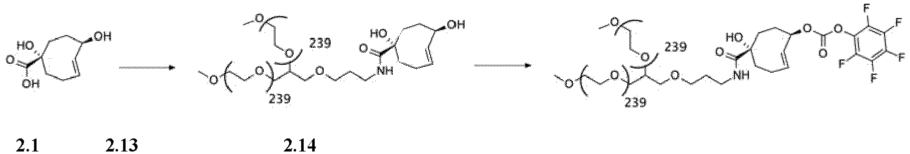

Compound **2.1** (2 eq), mPEG₂₀ₖ-amine (200 mg, ca. 9.8 µmol), PyBOP (1.5 eq) and DiPEA (5 eq) were dissolved in dry CH₂Cl₂ and agitated for 16h. The reaction mixture was filtered over a plug of cotton and added dropwise to Et₂O (45 mL) in a falcon tube. After spinning and decanting of the supernatant, the residue was washed twice more (2x45 mL Et₂O). Intermediate **2.13** was isolated after filtration and drying under vacuum (180 mg, ca. 8.8 µmol). **2.13,** (180 mg, ca. 8.8 µmol), pentafluorophenyl carbonate (10 eq), DMAP (5 eq) and DiPEA (5 eq) were dissolved in dry CH₂Cl₂ (2 mL) and agitated for 5 days at rt. The reaction mixture was filtered over a plug of cotton and added dropwise to Et₂O (45 mL) in a falcon tube. After spinning and decanting of the supernatant, the residue was washed twice more (2x45 mL Et₂O). The residue was dried under vacuum to yield **2.14** as a white powder in 140 mg yield (ca. 6.8 µmol).

### Example 3: Synthesis of conjugatable tetrazine-PEG masking moieties

### 1-{6-[4-({[(Tert-butoxy)carbonyl]amino}methyl)phenyl]-1,2,4,5-tetrazin-3-yl}ethyl 2,5-dioxopyrrolidin-1-yl carbonate (3.2)

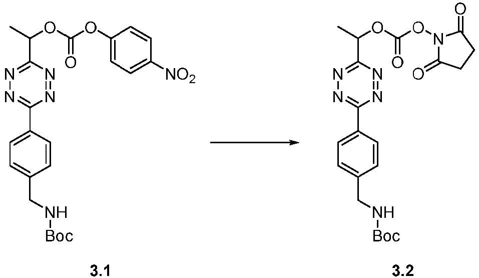

Compound **3.1** (33.1 mg, 66.7 µmol) was dissolved in dry CH₂Cl₂ (2 mL). N-hydroxysuccinimide (68.0 mg, 591 µmol) and DiPEA (0.02 mL, 0.1 mmol) were added and the mixture was stirred overnight at 40°C. The solvent was removed *in vacuo* followed by redissolving in CH₂Cl₂ (15 mL). The organic phase was washed with 0.1 M HCl (3x10 mL), sat. NaHCO₃ (5x10 mL) and brine (10 mL), dried over Na₂SO₄ and filtered. Following concentration, the reaction mixture was applied to a silica column in CH₂Cl₂ and eluted over a CH₂Cl₂:EtOAc gradient (100:0 to 80:20) to afford 3.2 as a pink solid (19.4 mg, 41.1 µmol, 61.6%). **Analysis 3.2:** ESI-MS calculated for C₂₁H₂₄N₆O₇: 472.17; found [M-*t*butyl+2H]⁺ 416.72. ¹H NMR (400 MHz, CDCl₃): δ 8.58 (d, *J* = 8.4 Hz, 2H), 7.50 (d, *J =* 8.3 Hz, 2H), 6.35 (q, *J =* 6.8 Hz, 1H), 5.05 (s, 1H), 4.43 (d, *J* = 6.2 Hz, 2H), 2.82 (s, 4H), 2.00 (d, *J* = 6.8 Hz, 3H), 1.47 (s, 10H) ppm.

### (4-{[(1-{6-[4-({[(Tert-butoxy)carbonyl]amino}methyl)phenyl]-1,2,4,5-tetrazin-3-yl}ethoxy)carbonyl]amino}phenyl)methyl 2,5-dioxopyrrolidin-1-yl carbonate (3.4)

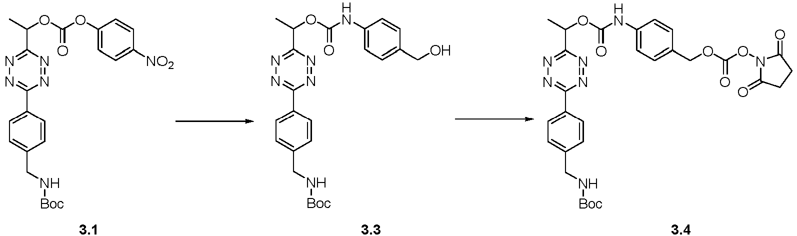

Compound **3.1** (18.8 mg, 37.9 µmol) was dissolved in dry DMF (1.5 mL). (4-Aminophenyl)methanol (15.0 mg, 122 µmol), DiPEA (0.10 mL, 0.57 mmol) and 1-hydroxybenzotriazole hydrate (5.0 mg, 37 µmol) were added and the mixture was stirred overnight at rt in the dark. The solvent was removed *in vacuo* followed by redissolving in CH₂Cl₂ (15 mL). The organic phase is washed with 0.1 M HCl (3x10 mL), sat. NaHCO₃ (4x10 mL) and brine (10 mL), and dried over Na₂SO₄. Intermediate **3.3** was isolated after filtration and concentration as a pink solid (17.0 mg, 35.4 µmol, 93.4%). **3.3** (15.5 mg, 32.3 µmol) and DMAP (8.2 mg, 67 µmol) were dissolved in dry CH₂Cl₂ (1.5 mL). N,N'-Disuccinimidyl carbonate (24.6 mg, 96.0 µmol) was added and the mixture was stirred for 1h at rt. CH₂Cl₂ (15 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂ and eluted over a CH₂Cl₂:EtOAc gradient (80:20 to 60:40) to afford **3.4** as a pink solid (14.2 mg, 22.8 µmol, 70.8%). **Analysis 3.3:** ¹H NMR (400 MHz, CDCl₃): δ 8.54 (d, *J* = 8.4 Hz, 2H), 7.48 (d, *J* = 8.1 Hz, 2H), 7.34 (d, *J =* 8.5 Hz, 2H), 7.26 (d, *J =* 8.5 Hz, 3H), 7.16 (s, 1H), 6.35 (q, *J =* 6.8 Hz, 1H), 5.05 (s, 1H), 4.61 (s, 2H), 4.42 (s, 2H), 1.89 (d, *J =* 6.9 Hz, 3H), 1.47 (s, 10H) ppm. **Analysis 3.4:** ESI-MS calculated for C₂₉H₃₁N₇O₉: 621.22; found [M-*t*boc+2H]⁺ 523.08. ¹H NMR (400 MHz, CDCl₃): δ 8.56 (d, *J =* 8.4 Hz, 2H), 7.50 (d, *J =* 8.1 Hz, 2H), 7.41 (d, *J =* 8.6 Hz, 2H), 7.32 (d, *J =* 8.7 Hz, 2H), 7.15 (s, 1H), 6.36 (q, *J =* 6.8 Hz, 1H), 5.24 (s, 2H), 5.01 (s, 1H), 4.43 (d, *J* = 5.9 Hz, 2H), 2.83 (s, 4H), 1.91 (d, *J* = 6.8 Hz, 3H), 1.48 (s, 9H) ppm.

### 1-{6-[4-({[(Tert-butoxy)carbonyl]amino}methyl)phenyl]-1,2,4,5-tetrazin-3-yl}ethyl N-[4-(hydroxymethyl)phenyl]-N-methylcarbamate (3.6)

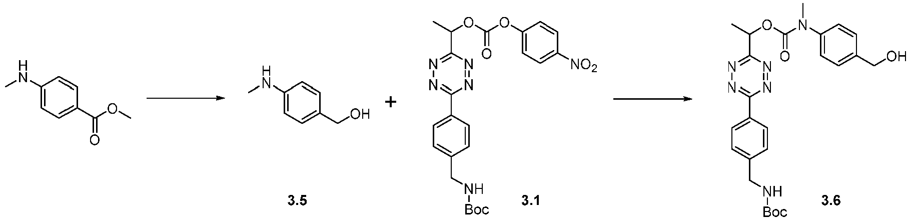

Methyl 4-(methylamino)benzoate (82.6 mg, 500 µmol) was dissolved in dry THF (2.5 mL). LiAlH₄ (38.6 mg, 1.02 mmol) was added and the mixture was stirred overnight at rt. The mixture was cooled to 0°C and quenched by addition of water (5 mL). The reaction was stirred for 40 min, filtered, and the solvent was removed *in vacuo.* The residue was redissolved in CH₂Cl₂ (30 mL), dried over MgSO₄ and filtered. Following concentration, the reaction mixture was applied to a silica column in CH₂Cl₂ and eluted over a CH₂Cl₂:EtOAc gradient (100:0 to 80:20) to afford the intermediate **3.5** as a yellow oil (38.3 mg, 279 µmol, 55.8%). **3.5** (35 mg, 0.26 mmol) was dissolved in CH₂Cl₂/pyridine (9:1 v/v, 1.5 mL). Chlorotrimethylsilane (90 µL, 0.71 mmol) was added and the mixture was stirred for 1h at rt. Subsequently, **3.1** (24 mg, 48 µmol), 1-hydroxybenzotriazole hydrate (2 mg, 0.01 mmol) and DiPEA (90 µL, 0.52 mmol) were added and the mixture was stirred overnight at rt. MeOH/H₂O (1:1 v/v, 4 mL) was added and the mixture was stirred for 30 min at rt. The solvent was removed *in vacuo* followed by redissolving in CH₂Cl₂ (20 mL). The organic phase was washed with water (10 mL), 0.1 M HCl (3x10 mL), sat. NaHCO₃ (5x10 mL) and brine (10 mL), dried over Na₂SO₄ and filtered. Following concentration, the reaction mixture was applied to a silica column in CH₂Cl₂ and eluted over a CH₂Cl₂:EtOAc gradient (100:0 to 75:25) to afford 3.6 as a pink solid (19.3 mg, 39.0 µmol, 81%). **Analysis 3.5:** ¹H NMR (500 MHz, CDCl₃) δ 7.19 (d, *J =* 8.5 Hz, 2H), 6.60 (d, *J =* 8.5 Hz, 2H), 4.53 (s, 2H), 2.83 (s, 3H) ppm. **Analysis 3.6:** ¹H NMR (500 MHz, CDCl₃) δ 8.56 (d, *J =* 8.5 Hz, 2H), 7.49 (d, *J =* 8.0 Hz, 2H), 7.34 (s, 4H), 6.25 (q, *J =* 7.1 Hz, 1H), 5.04 (s, 1H), 4.67 (s, 2H), 4.43 (d, *J =* 6.1 Hz, 2H), 3.34 (s, 3H), 1.79 (s, 3H), 1.48 (s, 9H) ppm.

### (4-{[(1-{6-[4-({[(Tert-butoxy)carbonyl]amino}methyl)phenyl]-1,2,4,5-tetrazin-3-yl}ethoxy)carbonyl](methyl)amino}phenyl)methyl 2,5-dioxopyrrolidin-1-yl carbonate (3.7)

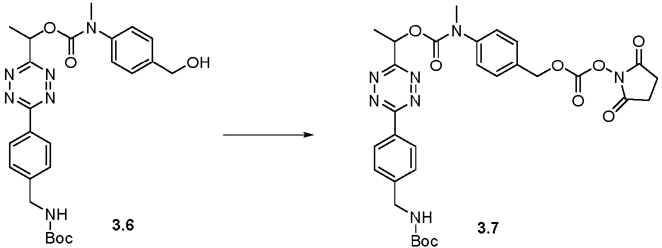

Compound **3.6** (10 mg, 20 µmol) was dissolved in dry CH₂Cl₂ (1 mL). DMAP (8.5 mg, 70 µmol) and N,N'-disuccinimidyl carbonate (20.1 mg, 78.5 µmol) were added and the mixture was stirred for 1h at rt. CH₂Cl₂ (15 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂ and eluted over a CH₂Cl₂:EtOAc gradient (100:0 to 90:10) to afford **3.7** as a pink solid (14.4 mg, 70% purity, 16 µmol, 78%). Analysis 3.7: ¹H NMR (400 MHz, CDCl₃): δ 8.57 (d, *J =* 8.4 Hz, 2H), 7.50 (d, *J =* 8.4 Hz, 2H), 7.40 (s, 3H), 6.26 (q,*J =* 6.9 Hz, 1H), 5.29 (s, 1H), 5.00 (s, 1H), 4.44 (d, *J =* 6.2 Hz, 2H), 3.37 (s, 3H), 2.83 (s, 3H), 1.81 (s, 3H), 1.48 (s, 9H) ppm.

### 1-{6-[4-(Aminomethyl)phenyl]-1,2,4,5-tetrazin-3-yl}ethanol (3.9)

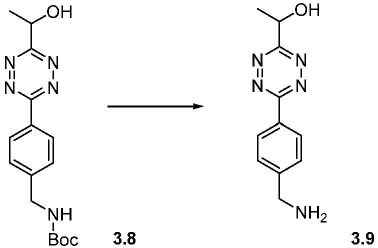

Compound **3.8** (48.0 mg, 145 µmol) was dissolved in CH₂Cl₂ (3 mL). TFA (1 mL) was added and the mixture was stirred for 1h at rt. Following concentration, the reaction mixture was applied to a silica column in CH₂Cl₂ and eluted over a CH₂Cl₂:MeOH gradient (100:0 to 85:15) to afford the TFA salt of **3.9** as a pink solid (36.0 mg, 105 µmol, 72.4%). **Analysis 3.9:** ESI-MS calculated for C₁₁H₁₃N₅O: 231.11; found [M+H]⁺ 231.92. ¹H NMR (500 MHz, CD₃OD): δ 8.64 (d, *J =* 8.3 Hz, 2H), 7.73 (d, *J* = 8.2 Hz, 2H), 5.41 (q, *J =* 6.7 Hz, 1H), 4.28 (s, 2H), 1.76 (d, *J =* 6.7 Hz, 3H) ppm.

### N-({4-[6-(1-Hydroxyethyl)-1,2,4,5-tetrazin-3-yl]phenyl}methyl)-2,5,8,11,14,17,20,23,26,29-decaoxadotriacontan-32-amide (3.10)

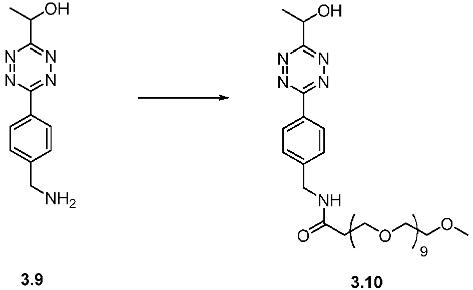

The TFA salt of **3.9** (34.7 mg, 101 µmol) and mPEG₁₀-COOH (74.1 mg, 148 µmol) were dissolved in dry DMF (3 mL) and DiPEA (125 µL, 718 µmol). PyBOP (155 mg, 298 µmol) was added and the mixture was stirred overnight at rt. CH₂Cl₂ (20 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂ and eluted over a CH₂Cl₂:MeOH gradient (100:0 to 95:5) to afford **3.10** as a pink solid (55.5 mg, 80% purity, 62 µmol, 62%). **Analysis 3.10:** ESI-MS calculated for C₃₃H₅₅N₅O₁₂: 713.38; found [M+H]⁺ 714.44. ¹H NMR (500 MHz, CDCl₃): δ 8.52 - 8.45 (m, 2H), 7.45 (d, *J =* 7.9 Hz, 2H), 7.33 (q, *J =* 7.4 Hz, 1H), 5.42 (q, *J =* 6.7 Hz, 1H), 4.51 (d, *J =* 6.0 Hz, 2H), 3.74 (t, *J =* 5.6 Hz, 2H), 3.64 - 3.48 (m, ca. 42H), 3.32 (s, 3H), 2.53 (t, *J =* 5.5 Hz, 2H), 1.77 (qd, *J=* 7.6, 3.3 Hz, 6H) ppm.

### 1-(6-{4-[(2,5,8,11,14,17,20,23,26,29-Decaoxadotriacontan-32-amido)methyl]phenyl}-1,2,4,5-tetrazin-3-yl)ethyl 2,3,4,5,6-pentafluorophenyl carbonate (3.11)

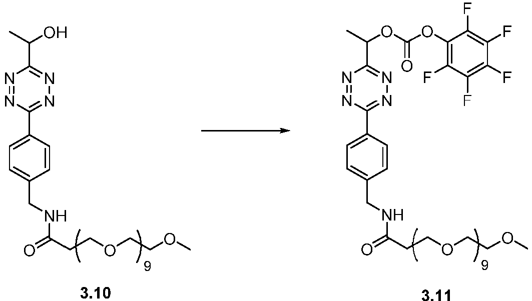

Compound **3.10** (55 mg, 80% purity, 62 µmol) and DMAP (9.6 mg, 79 µmol) were dissolved in dry CH₂Cl₂ (3 mL). Pentafluorophenyl carbonate (124 mg, 315 µmol) was added as a solution in dry CH₂Cl₂ (3 mL) at once, and the mixture was stirred overnight at rt. CH₂Cl₂ (20 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂, washed with EtOAc and eluted over a CH₂Cl₂:MeOH gradient (100:0 to 95:5) to afford 3.11 as a pink solid (29.7 mg, 32.1 µmol, 52%). **Analysis 3.11:** ESI-MS calculated for C₄₀H₅₄F₅N₅O₁₄: 923.36; found [M+H]⁺ 924.44. ¹H NMR (500 MHz, CDCl₃): δ 8.56 (d, *J =* 8.4 Hz, 2H), 7.53 (d, *J =* 8.4 Hz, 2H), 7.30 (t, *J =* 6.1 Hz, 1H), 6.35 (q, *J =* 6.8 Hz, 1H), 4.57 (d, *J =* 6.0 Hz, 2H), 3.78 (t, *J =* 5.6 Hz, 2H), 3.66 - 3.51 (m, ca. 37H), 3.35 (s, 3H), 2.56 (t, *J =* 5.6 Hz, 2H), 2.01 (d, *J =* 6.8 Hz, 3H) ppm.

### 1-{6-[4-(Aminomethyl)phenyl]-1,2,4,5-tetrazin-3-yl}ethyl N-[4-(hydroxymethyl)phenyl]-N-methylcarbamate (3.12)

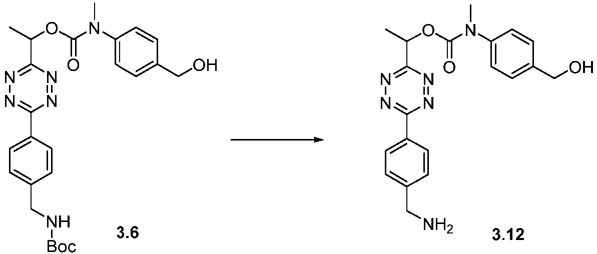

Compound **3.6** (19 mg, 38 µmol) was dissolved in CH₂Cl₂ (1.5 mL). TFA (0.5 mL) was added and the mixture was stirred for 1h at rt. Following concentration, the reaction mixture was applied to a silica column in CH₂Cl₂ and eluted over a CH₂Cl₂:MeOH gradient (100:0 to 80:20) to afford the TFA salt of **3.12** as a pink solid (9.0 mg, 18 µmol, 46%). **Analysis 3.12:** ¹H NMR (500 MHz, CD₃OD): δ 8.74 - 8.63 (m, 2H), 7.79 - 7.71 (m, 2H), 7.39 (d, *J* = 14.7 Hz, 4H), 6.20 (d, *J =* 12.2 Hz, 1H), 4.62 (d, *J =* 18.4 Hz, 2H), 4.29 (d, *J =* 18.5 Hz, 2H), 3.34 (s, 3H), 1.76 (s, 3H) ppm.

### 1-(6-{4-[(2,5,8,11,14,17,20,23,26,29-Decaoxadotriacontan-32-amido)methyl]phenyl}-1,2,4,5-tetrazin-3-yl)ethyl N-[4-(hydroxymethyl)phenyl]-N-methylcarbamate (3.13)

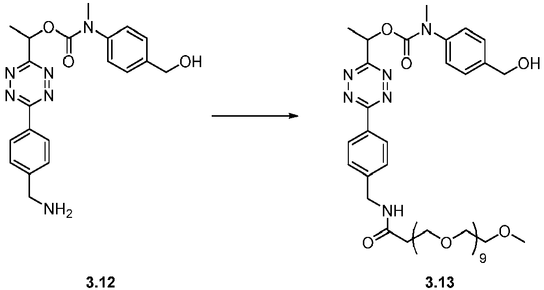

The TFA salt of **3.12** (9.0 mg, 18 µmol) and mPEG₁₀-COOH (21.5 mg, 42.9 µmol) were dissolved in dry DMF (2 mL) and DiPEA (30 µL, 0.17 mmol). PyBOP (52 mg, 0.10 mmol) was added and the mixture was stirred overnight at rt. CH₂Cl₂ (15 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂ and eluted over a CH₂Cl₂:MeOH gradient (100:0 to 90:10) to afford **3.13** as a pink solid (8.4 mg, 90% purity, 8.6 µmol, 49%). **Analysis 3.13**: ¹H NMR (500 MHz, CDCl₃): δ 8.54 (d, *J =* 6.9 Hz, 2H), 7.52 (d, *J =* 7.4 Hz, 2H), 7.34 (d, *J =* 3.7 Hz, 4H), 6.25 (q, *J =* 6.8 Hz, 1H), 4.67 (s, 2H), 4.58 (d, *J =* 5.4 Hz, 2H), 3.79 (t, *J =* 5.2 Hz, 2H), 3.67 - 3.51 (m, ca. 51H), 3.37 (dd, *J =* 4.2, 0.8 Hz, 6H), 2.63 - 2.57 (m, 2H), 1.80 (s, 3H) ppm.

### [4-({[1-(6-{4-[(2,5,8,11,14,17,20,23,26,29-Decaoxadotriacontan-32-amido)methyljphenyl}-1,2,4,5-tetrazin-3-yl)ethoxy]carbonyl}(methyl)amino)phenyl]methyl 2,3,4,5,6-pentafluorophenyl carbonate (3.14)

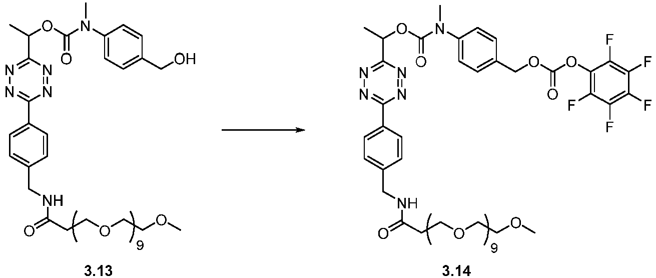

Compound **3.13** (8.2 mg, 90% purity, 8.4 µmol) was dissolved in dry CH₂Cl₂ (1.5 mL). DMAP (5.1 mg, 42 µmol) and pentafluorophenyl carbonate (17.7 mg, 44.9 µmol) were added and the mixture was stirred for 1h at rt. Pentafluorophenyl carbonate (13.8 mg, 35.0 µmol) was added and the mixture was stirred for 1h at rt. CH₂Cl₂ (15 mL) was added and the organic phase was washed with 0.1 M HCl (3x10 mL), sat. NaHCO₃ (3x10 mL) and brine (10 mL), dried over Na₂SO₄ and filtered. Following concentration, the reaction mixture was applied to a silica column in CH₂Cl₂ and eluted over a CH₂Cl₂:MeOH gradient (100:0 to 90:10) to afford **3.14** as a pink solid (6.4 mg, 5.9 nmol, 70%). **Analysis 3.14:** ESI-MS calculated for C₄₉H₆₃F₅N₆O₁₆: 1086.42; found [M+H]⁺ 1087.32. ¹H NMR (500 MHz, CDCl₃): δ 8.56 (d, *J =* 8.4 Hz, 2H), 7.69 (s, 1H), 7.53 (d, *J =* 8.5 Hz, 2H), 7.42 (s, 4H), 6.27 (q, *J =* 6.8 Hz, 1H), 5.31 (s, 2H), 4.59 (d, *J =* 5.4 Hz, 2H), 3.81 (t, *J =* 5.6 Hz, 2H), 3.67 - 3.53 (m, ca. 40H), 3.37 (s, 6H), 2.65 (t, *J* = 5.6 Hz, 2H), 1.82 (s, 3H) ppm.

### Example 4 - Synthesis of conjugatable TCO-keto moieties

### (1R,2E,6S)-6-{[(4-Acetylphenyl)methyl]carbamoyl}-6-hydroxycyclooct-2-en-1-yl 2,3,4,5,6-pentafluorophenyl carbonate (4.2)

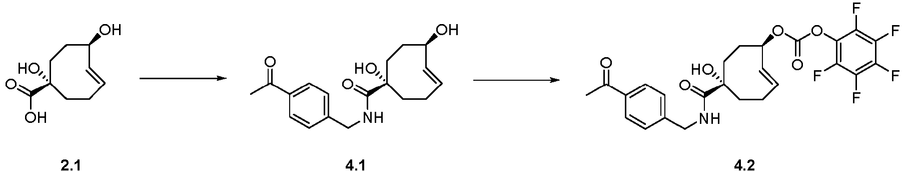

Compound **2.1** (76.1 mg, 409 µmol) was suspended in dry MeCN (4 mL). 1-[4-(aminomethyl)phenyl]ethan-1-one HCl salt (77.2 mg, 416 µmol), DiPEA (0.40 mL, 2.3 mmol) and PyBOP (255.6 mg, 491.2 µmol) were added and the mixture was stirred for 5h at rt. Following concentration, the reaction mixture was applied to a silica column in CH₂Cl₂ and eluted over a CH₂Cl₂:MeOH gradient (100:0 to 90:10) to give the intermediate **4.1** as a yellow solid (199.6 mg, 40% purity, 0.25 mmol, 62%). **4.1** (195 mg, 40% purity, 0.25 mmol) was dissolved in dry CH₂Cl₂ (4 mL). Pentafluorophenyl carbonate (304 mg, 770 µmol) and DiPEA (0.17 mL, 0.98 mmol) were added and the mixture was stirred overnight at rt. Following concentration, the reaction mixture was applied to a silica column in CH₂Cl₂ and eluted over a CH₂Cl₂:EtOAc gradient (100:0 to 70:30) to afford **4.2** as a white solid (75.0 mg, 142 µmol, 57%). **Analysis 4.1:** ¹H NMR (400 MHz, CDCl₃): δ 7.90 (t, *J =* 6.1 Hz, 1H), 7.82 (d, *J =* 8.3 Hz, 2H), 7.32 (d, *J =* 8.2 Hz, 2H), 6.00 (ddd, *J =* 14.9, 11.4, 2.8 Hz, 1H), 5.72 (dd, *J* = 16.4, 2.3 Hz, 1H), 4.77 (s, 1H), 4.46 - 4.30 (m, 3H), 2.53 (s, 3H), 2.44 (ddd, *J =* 13.4, 11.0, 3.5 Hz, 1H), 2.21 - 2.00 (m, 4H), 1.94 (dd, *J* = 12.3, 4.6 Hz, 1H), 1.66 - 1.58 (m, 1H), 1.41 - 1.33 (m, 1H) ppm. **Analysis 4.2:** ESI-MS calculated for C₂₅H₂₂F₅NO₆: 527.14; found [M+H]⁺ 527.72. ¹H NMR (400 MHz, CDCl₃): δ 7.84 (d, *J =* 8.3 Hz, 2H), 7.29 (d, *J =* 8.2 Hz, 2H), 6.85 (t, *J =* 6.0 Hz, 1H), 5.99 (ddd, *J* = 15.5, 11.2, 3.4 Hz, 1H), 5.74 (dd, *J* = 16.5, 2.3 Hz, 1H), 5.31 (s, 1H), 4.52 - 4.38 (m, 2H), 3.57 (s, 1H), 2.55 (s, 3H), 2.48 (td, *J =* 12.0, 4.7 Hz, 1H), 2.32 (d, *J* = 12.1 Hz, 1H), 2.29 - 2.13 (m, 2H), 2.09 - 1.99 (m, 4H), 1.81 (dd, *J* = 15.5, 5.6 Hz, 1H) ppm.

### Tert-butyl N-(26-{[(4-acetylphenyl)methyl]carbamoyl}-3,6,9,12,15,18,21,24-octaoxahexacosan-1-yl)carbamate (4.3)

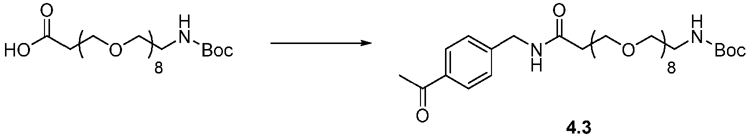

Boc-NH-PEG₈-COOH (50 mg, 92 µmol) was dissolved in dry DMF (2 mL) and DiPEA (0.10 mL, 0.57 mmol). PyBOP (85.7 mg, 165 µmol) was added and the mixture was stirred for 30 min at rt. 1-[4-(aminomethyl)phenyl]ethan-1-one HCl salt (28.6 mg, 154 µmol) was added and the mixture was stirred overnight at rt. Following concentration, the reaction mixture was applied to a silica column in CH₂Cl₂ and eluted over a CH₂Cl₂:MeOH gradient (100:0 to 90:10) to afford **4.3** as a thick oil (157 mg, 35% purity, 82 µmol, 88%). **Analysis 4.3:** ESI-MS calculated for C₃₃H₅₆N₂O₁₂: 672.38; found [M+H]⁺ 672.96. ¹H NMR (500 MHz, CDCl₃): δ 7.91 (d, *J =* 8.3 Hz, 2H), 7.73 (t, *J* = 5.9 Hz, 1H), 7.41 (d, *J* = 8.1 Hz, 2H), 5.38 (s, 1H), 4.50 (d, *J =* 6.1 Hz, 2H), 3.79 (t, *J =* 5.9 Hz, 2H), 3.71 - 3.59 (m, ca. 37H), 3.55 (t, *J =* 5.2 Hz, 2H), 3.30 (q, *J =* 5.5 Hz, 2H), 2.60 (d, *J =* 2.8 Hz, 5H), 1.45 (s, 10H) ppm.

### N-[(4-Acetylphenyl)methyl]-1-{[(1S,4E,6R)-1,6-dihydroxycyclooct-4-en-1-yl]formamido}-3,6,9,12,15,18,21,24-octaoxaheptacosan-27-amide (4.5)

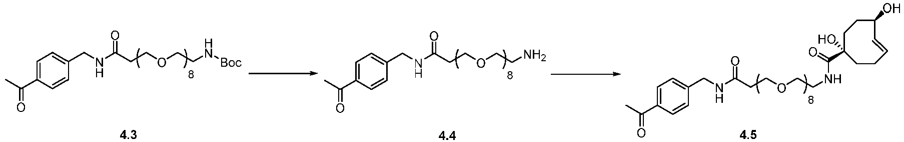

Compound **4.3** (155 mg, 35% purity, 81 µmol) was dissolved in CH₂Cl₂ (3 mL). TFA (1 mL) was added and the mixture was stirred for 3 h at rt. Following concentration, the reaction mixture was applied to a silica column in CH₂Cl₂ and eluted over a CH₂Cl₂:MeOH gradient (100:0 to 80:20). Following concentration, the residue was precipitated with Et₂O, the supernatant was decanted and the residue was dried *in vacuo* to give the TFA salt of intermediate **4.4** as a colourless oil. **2.1** (18.4 mg, 98.8 µmol) was dissolved in dry DMF (1 mL) and DiPEA (0.10 mL, 0.57 mmol). PyBOP (51.9 mg, 99.7 µmol) was added and the mixture was stirred for 30 min at rt. **4.4** in dry DMF (2 mL) was added and the mixture was stirred for 2.5 days at rt. The reaction mixture was concentrated and applied to a C₁₈ reverse phase preparative HPLC column followed by elution using a 5% to 95% gradient of MeCN in H₂O over 40 min. After lyophilization, the residue was dissolved in CH₂Cl₂ and dried over MgSO₄. **4.5** was isolated after filtration and concentration in 19.8 mg yield (26.7 µmol, 33% over 2 steps). **Analysis 4.4:** ¹H NMR (500 MHz, CD₃OD): δ 7.96 (d, *J* = 8.3 Hz, 2H), 7.43 (d, *J =* 8.1 Hz, 2H), 4.47 (s, 2H), 3.80 - 3.59 (m, ca. 41H), 2.59 (s, 3H), 2.55 (t, *J* = 6.0 Hz, 2H) ppm. **Analysis 4.5:** ESI-MS calculated for C₃₇H₆₀N₂O₁₃: 740.41; found [M+H]⁺ 741.44. ¹H NMR (500 MHz, CDCl₃): δ 7.89 (dt, *J* = 8.3, 1.8 Hz, 2H), 7.38 (d, *J* = 8.3 Hz, 2H), 7.34 (s, 1H), 6.81 (s, 1H), 6.00 (ddd, *J =* 15.6, 11.4, 3.3 Hz, 1H), 5.74 (dd, *J =* 16.5, 2.2 Hz, 1H), 4.52 - 4.45 (m, 3H), 3.76 (t, *J* = 5.7 Hz, 2H), 3.67 - 3.49 (m, ca. 32H), 3.43 - 3.37 (m, 2H), 2.59 - 2.52 (m, 5H), 2.45 (qd, *J* = 11.9, 4.5 Hz, 1H), 2.25 - 2.19 (m, 1H), 2.10 - 1.89 (m, 4H), 1.72 (dd, *J* = 14.4, 6.0 Hz, 1H), 1.61 (dd, *J* = 15.0, 6.2 Hz, 1H) ppm.

### (1R,2E,6S)-6-[(26-{[(4-Acetylphenyl)methyl]carbamoyl}-3,6,9,12,15,18,21,24-octaoxahexacosan-1-yl)carbamoyl]-6-hydroxycyclooct-2-en-1-yl 2,3,4,5,6-pentafluorophenyl carbonate (4.6)

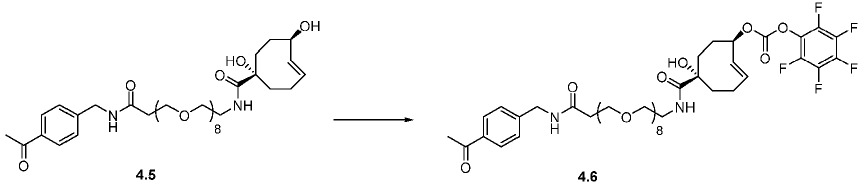

Compound **4.5** (19.5 mg, 26.3 µmol) was dissolved in dry CH₂Cl₂ (2 mL) and DiPEA (30 µL, 0.17 mmol). Pentafluorophenyl carbonate (49.6 mg, 126 µmol) was added and the mixture was stirred overnight at rt. Pentafluorophenyl carbonate (30.1 mg, 76.4 µmol) was added and the mixture was stirred for 2 h at rt. Following concentration, the reaction mixture was applied to a silica column in CH₂Cl₂ and eluted over a CH₂Cl₂:MeOH gradient (100:0 to 90:10) to afford **4.6** as a colorless oil (11.1 mg, 11.7 µmol, 44.3%). **Analysis 4.6:** ESI-MS calculated for C₄₄H₅₉F₅N₂O₁₅: 950.38; found [M+H]⁺ 951.20. ¹H NMR (500 MHz, CDCl₃): δ 7.90 (d, *J =* 8.3 Hz, 2H), 7.39 (t, *J =* 7.5 Hz, 3H), 6.90 (s, 1H), 5.99 (ddd, *J* = 15.4, 11.2, 3.4 Hz, 1H), 5.77 (dd, *J =* 16.6, 2.3 Hz, 1H), 5.31 (s, 1H), 4.51 (d, *J =* 6.0 Hz, 2H), 3.77 (t, *J =* 5.6 Hz, 2H), 3.66 - 3.51 (m, ca. 31H), 3.50 - 3.35 (m, 2H), 2.58 (d, *J* = 6.2 Hz, 5H), 2.50 (qd, *J =* 11.9, 4.6 Hz, 2H), 2.32 - 2.20 (m, 2H), 2.15 (td, *J* = 13.5, 4.6 Hz, 1H), 2.05 - 1.95 (m, 3H), 1.78 (dd, *J* = 15.5, 5.7 Hz, 1H) ppm.

### Example 5 - Synthesis of conjugatable hydroxylamine-peptide moieties

Four peptides (5.1-5.4) inclusive of an aminooxy moiety for oxime ligation purposes and a peptide sequence based on reported binding to fibronectin EDB and/or Tenascin C, both targetable structures present in the extracellular matrix in tumors, were synthesized (See table 1).

**Table 1: Structure information targeting peptides (HA denotes hydroxylamine).**

| ***Nr.*** | ***AA sequence*** | ***Orientation*** | ***Binding to*** | ***Reference*** |
|---|---|---|---|---|
| **5.1** | HA-PEG₂-PPRRGLIKLKTS (Peptide 1) | Linear | Tenascin-C & Fibronectin EDB | Lingasamy et al.; 2019 |
| **5.2** | HA-PEG₂-FHKHKSPALSPV (Peptide 2) | Linear | Tenascin C | Kim et al.; 2012 |
| **5.3** | HA-PEG₂-[KTVRTSADE-NH₂] (Peptide 3) | Cyclized via side chain of K and E | Fibronectin EDB | Park et al.; 2019 |
| **5.4** | CSSPIQGSWTWENGK[PEG₂-HA]WTWGIIRLEQ (Peptide 4) | Bipodal aptide | Fibronectin EDB | Saw et al., 2021 |

For all peptides, the aminooxy moiety was introduced into the peptide sequence by incorporation of building block t-Boc-aminooxy-PEG₂-CH₂CO₂H. Amino acids and the t-Boc-aminooxy-PEG₂-CH₂CO₂H were coupled using standard Fmoc solid phase peptide synthesis conditions. Treatment with standard TFA/scavenger conditions resulted in cleavage of the peptide and removal of protecting groups followed by concentration and preparative HPLC purification. After freeze drying all peptides were isolated in > 5.0 mg yield with TFA as counter ion. Peptides 5.1-5.4 were obtained in >90% purity as determined by HPLC analysis at 214 nm. Peptide 5.1 Analysis: MW (calc.) 1527 Da, [M+2H]²⁺ 765 Da. Peptide 5.2 Analysis: MW (calc.) 1509 Da, [M+H]⁺ 1510 Da. Peptide 5.3 Analysis: MW (calc.) 1148 Da, [M+H]⁺ 1149 Da. Peptide 5.4 Analysis: MW (calc.) 3124 Da, [M+H]⁺ 1563 Da.

### Example 6 - Synthesis of conjugatable TCO activators

### N,N-bis(2-{2-[2-(2-{2-[(4E)-Cyc1ooct-4-en-1-yloxy]acetamido}ethoxy)ethoxy]ethoxy}ethyl)-1-[3-(2,5-dioxo-2,5-dihydro-1H-pyrrol-1-yl)propanamido]3,6,9,12,15,18-hexaoxahenicosan-21-amide (6.1)

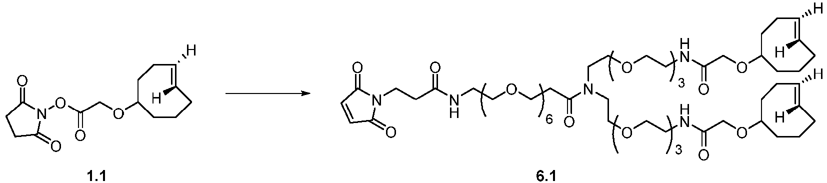

Compound 1.1 (49.0 mg, 174 µmol) and N-(Mal-PEG₆)-N-bis(PEG₃-amine) TFA salt (21 mg, 19 µmol) were dissolved in dry DMF (2 mL). DiPEA (50 µL, 0.29 mmol) was added and the mixture was stirred for 2.5 h at rt. The reaction mixture was concentrated and applied to a C₁₈ reverse phase preparative HPLC column followed by elution using a 25% to 75% gradient of MeCN in H₂O over 55 min, yielding 6.1 in 10 mg yield (8.4 µmol, 43%) after lyophilization. **Analysis 6.1**: ESI-MS calculated for C₅₈H₉₉N₅O₂₀: 1185.69; found [M+H]⁺ 1186.56. ¹H NMR (500 MHz, CDCl₃): δ 7.05 (s, 2H), 6.69 (s, 2H), 6.48 (s, 1H), 5.61 - 5.45 (m, 4H), 3.97 - 3.81 (m, 6H), 3.75 (t, *J =* 6.9 Hz, 2H), 3.67 - 3.46 (m, ca. 57H), 3.41 (q, *J =* 5.1 Hz, 2H), 2.68 (t, *J =* 7.0 Hz, 2H), 2.51 (t, *J =* 7.2 Hz, 2H), 2.35 - 2.15 (m, 8H), 2.06 (dd, *J =* 11.2, 3.1 Hz, 4H), 1.89 - 1.77 (m, 4H), 1.72 (qd, *J =* 13.0, 4.3 Hz, 2H), 1.54 (td, *J* = 14.4, 5.5 Hz, 2H), 1.25 - 1.17 (m, 2H) ppm.

### Example 7 - Synthesis of conjugatable tetrazine activators

### 2-Triisopropylsilyloxymethyl-3,4-dihydro-2H-pyran (7.1)

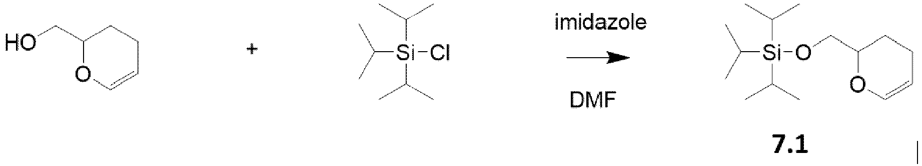

3,4-Dihydro-2H-pyran-2-methanol (2.50 g, 21.9 mmol) was dissolved in DMF (30 mL). Imidazole (3.73 g, 55 mmol) was added, and the mixture was cooled to 0°C. Triisopropylsilyl chloride (4.86 g, 25.2 mmol) was added, and the mixture was stirred under an atmosphere of argon at 0°C for 15 min. Then it was allowed to warm to 20°C and stirred for 4 hrs. Water (90 mL) was added and the turbid mixture was extracted with Et₂O (2×50 mL). The organic phase was dried over MgSO₄, filtered, and concentrated *in vacuo.* The crude product was purified by distillation (140°C, 0.5 mbar), to yield the product **7.1** as a colorless liquid (5.26 g, 89%). ¹H NMR (400 MHz, CDCl₃): δ 6.36 (d, 1H), 4.67 (m, 1H), 3.92 - 3.82 (m, 2H), 3.69 (m, 1H), 2.15 - 2.02 (m, 1H), 2.02 - 1.89 (m, 2H), 1.67 (m, 1H), 1.07 (m, 21H) ppm. ¹³C NMR (101 MHz, CDCl₃): δ 143.59, 100.48, 75.58, 65.69, 24.54, 19.24, 17.97, 11.97 ppm

### 2-Triisopropylsilyloxymethyl-3,4-dihydro-2H-pyran-6-yltributylstannane (7.2)

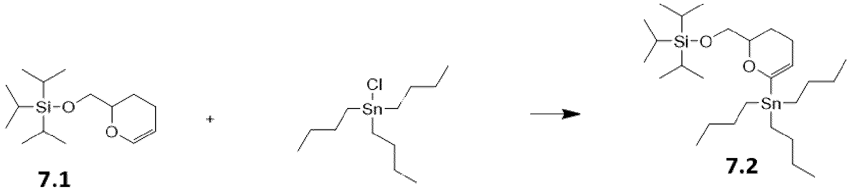

Potassium *tert*-butoxide (1.00 g, 8.92 mmol) was suspended in pentane (15 ml) and cooled to -78°C. Tetramethylethylenediamine (1.38 g, 11.88 mmol) was added, and n-BuLi (5.57 mL 1.6 M solution in hexane, 8.92 mmol) was added dropwise. After stirring for 10 min, the mixture was allowed to warm to -15°C to give a clear, yellow solution. Subsequently, it was recooled to -78°C, and **7.1** (1.60 g, 5.94 mmol) dissolved in pentane (1.5 mL) was added dropwise. The mixture was allowed to warm to -15°C over 1 h to give a precipitate. The turbid mixture was recooled to - 78°C, and tributyltin chloride (3.86 g, 11.88 mmol) was added. The turbid, yellow mixture was allowed to warm to 20°C and quenched with saturated NH₄Cl(aq). The organic phase was isolated, dried over MgSO₄, and concentrated to give a residue which was purified by flash column chromatography (silica neutralized with EtN₃, hexane) to afford the product **7.2** as a colorless oil (3.08 g, 93%). ¹H NMR (400 MHz, CDCl₃): δ 4.71 (m, 1H), 3.78 (m, 2H), 3.65 (m, 1H), 2.10 (m, 1H), 1.95 (m, 2H), 1.66 (m, 1H), 1.51 (m, 6H), 1.31 (m, 6H), 1.07 (m, 21H), 0.89 (m, 15H) ppm. ¹³C NMR (101 MHz, CDCl₃): δ 162.10, 111.87, 75.60, 66.05, 29.00, 27.22, 24.79, 20.94, 18.00, 13.72, 12.00, 9.44 ppm.

### 3-(2-Triisopropylsilyloxymethyl-3,4-dihydro-2H-pyran-6-yl)-6-(3,4-dihydro-2H-pyran-6-yl)-1,2,4,5-tetrazine (7.3)

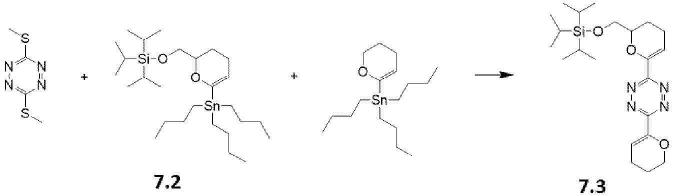

3,6-Bis-(methylthio)-1,2,4,5-tetrazine (164 mg, 0.94 mmol) was dissolved in dioxane (70 mL), and copper(I) thiophene-2-carboxylate (714 mg, 3.76 mmol), tetrakis(triphenylphosphine)-palladium(0) (163 mg, 0.14 mmol), tributyl(5,6-dihydro-4H-pyran-2-yl)stannane (700 mg, 1.88 mmol), and **7.2** (1050 mg, 1.88 mmol) were added. The dark suspension was heated at 100°C under an atmosphere of argon for 90 min. After allowing to cool to 20°C, it was filtered over diatomaceous earth, concentrated in vacuo, and redissolved in CH₂Cl₂ (10 mL). The pink solution was washed with Na₂CO₃(aq) (2×15 mL), dried over Na₂SO₄, filtered and concentrated. The crude product was purified by flash column chromatography (silica, hexane/ CH₂Cl₂) to afford **7.3** as a pink oil (64 mg, 16%). ¹H NMR (400 MHz, CDCl₃): δ 6.70 (t, J = 4.4 Hz, 2H), 4.34 (t, J = 5.1 Hz, 2H), 4.23 (dddd, J = 9.7, 7.2, 4.5, 2.4 Hz, 1H), 4.12 (dd, J = 9.9, 4.5 Hz, 1H), 3.87 (dd, J = 9.9, 7.5 Hz, 1H), 2.41 (m, 4H), 2.22 (m, 1H), 2.04 (m, 2H), 1.88 (m, 1H), 1.18 - 1.00 (m, 21H) ppm. ¹³C NMR (101 MHz, CDCl₃): δ 160.81, 160.62, 146.70, 146.32, 110.57, 110.46, 67.08, 64.91, 23.66, 21.82, 21.16, 20.63, 17.99, 17.97, 11.93 ppm. ESI-MS: m/z Calc. for C₂₂H₃₆N₄O₃Si 432.26 Da; Obs. [M+H]⁺ 433.33 Da.

### 3-(Hydroxymethyl-3,4-dihydro-2H-pyran-6-yl)-6-(3,4-dihydro-2H-pyran-6-yl)-1,2,4,5-tetrazine (7.4)

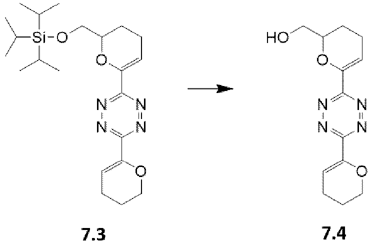

Compound **7.3** (40 mg, 0.093 mmol) was dissolved in MeCN (2 mL) and diluted with water (2 mL). Formic acid (0.080 mL) was added, and the mixture was stirred at 20°C for 68 hrs. The solvent was removed in vacuo and the residue was purified by reversed-phase column chromatography (C₁₈, water/MeCN) and lyophilization to give **7.4** as a pink solid (27 mg, 100%). ¹H NMR (400 MHz, CDCl₃): δ 6.73 (m, 2H), 4.35 (dd, J = 6.2, 4.2 Hz, 2H), 4.24 (ddt, J = 9.8, 6.2, 3.0 Hz, 1H), 3.89 (qd, J = 12.0, 4.9 Hz, 2H), 2.65 (br.s, 1H), 2.58 - 2.35 (m, 4H), 2.04 (dt, J = 10.3, 6.1 Hz, 2H), 1.99 (m, 1H), 1.92 (m, 1H) ppm. ¹³C NMR (101 MHz, CDCl₃): δ 146.64, 146,20, 110.76, 110.62, 67.10, 65.10, 23.06, 21.80, 21.18 ppm. ESI-MS: m/z Calc. for C₁₃H₁₆N₄O₃ 276.12 Da; Obs. [M+H]⁺ 277.17 Da.

### {6-[6-(3,4-Dihydro-2H-pyran-6-yl)-1,2,4,5-tetrazin-3-yl]-3,4-dihydro-2H-pyran-2-yl}methyl 2,5-dioxopyrrolidine-1-carboxylate (7.5)

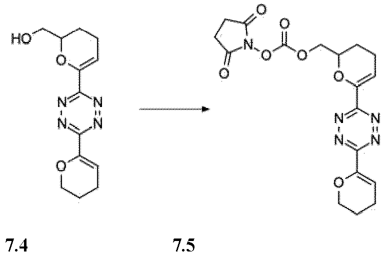

Compound **7.4** (8.6 mg, 0.0312 mmol) was dissolved in MeCN (1 mL), and bis(2,5-dioxopyrrolidin-1-yl) carbonate (16 mg, 0.0624 mmol), DIPEA (12 mg, 0.094 mmol), and DMAP (0.4 mg, 0.0031 mmol) were added. The mixture was stirred at 20°C overnight, and then concentrated, redissolved in CH₂Cl₂ (2 mL), and washed with 0.5 M citric acid (aq) (2×1 mL) and NaHCO₃ (2×1 mL). The pink solution was dried over Na₂SO₄, filtered and concentrated to yield **7.5** as a pink solid (13 mg, 95%). ¹H NMR (400 MHz, CDCl₃): δ 6.72 (m, 2H), 4.62 (m, 2H), 4.46 (m, 1H), 4.35 (dd, J = 6.2, 4.2 Hz, 2H), 2.85 (s, 4H), 2.49 (m, 2H), 2.41 (td, J = 6.3, 4.3 Hz, 2H), 2.17 - 2.09 (m, 1H), 2.05 (m, 2H), 1.95 (m, 1H) ppm. ESI-MS: m/z Calc. for C₁₈H₁₉N₅O₇ 417.13 Da; Obs. [M+H]⁺ 418.25 Da.

### {6-[6-(3,4-Dihydro-2H-pyran-6-yl)-1,2,4,5-tetrazin-3-yl]-3,4-dihydro-2H-pyran-2-yl}methyl N-[2-(2,5-dioxo-2,5-dihydro-1H-pyrrol-1-yl)ethyl]carbamate (7.6)

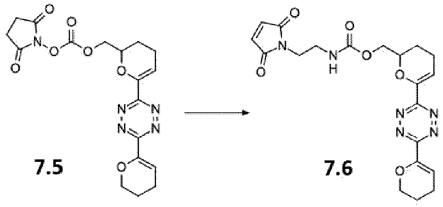

To a solution of compound **7.5** (5.3 mg, 0.0067 mmol) in DMF (0.5 mL) was added DIPEA (5 eq), N-(2-aminoethyl)maleimide TFA salt (1.2 eq), and PyBOP (1.5 eq). The pink solution is stirred at 20°C for 30 min, and subsequently formic acid (10 mg) and water (1 mL) were added. The product was purified by reversed-phase column chromatography (C₁₈, water/MeCN). The affluent is concentrated in vacuo to remove MeCN, and the aqueous solution was extracted with CH₂Cl₂ (10 mL). The organic layer was dried over Na₂SO₄, filtered, and concentrated in vacuo to yield compound **7.6.**

### 2,5-Dioxopyrrolidin-1-yl 1-(6-(6-(3,4-dihydro-2H-pyran-6-yl)-1,2,4,5-tetrazin-3-yl)-3,4-dihydro-2H-pyran-2-yl)-3-oxo-2,7,10,13,16,19,22,25,28,31-decaoxa-4-azatetratriacontan-34-oate (7.8)

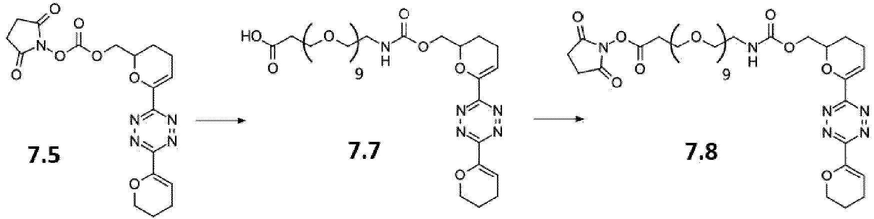

To a solution of compound **7.5** (16.7 mg, 0.0312 mmol) in MeCN (0.5 mL) was added a solution of 1-amino-3,6,9,12,15,18,21,24,27-nonaoxatriacontan-30-oic acid (29 mg, 0.0598 mmol) in MeCN (0.5 mL), followed by DIPEA (12 mg, 0.0936 mmol). The pink solution was stirred at 20°C for 30 min, and subsequently formic acid (16 mg) and water (2 mL) were added. The product was purified by reversed-phase column chromatography (C₁₈, water/MeCN) and lyophilization to yield compound **7.7** as a pink solid (18 mg, 73%). To a solution of compound **7.7** (18.0 mg, 0.0229 mmol) in MeCN (1 mL) was added DIPEA (14.8 mg, 0.115 mmol) and bis(2,5-dioxopyrrolidin-1-yl) carbonate (7.6 mg, 0.030 mmol). The pink solution was stirred at 20°C for 30 min, and subsequently formic acid (20 mg) and water (1.5 mL) were added. The product was purified by reversed-phase column chromatography (C₁₈, water/MeCN) and lyophilization to yield compound **7.8** as a pink oil (16.3 mg, 80%). **Analysis 7.7** ¹H NMR (400 MHz, CDCl₃): δ 6.71 (m, 2H), 5.57 (t, J = 5.3 Hz, 1H), 4.43 - 4.31 (m, 4H), 3.77 (t, J = 6.1 Hz, 2H), 3.71 (s, 2H), 3.65 (m, 32H), 3.56 (t, J = 5.1 Hz, 2H), 3.38 (q, J = 5.4 Hz, 2H), 2.60 (t, J = 6.0 Hz, 2H), 2.48 - 2.34 (m, 4H), 2.04 (m, 2H), 1.86 (m, 1H) ppm. ESI-MS: m/z Calc. for C₃₅H₅₇N₅O₁₅ 787.39 Da; Obs. [M+H]⁺ 788.42 Da. **Analysis 7.8** ¹H NMR (400 MHz, CDCl₃): δ 6.70 (m, 2H), 5.47 (t, J = 5.5 Hz, 1H), 4.35 (m, 5H), 3.85 (t, J = 6.5 Hz, 2H), 3.67 - 3.59 (m, 32H), 3.56 (t, J = 5.1 Hz, 2H), 3.38 (q, J = 5.4 Hz, 2H), 2.90 (t, J *=* 6.5 Hz, 2H), 2.84 (s, 4H), 2.43 (m, 4H), 2.04 (m, 3H), 1.88 (m, 1H) ppm. ESI-MS: m/z Calc. for C₃₉H₆₀N₆O₁₇ 884.40 Da; Obs. [M+H]⁺ 885.42 Da.

### {6-[6-(3,4-dihydro-2H-pyran-6-yl)-1,2,4,5-tetrazin-3-yl]-3,4-dihydro-2H-pyran-2-yl}methyl N-(29-{[2-(2,5-dioxo-2,5-dihydro-1H-pyrrol-1-yl)ethyl]carbamoyl}-3,6,9,12,15,18,21,24,27-nonaoxanonacosan-1-yl)carbamate (7.9)

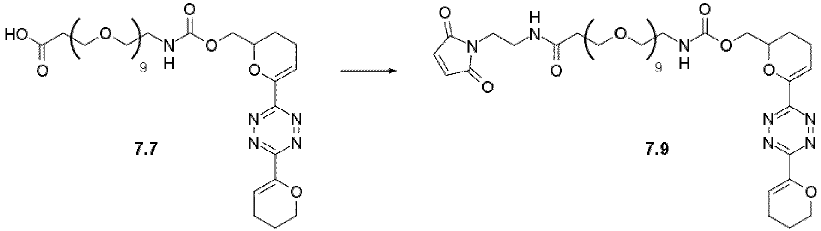

To a solution of compound **7.7** (5.3 mg, 0.0067 mmol) in DMF (0.5 mL) was added DIPEA (4.3 mg, 0.033 mmol), N-(2-aminoethyl)maleimide TFA salt (2.1 mg, 0.0081 mmol), and PyBOP (5.3 mg, 0.010 mmol). The pink solution was stirred at 20°C for 30 min, and subsequently formic acid (10 mg) and water (1 mL) were added. The product was purified by reversed-phase column chromatography (C₁₈, water/MeCN). The affluent was concentrated in vacuo to remove MeCN, and the aqueous solution was extracted with (10 mL). The organic layer was dried over Na₂SO₄, filtered, and concentrated in vacuo to yield compound **7.9** as a pink oil (4.0 mg, 66%). ¹H NMR (400 MHz, CDCl₃): δ 6.84 (br.s, 1H), 6.71 (m, 4H), 5.44 (t, J = 5.4 Hz, 1H), 4.43 - 4.31 (m, 4H), 3.8 - 3.5 (m, 40H), 3.56 (t, J = 5.1 Hz, 2H), 3.45 (m, 2H), 3.38 (q, J = 5.2 Hz, 2H), 2.43 (m, 4H), 2.04 (m, 2H), 1.88 (m, 1H) ppm. ESI-MS: m/z Calc. for C₄₁H₆₃N₇O₁₆ 909.43 Da; Obs. [M+H]⁺ 910.50 Da.

### Example 8 - Synthesis of small molecule tetrazine activators

### 2,2',2"-(10-(2,40,44-Trioxo-44-((6-(6-(pyridine-2-yl)-1,2,4,5-tetrazin-3-yl)pyridine-3-yl)amino)-6,9,12,15,18,21,24,27,30,33,36-undecaoxa-3,39-diazatetratetracontyl)-1,4,7,10-tetraazacyclododecane-1,4,7-triyl)triacetic acid (8.1)

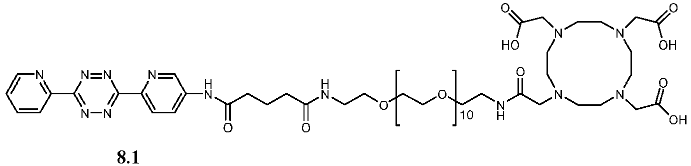

The synthesis of compound **8.1** was reported in Rossin et al., Angew. Chem. Int. Ed. 2010, 49, 3375 -3378.

### 2,2'-((2-((Carboxymethyl)(2-oxo-2-((6-(6-(pyridin-2-yl)-1,2-dihydro-1,2,4,5-tetrazin-3-yl)pyridin-3-yl)amino)ethyl)amino)ethyl)azanediyl)diacetic acid (8.3)

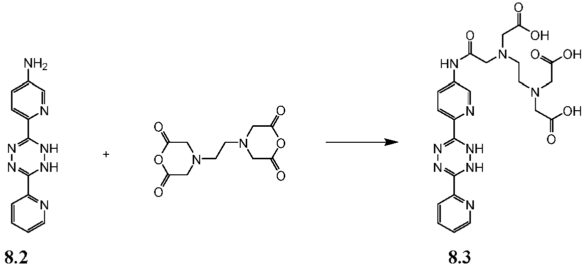

Ethylenediaminetetraacetic dianhydride (1.86 g, 7.26 mmol) was dissolved in dry DMSO (3 mL) by gentle heating. The mixture was allowed to cool to room temperature and a solution of **8.2** (450 mg, 1.78 mmol) in DMSO (11 mL) was slowly added. The orange, hazy mixture was stirred at room temperature under an atmosphere of argon for 5 h. Subsequently water (0.1 mL) was added and the mixture was stirred for 30 min. **8.3** mixture was used without further purification. ESI-MS: m/z Calc. for C₂₂H₂₅N₉O₇ 527.19 Da; Obs. [M+H]⁺ 528.42 Da and [M-H⁺]⁻ 526.50 Da.

### 2,2'-((2-((Carboxymethyl)(2-oxo-2-((6-(6-(pyridin-2-yl)-1,2,4,5-tetrazin-3-yl)pyridin-3-yl)amino)ethyl)amino)ethyl)azanediyl)diacetic acid (8.4)

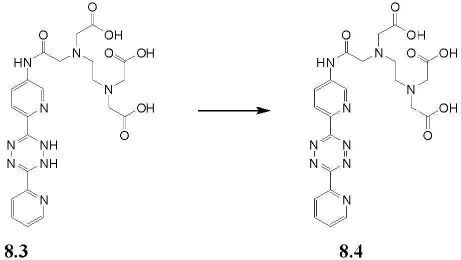

The crude reaction mixture of **8.3** was diluted with water (15 mL) and acidified by addition of formic acid (0.2 mL). Sodium nitrite (400 mg, 5.79 mmol) was added and the pink mixture was stirred in a closed flask at room temperature for 1 h. An aqueous solution of 1 M ammonium acetate (30 mL) was added and the pink suspension was centrifuged at 3000 rpm for 10 min. The clear, dark pink supernatant was isolated and purified by reversed-phase chromatography (C18 column, gradient of 5% MeCN / 0.1 M aqueous ammonium acetate to 25%). The combined product fractions were freeze dried, redissolved in water (25 mL), and again freeze dried and redissolved in water. To the pink solution was added formic acid (0.25 mL), which caused the product to precipitate. The suspension was centrifuged at 3000 rpm for 10 min, after which the clear, faint pink supernatant was discarded. The pink solid was washed with water (25 mL) and centrifuged, for two more times, and then washed with MeCN and centrifuged, for two more times. The remaining pink solid was dried in vacuo to give 486 mg (52% overall yield) of **8.4.** ¹H NMR (400 MHz, DMSO-d₆): δ 12.41 (br.s, 3H), 10.78 (s, 1H), 9.13 (d, J = 2.5 Hz, 1H), 8.94 (dd, J = 4.8, 1.7 Hz, 1H), 8.62 (m, 2H), 8.52 (dd, J = 8.7, 2.5 Hz, 1H), 8.16 (td, J = 7.8, 1.8 Hz, 1H), 7.73 (dd, J = 7.8, 4.7 Hz, 1H), 3.53 (m, J = 10.4 Hz, 8H), 2.85 (s, 4H) ppm. ¹³C NMR (101 MHz, DMSO-d6): δ 173.40, 172.97, 171.94, 163.52, 163.27, 151.08, 150.67, 144.47, 142.13, 138.49, 138.28, 127.05, 126.75, 125.28, 124.66, 58.55, 55.68, 55.37, 52.58, 52.22 ppm. ESI-MS: m/z Calc. for C₂₂H₂₃N₉O₇ 525.17 Da; Obs. [M+H]⁺ 526.33 Da and [M-H]⁻ 524.42 Da.

### Calcium sodium 2-({2-[bis(carboxylatomethyl)amino]ethyl}[({6-[6-(pyridin-2-yl)-1,2,4,5-tetrazin-3-yl]pyridin-3-yl}carbamoyl)methyl]amino)acetate (8.5)

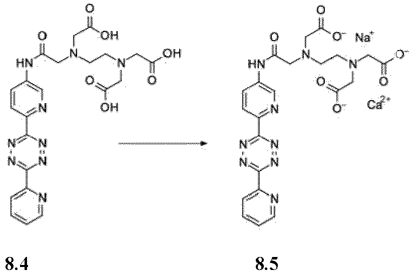

1M Sodium acetate was prepared by dissolving sodium acetate trihydrate in milliQ-H₂O (pH~9.0) followed by acidification to pH=6.4 acidic with AcOH glacial. To an acidic suspension of **8.4** (172 mg, 0.33 mmol; 10 mg/mL) in MQ-H₂O was added dropwise CaCO₃ (1.67 mL, 0.53 mmol of a 32 mg/mL homogenous suspension). Upon increasing pH, the tetrazine dissolved at pH=5.8 and CaCO₃ addition was halted at pH=6.5. Subsequently, 1.0 M sodium acetate (pH=6.4) was added to the tetrazine to obtain a final 0.1 M NaAc concentration. The solution was applied to a SEP-PAK column (10 gr, Waters) for purification (i.e. removal of excess calcium and NaAc components). The tetrazine retained at the top of the column and was rinsed once with 0.1 M sodium acetate followed by rinsing with milliQ-H₂O (6 volumes) prior to elution with milliQ-H₂O:MeOH (1:1) aided by vacuum pull. Tetrazine containing fractions were combined, reduced 80% by volume in vacuo, diluted with milliQ-H₂O, and lyophilized after micropore filter filtration. The lyphillized residue was redissolved in milliQ-H₂O at 50 mg/mL, micropore filtered once more, and freezedried to obtain **8.5** as a homogenous pink fluffy powder. ¹H NMR (400 MHz, D₂O) δ 8.89 (dd, *J* = 2.6, 0.6 Hz, 1H), 8.79 (ddd, *J* = 4.8, 1.7, 0.9 Hz, 1H), 8.63 - 8.55 (m, 2H), 8.37 (dd, *J* = 8.7, 2.6 Hz, 1H), 8.16 (td, *J* = 7.8, 1.7 Hz, 1H), 7.74 (ddd, *J* = 7.7, 4.7, 1.1 Hz, 1H), 3.58 (s, 2H), 3.32 - 3.06 (m, 6H), 2.77 - 2.50 (m, 4H) ppm. ¹³C NMR (100 MHz, D₂O) δ 179.9, 179.3, 174.1, 162.9, 162.5, 150.3, 148.3, 143.9, 142.0, 139.0, 137.3, 128.9, 127.6, 125.3, 124.7, 60.5, 59.8, 54.8 ppm. ESI-MS cal. for C₂₂H₂₃N₉O₇ 525.17 (excl. sodium calcium), found. M+H⁺ 526.25. Elemental anal. calcd for C₂₂H₂₀CaN₉NaO₇: Composition: C (45.1%), Ca (6.8%), N (21.5%), Na (3.9%). Measured: C (44.0%), Ca (7.2%), N (20.5%), Na (3.1%).

### 3-(2-(2-(3-Oxo-3-((6-(6-(pyridin-2-yl)-1,2-dihydro-1,2,4,5-tetrazin-3-yl)pyridin-3-yl)amino)propoxy)ethoxy)ethoxy)propanoic acid (8.6)

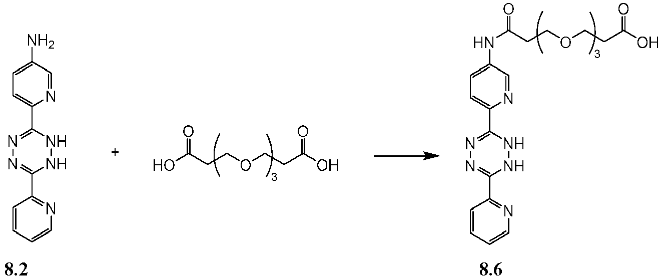

3,3'-((Oxybis(ethane-2,1-diyl))bis(oxy))dipropionic acid (5.90 g, 23.6 mmol) was dissolved in chloroform (100 mL), and pyridinium p-toluenesulfonate (0.15 g, 0.597 mmol) and EDC HCl (1.13 g, 5.92 mmol) were added. The solution was stirred at room temperature under an atmosphere of argon for 30 min. Subsequently, 6-(6-(pyridin-2-yl)-1,2-dihydro-1,2,4,5-tetrazin-3-yl)pyridin-3-amine (1.50 g, 5.92 mmol) was added, followed by DMAP (0.36 g, 2.95 mmol). The orange solution was stirred at room temperature under an atmosphere of argon for 90 min, and then washed twice with 0.5 M aqueous citric acid (60 mL). The combined organic layers were dried with sodium sulfate, filtered, and concentrated. Crude **8.6** was used without further purification. ESI-MS: m/z Calc. for C₂₂H₂₇N₇O₆ 485.20 Da; Obs. [M+H]⁺ 486.25 Da and [M-H⁺]⁻ 484.33 Da.

### 3-(2-(2-(3-Oxo-3-((6-(6-(pyridin-2-yl)-1,2,4,5-tetrazin-3-yl)pyridin-3-yl)amino)propoxy)ethoxy)ethoxy)propanoic acid (8.7)

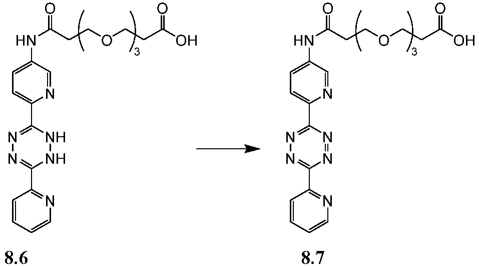

The crude product **8.6** was dissolved in MeCN (30 mL) and water (30 mL), and formic acid (1.5 mL) was added, followed by sodium nitrite (1.23 g, 17.8 mmol). The pink/red solution was stirred in a closed flask at room temperature for 30 min, and then diluted with water (90 mL). The mixture was filtered and purified by reversed-phase chromatography (C18 column, gradient of 15% MeCN / 0.1% aqueous formic acid to 30%). The combined product fractions were freeze-dried to give **8.7** as a pink, fluffy solid (1.32 g, 46% overall yield). ¹H NMR (400 MHz, CDCl₃): δ 9.61 (br.s, 1H), 8.97 (dt, J = 4.6, 1.4 Hz, 1H), 8.79 (m, J = 6.3, 2.6 Hz, 2H), 8.77 - 8.62 (m, 2H), 8.01 (td, J = 7.8, 1.8 Hz, 1H), 7.58 (ddd, J = 7.6, 4.7, 1.2 Hz, 1H), 3.85 (t, J = 5.5 Hz, 2H), 3.80 (t, J = 5.8 Hz, 2H), 3.74 (s, 4H), 3.72 - 3.59 (m, 4H), 2.76 (t, J = 5.5 Hz, 2H), 2.65 (t, J = 5.8 Hz, 2H) ppm. ¹³C NMR (101 MHz, CDCl₃): δ 174.18, 171.32, 163.53, 163.02, 150.91, 150.07, 143.56, 141.93, 138.55, 137.57, 127.36, 126.55, 125.27, 124.38, 70.70, 70.48, 70.20, 67.02, 66.78, 37.91, 35.40 ppm. ESI-MS: m/z Calc. for C₂₂H₂₅N₇O₆ 483.19 Da; Obs. [M+H]⁺ 484.50 Da and [M-H]⁻ 482.33 Da.

### 2,2'-((2-Oxo-2-((6-(6-(pyridin-2-yl)-1,2-dihydro-1,2,4,5-tetrazin-3-yl)pyridin-3-yl)amino)ethyl)azanediyl)diacetic acid (8.8)

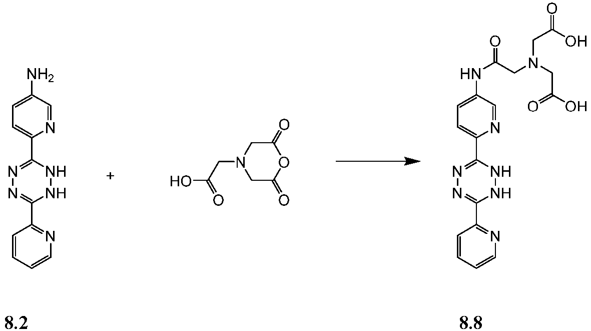

To a solution of **8.2** (168 mg, 0.66 mmol) in DMF (5 mL) was slowly added a solution of nitrilotriacetic anhydride (114 mg, 0.66 mmol) in DMF (1 mL). The solution was stirred at room temperature under an atmosphere of argon for 20 h. Water (30 mL) and formic acid (0.3 mL) were added to precipitate the product, which was isolated by centrifugation and decantation. The precipitate was washed with MeCN (30 mL), and dried in vacuo to yield **8.8** as orange powder (217 mg, 77% yield). ¹H NMR (400 MHz, DMSO-d6): δ 12.63 (br.s, 2H), 10.71 (br.s, 1H), 8.95 (s, 1H), 8.88 (s, 1H), 8.84 (d, J = 2.4 Hz, 1H), 8.64 (dd, J = 4.8, 1.6 Hz, 1H), 8.19 (dd, J = 8.7, 2.5 Hz, 1H), 8.07 - 7.83 (m, 3H), 7.53 (ddd, J = 6.9, 4.8, 1.6 Hz, 1H), 3.58 (s, 4H), 3.55 (s, 2H) ppm. ESI-MS: m/z Calc. for C₁₈H₁₈N₈O₅ 426.14 Da; Obs. [M+H]⁺ 427.33 Da and [M-H]⁻ 425.42 Da.

### 2,2'-((2-oxo-2-((6-(6-(pyridin-2-yl)-1,2,4,5-tetrazin-3-yl)pyridin-3-yl)amino)ethyl)azanediyl)diacetic acid (8.9)

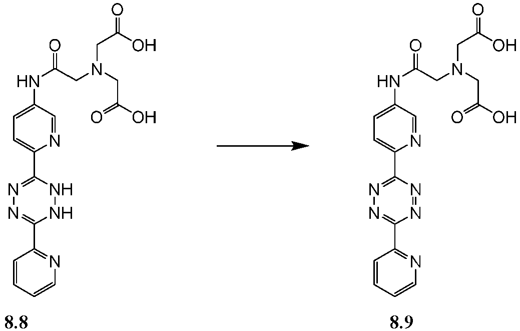

**8.8** (120 mg, 0.282 mmol) was suspended in water (20 mL), and sodium nitrite (97 mg, 1.41 mmol) was added. The suspension was stirred at room temperature under an atmosphere of argon, and turned pink and became clear within 10 min. After 1 h, a precipitate was formed again and the mixture was stored at 4°C for 1 h. The precipitate was isolated by centrifugation, washed with subsequently water (20 mL) and acetonitrile (25 mL), and dried in vacuo, to yield **8.9** as a pink powder (110 mg, 92%). ¹H NMR (400 MHz, DMSO-d6): δ 12.53 (br.s, 2H), 10.92 (br.s, 1H), 9.07 (d, J = 2.5 Hz, 1H), 8.94 (d, J = 4.2 Hz, 1H), 8.66 (d, J = 8.7 Hz, 1H), 8.60 (d, J = 7.9 Hz, 1H), 8.47 (dd, J = 8.7, 2.6 Hz, 1H), 8.16 (td, J = 7.8, 1.8 Hz, 1H), 7.73 (ddd, J = 7.7, 4.7, 1.2 Hz, 1H), 3.62 (m, 6H) ppm. ¹³C NMR (101 MHz, DMSO-d6): δ 173.86, 172.77, 171.68, 163.55, 163.24, 151.09, 150.71, 144.69, 141.66, 138.33, 138.28, 127.08, 126.50, 125.46, 124.69, 59.58, 56.45 ppm. ESI-MS: m/z Calc. for C₁₈H₁₆N₈O₅ 424.12 Da; Obs. [M+H]⁺ 425.33 Da and [M-H]⁻ 423.42 Da.

### Example 9 - IL12 conjugation with TCO-PEG masking moieties

A stock solution of IL12 in PBS was added with various amounts of the PFP-activated TCO-PEG masking moieties (in dry DMSO; **2.3:** 10 mg/mL; **2.5:** 10 mg/mL; **2.7:** 20 mg/mL; see Table 2 for equivalents added) and diluted with PBS to a 0.40 mg/mL final protein concentration. The pH was adjusted to ca 8.5 with sodium carbonate and the reaction mixtures were incubated for 1-2h at rt in the dark under gentle shaking followed by overnight incubation at +4°C. The IL12-TCO-PEG conjugates were then purified by SEC. The collected fractions were concentrated using Amicon centrifugal devices (MW cut-off 30 kDa) and the conjugate concentration in the obtained solutions was measured using a BCA assay (Thermo Fisher). The compound numbering of the resulting IL12-TCO-PEG conjugates are listed in Table 2. SDS-PAGE analysis of the conjugates showed the presence of a single species with MW ca 65 kDa for IL12-TCO-PEG₂ conjugates (Figure 5, lanes 2-5), and of single species with increasing MW (up to ca. 100 kDa) for IL12-TCO-PEG_{1K} conjugates with increasing functionalization grades (Figure 5, lanes 10-13). The IL12-TCO-PEG conjugates were then reacted with an excess (5 eq with respect to TCO) of tetrazine **8.5** in PBS for 3h at 37°C followed by SDS-PAGE analysis, confirming IL12 unmasking in vitro upon reaction with a tetrazine activator (Figure 5, lane 6-9 and 14-17).

**Table 2: Molar equivalents of TCO-PEG masks used in the conjugation reaction and average functionalization grades measured for the IL12-TCO-PEG conjugates using tetrazine titration.**

| Masking moiety | molar equivalents added | functionalization grade | IL12-TCO-PEG conjugates codes |
|---|---|---|---|
| TCO-PEG₂ (**2.3**) | 10 | 6.8 | **9.1** |
| | 20 | 11.2 | **9.2** |
| | 30 | 19.9 | **9.3** |
| | 50 | 24.5 | **9.4** |
| | 100 | 30.9 | **9.5** |
| TCO-PEG_{1K}(**2.5**) | 5 | 2.2 | **9.6** |
| | 10 | 2.8 | **9.7** |
| | 30 | 6.3 | **9.8** |
| | 50 | 9.3 | **9.9** |
| | 100 | 13.3 | **9.10** |

### Example 10 - Functionalization grade measurement of IL12-TCO-PEG

The functionalization grades of IL12-TCO-PEG conjugates were measured by a tetrazine titration method (R. Rossin et al., Angew Chem Int Ed, 2010, 49, 3375-3378) with minor modifications. 2 µg aliquots of the IL12-TCO-PEG conjugates **(9.1-9.10)** were reacted with a known excess of ¹¹¹In-labeled DOTA-tetrazine in 40 µl PBS. After 2h incubation at 37°C, the proteins were precipitated from the reaction mixture by adding 50 µl ice-cold MeCN. After 30 min at -20°C, the vials were centrifuged at 14000 rpm, the supernatant was two-fold diluted with H₂O and analyzed by RP-HPLC using gradient 1 (IL12-TCO-PEG₂) or gradient 2 (IL12-TCO-PEG_{1K}). The IL12 functionalization grades were then determined from the ratio between the ¹¹¹In-tetrazine peak and that of the released reaction product in the radio-chromatogram (Table 2).

### Example 11 - Bioactivity assay with IL12-TCO-PEG conjugates on HEK Blue cells, and detection by ELISA

The IL12-TCO-PEG conjugates **(9.3, 9.4, 9.5, 9.7, 9.9, 9.10)** and a tetrazine activator **8.4** (100 eqs with respect to IL12) were reacted in serum-free culture medium (DMEM, 1h, 37°C) in a Costar round-bottom plate. Next, a 1:2-step titration was made, after which 80.000 reporter cells (HEK-Blue IL12 cells; InvivoGen) were added in complete medium containing 10% FCS. IL12 bioactivity was determined after 20-22h, based on colorimetric quantification of Secreted Alkaline Phosphatase (SEAP) using the reagent QUANTI-Blue (InvivoGen) at OD630nm. Alternatively, IL12-TCO-PEG constructs were detected using a hIL12-p70 ELISA assay ('sandwich setup'; Invitrogen). IL12-TCO-PEG₂ **9.4,** or IL12-TCO-PEG_{1K} **9.10** were reacted with 100 molar equivalents of tetrazine **8.5** in PBS for 1h at 37C. Next, native IL12, reacted, or unreacted IL12 constructs were added to capture antibody-coated plates in PBS in titration and incubated overnight at 4□C. Sample IL12 content was determined according to kit manufacturer's instructions. The results show complete IL12 deactivation when the protein was functionalized with ca. 9 TCO-PEG masks (Figure 6-8, Table 2) while at lower functionalization grades partial IL12 deactivation was achieved. In all cases, upon reaction with a tetrazine the masking moiety is removed and functional detection by ELISA as well as IL12 bioactivity is re-established.

### Example 12: IL12 conjugation with Tz-PEG masking moieties

A stock solution of hIL12 was added with various amounts of the PFP-activated Tz-PEG masking moieties **3.11** and **3.14** (10 mM in dry DMSO) and diluted with PBS to a 2 mg/ml final protein concentration. The pH was adjusted to ca. 8.5 with 0.1M sodium carbonate and the reaction mixtures were incubated overnight at rt in the dark under gentle shaking. The IL12-Tz-PEG conjugates were then purified by SEC. The collected fractions were concentrated using Amicon centrifugal devices (MW cut-off 30 kDa) and the conjugate concentration in the obtained solutions was measured using a BCA assay. The compound numbering of the resulting IL12-Tz-PEG conjugates are listed in Table 3. Conjugate **12.3** was then reacted with an excess (200 eq with respect to IL12) of a small-molecule TCO activator **(1.4)** in PBS for 18 hrs at 37°C followed by SDS-PAGE analysis, confirming near complete IL12 unmasking in vitro upon reaction with the activator (Figure 9, lanes 2 and 3).

**Table 3: Molar equivalents of PEG-Tz masks used in the conjugation reaction and average functionalization grades measured for the IL12-Tz-PEG conjugates using Sulfo-Cy5-TCO.**

| Masking moiety | molar equivalents added | functionalization grade | IL12-Tz-PEG conjugates code |
|---|---|---|---|
| mPEG₁₀-Tz (**3.11**) | 38 | 4.1 | **12.1** |
| mPEG₁₀-Tz-MePABC (**3.14**) | 10 | 5.0 | **12.2** |
| | 20 | 10.6 | **12.3** |
| | 38 | 17.8 | **12.4** |

### Example 13: Functionalization grade measurement of IL12-Tz-PEG

1 µg aliquots of the IL12-Tz-PEG conjugates **(12.1-12.4)** were reacted with a known excess of sulfo-Cy5-TCO (Broadpharm) in 30 µl PBS. After 2h incubation at 37°C, the mixtures were analyzed by SEC with absorbance measurement at 600 nm. The IL-12 functionalization grades were then determined from the ratio between the sulfo-Cy5-TCO peak and that of the reaction products in the chromatogram (Table 3).

### Example 14 - Bioactivity assay with IL12-Tz-PEG conjugates on HEK Blue cells, and detection by ELISA

The IL12-Tz-PEG conjugate **12.4** and small-molecule TCO activator **1.4** (100 eq with respect to IL12) were reacted for 3hr at 37°C in DMEM culture medium containing 10% FCS. Next, a 1:2-step titration was made, after which 80.000 reporter cells (HEK-Blue IL12 cells) were added in DMEM containing 10% FCS. IL12 bioactivity was determined after 20-22h, based on colorimetric quantification of SEAP using QUANTI-Blue at OD630nm. The results show complete IL12 deactivation when the protein was functionalized with ca. 18 Tz-PEG masks (Figure 10A, Table 3) and recovery upon reaction with TCO. Alternatively, the IL12-Tz-PEG conjugate **12.4** (1ng/ml) was added together with **1.4** (100 eqs) and 10% human serum, during the antigen capture step of the hIL12-p70 ELISA assay (18hrs incubation at 4C). Figure 10B shows no/low ELISA detection of Tz-masked protein construct, while detection is regained following release by TCO activator.

### Example 15 - IL12 conjugation with TCO-PEG8-keto

A stock solution of IL-12 was added to a solution containing PBS (pH 8.3) and DMSO. Subsequently, compound **4.6** (40 mg/ml in DMSO) was added at 10, 20, 30, 40 or 50 molar eqs with respect to IL-12 and the reaction mixture was incubated for 18hrs at rt in the dark under gentle shaking. Final protein concentration was 0.5 mg/mL, DMSO content was 20%, and the pH was ca 8.3. The obtained IL12-TCO-PEG8-keto constructs **(15.1, 15.2, 15.3, 15.4, 15.5** resp.) were then purified by SEC. The collected fractions were concentrated using Amicon centrifugal devices and concentration was measured by UV at 280 nm. SDS-PAGE analysis showed the presence of single species with increased MW (Fig. 11, lane 2-6 ) compared to that of native IL-12 (ca 65 kDa, Fig. 11, lane 1 or 7). The conjugate was then reacted with an excess (200 molar eqs with respect to IL-12) of tetrazine **8.5** in PBS for 30 min at 37°C followed by SDS-PAGE analysis, confirming IL-12 unmasking (Fig. 11A, lane 8-12). The IL-12 functionalization grades were determined similarly as for the IL12-TCO-PEG conjugates **(9.1-9.10)** using 111In-labeled DOTA-tetrazine. This time, however, RP-HPLC gradient 3 was used for the analysis. The functionalization grades and codes of the resulting IL12-TCO-PEG8-keto constructs are given in Table 4

**Table 4: Molar equivalents of masks used in the conjugation reaction and average functionalization grades measured for the IL12-TCO-PEG8-keto conjugates using tetrazine titration.**

| Masking moiety | molar equivalents added | Functionalization grade | IL12-TCO-PEG8-keto conjugates code |
|---|---|---|---|
| TCO-PEG₈-keto (**4.6**) | 10 | 3.4 | **15.1** |
| | 20 | 7.3 | **15.2** |
| | 30 | 12 | **15.3** |
| | 40 | 12 | **15.4** |
| | 50 | 13 | **15.5** |

### Example 16 - IL12-TCO-PEG8-keto conjugation with targeting peptides

A solution of IL12-TCO-PEG8-keto **15.4** in PBS was rebuffered (Zeba desalting column) to peptide conjugation buffer (20 mM NaOAc, 150 mM NaCl, 1 mM EDTA, pH 4.6) and concentrated using an Amicon centrifugal device to 2.9 mg/mL. Rebuffered **15.4** was reacted with various targeting peptides (HA-PEG-peptides **5.1-5.4,** also in broader context referred to as "peptide 1-4"); 50 molar eqs with respect to IL-12; 10 mg/mL in water; final protein concentration of 1.3 mg/mL), and the mixtures were incubated at 25°C in the dark for 40h while shaking. The IL12-TCO-peptide conjugates **(16.1, 16.2, 16.3,** and **16.4** resp.) were then purified by SEC. The collected fractions were concentrated using Amicon centrifugal devices and concentrations were measured by UV. Confirmation of coupling of the peptides to the IL12-TCO-PEG8-keto constructs was obtained by MALDI. SDS-PAGE analysis of the conjugates **16.1-16.3** showed the presence of a single species with increased MW (ca 75 kDa, figure 12, lanes 3-5) compared to that of IL12 (lane 1) or IL12-TCO-PEG8-keto (lane 2) . The conjugate was then reacted with an excess (200 molar eqs with respect to IL-12) of tetrazine **8.5** in PBS for 30 min at 37°C followed by SDS-PAGE analysis, confirming IL-12 unmasking (lanes 6-9).

### Example 17 - Bioactivity assay with IL12-TCO-PEG8-keto conjugates

The IL12-TCO-PEG8-keto conjugates **15.1-15.5** with or without a tetrazine activator **8.5** (100 molar eqs with respect to IL12) were reacted in culture medium containing 10% FCS (1h, 37°C). Next, IL12 bioactivity was assessed using HEK-Blue reporter cells as described above. The results show increasing attenuation of biological activity with increased modification grade (Figure 13A) and complete recovery upon reaction with the tetrazine (Figure 13B).

### Example 18 - Bioactivity assay with IL12-TCO-peptide conjugates

The IL12-TCO-peptide conjugates **16.1-16.3** with or without tetrazine activator **8.5** (100 molar eqs with respect to IL12) were reacted in culture medium containing 10% FCS (1h, 37°C). Next, IL12 bioactivity was assessed using HEK-Blue reporter cells as described above. The results showed IL12 deactivation when the protein was functionalized with the peptide masks (Figure 14) Upon reaction with the tetrazine the masking moiety is removed and IL12 bioactivity is re-established.

### Example 19 - Blood kinetics of peptide-masked IL12 constructs.

Peptide-masked IL12 constructs **16.1-16.3** were radiolabelled with ¹²⁵I using an established Bolton-Hunter method (van Duijnhoven, S. M. J., et al. Diabody Pretargeting with Click Chemistry In Vivo. J. Nucl. Med. 56, 1422-1428 (2015)). The radiolabeled constructs were injected i.v. in tumour-free mice (ca. 10 µg, ca. 0.4 MBq in 100 µL PBS per mouse; male BALB/c nude mice, ca. 20 g body weight, Janvier; n=3/group), followed by detection of radioactivity in blood samples collected at various timepoints (2, 30, 60min, 3, 6, 24, and 72hrs post injection). Weight of the blood samples and counts as measured by a gamma-counter (together with standards) were used to calculate the percentage of the injected dose per gram (%ID/g). Figure 15 shows that all constructs have a comparable clearance pattern as wildtype IL12 (See table 5)

**Table 5: Calculated half-lives for a two phase decay peptide-masked constructs.**

| **Construct** | **T_{1/2, α} (hrs) (%)** | **T_{1/2, β} (hrs)** |
|---|---|---|
| IL12 native | 0.22 (55.3) | 5.15 |
| IL12-keto-peptide 1 (**16.1**) | 0.12 (87.6) | 8.84 |
| IL12-keto-peptide 2 (**16.2**) | 0.28 (53.1) | 4.31 |
| IL12-keto-peptide 3 (**16.3**) | 0.30 (50.9) | 6.55 |

### Example 20 - Detection of ¹²⁵I-labelled peptide-masked IL12 constructs in tumors and plasma, and their unmasking.

Male BALB/c nude mice (ca. 20 g body weight, Janvier) were subcutaneously injected 2x10⁶ PC3 tumor cells at the lower flank. Injections (100µL) were done in a 1:3 medium:Matrigel solution (Corning). Peptide-masked IL12 constructs **16.1-16.3** were radiolabelled with ¹²⁵I and injected i.v. when tumors reached ca. 0.2 cm³ in size (ca. 10 µg construct, ca. 0.4 MBq in 100 µL PBS per mouse; n=4/group). Twenty-four hours after injection of the constructs some mice received an i.v. injection of Tz activator **8.5** (100 ug in 100ul PBS), followed by euthanasia 1hr later. Upon isolation, the tumors were collected in MagNA lyser green bead tubes (Roche), added with EDTA-free protease inhibitor cocktail (Roche; 4 mL/g tumor) and stored on ice. Blood was collected in heparinized vials on ice and plasma was separated by centrifugation (5 min, 3000 rpm). Plasma samples were then 2-fold diluted with protease inhibitor cocktail. Next, radioactivity was measured using a gamma-counter, after which the tumors were homogenized and debris was removed by centrifugation (13000 rpm, 5 min). The amount of IL12-construct in tumors was estimated using ¹²⁵I activity (%ID/g). Data in figure 16 show significantly less tumor retention of wildtype IL12, as compared to the peptide-targeted constructs **16.1** and **16.3,** and relatively low washout after Tz unmasking.

The presence of IL12 constructs in tumor homogenates and plasma samples was evaluated by ELISA. For this, the tumor homogenates and plasma samples were 3-fold diluted in the kit's ELISPOT buffer, 100 µL was transferred to the ELISA plates, followed by incubation for 18 hrs at 4°C. Figures 17A and B show that significantly more IL12 can be detected in the tumors and blood of mice that were administered the masked constructs followed by Tz activator compared to the mice that received only the masked IL12 constructs, thus confirming in vivo unmasking.

### Example 21 - anti-TAG72 antibody and diabody functionalization with tetrazine moieties.

A stock solution of anti-TAG72 IgG (CC49) in PBS was diluted to 4-5 mg/mL and added with 20 equiv. of tetrazines **7.5, 7.8** or NHS-activated bis-pyridyl-tetrazine **1.2,** dissolved at 10 mg/mL in dry DMSO. The pH was adjusted to 8.5 with sodium carbonate, after which the reaction mixtures were incubated for 2h at rt. After incubation, the resulting tetrazine-functionalized antibodies were purified by SEC and the collected fractions were pooled and concentrated via Amicon centrifugal devices (MW cut-off 30 kDa). A BCA assay was used to measure the protein concentration and UV measurements showed the presence of 8.2, 5.6 and 2.5 tetrazine moieties per mAb molecule, respectively.

The anti-TAG72 diabody (AVP0458) was functionalized with tetrazine-PEG-maleimide **(7.9)** as previously described (Rossin et al., Nature Commun 2018). Briefly, the diabody was reduced with 6 mM DTT for 2h at rt followed by purification via PD-10 column, pre-equilibrated with 100 mM phosphate buffer pH 6.8, containing 2 mM EDTA. The reduced diabody was then added with tetrazine **7.9** (5 eq. per SH) dissolved in dry DMSO. The mixture was incubated overnight at +4°C. The Tz-functionalized diabody was then purified by SEC , the collected fractions were concentrated via Amicon centrifugal devices and a BCA assay was used to measure the protein concentration. HPLC-QTOF-MS analysis confirmed the presence of four Tz moieties per diabody molecule.

A stock solution of AVP0458 in PBS was diluted to 2 mg/ml and added with 10 equiv. of tetrazine-PEG-OSu **(7.8)** dissolved at 10 mg/mL in dry DMSO. The pH was adjusted to 8.5 with 0.1M sodium carbonate and the reaction mixture was incubated overnight at +4°C in the dark under gentle shaking. The Tz-functionalized diabody was then purified by SEC, the collected fractions were concentrated using Amicon centrifugal devices and a BCA assay was used to measure the protein concentration. Measurements with sulfo-Cy5-TCO showed the presence of 7.3 Tz moieties per diabody molecule.

AVP0458 was functionalized with mPEG24-maleimide (Broadpharm) as described for **7.9,** thus obtaining PEGylated diabody **21.1.** HPLC-QTOF-MS analysis confirmed the presence of four PEG moieties per diabody molecule.

Compound **21.1** was added with 7 equiv. of tetrazine-PEG-OSu **(7.8)** dissolved at 10 mg/mL in dry DMSO. The pH was adjusted to 8.5 with 0.1M sodium carbonate and the reaction mixture was incubated overnight at rt in the dark under gentle shaking. The Tz- and PEG-functionalized diabody **21.7** was then purified by SEC, the collected fractions were concentrated using Amicon centrifugal devices and a BCA assay was used to measure the protein concentration. Measurements with sulfo-Cy5-TCO showed the presence of 5.0 Tz moieties per diabody molecule.

The compound numbering of the resulting CC49-Tz and AVP0458-Tz conjugates is shown in table .6

**Table 6: Tz used in the conjugation reaction and average functionalization grades measured for the CC49-Tz and AVP0458-Tz conjugates**

| Targeting protein | Tetrazine conjugated | Functionalization grade | Resulting conjugate name |
|---|---|---|---|
| CC49 (IgG) | **7.5** | 8.2 | **21.2** |
| | **7.8** | 5.6 | **21.3** |
| | **1.2** | 2.5 | **21.4** |
| AVP0458 (diabody) | **7.8** | 7.3 | **21.5** |
| | **7.9** | 4.0 | **21.6** |
| AVP0458-PEG₂₄ (**21.1**) | **7.8** | 5.0 | **21.7** |

### Example 22 - anti-TAG72 antibody and diabody functionalization with TCO moieties

A stock solution of CC49 or AVP0458-PEG₂₄ **(21.1)** in PBS was diluted to 4-5 mg/mL and added with compound **1.1** dissolved at 5 mg/mL in DMSO. 30 and 8 molar eqs of **1.1** were used for CC49 and AVP0458-PEG₂₄, respectively. The pH was adjusted to 8.5 with 1M sodium carbonate and the reaction mixtures were incubated for 3h at rt in the dark under gentle shaking. The TCO-functionalized proteins were then purified via PD-10 column, pre-equilibrated with PBS. The obtained protein solutions were concentrated via Amicon centrifugal devices and a BCA assay was used to measure the protein concentration. TCO quantification by tetrazine titration showed the presence of 21 TCO moieties per IgG for CC49, and 6 TCO moieties per AVP0458-PEG₂₄.

AVP0458 was functionalized with TCO-PEG₂-maleimide **(1.3)** or maleimide-PEG-TCO₂ **(6.1)** as described for **7.9.** HPLC-QTOF-MS analysis confirmed the presence of four moieties (4 TCO's when **1.3** was used or 8 TCO's when **6.1** was used) per diabody molecule.

The compound numbering of the resulting CC49-TCO and AVP0458-TCO conjugates are shown in Table 7.

**Table 7: TCO used in the conjugation reaction and average functionalization grades measured for the CC49-TCO and AVP0458-TCO conjugates**

| Targeting protein | TCO conjugated | Functionalization grade | Resulting conjugate name |
|---|---|---|---|
| CC49 (IgG) | **1.1** | 21 | **22.1** |
| AVP0458 (diabody) | **1.3** | 4 | **22.2** |
| | **6.1** | 8 (i.e. 2x4) | **22.3** |
| AVP0458-PEG₂₄ (**21.1**) | **1.1** | 6 | **22.4** |

### Example 23 - Bioactivity of IL12-TCO-PEG conjugates following activation by a tetrazine activator coupled to tumor targeting moieties (in vitro)

TCO-masked IL12 constructs **9.4** and **9.10** were plated in a 1:2-step titration in serum-free culture medium (DMEM). In a first experiment, medium, unconjugated CC49 or CC49-Tz **21.4** were added at 50 µg/mL (16.6 molar eq. to IL12 at the highest concentration in the graph), followed by incubation for 1hr at 37°C. Next, IL12 bioactivity was assessed using the HEK-Blue-IL12 reporter cells as described above. Alternatively, TCO-masked IL12 **9.2** was incubated with AVP0458-Tz conjugates **21.5, 21.6,** and **21.7** or CC49-Tz **21.4** (all at 100 molar eqs of Tz with respect to IL12), in DMEM culture medium containing 10%FCS, for 1hr at 37°C. After incubation, a 1:2-step titration was made and IL12 bioactivity was assessed. A small-molecule Tz activator **8.5** was used as positive control. Figure 18 and 19 show complete reconstitution of masked IL12 activity when the various constructs were activated by free, antibody- or diabody-targeted tetrazine activator.

**Example 24 - Blood kinetics of masked IL12 constructs for pre-targeting approach.** TCO/Tz-PEG-masked IL12 constructs **9.2** and **12.3** were radiolabelled with ¹²⁵I and injected i.v. in tumour-free mice (ca 10 µg, ca. 0.4 MBq in 100 µL PBS per mouse; female BALB/c nude mice, ca. 20 g body weight, Janvier, n=3), followed by detection of radioactivity in blood samples collected at various timepoints (2, 30, 60min, 3, 6, 24, and 72hrs post injection). Weight of the blood samples and counts as measured by a gamma-counter were used to calculate the percentage of the injected dose per gram (%ID/g). Figure 20 shows that both masked IL12 constructs have a comparable clearance pattern (See Table 8).

**Table 8: Calculated half-lifes for a two phase decay TCO/Tz-masked IL12 constructs**

| **Construct** | **T_{1/2, α} (hrs) (%)** | **T_{1/2, β} (hrs)** |
|---|---|---|
| IL12-TCO-PEG (**9.2**) | 0.35 (65.7) | 7.63 |
| IL12-Tz-PEG (**12.3**) | 0.22 (60.3) | 3.39 |

### Example 25 - Detection of ¹²⁵I-labelled Tz-masked IL12 constructs in tumors and plasma, after activator pre-targeting.

Female BALB/c nude mice (ca. 20 g body weight, Janvier) were subcutaneously injected 1x10⁶ LS174T tumor cells at the lower flank. Injections (100µL) were done in a serum free RPMI medium. TCO-functionalized A VP0458 compounds **22.2** and **22.4** were injected i.v. when tumors reached ca. 0.3 cm³ in size (ca. 40ug in 100 µL PBS per mouse; n=4). 24h **(22.2),** or 48h **(22.4)** later, when diabodies were virtually cleared from blood, ¹²⁵I -labeled Tz-PEG-masked IL12 construct **(12.3)** was injected i.v. (10ug/mouse). 24hrs later, plasma and tumors were isolated and IL12 content was determined using ¹²⁵I counts. Figure 21 shows more tumor retention of masked IL12-Tz-PEG when TCO activator was pre-targeted via the diabodies.

### Example 26 - Detection of TCO-masked IL12 constructs in tumors and plasma after activator pre-targeting.

Compound **21.6** was injected i.v. in mice bearing LS174T tumors of ca. 0.3 cm³ in size (ca. 40ug in 100 µL PBS per mouse; n=4). Twenty-four hours later, when **21.6** was virtually cleared from blood, TCO-masked IL12 construct **9.2** was injected i.v. in some mice (ca. 10 µg in 100 µL PBS per mouse) and the mice were euthanized 24hrs later. Upon isolation, tumors were collected in MagNA lyser green bead tubes on ice, and added with protease inhibitor cocktail (2 mL/g tumor) followed by homogenization and debris removal. Blood was collected in heparized vials on ice and plasma was separated and 2-fold diluted with protease inhibitor cocktail. Detection of IL12 constructs in the tumor homogenates and plasma was carried out by ELISA. For this, the samples were 3-fold diluted in the kit's ELISPOT buffer, with or without addition of 100 eqs of small-molecule tetrazine activator **8.5** to unmask IL12 constructs ex vivo. 100 µl of the mixtures were transferred to the ELISA plates, followed by incubation for 18 hrs at 4C. Consecutive steps were carried out according to manufacturer's instruction. Figure 22 shows no IL12 unmasking in blood in vivo, due to low blood level of activator at the time of **9.2** injection. On the contrary, **9.2** could be unmasked ex vivo following addition of small-molecule tetrazine activator **8.5** before ELISA, confirming that the mask was intact and reactive. In the tumor, the pre-localized Tz activator **21.6** was able to unmask the IL12 construct locally. In this case, the addition of extra activator ex vivo, before ELISA, did not increase the amount of free IL12, confirming that in vivo on-tumor IL12 unmasking was complete.

### References

1. Rossin, R. et al. (2014). Trans-cyclooctene tag with improved properties for tumor pretargeting with the Diels-Alder reaction. Molecular pharmaceutics, 11(9), 3090-3096.
2. Rossin R, et al. Diels-Alder reaction for tumor pretargeting: in vivo chemistry can boost tumor radiation dose compared with directly labeled antibody. J Nucl Med. 2013 Nov;54(11):1989-95.
3. van Onzen, A. H., et al. (2020). Bioorthogonal tetrazine carbamate cleavage by highly reactive trans-cyclooctene. Journal of the American Chemical Society, 142(25), 10955-10963.
4. Rossin R, et al. In vivo chemistry for pretargeted tumor imaging in live mice. Angew Chem Int Ed Engl. 2010 Apr 26;49(19):3375-8.
5. Lingasamy P, et al. Bi-specific tenascin-C and fibronectin targeted peptide for solid tumor delivery. Biomaterials. 2019 Oct;219:119373.
6. Kim MY, et al. Selection and characterization of tenascin C targeting peptide. Mol Cells. 2012 Jan;33(1):71-7.
7. Park SE, et al. EDB-FN Targeted Peptide-Drug Conjugates for Use against Prostate Cancer. Int J Mol Sci. 2019 Jul 4;20(13):3291.
8. Saw PE, et al. Extra-domain B of fibronectin as an alternative target for drug delivery and a cancer diagnostic and prognostic biomarker for malignant glioma. Theranostics. 2021 Jan 1;11(2):941-957.

## Claims

1. A masked IL12 protein, or a masked subunit thereof, or a salt, hydrate, or solvate of said masked IL12 protein or said masked subunit;
wherein at least one lysine residue of the masked IL12 protein or the masked subunit has a structure according to Formula (1): 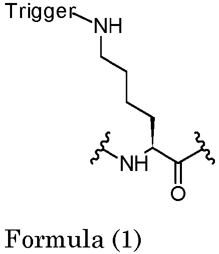
wherein the wiggly lines indicate bonds to other amino acid residues of the masked IL12 protein or the masked subunit;
wherein the Trigger is a dienophile or a tetrazine;
the dienophile or the tetrazine is linked to the ε-amine of the lysine residue of Formula (1) via a bond C^{A};
the dienophile comprises an eight-membered non-aromatic cyclic mono-alkenylene moiety comprising at least one allylic carbon, preferably two allylic carbons, and optionally comprising one or more heteroatoms;
the at least one allylic carbon is
(a) directly linked to the ε-amine of the lysine residue via a moiety M^{R} selected from the group consisting of -OC(O)-, -OC(S)-, -SC(O)-, and -SC(S)-, forming a carbamate, thiocarbamate or dithiocarbamate moiety together with said ε-amine and C^{A}; preferably M^{R} is -OC(O)-; or
(b) directly linked to a first end of a self-immolative linker via a cleavable moiety M^{B} selected from the group consisting of carbamate, thiocarbamate, carbonate, thiocarbonate, ether, ester, amine, amide, thioether, thioester, sulfoxide, and sulfonamide; and a second end of the self-immolative linker is directly linked to the ε-amine of the lysine residue via a moiety M^{R} that is part of the self-immolative linker, forming a carbamate, thiocarbamate or dithiocarbamate moiety together with said ε-amine and C^{A};
wherein the tetrazine is according to Formula (2): 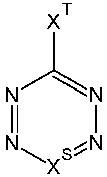
Formula (2); wherein X^{S} is an optionally substituted carbon atom; wherein X^{T} is -A^{T}-(B^{T})_{b}-X^{C}-C(C^{T})-(L^{C})_{f}-C^{A}; A^{T} and B^{T} are each independently selected from the group consisting of an optionally substituted carbon atom, an optionally substituted nitrogen atom, O, S, S(=O), and S(=O)₂; optionally the bond between A^{T} and B^{T} is a double bond;
b is 0 or 1; when b is 0, A^{T} is an optionally substituted carbon atom; when b is 1, B^{T} is an optionally substituted carbon atom; X^{C} is O, S, or an optionally substituted nitrogen atom; C^{T} is O or S; f is 0 or 1; L^{C} is a self-immolative linker; when f is 1, L^{C} is directly linked to C(C^{T}) via a moiety selected from the group consisting of O, S, a secondary amine, and a tertiary amine, wherein said moiety is part of L^{C}, and L^{C} is directly linked to the ε-amine of the lysine residue via a moiety M^{R} that is part of L^{C}, forming a carbamate, thiocarbamate or dithiocarbamate moiety together with said ε-amine and C^{A}; wherein optionally said dienophile or tetrazine comprises at least one C^{B}, wherein C^{B} is an organic molecule or an inorganic molecule;
wherein the masked subunit is a masked p35 subunit or a masked p40 subunit; wherein the masked p35 subunit has a sequence similarity of at least 80% with an amino acid sequence of SEQ ID NO: 1 or SEQ ID NO: 14; and wherein the masked p40 subunit has a sequence similarity of at least 80% with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13.

2. The masked IL12 protein or masked subunit according to claim 1, wherein at least five lysine residues, preferably at least ten lysine residues have a structure according to Formula (1).

3. The masked IL12 protein or masked subunit according any one of the preceding claims, wherein the masked IL12 protein comprises a masked p35 subunit and/or a masked p40 subunit; wherein the masked p35 subunit has a sequence similarity of at least 80% with an amino acid sequence of SEQ ID NO: 1 or SEQ ID NO: 14; and wherein the masked p40 subunit has a sequence similarity of at least 80% with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13.

4. The masked IL12 protein or masked subunit according any one of the preceding claims, wherein for the masked p35 subunit the at least one lysine residue having the structure according to Formula (1) is selected from the group consisting of K122, K128, K168, and K170, wherein said numbers refer to the amino acid residues of SEQ ID NO:1 or equivalent positions thereof in SEQ ID NO:14; and/or wherein for the masked p40 subunit the at least one lysine residue having the structure according to Formula (1) is selected from the group consisting of K58, K195, and K197, wherein said numbers refer to the amino acid residues of SEQ ID NO: 2 or equivalent positions thereof in any one of SEQ ID NO: 3 up to and including SEQ ID NO: 13.

5. The masked IL12 protein or masked subunit according any one of the preceding claims, wherein the dienophile and/or the tetrazine comprises at least one C^{B}, and wherein preferably each C^{B} is independently selected from the group consisting of polymer, peptide, protein, peptoid, amino acid, oligonucleotide, nucleic acid, nucleotide, carbohydrate, and combinations thereof; more preferably C^{B} comprises a polymer, most preferably polyethylene glycol.

6. The masked IL12 protein or masked subunit according any one of the preceding claims, wherein the dienophile is according to Formula (3b): 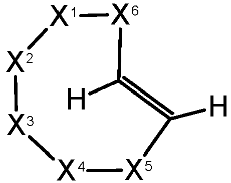 Formula (3b), wherein
X⁶ is -CHR₄₈; R₄₈ is -M^{R}-C^{A} or -M^{B}-L^{C}-C^{A};
L^{C} is a self-immolative linker directly linked via a group M^{R}, that is part of L^{C}, and the bond C^{A} to the ε-amine of the at least one lysine residue of Formula (1), and wherein optionally L^{C} is linked to one or more groups -(S^{P})ᵢ-C^{B} and/or one or more groups -(S^{P})ᵢ-D^{D} with i being an integer in a range of from 0 to 4, preferably i is 1;
the optional at least one C^{B}, if present, is linked, directly or via a spacer S^{P}, to a moiety selected from the group consisting of X¹, X², X³, X⁴, X⁵, and L^{C}; wherein if C^{B} is linked, directly or via a spacer S^{P}, to a moiety selected from the group consisting of X¹, X², X³, X⁴, and X⁵, C^{B} or -S^{P}-C^{B} is considered to be R₄₇;
X⁵ is -C(R₄₇)₂- or -CHR₄₉, preferably X⁵ is -C(R₄₇)₂-;
each of X¹, X², X³, and X⁴ is independently selected from the group consisting of -C(R₄₇)₂-, -NR₃₇-, -C(O)-, and -O-, such that at most two of X¹, X², X³, X⁴ are not -C(R₄₇)₂-, and with the proviso that no sets consisting of adjacent atoms are present selected from the group consisting of -O-O-, -O-N-, -C(O)-O-, N-N-, and -C(O)-C(O)-;
each of C^{B}, R₄₇ and R₃₇ are independently selected from the group consisting of hydrogen, organic molecules, and inorganic molecules;
R₄₉ is selected from the group consisting of -M^{R}-C^{B}, -M^{B}-L^{C}-C^{B}; -M^{R}- D^{D}, and - M^{B}-L^{C}-D^{D}; and D^{D} is a drug.

7. A combination comprising the masked IL12 protein or masked subunit according to any one of the preceding claims, wherein
(a) if the Trigger is a dienophile, the combination further comprises a diene, preferably a tetrazine; or
(b) if the Trigger is a tetrazine, the combination further comprises a dienophile, preferably a dienophile comprising an eight-membered non-aromatic cyclic mono-alkenylene moiety, and optionally comprising one or more heteroatoms.

8. The combination according to claim 7, wherein the diene is a tetrazine satisfying Formula (4) or a salt, solvate, or hydrate thereof: 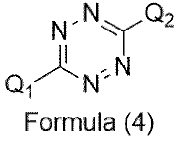 wherein
each moiety Q₁ and Q₂ is independently selected from the group consisting of hydrogen, organic molecules, and inorganic molecules;
and preferably at least one of moieties Q₁ and Q₂ is not hydrogen.

9. The combination according to claim 8, wherein Q₁ and Q₂ are selected from the group of hydrogen, (cyclo)alkyl, (cyclo)alkenyl, (cyclo)alkynyl, hetero(cyclo)alkyl, hetero(cyclo)alkenyl, hetero(cyclo)alkynyl, phenyl, 2-pyridyl, 3-pyridyl, 4-pyridyl, 2,6-pyrimidyl, 2,5-pyrimidyl, 3,5-pyrmidyl, 2,4-pyrimidyl, and linear or cyclic vinyl ethers; and Q₁ and Q₂ not being hydrogen are optionally substituted.

10. A method for preparing a masked IL12 protein or masked subunit according to any one of claims 1 to 6, wherein said method comprises the step of:
(a1) contacting an activated dienophile or a salt, solvate, or hydrate thereof, with an IL12 protein or a subunit thereof; or
(a2) contacting an activated tetrazine or a salt, solvate, or hydrate thereof, with an IL12 protein or a subunit thereof;
wherein the subunit of the IL12 protein is a p35 subunit or a p40 subunit;
wherein the IL12 protein comprises a p35 subunit and a p40 subunit; wherein the activated dienophile comprises an eight-membered non-aromatic cyclic mono-alkenylene moiety comprising at least one allylic carbon, preferably two allylic carbons, and optionally comprising one or more heteroatoms;
the at least one allylic carbon is directly linked to a moiety -M^{R}-R^{R} or -M^{B}-L^{C}-R^{R};
M^{R}, M^{B}, and L^{C} are as defined in any one of claims 1 to 5; wherein L^{C} comprises a moiety M^{R} that is directly linked to the R^{R} moiety; R^{R} is selected from the group consisting of -OH, -Cl, -F, -O-N-succinimidyl, -O-4-nitrophenyl, -O-tetrafluorophenyl, and -O-pentafluorophenyl; wherein optionally said activated dienophile comprises at least one C^{B} as defined in any one of claims 1 to 6;
wherein the activated tetrazine is according to Formula (2a): 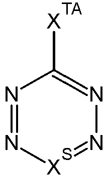
Formula (2a); wherein X⁵ is an optionally substituted carbon atom; wherein X^{TA} is -A^{T}-(B^{T})_{b}-X^{C}-C(C^{T})-(L^{C})_{f}-R^{R}; wherein A^{T}, B^{T}, b, X^{C}, C^{T}, L^{C}, and f are as defined in any one of claims 1 to 5; wherein optionally said activated tetrazine comprises at least one C^{B} as defined in any one of claims 1 to 6; wherein the p35 subunit has a sequence similarity of at least 80% with an amino acid sequence of SEQ ID NO: 1 or SEQ ID NO: 14; and wherein the p40 subunit has a sequence similarity of at least 80% with an amino acid sequence selected from the group consisting of SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, and SEQ ID NO: 13.

11. The method according to claim 10, wherein the molar ratio of the IL12 protein or a subunit thereof as compared to the activated dienophile or activated tetrazine is at least 1:10, preferably at least 1:25, more preferably at least 1:50, and most preferably at least 1:100.

12. A non-therapeutic method for unmasking the masked IL12 protein or masked subunit according to any one of claims 1 to 6, said non-therapeutic method comprising the step of contacting said IL12 protein with either:
(a) a diene as defined in any one of claims 7 to 9 if the Trigger is a dienophile; or
(b) a dienophile as defined in claim 7 if the Trigger is a tetrazine.

13. A non-therapeutic method for imaging the masked IL12 protein or masked subunit according to any one of claims 1 to 6, in a subject, preferably a human, said non-therapeutic method comprising the steps of
(a) administering the masked IL12 protein according to any one of claims 1 to 6 comprising a label, to the subject; and
(b) imaging said masked IL12 protein present in the subject;
wherein the label is selected from the group consisting of radionuclides, fluorescent dyes, and phosphorescent dyes.

## Patentansprüche

1. Maskiertes IL12-Protein oder maskierte Untereinheit davon, oder ein Salz, Hydrat oder Solvat des maskierten IL12-Proteins oder der maskierten Untereinheit; wobei mindestens ein Lysinrest des maskierten IL12-Proteins oder der maskierten Untereinheit eine Struktur gemäß Formel (1) hat: 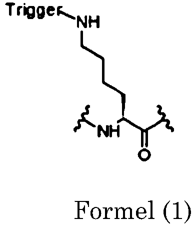
wobei die geschlängelten Linien Bindungen zu anderen Aminosäureresten des maskierten IL12-Proteins oder der maskierten Untereinheit anzeigen;
wobei der Trigger ein Dienophil oder ein Tetrazin ist;
das Dienophil oder das Tetrazin mit dem ε-Amin des Lysinrestes der Formel (1) über eine Bindung C^{A} verbunden ist;
das Dienophil eine achtgliedrige nichtaromatische cyclische Monoalkenyleneinheit umfasst, die mindestens einen allylischen Kohlenstoff, vorzugsweise zwei allylische Kohlenstoffe, und optional ein oder mehrere Heteroatome umfasst;
der mindestens eine allylische Kohlenstoff
(a) direkt mit dem ε-Amin des Lysinrestes über eine Einheit M^{R} verbunden ist, ausgewählt aus der Gruppe bestehend aus -OC(O)-, -OC(S)-, -SC(O)- und -SC(S)-, wobei eine Carbamat-, Thiocarbamat- oder Dithiocarbamat-Einheit zusammen mit dem ε-Amin und C^{A} gebildet wird; wobei M^{R} vorzugsweise-OC(O)- ist; oder
(b) direkt an ein erstes Ende eines selbst-immolierenden Linkers über eine spaltbare Einheit M^{B}, ausgewählt aus der Gruppe bestehend aus Carbamat, Thiocarbamat, Carbonat, Thiocarbonat, Ether, Ester, Amin, Amid, Thioether, Thioester, Sulfoxid und Sulfonamid, gebunden ist; und ein zweites Ende des selbst-immolierenden Linkers direkt mit dem ε-Amin des Lysinrestes über eine Einheit M^{R} verbunden ist, die Teil des selbst-immolierenden Linkers ist, wobei eine Carbamat-, Thiocarbamat- oder Dithiocarbamat-Einheit zusammen mit dem ε-Amin und C^{A} gebildet wird;
wobei das Tetrazin der Formel (2) entspricht: 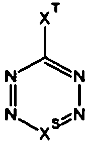 Formel (2); wobei X^{S} ein optional substituiertes Kohlenstoffatom ist; wobei X^{T} -A^{T}-(B^{T})_{b}-X^{C}-C(C^{T})-(L^{C})_{f}-C^{A} ist; A^{T} und B^{T} jeweils unabhängig voneinander ausgewählt sind aus der Gruppe bestehend aus einem optional substituierten Kohlenstoffatom, einem optional substituierten Stickstoffatom, O, S, S(=O) und S(=O)₂; die Bindung zwischen A^{T} und B^{T} optional eine Doppelbindung ist;
b 0 oder 1 ist; wenn b 0 ist, A^{T} ein optional substituiertes Kohlenstoffatom ist; wenn b 1 ist, B^{T} ein optional substituiertes Kohlenstoffatom ist; X^{C} O, S oder ein optional substituiertes Stickstoffatom ist; C^{T} O oder S ist; f 0 oder 1 ist; L^{C} ein selbst-immolierender Linker ist; wenn f 1 ist, L^{C} direkt mit C(C^{T}) über eine Einheit verbunden ist, ausgewählt der Gruppe bestehend aus O, S, einem sekundären Amin und einem tertiären Amin, wobei die Einheit Teil von L^{C} ist, und L^{C} direkt mit dem ε-Amin des Lysinrestes über eine Einheit M^{R} verbunden ist, die Teil von L^{C} ist, wobei eine Carbamat-, Thiocarbamat- oder Dithiocarbamat-Einheit zusammen mit dem ε-Amin und C^{A} gebildet wird; wobei optional das Dienophil oder Tetrazin mindestens ein C^{B} umfasst, wobei C^{B} ein organisches Molekül oder ein anorganisches Molekül ist;
wobei die maskierte Untereinheit eine maskierte p35-Untereinheit oder eine maskierte p40-Untereinheit ist; wobei die maskierte p35-Untereinheit eine Sequenzähnlichkeit von mindestens 80% mit einer Aminosäuresequenz von SEQ ID NO: 1 oder SEQ ID NO: 14 hat; und wobei die maskierte p40-Untereinheit eine Sequenzähnlichkeit von mindestens 80 % mit einer Aminosäuresequenz hat, ausgewählt aus der Gruppe bestehend aus SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, und SEQ ID NO: 13.

2. Maskiertes IL12-Protein oder maskierte Untereinheit nach Anspruch 1, wobei mindestens fünf Lysinreste, vorzugsweise mindestens zehn Lysinreste, eine Struktur gemäß der Formel (1) haben.

3. Maskiertes IL12-Protein oder maskierte Untereinheit nach einem der vorhergehenden Ansprüche, wobei das maskierte IL12-Protein eine maskierte p35 -Untereinheit und/oder eine maskierte p40-Untereinheit umfasst; wobei die maskierte p35-Untereinheit eine Sequenzähnlichkeit von mindestens 80 % mit einer Aminosäuresequenz von SEQ ID NO: 1 oder SEQ ID NO: 14 hat; und wobei die maskierte p40-Untereinheit eine Sequenzähnlichkeit von mindestens 80 % mit einer Aminosäuresequenz hat, ausgewählt aus der Gruppe bestehend aus SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, und SEQ ID NO: 13.

4. Maskiertes IL12-Protein oder maskierte Untereinheit nach einem der vorhergehenden Ansprüche, wobei für die maskierte p35-Untereinheit der mindestens eine Lysinrest mit der Struktur gemäß Formel (1) ausgewählt ist aus der Gruppe bestehend aus K122, K128, K168 und K170, wobei sich die Zahlen auf die Aminosäurereste von SEQ ID NO:1 oder äquivalente Positionen davon in SEQ ID NO:14 beziehen; und/oder wobei für die maskierte p40-Untereinheit der mindestens eine Lysinrest mit der Struktur gemäß Formel (1) ausgewählt ist aus der Gruppe bestehend aus K58, K195 und K197, wobei sich die Zahlen auf die Aminosäurereste von SEQ ID NO: 2 oder äquivalente Positionen davon in einer der SEQ ID NO: 3 bis zu und einschließlich SEQ ID NO: 13 beziehen.

5. Maskiertes IL12-Protein oder maskierte Untereinheit nach einem der vorhergehenden Ansprüche, wobei das Dienophil und/oder das Tetrazin mindestens ein C^{B} umfasst und wobei vorzugsweise jedes C^{B} unabhängig ausgewählt ist aus der Gruppe bestehend aus Polymer, Peptid, Protein, Peptoid, Aminosäure, Oligonucleotid, Nucleinsäure, Nucleotid, Kohlenhydrat und Kombinationen davon; C^{B} bevorzugter ein Polymer, am bevorzugtesten Polyethylenglykol umfasst.

6. Maskiertes IL12-Protein oder maskierte Untereinheit nach einem der vorhergehenden Ansprüche, wobei das Dienophil der Formel (3b) entspricht: 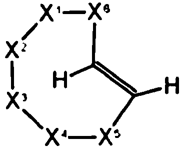 Formel (3b), wobei
X⁶ -CHR₄₈ ist; R₄₈ -M^{R}-C^{A} oder -M^{B}-L^{C}-C^{A} ist;
L^{C} ein selbst-immolativer Linker ist, der direkt über eine Gruppe M^{R}, die Teil von L^{C} ist, und die Bindung C^{A} an das ε-Amin des mindestens einen Lysinrestes der Formel (1) gebunden ist, und wobei optional L^{C} an eine oder mehrere Gruppen -(S^{P})ᵢ-C^{B} und/oder eine oder mehrere Gruppen -(S^{P})ᵢ-D^{D} gebunden ist, wobei i eine ganze Zahl im Bereich von 0 bis 4 ist, und i vorzugsweise 1 ist;
das optional mindestens eine C^{B}, falls vorhanden, direkt oder über einen Spacer S^{P}, an eine Einheit, ausgewählt aus der Gruppe bestehend aus X¹, X², X³, X⁴, X⁵, und L^{C}, gebunden ist; wobei, wenn C^{B} direkt oder über einen Spacer S^{P} an eine Einheit, ausgewählt aus der Gruppe bestehend aus X¹, X², X³, X⁴ und X⁵, gebunden ist, C^{B} oder -S^{P}-C^{B} als R₄₇ angesehen wird;
X⁵ -C(R₄₇)₂- oder -CHR₄₉, vorzugsweise X⁵ -C(R₄₇)₂- ist;
wobei jedes von X¹, X², X³, und X⁴ unabhängig ausgewählt ist aus der Gruppebestehend aus -C(R₄₇)₂-, -NR₃₇-, -C(O)-, und -O-, so dass höchstens zwei von X¹, X², X³, X⁴ nicht-C(R₄₇)₂- sind, und mit der Maßgabe, dass keine aus benachbarten Atomen bestehenden Sätze vorhanden sind, ausgewählt aus der Gruppe bestehend aus -O-O-, -O-N-, -C(O)-O-, N-N-, und -C(O)-C(O)-;
jedes von C^{B}, R₄₇, und R₃₇ unabhängig ausgewählt sind aus der Gruppe bestehende aus Wasserstoff, organischen Molekülen und anorganischen Molekülen besteht;
R₄₉ ausgewählt ist aus der Gruppe bestehend aus -M^{R}-C^{B}, -M^{B}-L^{C}-C^{B}, -M^{R}-D^{D} und -M^{B}-L^{C}-D^{D}; und D^{D} ein Arzneimittel ist.

7. Kombination, umfassend das maskierte IL12-Protein oder die maskierte Untereinheit nach einem der vorangegangenen Ansprüche, wobei
(a) wenn der Trigger ein Dienophil ist, die Kombination ferner ein Dien, vorzugsweise ein Tetrazin umfasst; oder
(b) wenn der Trigger ein Tetrazin ist, die Kombination ferner ein Dienophil umfasst, vorzugsweise ein Dienophil umfassend eine achtgliedrige nicht-aromatische zyklische Monoalkenylen-Einheit und optional umfassend ein oder mehrere Heteroatome.

8. Kombination nach Anspruch 7, wobei das Dien ein Tetrazin ist, das der Formel (4) erfüllt, oder ein Salz, Solvat oder Hydrat davon: 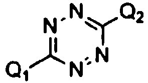 Formel (4); wobei
jede Einheit Q₁ und Q₂ unabhängig ausgewählt ist aus der Gruppe bestehend aus Wasserstoff, organischen Molekülen und anorganischen Molekülen;
und vorzugsweise mindestens einer der Reste Q₁ und Q₂ nicht Wasserstoff ist.

9. Kombination nach Anspruch 8, wobei Q₁ und Q₂ ausgewählt sind aus der Gruppe bestehend aus Wasserstoff, (Cyclo)alkyl, (Cyclo)alkenyl, (Cyclo)alkinyl, Hetero(cyclo)alkyl, Hetero(cyclo)alkenyl, Hetero(cyclo)alkinyl, Phenyl, 2-Pyridyl, 3-Pyridyl, 4-Pyridyl, 2,6-Pyrimidyl, 2,5-Pyrimidyl, 3,5-Pyrmidyl, 2,4-Pyrimidyl, und linearen oder cyclischen Vinylethern; und Q₁ und Q₂, die nicht Wasserstoff sind, optional substituiert sind.

10. Verfahren zur Herstellung eines maskierten IL12-Proteins oder einer maskierten Untereinheit nach einem der Ansprüche 1 bis 6, wobei das Verfahren die Schritte umfasst von:
(a1) Inkontaktbringen eines aktivierten Dienophils oder eines Salzes, Solvats oder Hydrats davon mit einem IL12-Protein oder einer Untereinheit davon; oder
(a2) Inkontaktbringen eines aktivierten Tetrazins oder eines Salzes, Solvats oder Hydrats davon mit einem IL12-Protein oder einer Untereinheit davon;
wobei die Untereinheit des IL12-Proteins eine p35-Untereinheit oder eine p40-Untereinheit ist;
wobei das IL12-Protein eine p35-Untereinheit und eine p40-Untereinheit umfasst; wobei das aktivierte Dienophil eine achtgliedrige nicht-aromatische cyclische Monoalkenyleneinheit umfasst, umfassend mindestens einen allylischen Kohlenstoff, vorzugsweise zwei allylische Kohlenstoffe, und optional umfassend ein oder mehrere Heteroatome; der mindestens eine allylische Kohlenstoff direkt an eine Einheit -M^{R}-R^{R} oder -M^{B}-L^{C}-R^{R} gebunden ist;
M^{R}, M^{B} und L^{C} sind wie in einem der Ansprüche 1 bis 5 definiert; wobei L^{C} eine Einheit M^{R} umfasst, die direkt mit der Einheit R^{R} verbunden ist; R^{R} ausgewählt ist aus der Gruppe bestehend aus -OH, -Cl, -F, *-*O*-N-*Succinimidyl, -O-4-Nitrophenyl, -O-Tetrafluorphenyl und -O-Pentafluorphenyl; wobei optional das aktivierte Dienophil mindestens ein C^{B}, wie in einem der Ansprüche 1 bis 6 definiert, umfasst; wobei das aktivierte Tetrazin der Formel (2a) entspricht: 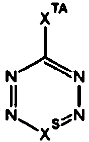 Formel (2a); wobei X^{S} ein optional substituiertes Kohlenstoffatom ist; wobei X^{TA} -A^{T}-(B^{T})_{b}-X^{C}-C(C^{T})-(L^{C})_{f}-R^{R} ist; wobei A^{T}, B^{T}, b, X^{C}, C^{T}, L^{C} und f wie in einem der Ansprüche 1 bis 5 definiert sind; wobei das optional aktivierte Tetrazin mindestens ein C^{B}, wie in einem der Ansprüche 1 bis 6 definiert, umfasst; wobei die p35-Untereinheit eine Sequenzähnlichkeit von mindestens 80 % mit einer Aminosäuresequenz von SEQ ID NO 1 oder SEQ ID NO: 14 hat; und wobei die p40-Untereinheit eine Sequenzähnlichkeit von mindestens 80 % mit einer Aminosäuresequenz hat, ausgewählt aus der Gruppe bestehend aus SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, und SEQ ID NO: 13.

11. Verfahren nach Anspruch 10, wobei das molare Verhältnis des IL12-Proteins oder einer Untereinheit davon im Vergleich zu dem aktivierten Dienophil oder aktivierten Tetrazin mindestens 1:10, vorzugsweise mindestens 1:25, bevorzugter mindestens 1:50 und am bevorzugtesten mindestens 1:100 ist.

12. Nicht-therapeutisches Verfahren zum Demaskieren des maskierten IL12-Proteins oder der maskierten Untereinheit nach einem der Ansprüche 1 bis 6, wobei das nicht-therapeutische Verfahren den Schritt des Inkontaktbringens des IL12-Proteins umfasst, mit entweder:
(a) einem Dien, wie in einem der Ansprüche 7 bis 9 definiert, wenn der Trigger ein Dienophil ist;
oder
(b) einem Dienophil wie in Anspruch 7 definiert, wenn der Trigger ein Tetrazin ist.

13. Nicht-therapeutisches Verfahren zum Abbilden des maskierten IL12-Proteins oder der maskierten Untereinheit nach einem der Ansprüche 1 bis 6 in einem Subjekt, vorzugsweise einem Menschen, wobei das nicht-therapeutische Verfahren die Schritte umfasst von:
(a) Verabreichung des maskierten IL12-Proteins nach einem der Ansprüche 1 bis 6, umfassend eine Markierung, an das Subjekt; und
(b) Abbilden des maskierten IL12-Proteins, das in dem Subjekt vorhanden ist;
wobei die Markierung ausgewählt ist aus der Gruppe bestehend aus Radionukliden, Fluoreszenzfarbstoffen und phosphoreszierenden Farbstoffen.

## Revendications

1. Protéine IL12 masquée, ou une sous-unité masquée de celle-ci, ou un sel, hydrate ou solvate de ladite protéine IL12 masquée ou de ladite sous-unité masquée ; dans lequel au moins un résidu de lysine de la protéine IL12 masquée ou de la sous-unité masquée a une structure de formule (1) : 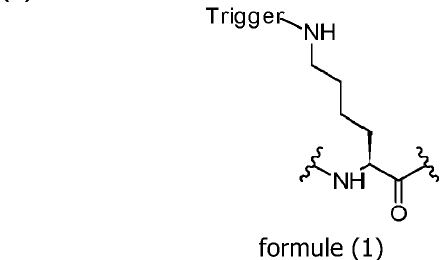
dans laquelle les lignes ondulées désignent des liaisons à d'autres résidus d'acides aminés de la protéine IL12 masquée ou de la sous-unité masquée ;
dans laquelle le déclencheur (Trigger) est un diénophile ou une tétrazine ;
le diénophile ou la tétrazine est lié à l'ε-amine du résidu de lysine de formule (1) via une liaison C^{A} ;
le diénophile comprend un fragment monoalcénylène cyclique non aromatique à huit chaînons comprenant au moins un carbone allylique, de préférence deux carbones allyliques, et comprenant éventuellement un ou plusieurs hétéroatomes ;
l'au moins un carbone allylique est
(a) directement lié à l'ε-amine du résidu de lysine via un fragment M^{R} choisi dans l'ensemble constitué par -OC(O)-, -OC(S)-, -SC(O)- et -SC(S)- en formant un fragment carbamate, thiocarbamate ou dithiocarbamate conjointement avec ladite ε-amine et C^{A} ; de préférence M^{R} est -OC(O)- ; ou
(b) directement lié à une première extrémité d'un lieur auto-immolable via un fragment clivable M^{B} choisi dans l'ensemble constitué par carbamate, thiocarbamate, carbonate, thiocarbonate, éther, ester, amine, amide, thioéther, thioester, sulfoxyde et sulfonamide ; et une deuxième extrémité du lieur auto-immolable est directement liée à l'ε-amine du résidu de lysine via un fragment M^{R} qui fait partie du lieur auto-immolable, en formant un fragment carbamate, thiocarbamate ou dithiocarbamate conjointement avec ladite ε-amine et C^{A} ;
dans laquelle la tétrazine est de formule (2) : 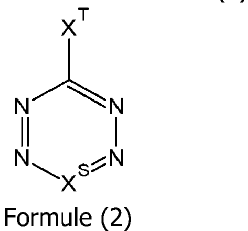
dans laquelle X^{S} est un atome de carbone éventuellement substitué ; dans laquelle X^{T} est -A^{T}-(B^{T})_{b}-X^{C}-C(C^{T})-(L^{C})_{f}-C^{A} ; chacun de A^{T} et B^{T} est indépendamment choisi dans l'ensemble constitué par un atome de carbone éventuellement substitué, un atome d'azote éventuellement substitué, O, S, S(=O) et S(=O)₂ ; éventuellement la liaison entre A^{T} et B^{T} est une double liaison ;
b vaut 0 ou 1 ; quand b vaut 0, A^{T} est un atome de carbone éventuellement substitué ; quand b vaut 1, B^{T} est un atome de carbone éventuellement substitué ; X^{C} est O, S ou un atome d'azote éventuellement substitué ; C^{T} est O ou S ; f vaut 0 ou 1 ; L^{C} est un lieur auto-immolable ; quand f vaut 1, L^{C} est directement lié à C(C^{T}) via un fragment choisi dans l'ensemble constitué par O, S, une amine secondaire et une amine tertiaire, dans laquelle ledit fragment fait partie de L^{C}, et L^{C} est directement lié à l'ε-amine du résidu de lysine via un fragment M^{R} qui fait partie de L^{C}, en formant un fragment carbamate, thiocarbamate ou dithiocarbamate avec ladite ε-amine et C^{A} ; dans laquelle éventuellement ledit diénophile ou ladite tétrazine comprend au moins un C^{B}, dans laquelle C^{B} est une molécule organique ou une molécule inorganique ;
dans lequel la sous-unité masquée est une sous-unité p35 masquée ou une sous-unité p40 masquée ; dans lequel la sous-unité p35 masquée a une similarité de séquence d'au moins 80 % avec une séquence d'acides aminés de la SEQ ID NO : 1 ou de la SEQ ID NO : 14 ; et dans lequel la sous-unité p40 masquée a une similarité de séquence d'au moins 80 % avec une séquence d'acides aminés choisie dans l'ensemble constitué par la SEQ ID NO : 2, la SEQ ID NO : 3, la SEQ ID NO : 4, la SEQ ID NO : 5, la SEQ ID NO : 6, la SEQ ID NO : 7, la SEQ ID NO : 8, la SEQ ID NO : 9, la SEQ ID NO : 10, la SEQ ID NO : 11, la SEQ ID NO : 12 et la SEQ ID NO : 13.

2. Protéine IL12 masquée ou sous-unité masquée selon la revendication 1, dans laquelle au moins cinq résidus de lysine, de préférence au moins dix résidus de lysine ont une structure de formule (1).

3. Protéine IL12 masquée ou sous-unité masquée selon l'une quelconque des revendications précédentes, dans laquelle la protéine IL12 masquée comprend une sous-unité p35 masquée et/ou une sous-unité p40 masquée ; dans laquelle la sous-unité p35 masquée a une similarité de séquence d'au moins 80 % avec une séquence d'acides aminés de la SEQ ID NO : 1 ou de la SEQ ID NO : 14 ; et dans laquelle la sous-unité p40 masquée a une similarité de séquence d'au moins 80 % avec une séquence d'acides aminés choisie dans l'ensemble constitué par la SEQ ID NO : 2, la SEQ ID NO : 3, la SEQ ID NO : 4, la SEQ ID NO : 5, la SEQ ID NO : 6, la SEQ ID NO : 7, la SEQ ID NO : 8, la SEQ ID NO : 9, la SEQ ID NO : 10, la SEQ ID NO : 11, la SEQ ID NO : 12 et la SEQ ID NO : 13.

4. Protéine IL12 masquée ou sous-unité masquée selon l'une quelconque des revendications précédentes, dans laquelle, pour la sous-unité p35 masquée, l'au moins un résidu de lysine ayant la structure de formule (1) est choisi dans l'ensemble constitué par K122, K128, K168 et K170, dans laquelle lesdits nombres se réfèrent aux résidus d'acides aminés de la SEQ ID NO : 1 ou à des positions équivalentes à ceux-ci dans la SEQ ID NO : 14 ; et/ou dans laquelle, pour la sous-unité p40 masquée, l'au moins un résidu de lysine ayant la structure de formule (1) est choisi dans l'ensemble constitué par K58, K195 et K197, dans laquelle lesdits nombres se réfèrent aux résidus d'acides aminés de la SEQ ID NO : 2 ou à des positions équivalentes à ceux-ci dans l'une quelconque des SEQ ID NO : 3 à SEQ ID NO : 13, y compris celles-ci.

5. Protéine IL12 masquée ou sous-unité masquée selon l'une quelconque des revendications précédentes, dans laquelle le diénophile et/ou la tétrazine comprennent au moins un C^{B}, et dans laquelle de préférence chaque C^{B} est indépendamment choisi dans l'ensemble constitué par un polymère, un peptide, une protéine, un peptoïde, un acide aminé, un oligonucléotide, un acide nucléique, un nucléotide, un hydrate de carbone, et leurs combinaisons ; mieux encore C^{B} comprend un polymère, tout spécialement le polyéthylèneglycol.

6. Protéine IL12 masquée ou sous-unité masquée selon l'une quelconque des revendications précédentes, dans laquelle le diénophile est de formule (3b) : 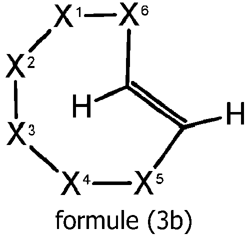 dans laquelle
X⁶ est -CHR₄₈ ; R₄₈ est -M^{R}-C^{A} ou -M^{B}-L^{C}-C^{A} ;
L^{C} est un lieur auto-immolable lié directement via un groupe M^{R}, qui fait partie de L^{C}, et la liaison C^{A} à l'ε-amine de l'au moins un résidu de lysine de formule (1), et dans laquelle éventuellement L^{C} est lié à un ou plusieurs groupes -(S^{P})ᵢ-C^{B} et/ou à un ou plusieurs groupes -(S^{P})ᵢ-D^{D} où i est un entier situé dans la plage allant de 0 à 4, de préférence i vaut 1 ;
l'au moins un C^{B} optionnel, s'il est présent, est lié, directement ou via un espaceur S^{P}, à un fragment choisi dans l'ensemble constitué par X¹, X², X³, X⁴, X⁵ et L^{C} ; dans laquelle, si C^{B} est lié, directement ou via un espaceur S^{P}, à un fragment choisi dans l'ensemble constitué par X¹, X², X³, X⁴ et X⁵, C^{B} ou -S^{P}-C^{B} est considéré être R₄₇ ;
X⁵ est -C(R₄₇)₂- ou -CHR₄₉, de préférence X⁵ est -C(R₄₇)₂- ;
chacun de X¹, X², X³ et X⁴ est indépendamment choisi dans l'ensemble constitué par -(C₄₇)₂-, -NR₃₇-, -C(O)- et -O-, de façon qu'au plus deux parmi X¹, X², X³, X⁴ ne soient pas -C(R₄₇)₂-, et sous réserve qu'aucuns jeux consistant en atomes adjacents, choisis dans l'ensemble constitué par -O-O-, -O-N-, -C(O)-O-, N-N- et -C(O)-C(O)-, ne soient présents ;
chacun de C^{B}, R₄₇ et R₃₇ est indépendamment choisi dans l'ensemble constitué par l'hydrogène, les molécules organiques, et les molécules inorganiques ;
R₄₉ est choisi dans l'ensemble constitué par -M^{R}-C^{B}, -M^{B}-L^{C}-C^{B}, -M^{R}-D^{D} et -M^{B}-L^{C}-D^{D} ; et D^{D} est un médicament.

7. Combinaison comprenant la protéine IL2 masquée ou la sous-unité masquée de l'une quelconque des revendications précédentes, dans laquelle
(a) si le déclencheur est un diénophile, la combinaison comprend en outre un diène, de préférence une tétrazine ; ou
(b) si le déclencheur est une tétrazine, la combinaison comprend en outre un diénophile, de préférence un diénophile comprenant un fragment monoalcénylène cyclique non aromatique à huit chaînons, et comprenant éventuellement un ou plusieurs hétéroatomes.

8. Combinaison selon la revendication 7, dans laquelle le diène est une tétrazine satisfaisant à la formule (4) ou un sel, solvate ou hydrate de celle-ci : 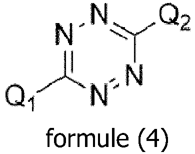 dans laquelle
chaque fragment Q₁ et Q₂ est indépendamment choisi dans l'ensemble constitué par l'hydrogène, les molécules organiques et les molécules inorganiques ;
et de préférence au moins l'un des fragments Q₁ et Q₂ n'est pas l'hydrogène.

9. Combinaison selon la revendication 8, dans laquelle Q₁ et Q₂ sont choisis dans l'ensemble constitué par l'hydrogène, (cyclo)alkyle, (cyclo)alcényle, (cyclo)alcynyle, hétéro(cyclo)alkyle, hétéro(cyclo)alcényle, hétéro(cyclo)alcynyle, phényle, 2-pyridyle, 3-pyridyle, 4-pyridyle, 2,6-pyrimidyle, 2,5-pyrimidyle, 3,5-pyrimidyle, 2,4-pyrimidyle, et les vinyléthers linéaires ou cycliques ; et Q₁ et Q₂, quand ils ne sont pas des hydrogènes, sont éventuellement substitués.

10. Procédé pour préparer une protéine IL-12 masquée ou une sous-unité masquée de l'une quelconque des revendications 1 à 6, lequel procédé comprend l'étape suivante :
(a1) mise en contact d'un diénophile activé ou d'un sel, solvate ou hydrate de celui-ci, avec une protéine IL12 ou une sous-unité de celle-ci ; ou
(a2) mise en contact d'une tétrazine activée ou d'un sel, solvate ou hydrate de celle-ci, avec une protéine IL12 ou une sous-unité de celle-ci ;
dans lequel la sous-unité de la protéine IL12 est une sous-unité p35 ou une sous-unité p40 ;
dans lequel la protéine IL12 comprend une sous-unité p35 et une sous-unité p40 ; dans lequel le diénophile activé comprend un fragment monoalcénylène cyclique non aromatique à huit chaînons comprenant au moins un carbone allylique, de préférence deux carbones allyliques, et comprenant éventuellement un ou plusieurs hétéroatomes ;
l'au moins un carbone allylique est directement lié à un fragment -M^{R}-R^{R} ou -M^{B}-L^{C}-R^{R} ;
M^{R}, M^{B} et L^{C} sont tels que définis dans l'une quelconque des revendications 1 à 5 ; dans lequel L^{C} comprend un fragment M^{R} qui est directement lié au fragment R^{R} ; R^{R} est choisi dans l'ensemble constitué par -OH, -Cl, -F, -O-*N*-succinimidyle, -O-4-nitrophényle, -O-tétrafluorophényle et -O-pentafluorophényle ; dans lequel éventuellement ledit diénophile activé comprend au moins un C^{B} tel que défini dans l'une quelconque des revendications 1 à 6 ;
dans lequel la tétrazine activée est de formule (2a) : 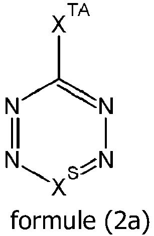
dans laquelle X^{S} est un atome de carbone éventuellement substitué ; dans laquelle X^{TA} est -A^{T}-(B^{T})_{b}-X^{C}-C(C^{T})-(L^{C})_{f}-R^{R} ; dans laquelle A^{T}, B^{T}, b, X^{C}, C^{T}, L^{C} et f sont tels que définis dans l'une quelconque des revendications 1 à 5 ; dans lequel éventuellement ladite tétrazine activée comprend au moins un C^{B} tel que défini dans l'une quelconque des revendications 1 à 6 ; dans lequel la sous-unité p35 a une similarité de séquence d'au moins 80 % avec une séquence d'acides aminés de la SEQ ID NO : 1 ou de la SEQ ID NO : 14 ; et dans lequel la sous-unité p40 a une similarité de séquence d'au moins 80 % avec une séquence d'acides aminés choisie dans l'ensemble constitué par la SEQ ID NO : 2, la SEQ ID NO : 3, la SEQ ID NO : 4, la SEQ ID NO : 5, la SEQ ID NO : 6, la SEQ ID NO : 7, la SEQ ID NO : 8, la SEQ ID NO : 9, la SEQ ID NO : 10, la SEQ ID NO : 11, la SEQ ID NO : 12 et la SEQ ID NO : 13.

11. Procédé selon la revendication 10, dans lequel le rapport molaire de la protéine IL12 ou d'une sous-unité de celle-ci au diénophile activé ou à la tétrazine activée est d'au moins 1/10, de préférence d'au moins 1/25, mieux encore d'au moins 1/50, et tout spécialement d'au moins 1/100.

12. Procédé non thérapeutique pour démasquer la protéine IL12 masquée ou la sous-unité-masquée de l'une quelconque des revendications 1 à 6, ledit procédé non thérapeutique comprend l'étape de mise en contact de la protéine IL12 avec :
(a) soit un diène tel que défini dans l'une quelconque des revendications 7 à 9 si le déclencheur est un diénophile ;
(b) soit un diénophile tel que défini dans la revendication 7 si le déclencheur est une tétrazine.

13. Procédé non thérapeutique pour imager la protéine IL12 masquée ou la sous-unité-masquée de l'une quelconque des revendications 1 à 6 chez un sujet, de préférence un humain, ledit procédé non thérapeutique comprenant les étapes suivantes :
(a) administration au sujet de la protéine IL12 masquée de l'une quelconque des revendications 1 à 6 comprenant un marqueur ; et
(b) imagerie de ladite protéine IL12 masquée présente chez le sujet ;
dans lequel le marqueur est choisi dans l'ensemble constitué par les radionucléides, les colorants fluorescents, et les colorants phosphorescents.
